# EUROPEAN PATENT APPLICATION

(11) **EP 2 664 616 A1**
(43) Date of publication of application: **20.11.2013**
(21) Application number: 12305541.0
(22) Date of filing: 15.05.2012
(51) Int. Cl.: C07D 233/78, C07D 233/86, C07D 401/04, C07D 401/06, C07D 401/12, C07D 403/06, C07D 409/06, C07D 409/14, C07D 413/12, C07D 417/12, A61K 31/4166, A61P 31/14

(54) **Hydantoin and thiohydantoin derivatives as antiviral drugs**

(71) Applicant: Vivalis, 49450 Roussay (FR)
(72) Inventor: Guedat, Philippe, 25260 Montenois (FR); Berecibar, Amaya, 44220 Coueron (FR); Ciapetti, Paola, 67120 Altorf (FR); Venkata Pithani ,Subhash, 67000 Strasbourg (FR); Trouche, Nathalie, 67150 Erstein Krafft (FR)
(74) Representative: Jacobson, Claude

(57) **Abstract**

The present invention relates to a compound of the following formula (I), or a salt, solvate, tautomer, enantiomer, diastereoisomer or racemic mixture thereof: 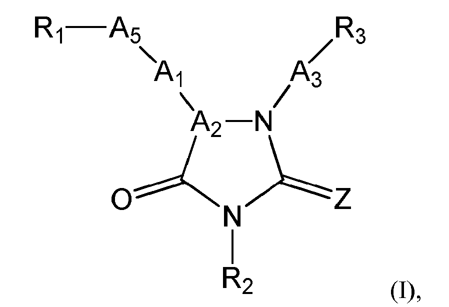 as well as its use as a drug, notably in the treatment of hepatitis C, its preparation process, and the pharmaceutical compositions containing such a compound.

## Description

The present invention concerns hydantoin and thiohydantoin derivatives, as well as the synthesis and the use of such compounds for treating a viral infection, in particular for the treatment of hepatitis C.

Hepatitis C is a global health problem with 170 million carriers' worldwide, 3 to 4 million new cases each year and a worldwide mortality estimated to 500,000 persons a year. Hepatitis C is an infectious disease affecting primarily the liver, caused by the hepatitis C virus (HCV). 84 % of the HCV infections detected during the period 2005-2009 were diagnosed in people between the ages of 15-44 years. 30 % of liver grafts are currently prescribed to patients infected with HCV.

HCV is spread primarily by direct contact with human blood. Transmission through blood transfusions that are not screened for HCV infection, through the re-use of inadequately sterilized needles and syringes or other medical equipment or through needle-sharing among drug users, is well documented. Sexual and perinatal transmission may also occur, although less frequently.

The incubation period of HCV infection before the onset of clinical symptoms ranges from 15 to 150 days. About 80 % of infected patients progress to develop chronic infection which can also be asymptomatic. Cirrhosis develops in about 10 % to 20 % of persons with chronic infection and liver cancer develops in 1 % to 5 % of persons with chronic infection over a period of 20 to 30 years.

HCV is an enveloped virus from the Flaviviridae family and is the only member of hepacivirus genus. HCV comprises 6 genotypes, more than 45 subtypes and quasi-species patient-specific. Its positive single strand linear RNA has about 9,600 nucleotides. RNA genome is flanked by two untranslated regions (UTR) that play a major role in translation and replication of the viral genome. Upon interaction and fusion of viral and cellular membranes, RNA genome is released into the cytoplasm of a newly infected cell and serves as template for RNA replication. Viral genome replication is a two step process: the positive RNA strand is used as a matrix for the synthesis of a negative polarity RNA which in turn serves as matrix for the synthesis of positive RNA strands that will be incorporated in new virions. Translation of HCV genome depends on an internal ribosome entry site and produces a large polyprotein which is proteolytically cleaved by cellular and viral proteases to produce 10 viral proteins. The amino terminal one third of the polyprotein encodes the structural proteins: core protein glycoproteins E1 + E2. After the structural region, comes a small integral protein, P7, which seems to function as an ion chemical. The remainder of the genome encodes the non structural proteins NS2, NS3, NS4A, NS4B, NS5A & NS5B which coordinate the intracellular processes of the virus life cycle (Lindenbach et al., 2005 Science 309:623-626). Replication complex is associated with membranes of the endoplasmic reticulum. Viral proteins involved in this complex are the NTPase/helicase/serine protease NS3-4A, NS4B which is involved in the formation of the replication web, NS5A which is in interaction with other viral and host proteins but whose function still remains to be elucidated and the RNA-dependent RNA polymerase NS5B. No vaccine is currently available to prevent hepatitis C. The landscape for treating hepatitis C virus is set to change in the wake of the significant developments of new variety of drugs that offering higher efficacy, convenience and tolerability over the current standard of care, which is pegylated alpha interferon (Moussalli et al., 1998 J. Viral. Hepatitis 5:73-82) combined with ribavirin (Hugle et al., 2003 Rev. Med. Virol. 13:361-71), with the recent addition of the first protease inhibitors: Vertex/Johnson & Johnson's telaprevir and Merck's boceprevir. Potential treatments for HCV infection has been discussed in different references including Balsano, 2008 Mini Rev. Med. Chem. 8(4):307-318, Rön et al. 2008 Current topics in Med. Chem. 8:533-562, Sheldon et al., 2007 Expert Opin. Investig. Drugs 16(8):1171-1181, and De Francesco et al., 2003 Antiviral Research 58:1-16.

Besides its relative efficacy, the pegylated alpha interferon combined with ribavirin combination therapy yields significant side effects (Fried Michael, 2002 Hepatology Vol. 32:S237-244). New treatment regimens are thus needed. To address inefficiency and specificity issues, investigators have focused in recent years on the identification of drugs that specifically inhibit viral enzymes playing a key role in the hepatitis C virus life-cycle.

There is a need to find new compounds which can be used in the treatment of hepatitis C, and in particular having a hepatitis C virus (HCV) inhibitory activity without the drawbacks of the prior art.

The present invention concerns thus a compound of the following formula (I), or a salt, solvate, tautomer, enantiomer, diastereoisomer or racemic mixture thereof: 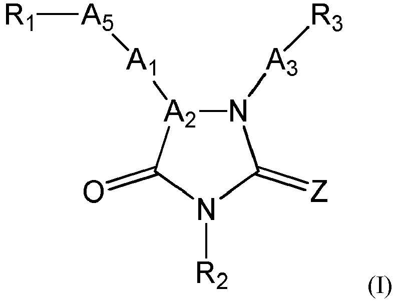 wherein:
- Z represents O or S,
- A₁ represents -OC(O)-, -C(O)O-, -NHC(O)- or -C(O)NH-,
- A₂ represents 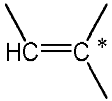 (the double bond having thus a Z or E configuration) or 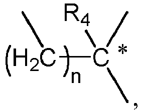 the carbon atom marked with a star being comprised in the hydantoin or thiohydantoin ring,
- A₃ represents -(CH₂)ₒ-, -CH=CH-, -C≡C-, or -(CH₂)ₘ-A₄-(CH₂)ₚ-,
- A₄ represents O, NH or 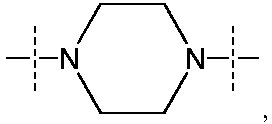
- A₅ represents 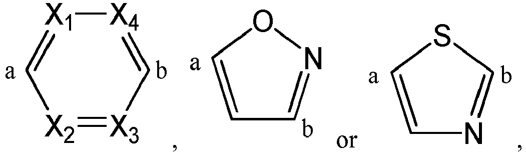 the carbon atoms marked with a being bound to R₁ and the carbon atoms marked with b being bound to A₁,
- X, represents N or CR₅,
- X₂ represents N or CR₆,
- X₃ represents N or CR₇,
- X₄ represents N or CR₈,
- R₁ represents a hydrogen atom, a halogen atom, R₉, OR₁₀, NR₁₁R₁₂, C(O)R₁₀, C(O)OR₁₀, OC(O)R₉, C(O)NR₁₁R₁₂ or NR₁₃C(O)R₁₄,
- R₂ represents a (C₁-C₆)alkyl or -(CH₂)_{q}-R₂₂ group,
- R₃ represents a (C₁-C₆)alkyl group, a (C₂-C₆)alkenyl group or a cycloalkyl, cycloalkenyl, heterocyclic, aryl or heteroaryl ring, the said ring being optionally substituted with one or several groups, preferably with one, chosen among a halogen atom, a oxo group, (C₁-C₆)alkyl, (C₁-C₆)haloalkyl, OR₂₃, NR₁₇R₁₈ and SO₂NR₁₉R₂₀,
- R₄ represents a hydrogen atom or an OH, (C₁-C₆)alkyl or (C₁-C₆)alkoxy group,
- R₅ and R₆ represent, independently of each other, a hydrogen atom, a halogen atom, a (C₁-C₆)alkyl group or a (C₁-C₆)alkoxy group,
- R₇ and R₈ represent, independently of each other, H or F,
- R₉ represents a (C₁-C₆)alkyl, (C₁-C₆)haloalkyl, or (C₁-C₆)alkyl-O-(C₁-C₆)alkanediyl group,
- R₁₀ represents a hydrogen atom or a (C₁-C₆)alkyl, (C₁-C₆)haloalkyl, or (C₁-C₆)alkyl-O-(C₁-C₆)alkanediyl group,
- R₁₁ and R₁₂ represent, independently of each other, a hydrogen atom or a (C₁-C₆)alkyl, (C₁-C₆)haloalkyl or (C₁-C₆)alkyl-O-(C₁-C₆)alkanediyl group, or R₁₁ and R₁₂ form, together with the nitrogen atom bearing them, a heterocycle optionally comprising another heteroatom chosen among O, S and N and optionally substituted with a (C₁-C₆)alkyl or oxo (C=O) group,
- R₁₃ represents a hydrogen atom or a (C₁-C₆)alkyl, (C₁-C₆)haloalkyl or (C₁-C₆)alkyl-O-(C₁-C₆)alkanediyl group,
- R₁₄ represents a (C₁-C₆)alkyl, (C₁-C₆)haloalkyl, or (C₁-C₆)alkyl-O-(C₁-C₆)alkanediyl group, or R₁₃ and R₁₄ form together a -(CH₂)₂- or -(CH₂)₃- chain,
- R₁₅ and R₁₆ represent, independently of each other, a hydrogen atom or a (C₁-C₆)alkyl or (C₁-C₆)haloalkyl group, or R₁₅ and R₁₆ form, together with the nitrogen atom bearing them, a heterocycle optionally comprising another heteroatom chosen among O, S and N and optionally substituted with a (C₁-C₆)alkyl or oxo group,
- R₁₇ and R₁₈ represent, independently of each other, a hydrogen atom or a (C₁-C₆)alkyl or (C₁-C₆)haloalkyl group, or R₁₇ and R₁₈ form, together with the nitrogen atom bearing them, a heterocycle optionally comprising another heteroatom chosen among O, S and N and optionally substituted with a (C₁-C₆)alkyl or oxo group,
- R₁₉ and R₂₀ represent, independently of each other, a hydrogen atom or a (C₁-C₆)alkyl or (C₁-C₆)haloalkyl group, or R₁₉ and R₂₀ form, together with the nitrogen atom bearing them, a heterocycle optionally comprising another heteroatom chosen among O, S and N and optionally substituted with a (C₁-C₆)alkyl group,
- R₂₂ represents a cycloalkyl, aryl or heteroaryl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆,
- R₂₃ represents a hydrogen atom or a (C₁-C₆)alkyl, (C₁-C₆)haloalkyl or (C₁-C₆)alkyl-OC(O)NH-(C₁-C₆)alkanediyl group,
- n represents 0 or 1,
- m represents 0, 1, 2 or 3,
- o represents 1, 2, 3, 4, 5 or 6,
- p represents 0, 1, 2 or 3, and
- q represents 0, 1, 2 or 3, preferably 0,
for use as a drug, in particular as an antiviral drug, advantageously intended to treat hepatitis C, for example as a hepatitis C virus replication inhibitor.

Chandrasekaran et al. Med. Chem. Res., published online on December 30, 2011, describes notably the following compound: 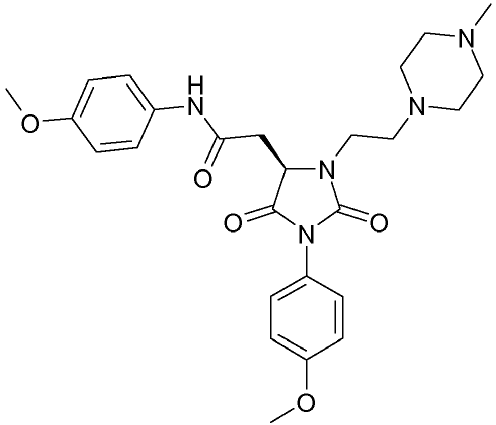 as a virtual lead compound with predicted Factor Xa (FXa) inhibitory activity and not for its use in the treatment of hepatitis C. No *in vitro* or *in vivo* test has been performed. The potential activity of this compound has only been evidenced *in silico.* Consequently, the possible use of this compound as drug has not been demonstrated in this article.

WO 2009/133294 discloses also hydantoin and thiohydantoin derivatives as a drug for the prevention or the treatment of diseases dependent on metabolic pathways involving Syk protein tyrosine kinase and not for the treatment of hepatitis C. The compounds disclosed in this patent application do not however describe the particular substituents of the compounds of the present invention.

FR 2 387 962 describes the synthesis of hydantoin and thiohydantoin derivatives, no biological activity for these derivatives being reported in this document. The compounds disclosed in this patent application do not however describe the particular substituents of the compounds of the present invention.

Preferably, the compound of formula (I) is not the compound of following formula:

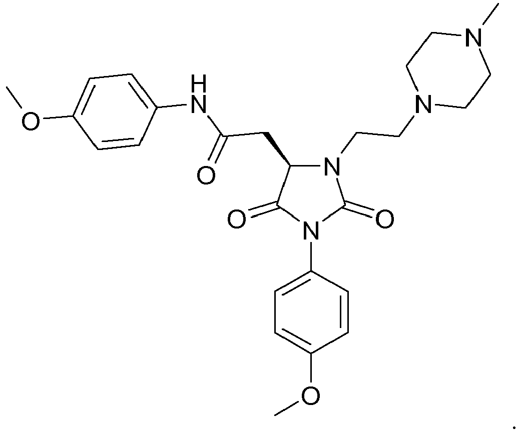

In a particular embodiment, A₁ represents -OC(O)- or -NHC(O)-, notably-NHC(O)- (C(O)- being bound to A₂).

A₂ will represent in particular 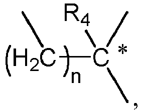 with n representing advantageously 1. In this case, R₄ represents advantageously H, OH or (C₁-C₆)alkoxy, preferably H or OH.

The compounds according to the invention can thus fit the following formula (Ia): 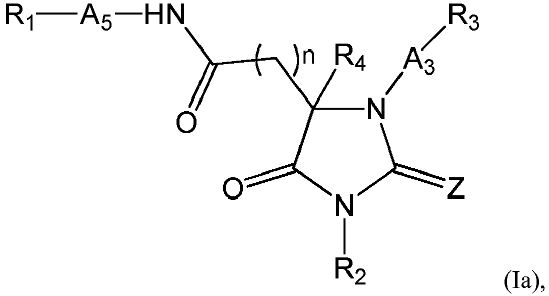 with n representing advantageously 1 and with R₄ representing advantageously H, OH or (C₁-C₆)alkoxy, preferably H or OH.

A₃ will represent advantageously O, NH, -CH=CH-, -(CH₂)ₒ-, or -(CH₂)ₘ-A₄-(CH₂)ₚ-, with A₄ representing O or 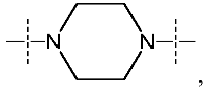 and notably with m = 2 or 3 and p = 0 or 1. A₃ can be in particular -(CH₂)ₒ-, in particular with o = 1, 2, 3 or 4, notably with o = 2.

Advantageously, A₅ will be

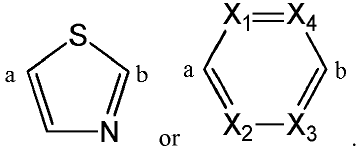

When A₅ represents 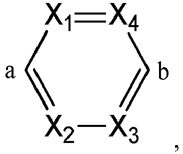 preferably at most two of X₁, X₂, X₃ and

X₄ represent N. In particular, 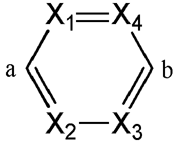 can be one of the following rings:

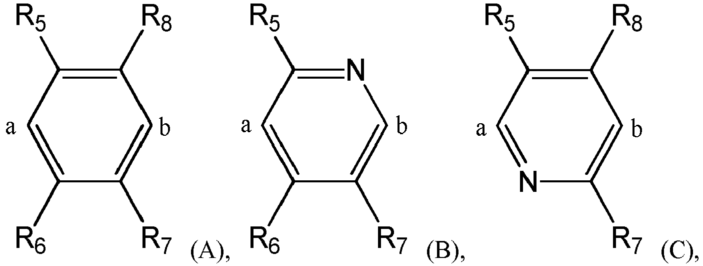

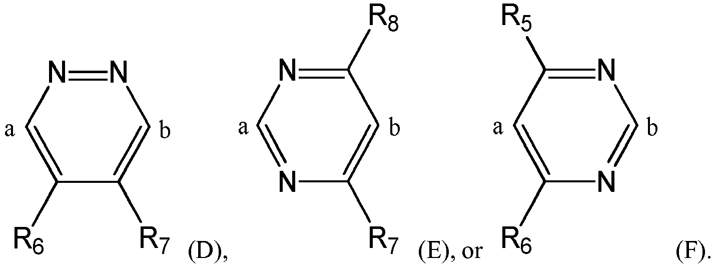

In particular, the ring 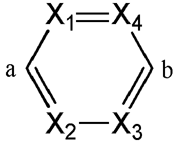 will be a ring (A), (B) or (F), preferably, a ring (B) or (F). R₅ and R₆, when present, will represent advantageously a hydrogen atom.

Advantageously, R₁ represents a hydrogen atom, a halogen atom, R₉, OR₁₀, NR₁₁R₁₂, C(O)OR₁₀, OC(O)R₉, C(O)NR₁₁R₁₂ or NR₁₃C(O)R₁₄. R₁ can represent in particular a hydrogen atom, a halogen atom, R₉, OR₁₀, NR₁₁R₁₂, C(O)OR₁₀ or C(O)NR₁₁R₁₂. Preferably, R₁ represents a hydrogen atom, a halogen atom, R₉, OR₁₀, or NR₁₁R₁₂.

Advantageously, R₂ represents an aryl or heteroaryl ring, notably a phenyl or pyridinyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆. R₂ can represent in particular a phenyl ring optionally substituted with one or several groups, preferably with one (in particular in *para* position), chosen among a halogen atom, OH, (C₁-C₆)alkyl, (C₁-C₆)haloalkyl, (C₁-C₆)alkoxy, (C₁-C₆)haloalkoxy and NR₁₅R₁₆; or a pyridinyl ring. Preferably, R₂ represents a phenyl ring optionally substituted with one or several groups, preferably with one (in particular in *para* position), chosen among a halogen atom, OH, (C₁-C₆)alkyl, (C₁-C₆)haloalkyl, (C₁-C₆)alkoxy, (C₁-C₆)haloalkoxy and NR₁₅R₁₆; or a pyridinyl ring. Most preferably, R₂ represents a phenyl ring optionally substituted in *para* position with one group chosen among a halogen atom, OH, (C₁-C₆)alkyl, (C₁-C₆)haloalkyl, (C₁-C₆)alkoxy, (C₁-C₆)haloalkoxy and NR₁₅R₁₆, or a pyridinyl ring.

Preferably, R₃ does not represent a substituted or unsubstitued pyridinyl group.

Advantageously, R₃ represents a cycloalkyl, cycloalkenyl, heterocyclic, aryl or heteroaryl ring, the said ring being optionally substituted with one or several groups, preferably with one, chosen among a halogen atom, a oxo group, (C₁-C₆)alkyl, (C₁-C₆)haloalkyl, OR₂₃, NR₁₇R₁₈ and SO₂NR₁₉R₂₀. Notably, R₃ represents a cyclohexenyl, morpholinyl, piperazinyl, pyrrolidinyl, piperidinyl, 1,3-benzodioxolyl, phenyl, pyridinyl, thienyl, imidazolyl, or indolyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy, NR₁₇R₁₈ and SO₂NR₁₉R₂₀. In particular, R₃ will represent a phenyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy, NR₁₇R₁₈ and SO₂NR₁₉R₂₀.

The compound of formula (I) can be in particular one of the following compounds:

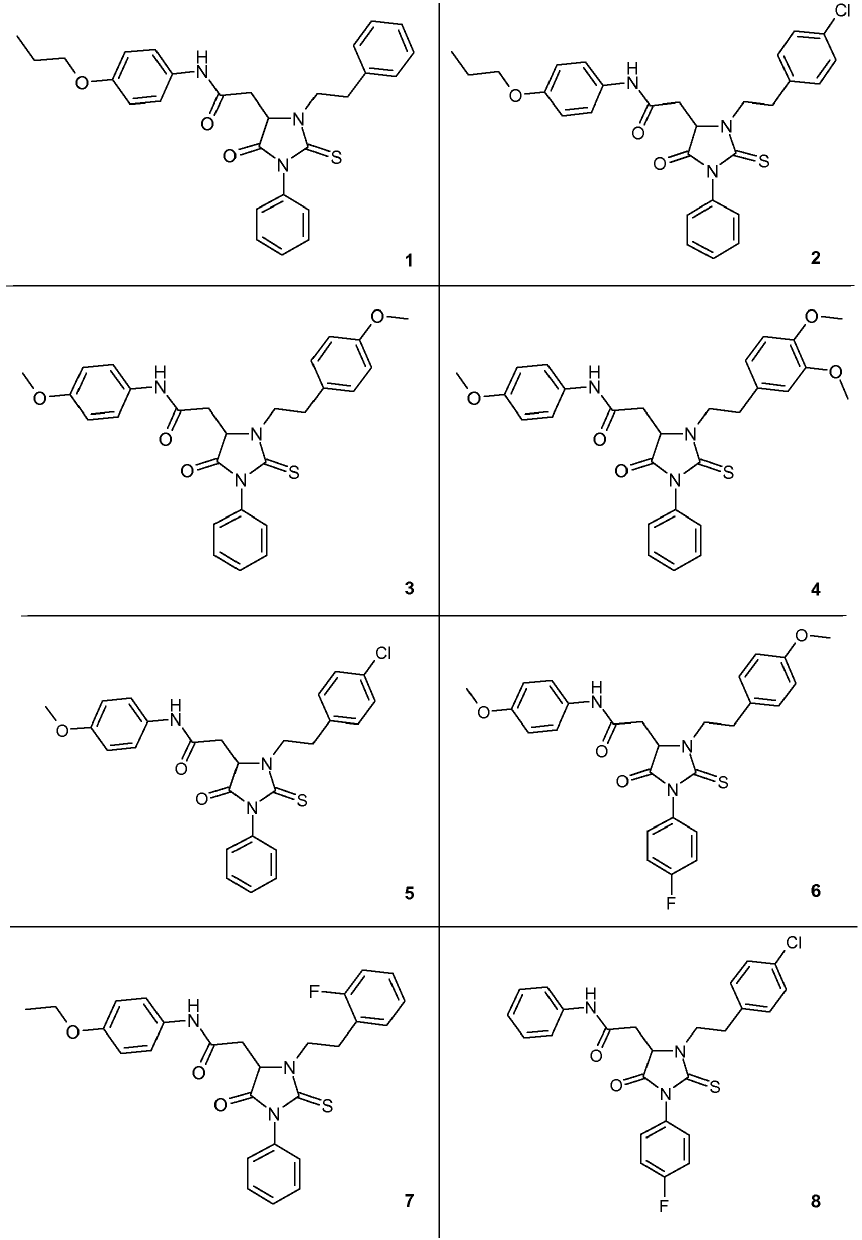

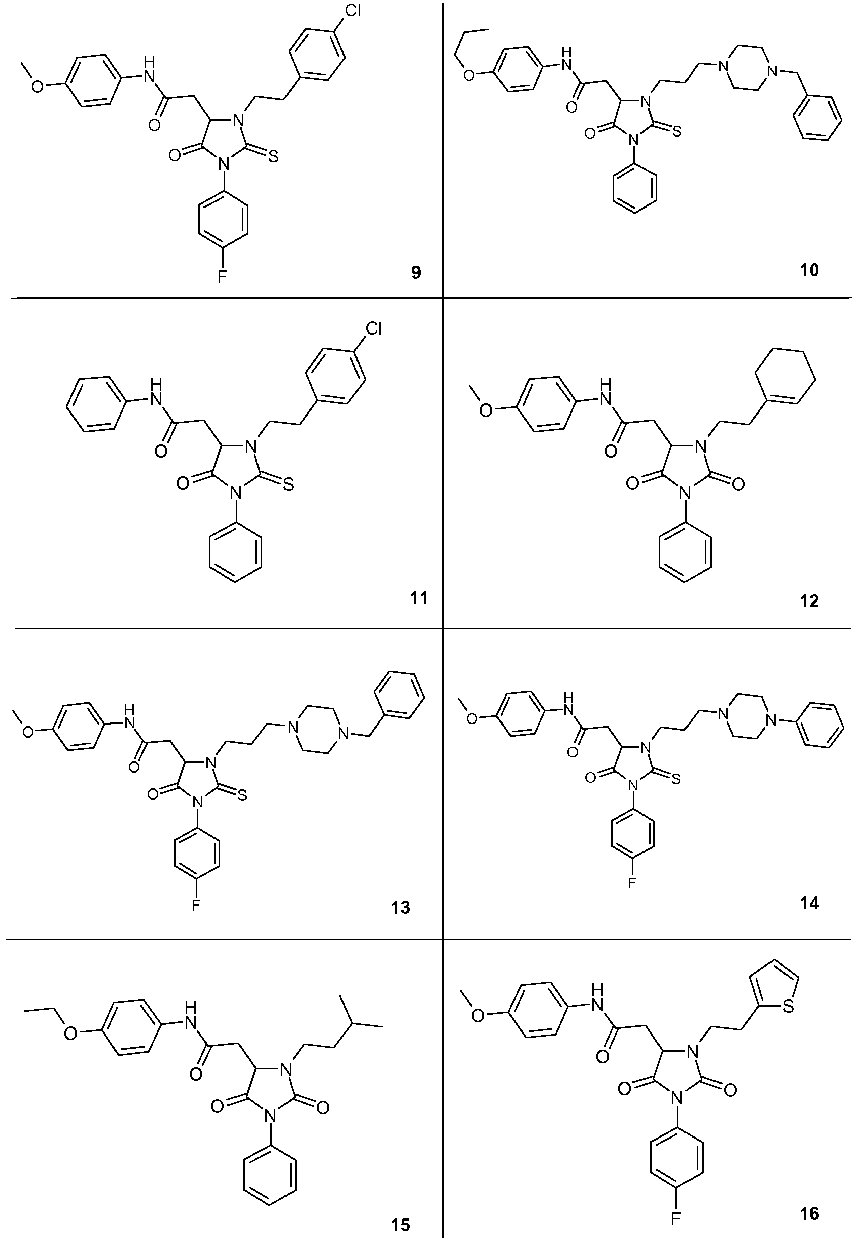

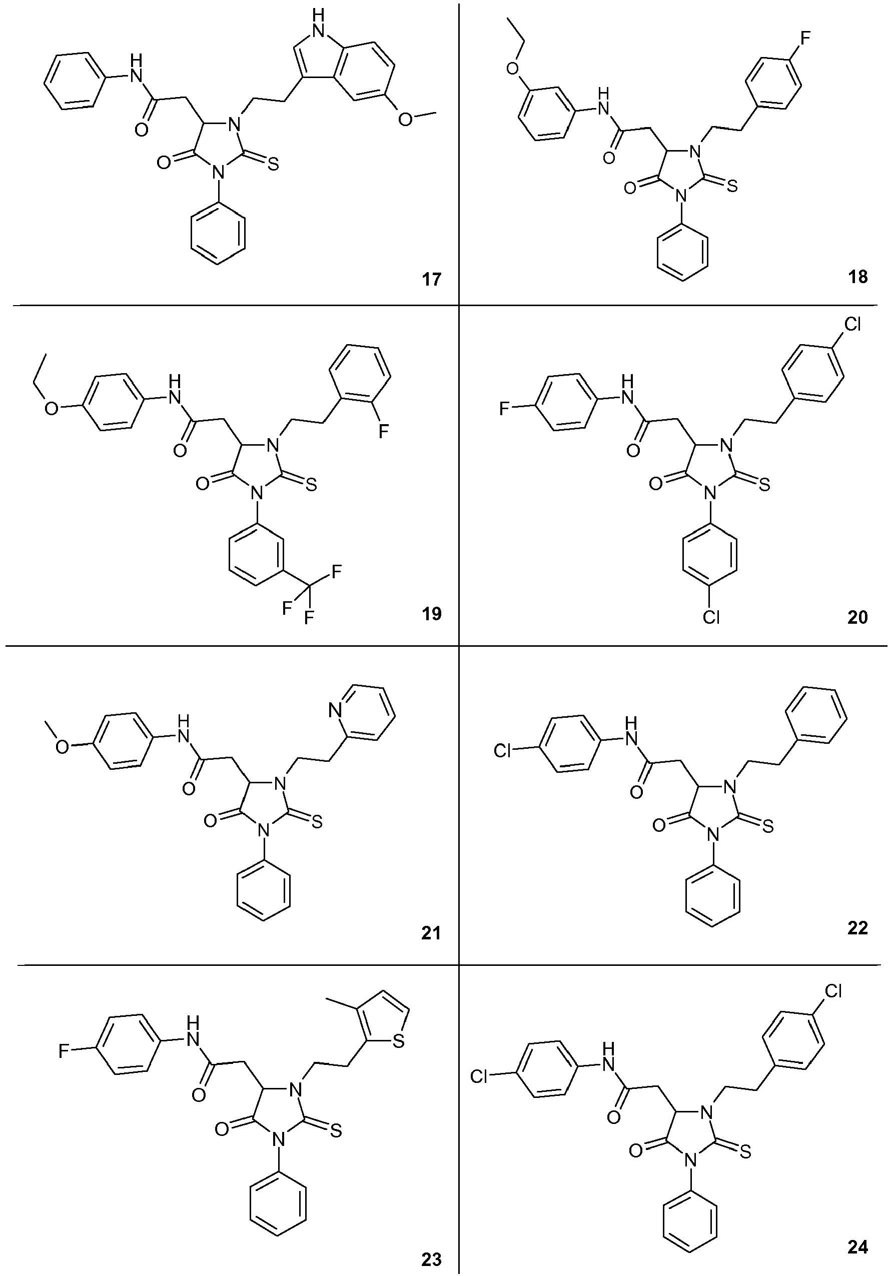

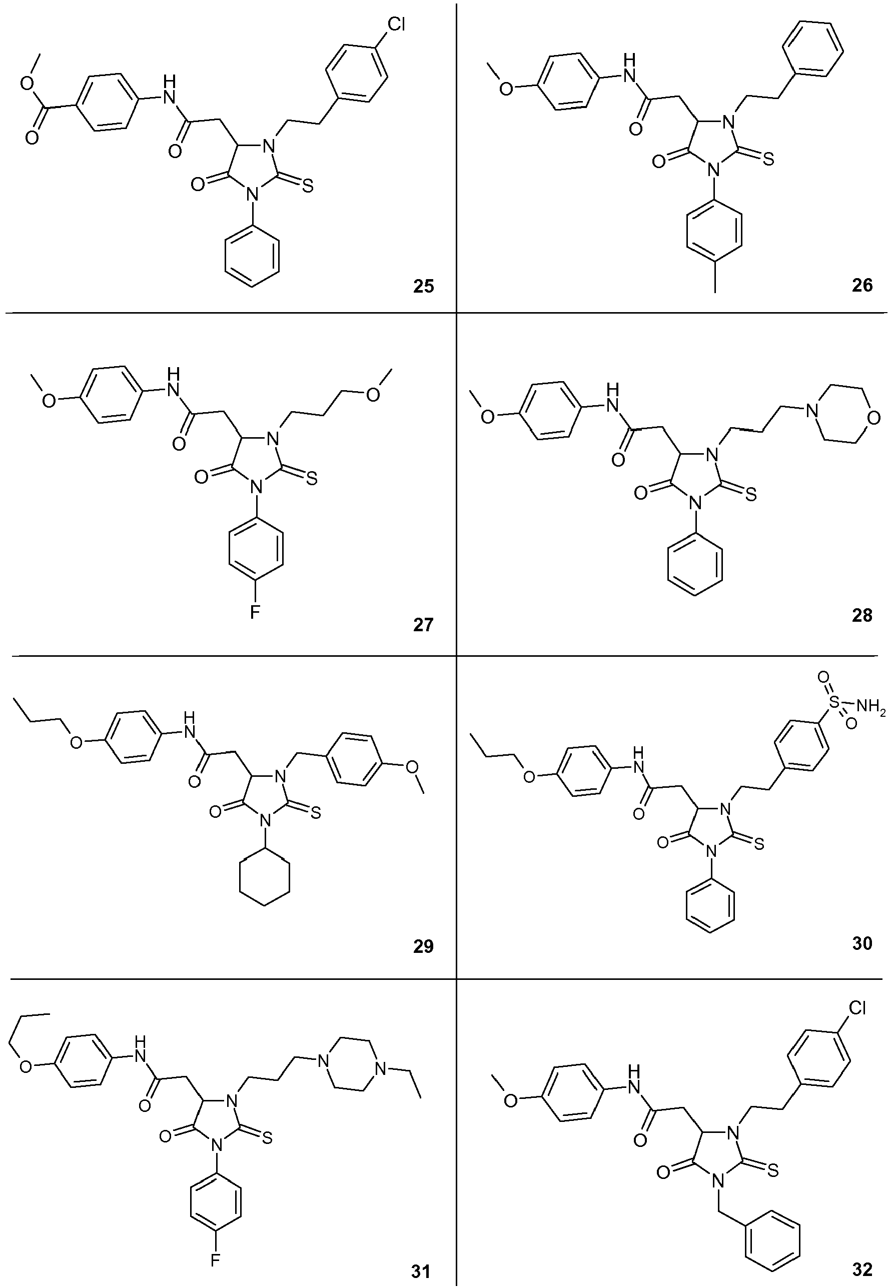

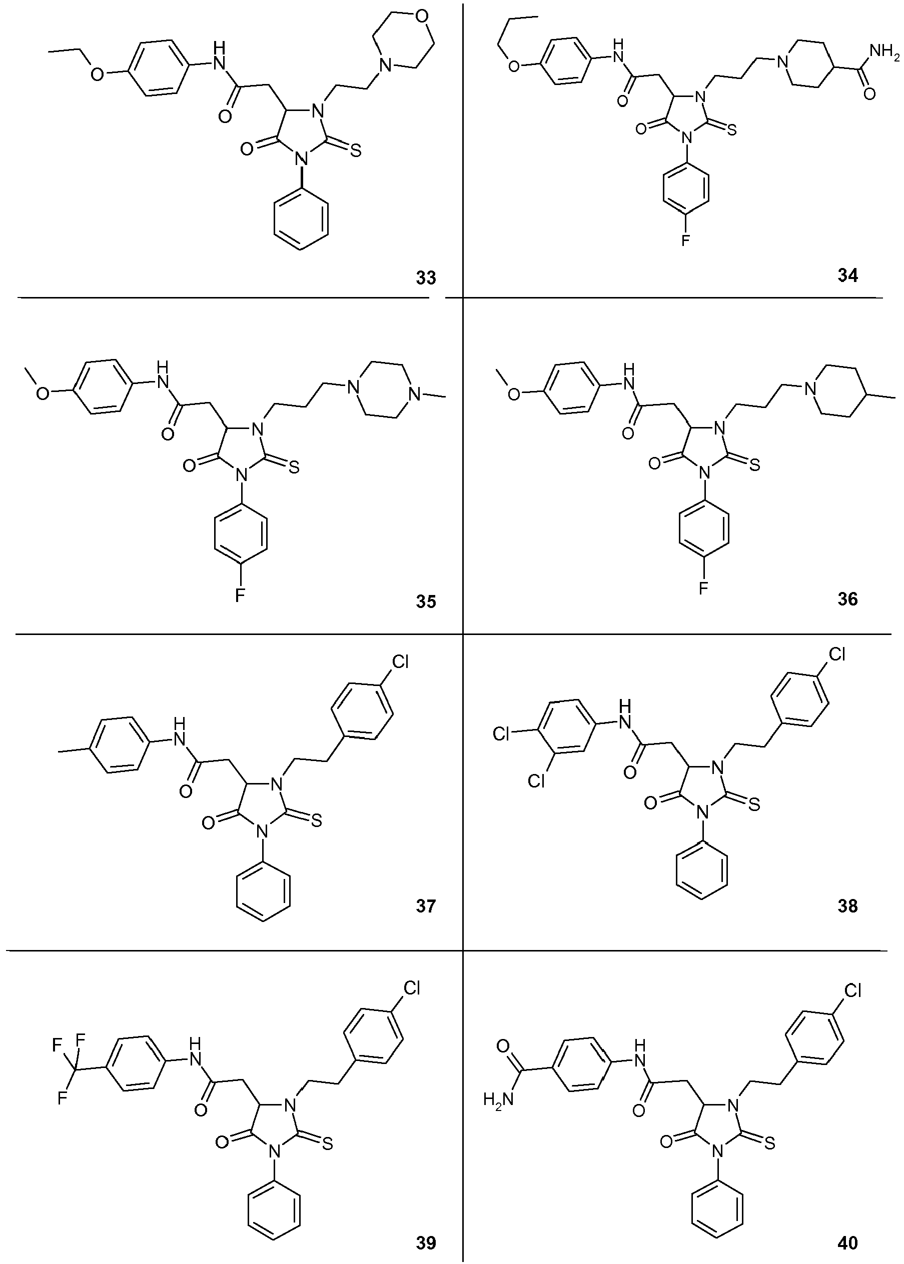

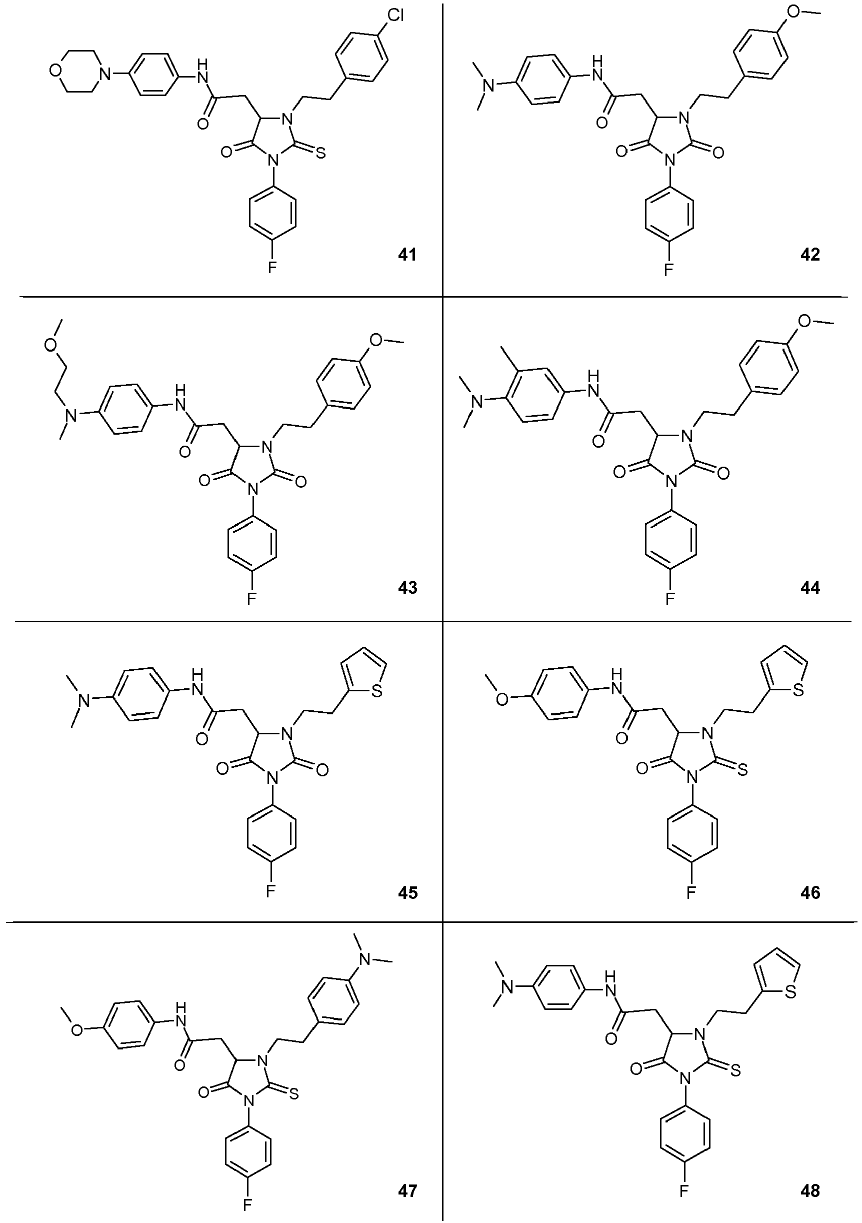

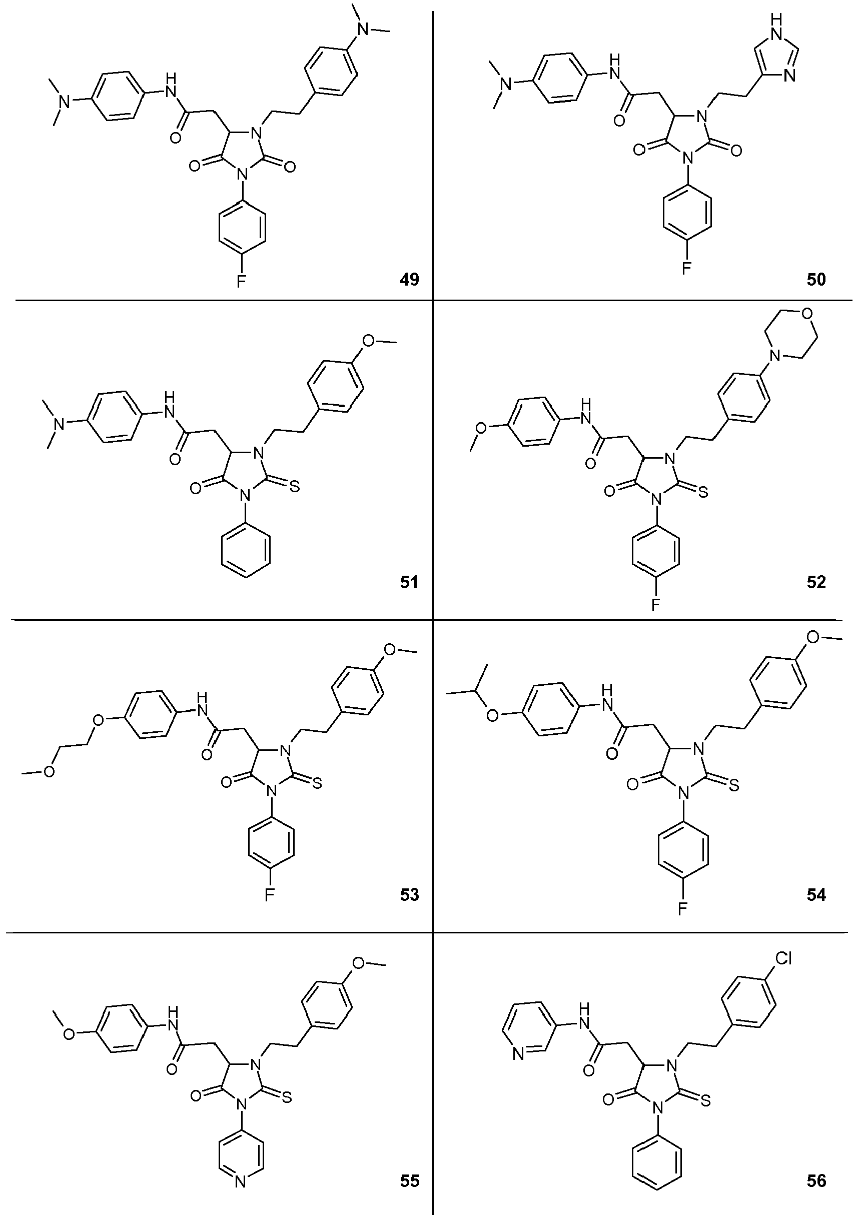

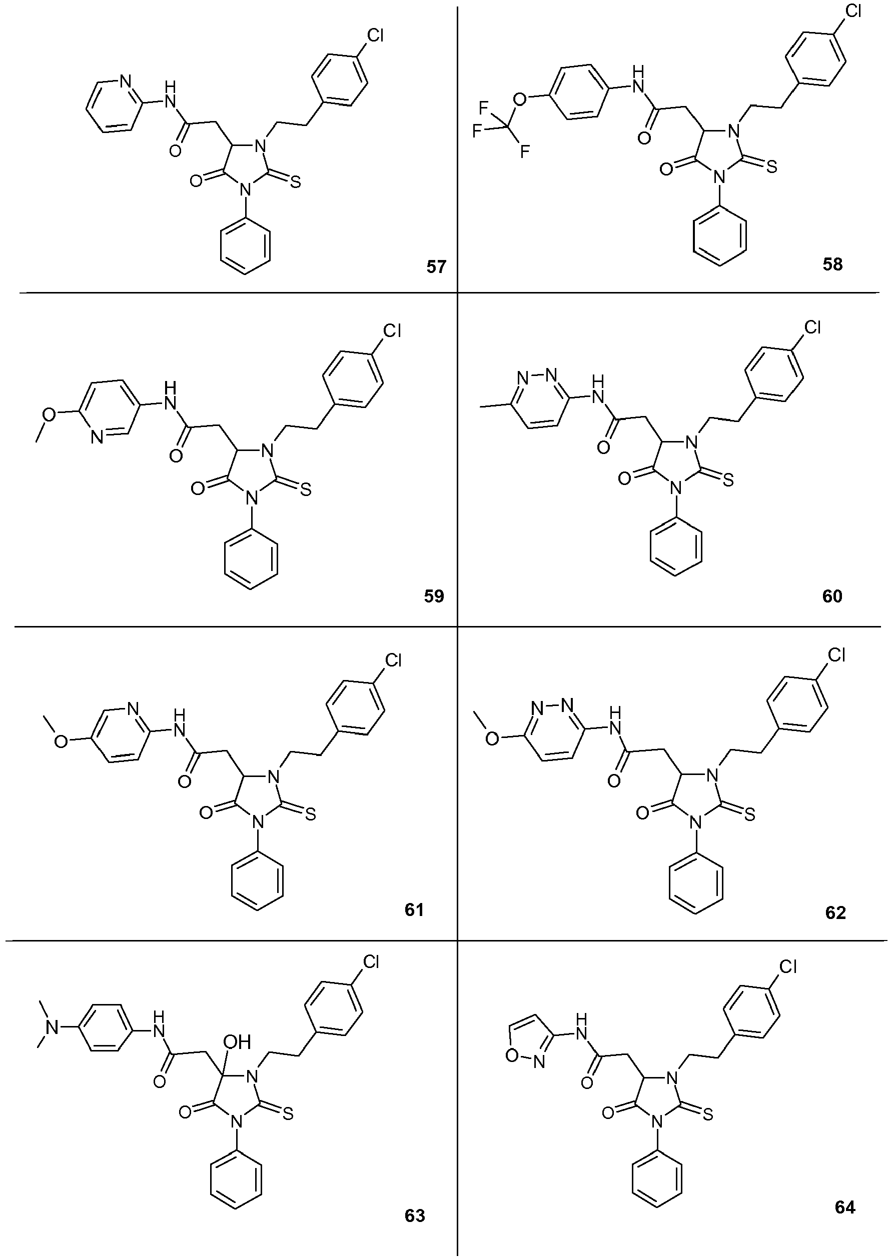

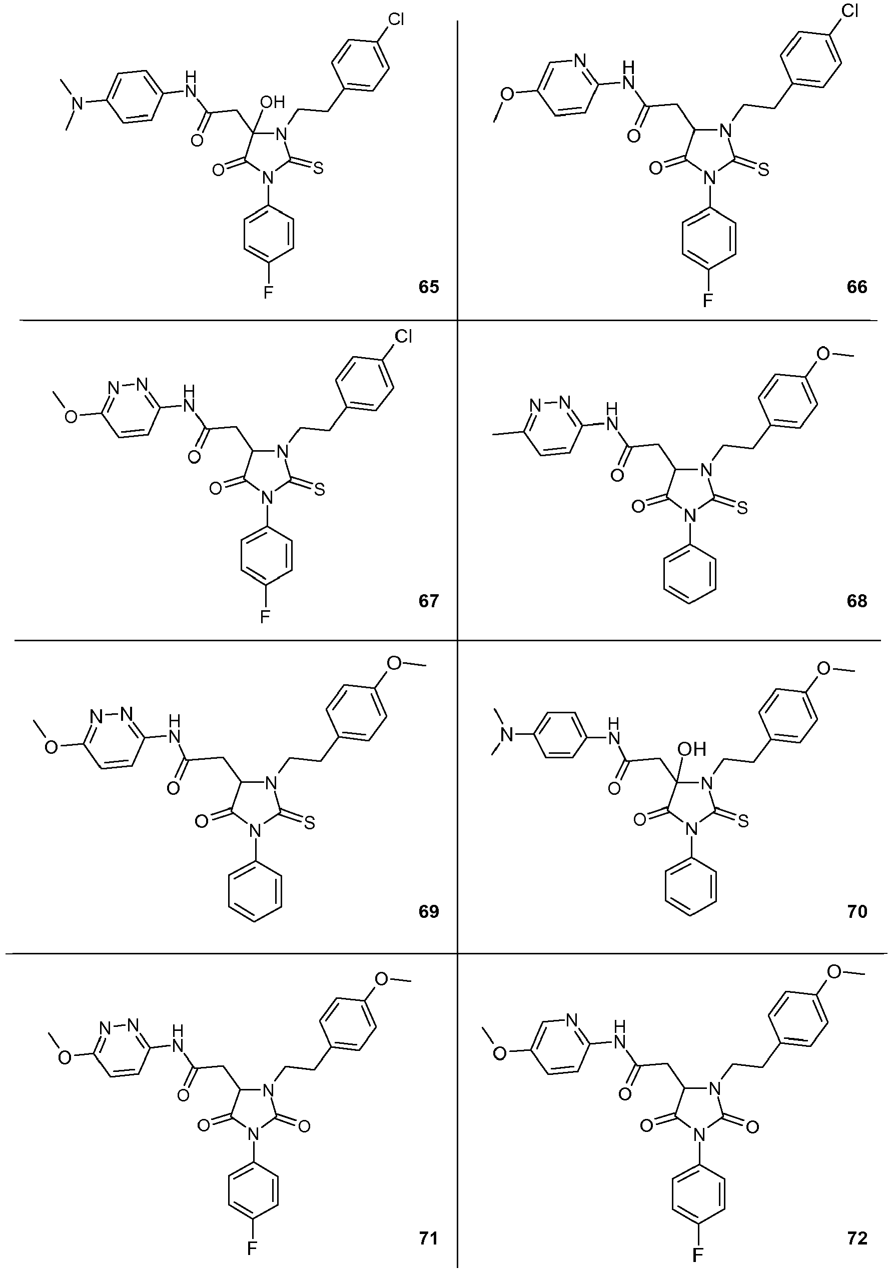

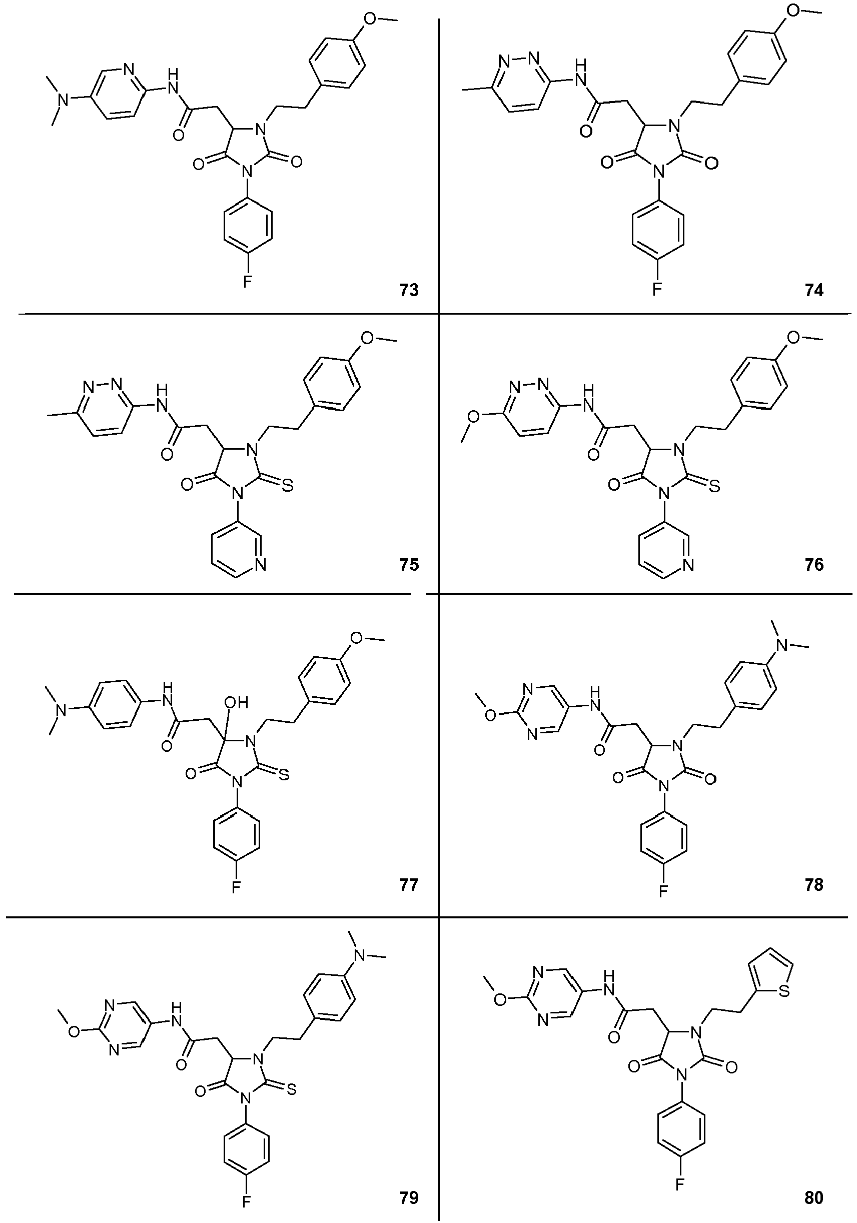

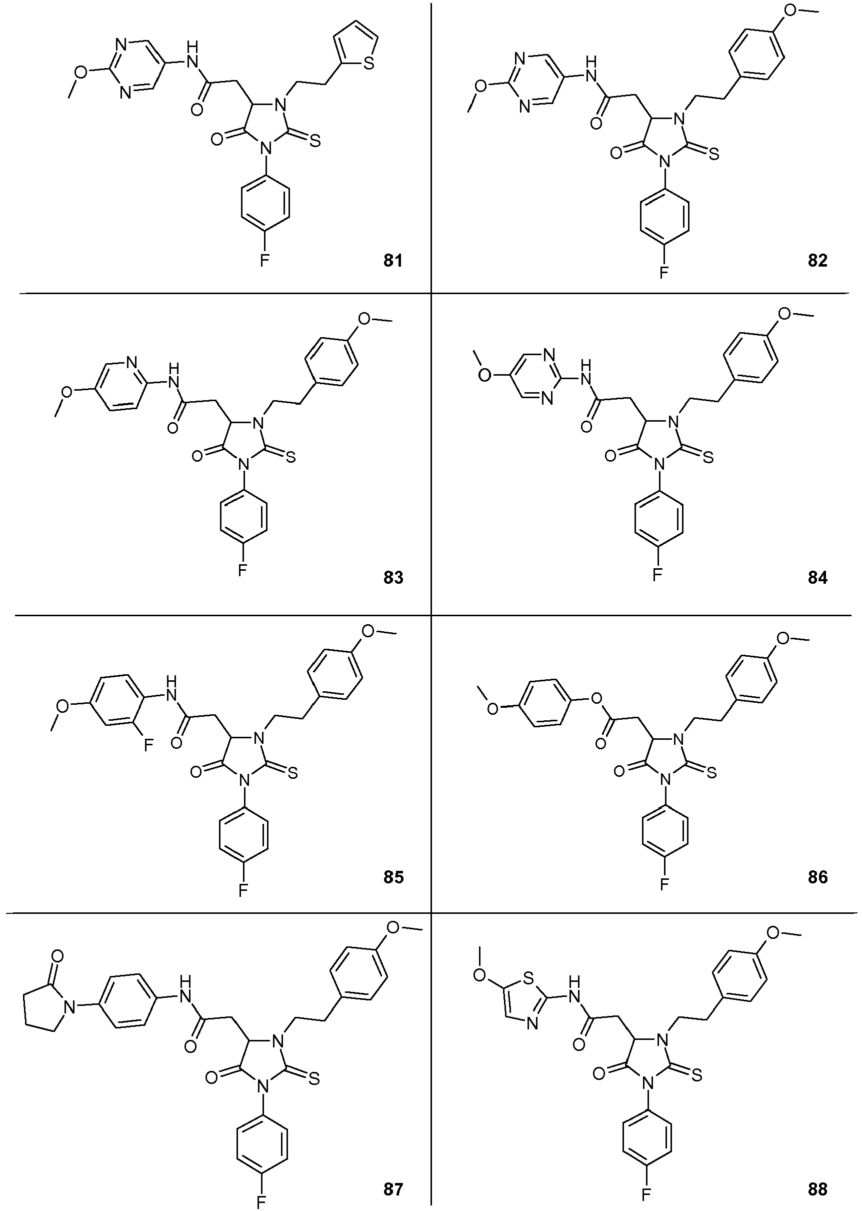

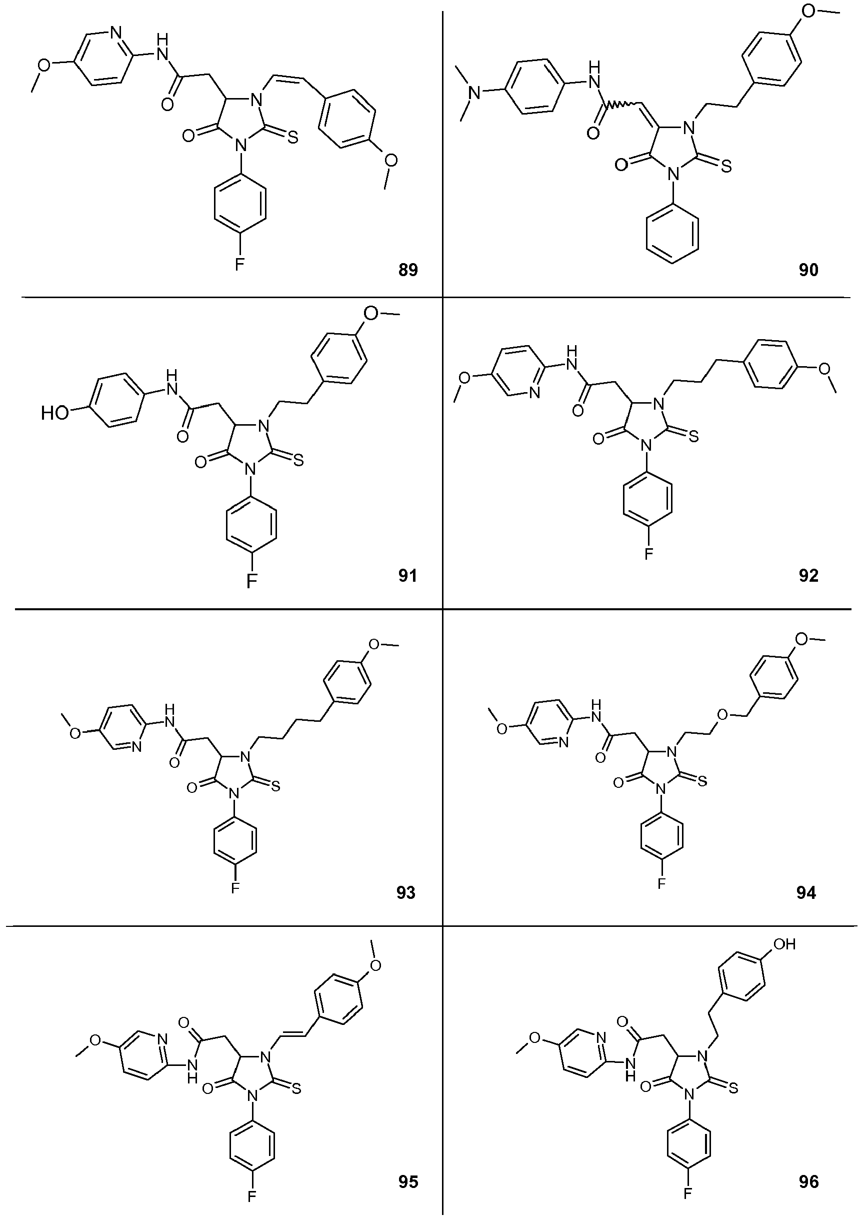

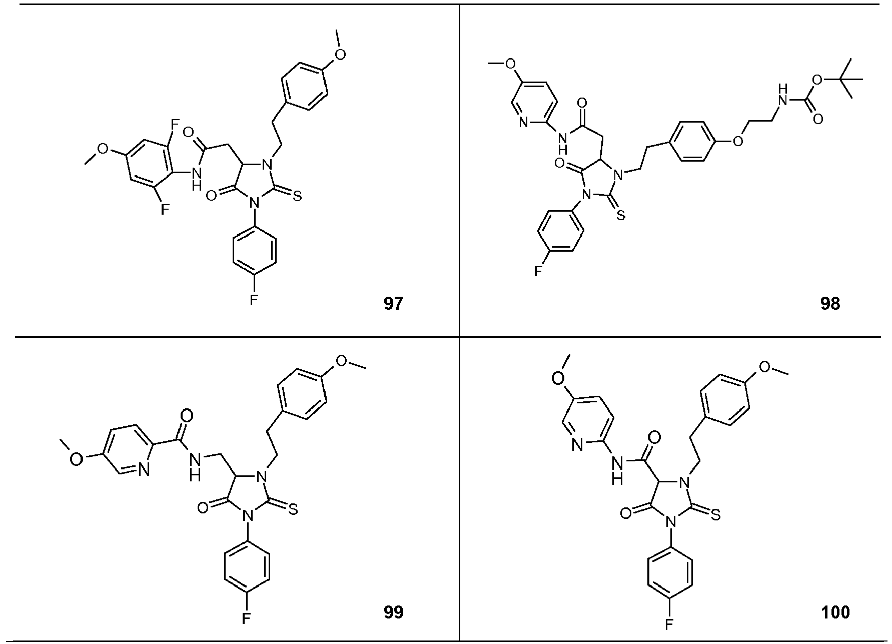

The present invention relates also to the use of a compound of formula (I) as described above, or a salt, solvate, tautomer, enantiomer, diastereoisomer or racemic mixture thereof, for the manufacturing of a drug, in particular of an antiviral drug, preferably intended to treat hepatitis C, for example as a hepatitis C virus replication inhibitor.

The present invention relates also to a method to treat a viral infection, in particular hepatitis C, by administering to a person in need thereof an efficient amount of a compound of formula (I) as defined above, or a salt, solvate, tautomer, enantiomer, diastereoisomer or racemic mixture thereof.

The present invention concerns also a pharmaceutical composition comprising a compound of formula (I) as defined above, or a salt, solvate, tautomer, enantiomer, diastereoisomer or racemic mixture thereof, in combination with pharmaceutically acceptable excipients.

Advantageously, the pharmaceutical composition according to the present invention contains a further antiviral agent, in particular selected in the group consisting of ribavirin, interferon, inhibitors of HCV NS3 helicase activity, inhibitors of HCV NS3 protease activity, inhibitors of HCV NS4A, inhibitors of HCV NS5A, inhibitors of HCV NS5B, inhibitors of NS2, agonists of toll-like receptor 7 (TLR7), inhibitors of cyclophilin, anti-HIV agents and a mixture thereof. Inhibitors of HCV replication complex proteins as well as other drugs with hepatitis C virus inhibitory activity are described in Caister Academin Press, 2011. Hepatitis C: antiviral drug discovery and development.

The present invention also concerns a compound of formula (I) as defined above, or a salt, solvate, tautomer, enantiomer, diastereoisomer or a racemic mixture thereof, for use as a combination therapy with at least another antiviral agent, preferably selected in the group consisting of ribavirin, interferon, inhibitors of HCV NS3 helicase activity, inhibitors of HCV NS3 protease activity, inhibitors of HCV NS4A, inhibitors of HCV NS5A, inhibitors of HCV NS5B, inhibitors of HCV NS5A, inhibitors of NS2, agonists of toll-like receptor 7 (TLR7), inhibitors of cyclophilin, anti-HIV agent and a mixture thereof, wherein said compounds are provided as separate pharmaceutical compositions for simultaneous or sequential administration.

Therefore the compound of formula (I) according to the present invention can be used as a bi-, tri-therapy or multi-therapy in order to treat hepatitis C, namely, the compound of formula (I) according to the present invention can be used as a combination therapy with two, three or more antiviral agents, respectively. In one embodiment, said one or more antiviral agent is an anti-hepatitis C antiviral agent, which is preferably selected from the group consisting of ribavirin, interferon, inhibitors of HCV NS3 helicase activity, inhibitors of HCV NS3 protease activity, inhibitors of HCV NS4A, inhibitors of HCV NS5B, inhibitors of HCV NS5A, inhibitors of NS2, agonists of TLR7, inhibitors of cyclophilin, or mixture thereof. In another embodiment, said antiviral agent is one or more anti-HIV antiviral agents for use in a combination therapy for treating hepatitis C in a patient having also HIV disease. In a further embodiment, the compound of formula (I) according to the present invention is used as a tri-therapy with another anti-hepatitis C antiviral agent and an anti-HIV antiviral agent in order to treat hepatitis C in a patient having also HIV disease.

The present invention concerns also a compound of the following formula (I), or a salt, solvate, tautomer, enantiomer, diastereoisomer or racemic mixture thereof: 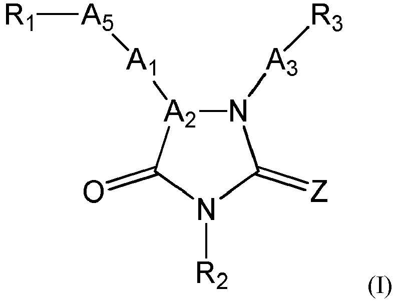 wherein:
- Z represents O or S,
- A₁ represents -OC(O)-, -C(O)O-, -NHC(O)- or -C(O)NH-,
- A₂ represents 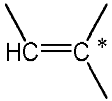 (the double bond having thus a Z or E configuration) or 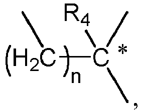 the carbon atom marked with a star being comprised in the hydantoin or thiohydantoin ring,
- A₃ represents -(CH₂)ₒ-, -CH=CH-, -C≡C-, or -(CH₂)ₘ-A₄-(CH₂)ₚ-,
- A₄ represents O, NH or 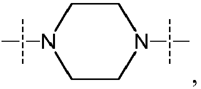
- A₅ represents 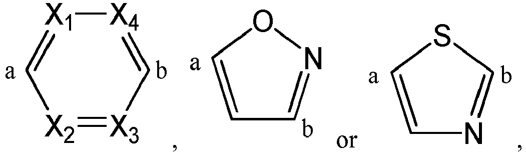 the carbon atoms marked with a being bound to R₁ and the carbon atoms marked with b being bound to (CH₂)ᵣA₁,
- X₁ represents N or CR₅,
- X₂ represents N or CR₆,
- X₃ represents N or CR₇,
- X₄ represents N or CR₈,
- R₁ represents a hydrogen atom, a halogen atom, R₉, OR₁₀, NR₁₁R₁₂, C(O)R₁₀, C(O)OR₁₀, OC(O)R₉, C(O)NR₁₁R₁₂ or NR₁₃C(O)R₁₄,
- R₂ represents a (C₁-C₆)alkyl or -(CH₂)_{q}-R₂₂ group,
- R₃ represents a (C₁-C₆)alkyl group, a (C₂-C₆)alkenyl group or a cycloalkyl, cycloalkenyl, heterocyclic, aryl or heteroaryl ring, the said ring being optionally substituted with one or several groups, preferably with one, chosen among a halogen atom, a oxo group, (C₁-C₆)alkyl, (C₁-C₆)haloalkyl, OR₂₃, NR₁₇R₁₈ and SO₂NR₁₉R₂₀,
- R₄ represents a hydrogen atom or an OH, (C₁-C₆)alkyl or (C₁-C₆)alkoxy group,
- R₅ and R₆ represent, independently of each other, a hydrogen atom, a halogen atom, a (C₁-C₆)alkyl or a (C₁-C₆)alkoxy group,
- R₇ and R₈ represent, independently of each other, H or F,
- R₉ represents a (C₁-C₆)alkyl, (C₁-C₆)haloalkyl, or (C₁-C₆)alkyl-O-(C₁-C₆)alkanediyl group,
- R₁₀ represents a hydrogen atom or a (C₁-C₆)alkyl, (C₁-C₆)haloalkyl, or (C₁-C₆)alkyl-O-(C₁-C₆)alkanediyl group,
- R₁₁ and R₁₂ represent, independently of each other, a hydrogen atom or a (C₁-C₆)alkyl, (C₁-C₆)haloalkyl or (C₁-C₆)alkyl-O-(C₁-C₆)alkanediyl group, or R₁₁ and R₁₂ form, together with the nitrogen atom bearing them, a heterocycle optionally comprising another heteroatom chosen among O, S and N and optionally substituted with a (C₁-C₆)alkyl or oxo group,
- R₁₃ represents a hydrogen atom or a (C₁-C₆)alkyl, (C₁-C₆)haloalkyl or (C₁-C₆)alkyl-O-(C₁-C₆)alkanediyl group,
- R₁₄ represents a (C₁-C₆)alkyl, (C₁-C₆)haloalkyl, or (C₁-C₆)alkyl-O-(C₁-C₆)alkanediyl group, or
   R₁₃ and R₁₄ form together a -(CH₂)₂- or -(CH₂)₃- chain,
- R₁₅ and R₁₆ represent, independently of each other, a hydrogen atom or a (C₁-C₆)alkyl or (C₁-C₆)haloalkyl group, or R₁₅ and R₁₆ form, together with the nitrogen atom bearing them, a heterocycle optionally comprising another heteroatom chosen among O, S and N and optionally substituted with a (C₁-C₆)alkyl or oxo group,
- R₁₇ and R₁₈ represent, independently of each other, a hydrogen atom or a (C₁-C₆)alkyl or (C₁-C₆)haloalkyl group, or R₁₇ and R₁₈ form, together with the nitrogen atom bearing them, a heterocycle optionally comprising another heteroatom chosen among O, S and N and optionally substituted with a (C₁-C₆)alkyl or oxo group,
- R₁₉ and R₂₀ represent, independently of each other, a hydrogen atom or a (C₁-C₆)alkyl or (C₁-C₆)haloalkyl group, or R₁₉ and R₂₀ form, together with the nitrogen atom bearing them, a heterocycle optionally comprising another heteroatom chosen among O, S and N and optionally substituted with a (C₁-C₆)alkyl or oxo group,
- R₂₂ represents a cycloalkyl, aryl or heteroaryl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆,
- R₂₃ represents a hydrogen atom or a (C₁-C₆)alkyl, (C₁-C₆)haloalkyl or (C₁-C₆)alkyl-OC(O)NH-(C₁-C₆)alkanediyl group,
- n represents 0 or 1,
- m represents 0, 1, 2 or 3,
- o represents 1, 2, 3, 4, 5 or 6,
- p represents 0, 1, 2 or 3, and
- q represents 0, 1, 2 or 3, preferably 0,
   with the exception of:
   ■ the compounds for which Z = O or S; A₁ = -NHC(O)-, C(O) being bound to A₂; 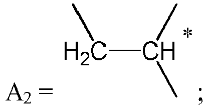 A₃ = -(CH₂)ₒ-; 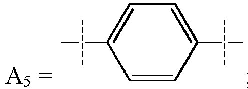 and R₁ R₂ and R₃ are as defined above (most of these compounds are commercially available; one of them is described in Turgeon et al. Antimicrob. Agents Chemother. 2011, 55(3), 983-991, no biological activity being described), and
   ■ the compound of the following formula:

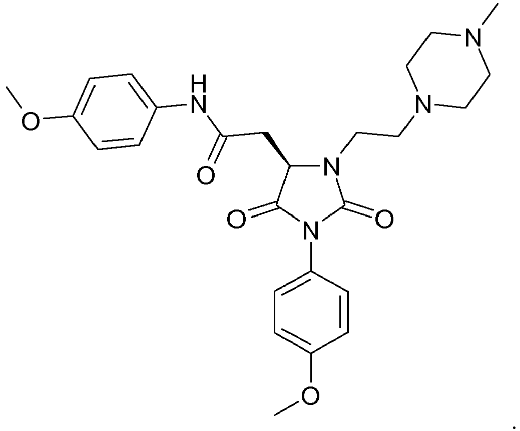

According to a particular embodiment, X₃ and/or X₄ represent CF or at least one, notably one or two, of X₁, X₂, X₃ and X₄ represents N.

Advantageously, A₁ represents -OC(O)- or -NHC(O)-, notably -NHC(O)-(C(O)- being bound to A₂).

A₂ will represent in particular 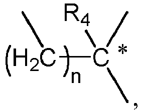 with n representing advantageously 1. In this case, R₄ represents advantageously H, OH or (C₁-C₆)alkoxy, notably H or OH.

The compounds according to the invention can thus fit the following formula (Ia): 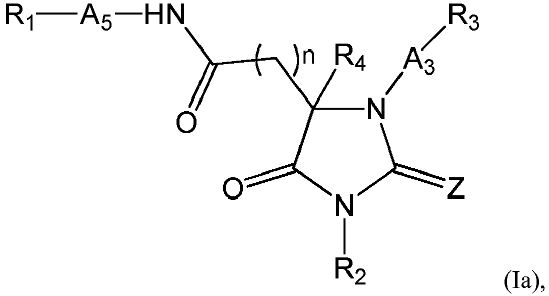 with n representing advantageously 1 and with R₄ representing advantageously H, OH or (C₁-C₆)alkoxy, notably H or OH.

A₃ will represent advantageously O, NH, -CH=CH-, -(CH₂)ₒ-, or -(CH₂)ₘ-A₄-(CH₂)ₚ-, with A₄ representing O or 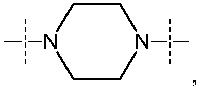 and notably with m = 2 or 3 and p = 0 or 1. A₃ can be in particular -(CH₂)ₒ-, in particular with o = 1, 2, 3 or 4, notably with o = 2.

Advantageously, A₅ will be

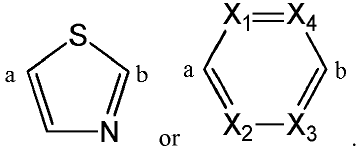

When A₅ represents 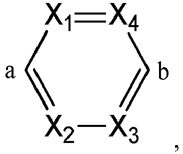 preferably at most two of X₁, X₂, X₃ and

X₄ represent N. In particular, 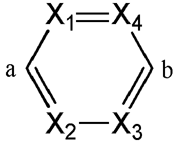 can be one of the following rings:

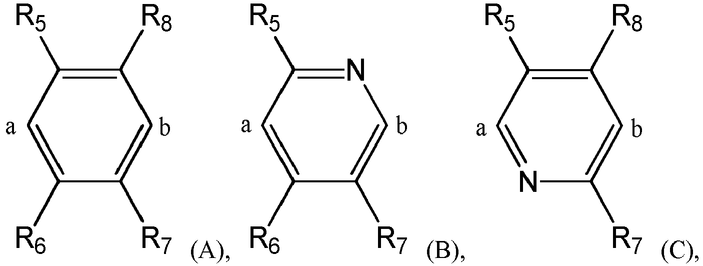

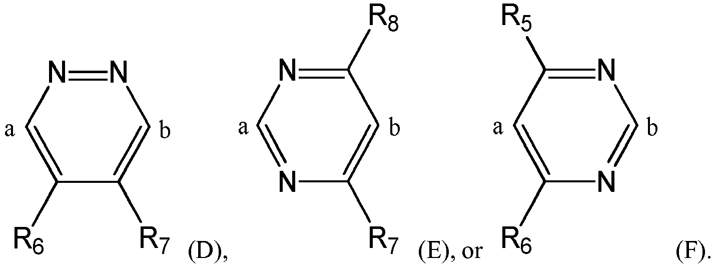

In particular, the ring 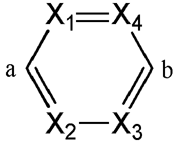 will be a ring (A), (B), or (F), preferably, a ring (B) or (F). R₅ and R₆, when present, will represent advantageously a hydrogen atom.

Advantageously, R₁ represents a hydrogen atom, a halogen atom, R₉, OR₁₀, NR₁₁R₁₂, C(O)OR₁₀, OC(O)R₉, C(O)NR₁₁R₁₂ or NR₁₃C(O)R₁₄. R₁ can represent in particular a hydrogen atom, a halogen atom, R₉, OR₁₀, NR₁₁R₁₂, C(O)OR₁₀ or C(O)NR₁₁R₁₂. Preferably, R₁ represents a hydrogen atom, a halogen atom, R₉, OR₁₀, or NR₁₁R₁₂.

Advantageously, R₂ represents an aryl or heteroaryl ring, notably a phenyl or pyridinyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆. R₂ can represent in particular a phenyl ring optionally substituted with one or several groups, preferably with one (in particular in *para* position), chosen among a halogen atom, OH, (C₁-C₆)alkyl, (C₁-C₆)haloalkyl, (C₁-C₆)alkoxy, (C₁-C₆)haloalkoxy and NR₁₅R₁₆; or a pyridinyl ring. Preferably, R₂ represents a phenyl ring optionally substituted with one or several groups, preferably with one (in particular in *para* position), chosen among a halogen atom, OH, (C₁-C₆)alkyl, (C₁-C₆)haloalkyl, (C₁-C₆)alkoxy, (C₁-C₆)haloalkoxy and NR₁₅R₁₆; or a pyridinyl ring. Most preferably, R₂ represents a phenyl ring optionally substituted in *para* position with one group chosen among a halogen atom, OH, (C₁-C₆)alkyl, (C₁-C₆)haloalkyl, (C₁-C₆)alkoxy, (C₁-C₆)haloalkoxy and NR₁₅R₁₆; or a pyridinyl ring.

Preferably, R₃ does not represent a substituted or unsubstitued pyridinyl group.

Advantageously, R₃ represents a cycloalkyl, cycloalkenyl, heterocyclic, aryl or heteroaryl ring, the said ring being optionally substituted with one or several groups, preferably with one, chosen among a halogen atom, a oxo group, (C₁-C₆)alkyl, (C₁-C₆)haloalkyl, OR₂₃, NR₁₇R₁₈ and SO₂NR₁₉R₂₀. Notably, R₃ represents a cyclohexenyl, morpholinyl, piperazinyl, pyrrolidinyl, piperidinyl, 1,3-benzodioxolyl, phenyl, pyridinyl, thienyl, imidazolyl, or indolyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy, NR₁₇R₁₈ and SO₂NR₁₉R₂₀. In particular, R₃ will represent a phenyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy, NR₁₇R₁₈ and SO₂NR₁₉R₂₀.

The compound of formula (I) can be in particular one of the compounds 37 to 100 indicated above.

The present invention concerns also a method to prepare a compound of formula (I) according to the present invention for which A₁ = -OC(O)- or -NHC(O)- and A₂ = 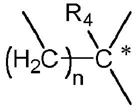 with R4 = H, OH or (C₁-C₆)alkoxy comprising the coupling of a compound of the following formula (II): 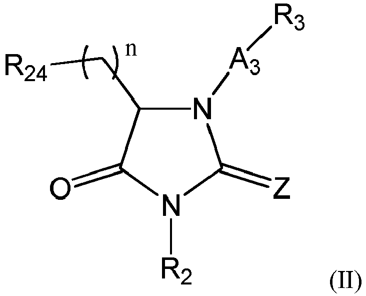 wherein Z, A₃, R₂, R₃ and n are as defined above and R₂₄ represents a carboxylic acid function (COOH) optionally in an activated form, with a compound of the following formula (III):

R₁——A₅—R₂₅ (III)

wherein R₁ and A₅ are as defined above and R₂₅ represents OH or NH₂, to give a compound of formula (I) with A₁ = -OC(O)- or -NHC(O)- and A₂ = 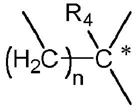 with R4 = H or OH, followed optionally, when R₄ = OH, by a substitution of the OH function to give a compound of formula (I) with A₁ = -OC(O)- or -NHC(O)- and 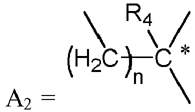 with R4 = (C₁-C₆)alkoxy.

In the context of the present invention, "activated form" of the COOH function means a derivative of a carboxylic function which is more active toward a nucleophile reagent in a nucleophile substitution reaction. It can be in particular an acyl choride (COCl).

When R₂₄ = COOH, the coupling can be carried out in the presence of a coupling agent, such as diisopropylcarbodiimide (DIC), dicyclohexylcarbodiimide (DCC), 1-(3-dimethylaminopropyl)-3-ethylcarbodiimide hydrochloride (EDC), carbonyldiimidazole (CDI), hexafluorophosphate 2-H-benzotriazole-1-yl)-1,1,3,3-tetraméthyluronium (HBTU), tetrafluoroborate 2-(1H-benzotriazole-1-yl)-1,1,3,3-tetramethyluronium (TBTU), hexafluorophosphate O-(7-azobenzotriazol-1-yl)-1,1,3,3-tetramethyluronium (HATU) or or (benzotriazol-1-yloxy)tripyrrolodinophosphonium hexafluorophosphate (PyBOP); optionally associated with an auxiliary coupling, such as N-hydroxy-succinimide (NHS), N-hydroxy-benzotriazole (HOBt), 3,4-dihydro-3-hydroxy-4-oxo-1,2,3-benzotriazole (HOOBt), 1-hydroxy-7-azabenzotriazole (HAt) or N-hydroxysylfosuccinimide (sulfo NHS), and optionally with a base such as N,N-diisopropylethylamine (DIPEA).

In this case, the coupling can be carried out notably in the presence of EDCI, HOBt and DIPEA; HATU and DIPEA; or TBTU and DIPEA.

A solvent such as dimethylformamide and/or dioxane can be used. The reaction of coupling can be performed notably at room temperature.

When R₂₄ represents an activated form of a carboxylic acid function, and in particular an acyl chloride (R₂₄ = COCl), this coupling can be carried out in the presence of a base such as DIPEA and pyridine.

A solvent such as dichloromethane can be used.

The acyl chloride can be obtained from the corresponding carboxylic acid by reaction with oxalyl chloride in the presence of DMF.

The substitution of the OH function can be carried out by methods well known to those skilled in the art.

The compound obtained can be separated from the reaction medium by methods well known to the person skilled in the art, such as by extraction, evaporation of the solvent or by precipitation or crystallisation (followed by filtration).

The compound can be also purified if necessary by methods well known to the person skilled in the art, such as by recrystallisation, by distillation, by chromatography on a column of silica gel or by high performance liquid chromatography (HPLC).

Compounds of formula (III) can be commercially available or prepared by methods well known to those skilled in the art.

Compounds of formula (II) with R₂₄ = COOH can be obtained by hydrolysis of a compound of the following formula (IV): 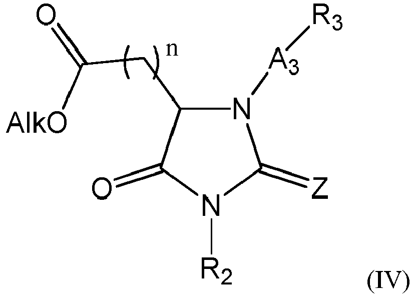 wherein Z, A₃, R₂, R₃ and n are as defined above and Alk represents a (C₁-C₆)alkyl group, such as ethyl.

The reaction of hydrolysis can be carried out by methods well known to those skilled in the art, notably by treatment of the compound of formula (II) with a hydroxide salt, such as NaOH, KOH, LiOH or TBAOH.

Compounds of formula (IV) can be obtained by reaction of a compound of the following formula (V): 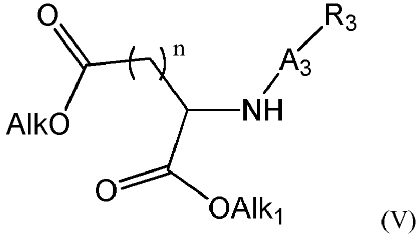 wherein A₃, R₃ and n are as defined above and Alk and Alk₁ represent, independently of each other, a (C₁-C₆)alkyl group, such as ethyl, with a compound of the following formula (VI):

R₂-NCZ (VI)

wherein Z and R₂ are as defined above.

This reaction can be carried out in a solvent such as dichloromethane, notably at room temperature.

Compounds of formula (VI) can be commercially available or prepared by methods well known to those skilled in the art.

When n = 1, compounds of formula (V) can be prepared by reaction between a compound of the following formula (VII): 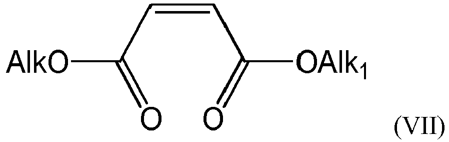 wherein Alk and Alk₁ are as defined above, and a compound of the following formula (VIII):

R₃-A₃-NH₂ (VIII)

wherein A₃ and R₃ are as defined above.

This reaction can be carried out in a solvent such as acetonitrile, notably at room temperature.

Compounds of formula (VII) and (VIII) can be commercially available or prepared by methods well known to those skilled in the art.

When n = 0, compounds of formula (V) can be prepared by reaction between a compound of the following formula (IX): 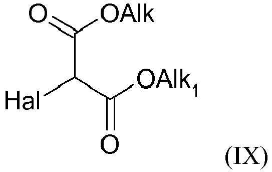 wherein Alk and Alk₁ are as defined above and Hal represents a halogen atom such as a brome,
and a compound of formula (VIII) as defined above.

Compounds of formula (IX) can be commercially available or prepared by methods well known to those skilled in the art.

The present invention concerns also a method to prepare a compound of formula (1) according to the present invention for which 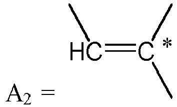 by dehydration of the corresponding compound of formula (I) for which 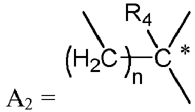 with R4 = OH.

Such a reaction can be carried out in the presence of *para*-toluenesulfonic acid.

Thus, the compounds according to the present invention can be prepared notably according to one of the following general schemes:

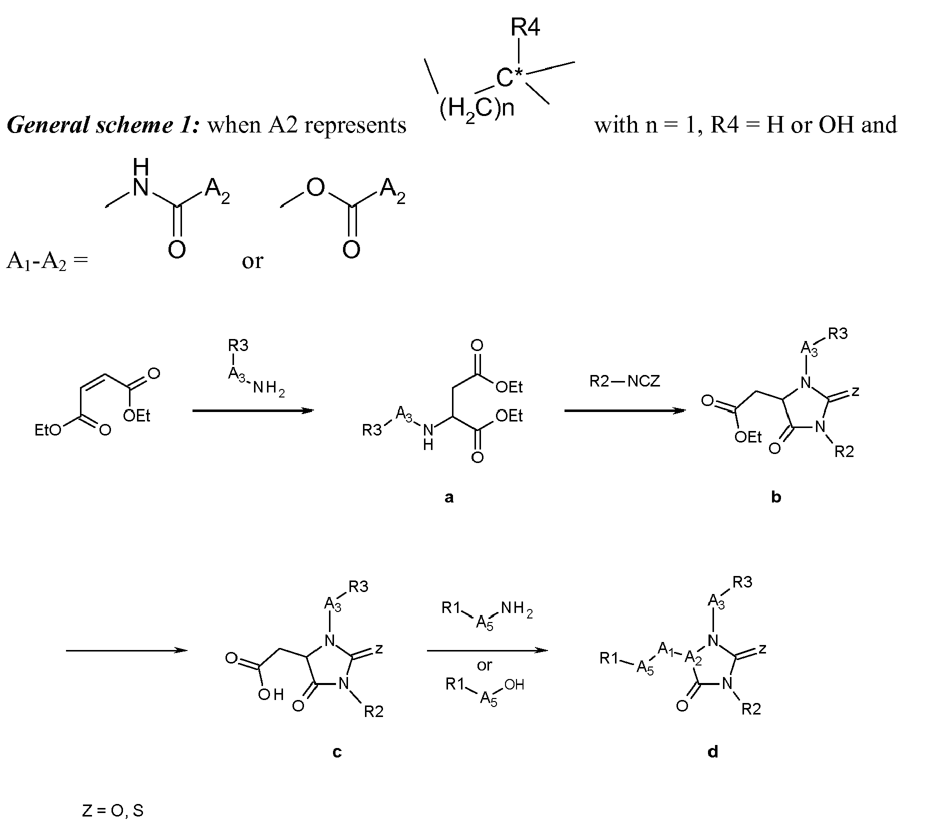

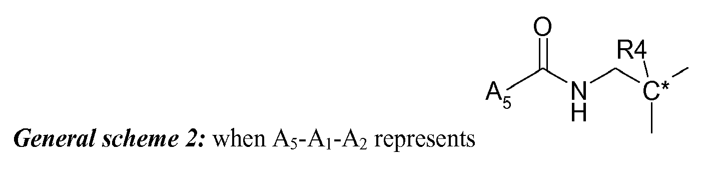

In one embodiment: route A

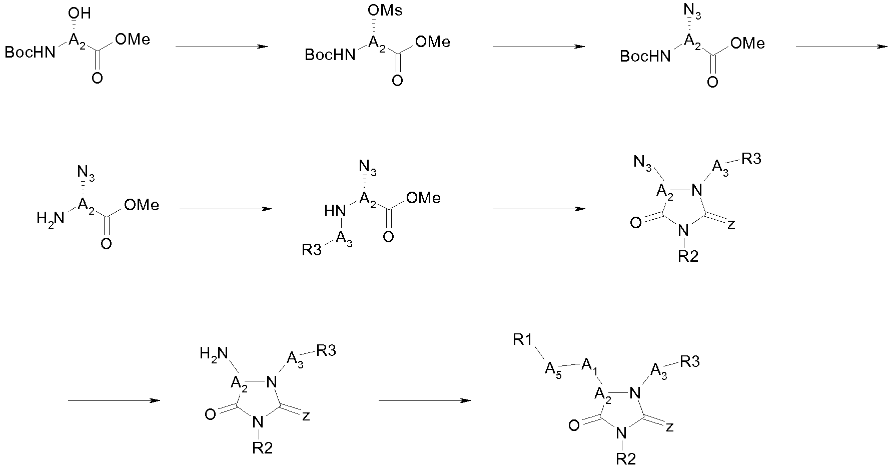

In a preferred embodiment: route B

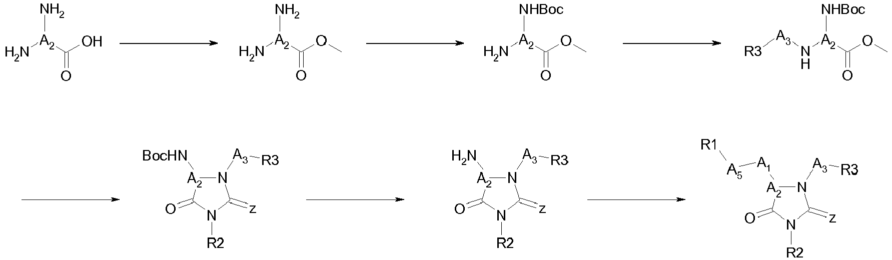

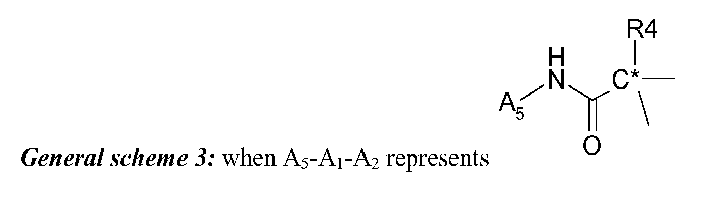

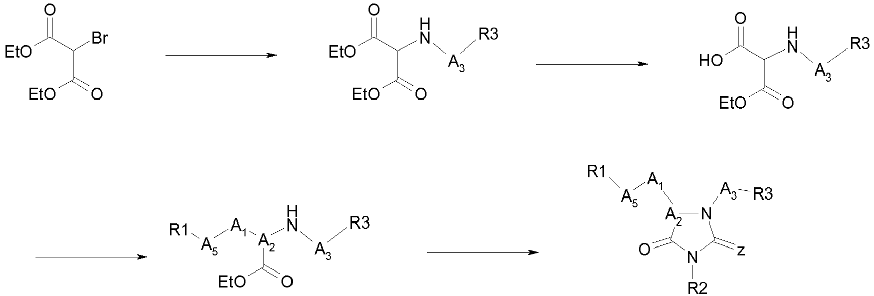

### DEFINITIONS

The terms "comprising" and "comprises" means "including" as well as "consisting" e.g. a composition "comprising" X may consist exclusively of X or may include something additional e.g. X + Y.

The word "substantially" does not exclude "completely" e.g. a composition which is "substantially free" from Y may be completely free from Y. Where necessary, the word "substantially" may be omitted from the definition of the invention.

"Optional" or "optionally" means that the subsequently described event or circumstances may or may not occur, and that the description includes instances where said event or circumstance occurs and instances in which it does not.

### Antiviral agent

By antiviral agent it is meant any of several drugs used to treat or prevent viral infections. The drugs act by interfering with a virus's ability to enter a host cell and replicate itself with the host cell's DNA or RNA. Some drugs block the virus's attachment or entry into the cell; others inhibit replication or prevent the virus from shedding the protein coat that surrounds the viral DNA or RNA.

Antiviral agents or drugs are now available for a wide variety of viral diseases. For example, Ribavirin, available since the mid-1980s, is used to treat respiratory syncytial virus (RSV), a cause of severe childhood respiratory infections. It is thought to inhibit messenger RNA. Amantadine and rimantadine, which are effective against strains of influenza A, act by interfering with viral uncoating.

### Pharmaceutical compositions

It is anticipated that the compounds of the invention can be administered by oral or parenteral routes, intestinal, ocular, vaginal, rectal nasal (intranasal), pulmonary or other mucosal, transdermal and topical administration, and inhalation, advantageously by oral route. Primary routes for parenteral administration include intravenous, intramuscular, and subcutaneous administration. Secondary routes of administration include intraperitoneal, intra-arterial, intra-articular, intracardiac, intracisternal, intradermal, intralesional, intraocular, intrapleural, intrathecal, intrauterine, and intraventricular administration.

For oral administration, the compounds of the invention will generally be provided in the form of tablets or capsules or as an aqueous solution or suspension. Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract. Capsules for oral use include hard gelatine capsules in which the active ingredient is mixed with a solid diluent and soft gelatine capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions of the present invention may, in particular, comprise more than one agent (multiple) of the present invention, *e.g.*, two or more agents. The invention also provides a pharmaceutical preparation or system, comprising (a) a first agent, which is an agent of the invention; and (b) a second pharmaceutical agent. Said multiple agents of the invention or said first and second agents are formulated either in admixture or as separate compositions, e.g. for simultaneous though separate, or for sequential administration (see below).

The compositions of the present invention can be delivered directly or in pharmaceutical compositions containing excipients (see above), as is well known in the art. The present methods of treatment involve administration of a therapeutically effective amount of an agent of the present invention to a subject. The term "therapeutically effective amount" as used herein refers to an amount of an agent according to the present invention needed to treat or ameliorate the targeted disease condition, or to exhibit a detectable therapeutic effect or a prolongation of survival in a patient. In general, the therapeutically effective dose can be estimated initially either in cell culture assays or in animal models, for example, in non-human primates, mice, rabbits, dogs, or pigs. The animal model may also be used to determine the appropriate concentration range and route of administration. Such information can then be used to determine useful doses and routes for administration in humans. Effective doses of the compounds of the present invention may be ascertained by conventional methods. The specific dosage level required for any particular patient will depend on a number of factors, including severity of the condition being treated, the route of administration, the general health of the patient (i.e. age, weight and diet) in particular in HIV patients, the gender of the patient, the time and frequency of administration, and tolerance/response to therapy.

In general, however, the daily dose (whether administered as a single dose or as divided doses) will be in the range 0.001 to 5000 mg per day, more usually from 1 to 2500 mg per day, and most usually from 10 to 1500 mg per day. Alternatively, dosages can be administered per unit body weight and in this instance a typical dose will be between 0.01 µg/kg and 50 mg/kg, especially between 10 µg/kg and 10 mg/kg, between 100 µg/kg and 2 mg/kg.

The present compositions may, if desired, be presented in a pack or dispenser device containing one or more unit dosage forms containing the active ingredient. Such a pack or device may, for example, comprise metal or plastic foil, such as a blister pack, or glass and rubber stoppers such as in vials. The pack or dispenser device may be accompanied by instructions for administration. Compositions comprising an agent of the invention formulated in a compatible pharmaceutical carrier may also be prepared, placed in an appropriate container, and labelled for treatment of an indicated condition.

### Chemical Definitions

The compounds of the present invention may be present in the form of pharmaceutically acceptable salts. For use in medicine, the salts of the compounds of this invention refer to non-toxic "pharmaceutically acceptable salts." Pharmaceutically acceptable salt forms include pharmaceutically acceptable acidic/anionic or basic/cationic salts. Pharmaceutically acceptable salts of the acidic or basic compounds of the invention can of course be made by conventional procedures, such as by reacting the free base or acid with at least a stoichiometric amount of the desired salt-forming acid or base. Pharmaceutically acceptable salts of the acidic compounds of the invention include salts with inorganic cations such as sodium, potassium, calcium, magnesium, zinc, and ammonium, and salts with organic bases. Suitable organic bases include N-methyl-D-glucamine, arginine, benzathine, diolamine, olamine, procaine and tromethamine. Pharmaceutically acceptable salts of the basic compounds of the invention include salts derived from organic or inorganic acids. Suitable anions include acetate, adipate, besylate, bromide, camsylate, chloride, citrate, edisylate, estolate, fumarate, gluceptate, gluconate, glucuronate, hippurate, hyclate, hydrobromide, hydrochloride, iodide, isethionate, lactate, lactobionate, maleate, mesylate, methylbromide, methylsulphate, napsylate, nitrate, oleate, pamoate, phosphate, polygalacturonate, stearate, succinate, sulphate, sulphosalicylate, tannate, tartrate, terephthalate, tosylate and triethiodide. Hydrochloride salts are particularly preferred.

The compounds of the present invention may be also present in the form of a "solvate", i.e. a combination of the said compounds with a solvent.

Where the compounds according to this invention have at least one chiral centre, they may accordingly exist as enantiomers. Where the compounds possess two or more chiral centres, they may additionally exist as diastereomers. Where the processes for the preparation of the compounds according to the invention give rise to mixture of stereoisomers, these isomers may be separated by conventional techniques such as preparative chromatography. The compounds may be prepared in racemic form or individual enantiomers may be prepared by standard techniques known to those skilled in the art, for example, by enantiospecific synthesis or resolution, formation of diastereomeric pairs by salt formation with an optically active acid, followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column. It is to be understood that all such isomers and mixtures thereof in all proportion are encompassed within the scope of the present invention.

Where any particular moiety is substituted, for example a phenyl group comprising a substituent on the aryl ring, unless specified otherwise, the term "substituted" contemplates all possible isomeric forms. For example, substituted phenyl includes all of the following *ortho-, meta-* and *para-* permutations:

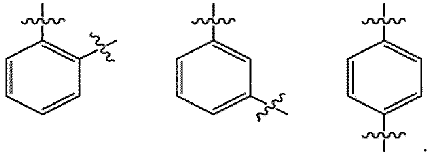

However, in general para substitution is preferred.

As used herein the term « tautomer » refers to isomers of the compounds according to the present invention that readily interconvert by a chemical reaction called tautomerization. Commonly this reaction results in the formal migration of a hydrogen atom or proton, accompanied by a switch of a single bond and adjacent double bond. In solutions where tautomerization is possible, a chemical equilibrium of the tautomers will be reached. The exact ratio of the tautomers depends on several factors, including temperature, solvent, and pH. The concept of tautomers that are interconvertible by tautomerizations is called tautomerism. Common tautomeric pairs are: ketone - enol; amide - imidic acid; lactam - lactim, an amide - imidic acid tautomerism in heterocyclic rings; enamine - imine; enamine - enamine. In particular it can include ring-chain tautomerism which occurs when the movement of the proton is accompanied by a change from an open structure to a ring.

The term "halogen" is used herein to refer to any of fluorine, chlorine, bromine and iodine. Most usually, however, halogen substituents in the compounds of the invention are chlorine, bromine and fluorine substituents, in particular fluorine substituents.

As used herein, the term "alkyl" refers to a straight or branched saturated monovalent hydrocarbon radical, having the number of carbon atoms as indicated. For example, the term "(C₁-C₆)alkyl" includes C₁, C₂, C₃, C₄, C₅ and C₆ alkyl groups. By way of non-limiting example, suitable alkyl groups include methyl, ethyl, propyl, isopropyl, butyl, iso-butyl, tert-butyl, pentyl and hexyl. In one aspect of the present invention ranges of alkyl groups are: (C₁-C₆)alkyl, (C₁-C₅)alkyl, (C₁-C₄)alkyl, (C₁-C₃)alkyl and (C₁-C₂)alkyl, in particular (C₁-C₃)alkyl.

As used herein, the term "alkanediyl" refers to a straight or branched saturated divalent hydrocarbon radical, having the number of carbon atoms as indicated. For example, the term "(C₁-C₆)alkanediyl" includes C₁, C₂, C₃, C₄, C₅ and C₆ alkyl groups. By way of non-limiting example, suitable alkanediyl groups include methanediyl, ethanediyl, propanediyl, iso-propanediyl, butanediyl, iso-butanediyl, tert-butanediyl, pentanediyl and hexanediyl. In one aspect of the present invention ranges of alkyl groups are: (C₁-C₆)alkanediyl, (C₁-C₅)alkanediyl, (C₁-C₄)alkanediyl, (C₁-C₃)alkanediyl and (C₁-C₂)alkanediyl, in particular (C₁-C₃)alkanediyl.

As used herein, the term "haloalkyl" refers to an alkyl group as defined above in which one or several hydrogen atoms have been replaced with a halogen atom as defined above. It can be in particular a trifluoromethyl group.

As used herein, the term "alkenyl" refers to a straight or branched unsaturated monovalent hydrocarbon radical comprising at least one double bond, having the number of carbon atoms as indicated. For example, the term "(C₂-C₆)alkenyl" includes C₂, C₃, C₄, C₅ and C₆ alkenyl groups. For example, a suitable alkenyl group is a vinyl group.

As used herein, the term "alkoxy" refers to an alkyl group as defined above bound to the molecule via an oxygen atom. The term "(C₁-C₆)alkoxy" includes thus C₁, C₂, C₃, C₄, C₅ and C₆ alkoxy groups. By way of non-limiting example, suitable alkoxy groups include methoxy, ethoxy, propoxy, iso-propoxy, butoxy, iso-butoxy, tert-butoxy, pentoxy and hexoxy. In one aspect of the present invention ranges of alkoxy groups are: (C₁-C₆)alkoxy, (C₁-C₅)alkoxy, (C₁-C₄)alkoxy, (C₁-C₃)alkoxy and (C₁-C₂)alkoxy, in particular (C₁-C₃)alkoxy.

As used herein, the term "haloalkoxy" refers to an alkoxy group as defined above in which one or several hydrogen atoms have been replaced with a halogen atom as defined above. It can be in particular a trifluoromethoxy group.

As used herein, the term "aryl" refers to monovalent aromatic carbocyclic radicals having one ring or two or three fused rings. In one aspect of the present invention, the term "aryl" refers to an aromatic monocyclic ring containing 6 carbon atoms (phenyl ring), which may be substituted on the ring with 1, 2, 3, 4 or 5 substituents as defined herein; an aromatic bicyclic ring system containing 10 carbon atoms (naphthyl or azulenyl ring), which may be substituted on the ring with 1, 2, 3, 4, 5, 6, 7 or 8 substituents as defined herein; or an aromatic tricyclic ring system containing 14 carbon atoms, which may be substituted on the ring with 1, 2, 3, 4, 5, 6, 7, 8, 9, or 10 substituents as defined herein. By way of non-limiting example, suitable non substituted aryl groups include phenyl, azulenyl, and naphthyl groups, advantageously a phenyl group.

As used herein, the term "heteroaryl" refers to monovalent aromatic heterocyclic radicals having one ring or two or three fused rings. At least one atom of the ring(s) is a N, O or S atom. Preferably, one, two or three atoms of the ring(s) are a N, O or S atom, the other atoms of the ring(s) being carbon atoms. By way of non-limiting example, suitable heteroaryl groups include furanyl, pyrrolyl, thienyl, imidazolyl, pyrazolyl, thiazolyl, isothiazolyl, oxazolyl, isoxazolyl, oxadiazolyl, pyridazinyl, tetrazolyl, triazolyl, pyridinyl, pyrazinyl, pyrimidinyl, indolyl.

As used herein, the term "heterocycle" or "heterocyclic ring" refers to a saturated or partially unsaturated but not aromatic ring having 5 to 8, preferably 5 or 6, members of which at least one member is N and which optionally contains one or two additional N, O or S atoms. Typically, heterocycles comprising peroxide groups are excluded from the definition of heterocyclic. By way of non-limiting example, suitable heterocyclic groups include 1,3-benzodioxolyl, pyrrolinyl, pyrrolidinyl, dioxolanyl, tetrahydrofuranyl, morpholinyl, imidazolinyl, imidazolidinyl, pyrazolidinyl, piperidinyl, dihydropyranyl, tetrahydropyranyl and piperazinyl.

As used herein, the term "cycloalkyl" refers to a monovalent saturated hydrocarbon monocycle or polycycle having two or three fused rings. In one aspect of the present invention, the term "cycloalkyl" refers to a monovalent saturated hydrocarbon monocycle containing 3 to 8, notably 5 or 6 carbon atoms; or a monovalent saturated hydrocarbon bicycle or tricycle, each cycle containing 3 to 8, notably 5 or 6 carbon atoms. By way of non-limiting example, suitable cycloalkyl groups include cyclopropyl, cyclobutyl, cyclopentyl, cyclohexyl, cycloheptyl, and cyclooctyl.

As used herein, the term "cycloalkenyl" refers to a monovalent unsaturated hydrocarbon monocycle or polycycle having two or three fused rings, comprising at least one double bond. In one aspect of the present invention, each cycle contains 5 or 6 carbon atoms. For example, a suitable cycloalkenyl group is a cyclohexenyl group.

### EXPERIMENTAL PART

The compounds according to the present invention have been prepared and tested as described below in a non-limiting way.

Most of compounds 1 to 36 are commercially available or disclosed in prior art.

The following abbreviations have been used in this part.

| | |
|---|---|
| Ac | Acetyl |
| ACN | Acetonitrile |
| BOP | Benzotriazol-1-yl-oxy-tris-(dimethylamino)phosphonium hexafluorophosphate |
| DCM | Dichloromethane |
| DIPEA | N,N-Diisopropylethylamine |
| DME | Dimethoxyethane |
| DMF | Dimethylformamide |
| EDCI | 1-Ethyl-3-(3-dimethylaminopropyl) carbodiimide |
| ESI | Electrospray ionization |
| Fmoc | Fluorenylmethyloxycarbonyl |
| HATU | N,N,N',N'-Tetramethyl-O-(7-azabenzotriazol-1-yl)uronium hexafluorophosphate |
| HOBt | N-Hydroxybenzotriazole |
| LAH | Lithium aluminium hydride |
| Me | Methyl |
| MS | Mass spectrum |
| NMR | Nuclear magnetic resonance |
| Py | Pyridine |
| RT | Room temperature |
| TBA | Tetrabutyl ammonium |
| TBTU | N,N,N',N'-Tetramethyl-O-(benzotriazo-1-yl)uronium tetrafluoroborate |
| TEA | Triethylamine |
| TFA | Trifluoroacetic acid |
| THF | Tetrahydrofuran |
| p-TsOH | *para*-Toluenesulfonic acid |

### I - Preparation of the compounds according to the present invention

### Example 37: Preparation of 2-{3-[2-(4-chlorophenyl)ethyl]-5-oxo-1-phenyl-2-sulfanylideneimidazolidin-4-yl}-N-(4-methylphenyl)acetamide.

### a) 1,4-diethyl2-{[2-(4-chlorophenyl)ethyl]amino}butanedioate (I-1)

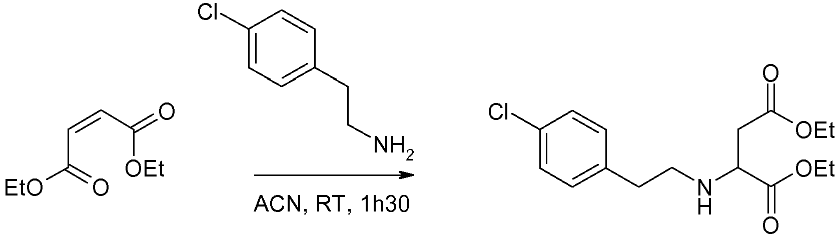

2-(4-chlorophenyl)ethan-1-amine (4.9 mL; 34.84 mmol; 4 eq) was added to a solution of 1,4-diethyl (2Z)-but-2-enedioate (1.41 mL; 8.71 mmol; 1 eq) in acetonitrile (30 mL). The reaction mixture was stirred at room temperature for 1 hour and 30 minutes, and then, concentrated to dryness. The crude was purified on silica gel chromatography using petroleum ether/ethyl acetate (90/10 to 70/30) as an eluent. The title compound, 1,4-diethyl 2-{[2-(4-chlorophenyl)ethyl]amino}butanedioate was obtained in 100% yield (2.85 g) as a colorless oil. ¹H-NMR (CDCl₃): δ (ppm) 1.24 (m, 6H), 1.73 (s, 1H), 2.73 (s, 5H), 2.93 (m, 1H), 3.63 (td, 1H, J = 6.7 Hz, 1.6 Hz), 4.19 (m, 4H), 7.12 (m, 2H), 7.26 (m, 2H); MS (ESI+): m/z = 327.9, 329.9 [M+H]⁺.

### b) Ethyl 2-{3-r2-(4-chlorophenyl)ethyl]-5-oxo-1-phenyl-2-sulfanylidene imidazolidin-4-yl}acetate (1-2)

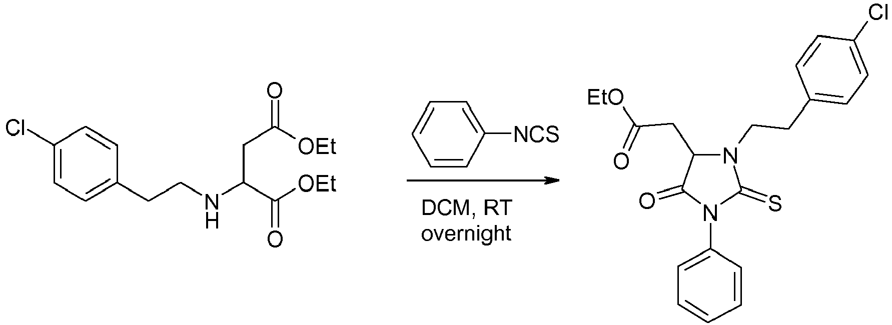

PhNCS (1.15 mL; 9.59 mmol; 1.1 eq) was added to a solution of 1,4-diethyl 2-{[2-(4-chlorophenyl) ethyl] amino}butanedioate (I-1) (2.85 g; 8.71 mmol; 1 eq) in dichloromethane (30 mL). The reaction mixture was stirred at room temperature overnight, and then, concentrated to dryness. The crude was purified by flash chromatography on silica gel using petroleum ether/ethyl acetate (90/10 to 50/50) as an eluent. The title compound, ethyl 2-{3-[2-(4-chlorophenyl)ethyl]-5-oxo-1-phenyl-2-sulfanylideneimidazolidin-4-yl}acetate was obtained in 86% yield (3.13 g) as a white solid. ¹H-NMR (DMSO-d₆): δ (ppm) 1.16 (m, 3H), 2.86 (m, 1H), 3.02 (m, 1H), 3.12 (m, 1H), 3.29 (m, 1H), 3.7 (m, 1H), 4.11 (m, 3H), 4.72 (m, 1H), 7.37 (m, 9H); MS (ESI+): m/z = 416.8 [M+H]⁺.

### c) 2-{3-[2-(4-chlorophenyl)ethyl]-5-oxo-1-phenyl-2-sulfanylideneimidazolidin-4-yl}acetic acid (1-3)

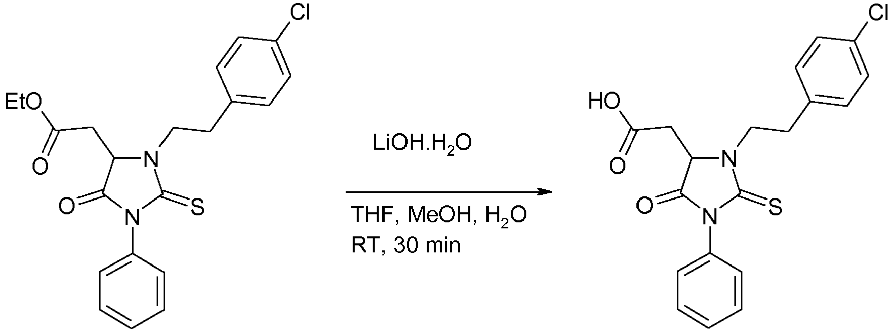

An aqueous solution of lithium hydroxide monohydrate (4.6 mL; 2.2 M aqueous solution; 10.7 mmol; 2 eq) was added to a solution of ethyl 2-{3-[2-(4-chlorophenyl)ethyl]-5-oxo-1-phenyl-2-sulfanylideneimidazolidin-4-yl}acetate (I-2) (2.1 g; 5.03 mmol; 1 eq) in tetrahydrofuran (14.4 mL) and methanol (9.1 mL). The reaction mixture was stirred at room temperature for 30 minutes, and then concentrated to dryness. Water (30 mL) was added followed by ethyl acetate (30 mL). The layers were separated. The aqueous layer was acidified with hydrochloric acid 1N until pH 1, and was extracted with ethyl acetate (2 x 30 mL). The combined organic layers was dried over sodium sulfate, filtered and concentrated under reduced pressure. The title **compound**, 2-{3-[2-(4-chlorophenyl)ethyl]-5-oxo-1-phenyl-2-sulfanylidene imidazolidin-4-yl}acetic acid was obtained in 82% yield (1.60 g) as a yellow solid. ¹H-NMR (CDCl₃): δ (ppm) 2.97 (m, 1H), 3.11 (m, 3H), 3.65 (m, 1H), 4.25 (t, 1H, J = 4.6 Hz), 4.37 (m, 1H), 7.25 (m, 6H), 7.48 (m, 3H); MS (ESI+): m/z = 388.9, 390.9 [M+H]⁺.

### d) 2-{3-[2-(4-chlorophenyl)ethyl]-5-oxo-1-phenyl-2-sulfanylideneimidazolidin-4-yl}-N-(4-methylphenyl)acetamide (example 37).

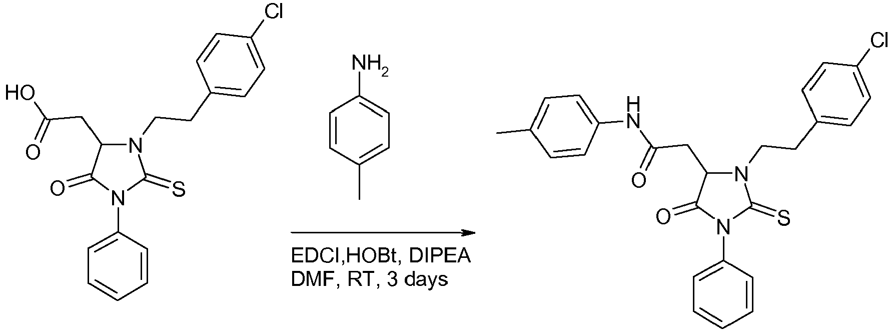

4-Methylaniline (29 mg; 0.27 mmol; 1.05 eq), followed by EDCI (54 mg; 0.28 mmol; 1.1 eq), HOBt (38 mg; 0.28 mmol; 1.1 eq) and DIPEA (98 µL; 0.565 mmol; 2.2 eq) were added to a solution of 2-{3-[2-(4-chlorophenyl)ethyl]-5-oxo-1-phenyl-2-sulfanylideneimidazolidin-4-yl}acetic acid (I-3) (100 mg; 0.26 mmol; 1 eq) in dimethylformamide (14 mL). The reaction mixture was stirred at room temperature for 3 days. Water (20 mL) was added and the aqueous layer was extracted with ethyl acetate (3 x 30 mL). The combined organic layers were washed with saturated ammonium chloride and saturated sodium chloride (3 x 30 mL), dried over sodium sulfate, filtered and concentrated under reduced pressure. The crude was purified on silica gel using dichloromethane/ethyl acetate (98/2) as an eluent. After trituration in diethyl ether/pentane, and recrystallization in dichloromethane/cyclohexane, the title compound 2-{3-[2-(4-chlorophenyl)ethyl]-5-oxo-1-phenyl-2-sulfanylideneimidazolidin-4-yl}-*N*-(4-methylphenyl)acetamide was obtained in 23% yield (30 mg) as a white solid. ¹H-NMR (DMSO-d₆): δ (ppm) 2.24 (s, 3H), 2.87 (m, 1H), 3.08 (m, 2H), 3.27 (m, 1H), 3.71 (m, 1H), 4.17 (m, 1H), 4.77 (t, 1H, J = 4.0 Hz), 7.11 (dd, 2H), 7.42 (m, 11H), 10.12 (s, 1H); MS (ESI+): m/z = 477.9, 479.8 [M+H]⁺.

### Example 56: Preparation of 2-{3-[2-(4-chlorophenyl)ethyl]-5-oxo-1-phenyl-2-sulfanylideneimidazolidin-4-yl}-N-(pyridin-3-ylmethyl)acetamide.

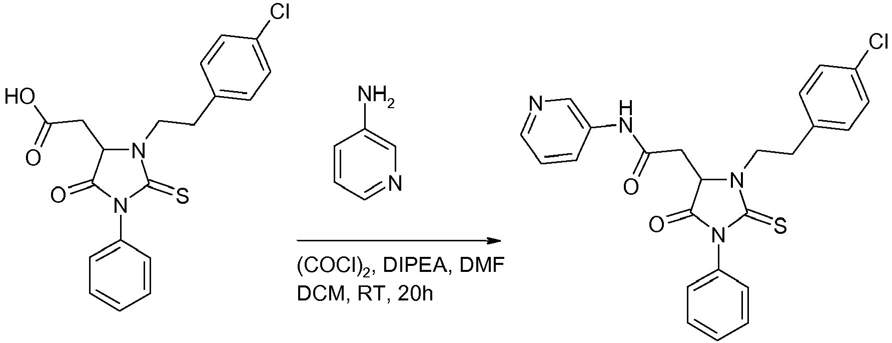

Oxalyl chloride (27 µL; 0.31 mmol; 1.2 eq) and dimethylformamide (0.2 mL) were added to a solution of 2-{3-[2-(4-chlorophenyl)ethyl]-5-oxo-1-phenyl-2-sulfanylideneimidazolidin-4-yl}acetic acid (I-3) (100 mg; 0.26 mmol; 1 eq) in dichloromethane (5.5 mL). The reaction mixture was stirred at room temperature for 3 hours. Then, 3-aminopyridine amine (48.94 mg; 0.52 mmol; 2 eq) and DIPEA (113 µL; 0.65 mmol; 2.5 eq) were added. The reaction mixture was stirred at room temperature for 17 hours. Saturated ammonium chloride (10 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on silica gel using dichloromethane/methanol (98/2 to 95/5) as an eluent, to afford the title compound 2-{3-[2-(4-chlorophenyl)ethyl]-5-oxo-1-phenyl-2-sulfanylideneimidazolidin-4-yl}-*N-*(pyridin-3-ylmethyl)acetamide in 34% yield (32 mg) as a green solid. ¹H-NMR (CDCl₃): δ (ppm) 2.89 (m, 1H), 3.12 (m, 3H), 3.72 (dt, 1H, J=17.6Hz, 4.2Hz), 4.39 (m, 1H), 4.52 (m, 1H), 7.25 (m, 7H), 7.48 (m, 3H), 7.6 (s, 1H), 8.11 (m, 1H), 8.41 (m, 1H), 8.57 (m, 1H); MS (ESI+): m/z = 464.8, 466.8 [M+H]⁺.

### Example 38: Preparation of 2-{3-[2-(4-chlorophenyl)ethyl]-5-oxo-1-phenyl-2-sulfanylideneimidazolidin-4-yl}-N-(3,4-dichlorophenyl)acetamide.

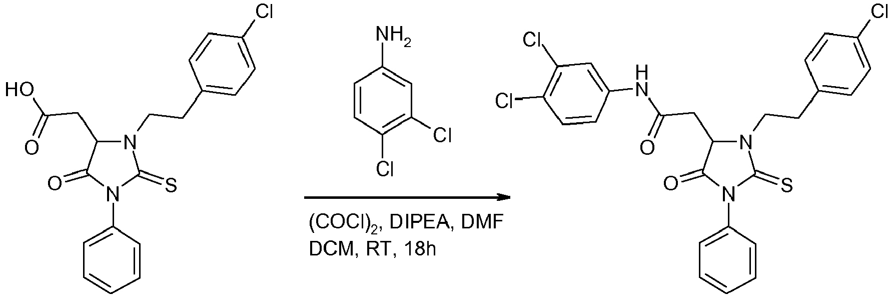

Oxalyl chloride (27 µL; 0.31 mmol; 1.2 eq) and dimethylformamide (0.2 mL) were added to a solution of 2-{3-[2-(4-chlorophenyl)ethyl]-5-oxo-1-phenyl-2-sulfanylideneimidazolidin-4-yl}acetic acid (I-3) (100 mg; 0.26 mmol; 1 eq) in dichloromethane (5.5 mL). The reaction mixture was stirred at room temperature for 30 minutes. Then, 3,4-dichloroaniline (84 mg; 0.52 mmol; 2 eq) and DIPEA (113 µL; 0.65 mmol; 2.5 eq) were added. The reaction mixture was stirred at room temperature for 17 hours and 30 minutes. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on silica gel using dichloromethane/ethyl acetate (98/2 to 95/5) as an eluent. After trituration in diethyl ether, the title compound 2-{3-[2-(4-chlorophenyl)ethyl]-5-oxo-1-phenyl-2-sulfanylideneimidazolidin-4-yl}-*N*-(3,4-dichlorophenyl)acetamide was obtained in 26% yield (27 mg) as a white solid. ¹H-NMR (Acetone-d₆): δ (ppm) 3.04 (m, 1H), 3.22 (m, 2H), 3.41 (dd, 1H, J = 16.6Hz, 3.4Hz), 3.84 (m, 1H), 4.36 (m, 1H), 4.73 (t, 1H, J = 4.4 Hz), 7.42 (m, 11H), 7.99 (d, 1H, J = 2.3Hz), 9.7 (s, 1H); MS (ESI+): m/z = 531.7, 533.7 [M+H]⁺.

### Example 57: Preparation of 2-{3-[2-(4-chlorophenyl)ethyl]-5-oxo-1-phenyl-2-sulfanylideneimidazolidin-4-yl}-N-(pyridin-2-yl)acetamide.

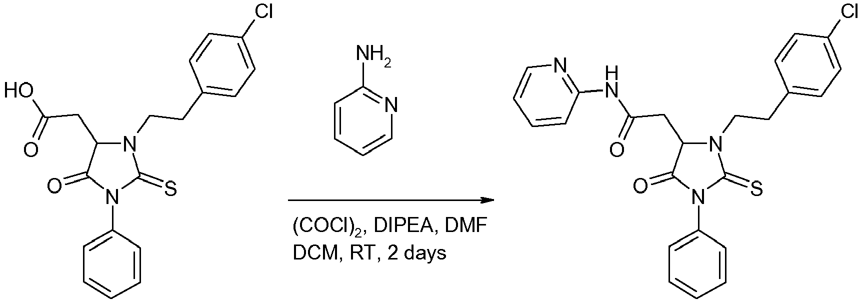

Oxalyl chloride (52.5 µL; 0.6 mmol; 1.2 eq) and dimethylformamide (0.2 mL) were added to a solution of 2-{3-[2-(4-chlorophenyl)ethyl]-5-oxo-1-phenyl-2-sulfanylideneimidazolidin-4-yl} acetic acid (1-3) (200 mg; 0.5 mmol; 1 eq) in dichloromethane (7 mL). The reaction mixture was stirred at room temperature for 3 hours, then, 2-amino-pyridine (96.82 mg; 1.03 mmol; 2 eq) and DIPEA (224 µL; 1.29 mmol; 2.5 eq) were added. The reaction mixture was stirred at room temperature for 18 hours. Saturated ammonium chloride (10 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on silica gel using dichloromethane/ethyl acetate (80/20) as an eluent. After trituration in dichloromethane/pentane, the title compound 2-{3-[2-(4-chlorophenyl)ethyl]-5-oxo-1-phenyl-2-sulfanylideneimidazolidin-4-yl}-*N*-(pyridin-2-yl)acetamide was obtained in 20% yield (48 mg) as a green solid. ¹H-NMR (CDCl₃): δ (ppm) 2.98 (m, 2H), 3.06 (m, 2H), 3.67 (m, 1H), 4.38 (m, 1H), 4.52 (m, 1H), 7.1 (dd, 1H, J = 7.4 Hz, 5.0 Hz), 7.22 (m, 4H), 7.37 (qd, 2H, J = 7.1 Hz, 2.0 Hz), 7.49 (m, 3H), 7.75 (td, 1H, J = 8.0 Hz, 1.7 Hz), 8.06 (m, 1H), 8.17 (m, 1H), 8.29 (d, 1H, J = 4.9 Hz); MS (ESI+): m/z = 464.8, 466.8 [M+H]⁺ .

### Example 58: Preparation of 2-{3-[2-(4-chlorophenyl)ethyl]-5-oxo-1-phenyl-2-sulfanylideneimidazolidin-4-yl}-N-[4-(trifluoromethoxy)phenyl]acetamide.

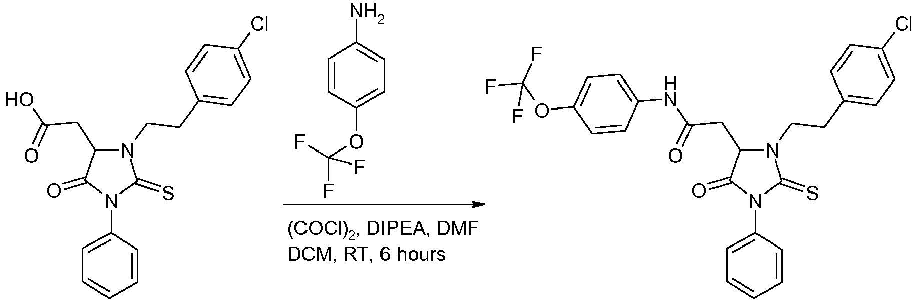

Oxalyl chloride (67.5 µL; 0.77 mmol; 1.5 eq) and dimethylformamide (0.2 mL) were added to a solution of 2-{3-[2-(4-chlorophenyl)ethyl]-5-oxo-1-phenyl-2-sulfanylideneimidazolidin-4-yl}acetic acid (1-3) (200 mg; 0.5 mmol; 1 eq) in dichloromethane (7 mL). The reaction mixture was stirred at room temperature for 3 hours. Then, 4-(trifluoromethoxy)aniline (139 µL; 1.03 mmol; 2 eq) and DIPEA (269 µL; 1.54 mmol; 3 eq) were added. The reaction mixture was stirred at room temperature for 3 hours. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. After trituration in dichloromethane/pentane, the title compound 2-{3-[2-(4-chlorophenyl)ethyl]-5-oxo-1-phenyl-2-sulfanylidene imidazolidin-4-yl}- -[4-(trifluoromethoxy)phenyl]acetamide was obtained in 58% yield (198 mg) as an off-white solid. ¹H-NMR (DMSO-d₆): δ (ppm) 2.88 (m, 1H), 3.05 (m, 1H), 3.16 (dd, 1H, J = 16.8 Hz, 4.1 Hz), 3.29 (dt, 1H, J = 7.7 Hz, 2.6 Hz), 3.71 (m, 1H), 4.18 (m, 1H), 4.79 (t, 1H, J = 4.0 Hz), 7.34 (m, 8H), 7.48 (m, 3H), 7.65 (m, 2H), 10.43 (s, 1H); MS (ESI+): m/z = 545.8, 547.7 [M+H]⁺.

### Example 59: Preparation of 2-{3-[2-(4-chlorophenyl)ethyl)-5-oxo-1-phenyl-2-sulfanylideneimidazolidin-4-yl}-N-(6-methoxypyridin-3-yl)acetamide.

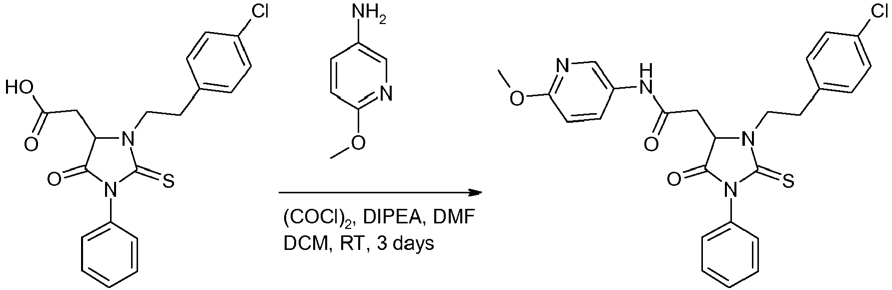

Oxalyl chloride (122.5 µL; 1.4 mmol; 2.8 eq) and dimethylformamide (0.2 mL) were added to a solution of 2-{3-[2-(4-chlorophenyl)ethyl]-5-oxo-1-phenyl-2-sulfanylideneimidazolidin-4-yl}acetic acid (1-3) (200 mg; 0.5 mmol; 1 eq) in dichloromethane (7 mL). The reaction mixture was stirred at room temperature for 5 hours and 30 minutes. Then, 6-methoxypyridin-3-amine (128 mg; 1.03 mmol; 2 eq) and DIPEA (540 µL; 3.1 mmol; 6 eq) were added. The reaction mixture was stirred at room temperature over the week-end. Saturated ammonium chloride (15 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on silica gel using dichloromethane/ethyl acetate (100/0 to 95/5) as an eluent. After trituration in dichloromethane/pentane, the title compound 2-{3-[2-(4-chlorophenyl)ethyl]-5-oxo-1-phenyl-2-sulfanylideneimidazolidin-4-yl}-N-(6-methoxypyridin-3-yl)acetamide was obtained in 12% yield (32 mg) as a off-white solid. ¹H-NMR (CDCl₃): δ (ppm) 2.85 (dd, 1H, J = 16.0 Hz, 6.0 Hz), 3.09 (m, 3H), 3.71 (m, 1H), 3.93 (s, 3H), 4.42 (m, 1H), 4.54 (dd, 1H, J = 5.5 Hz, 4.7 Hz), 6.76 (d, 1H, J = 9.0 Hz), 7.22 (m, 4H), 7.33 (d, 2H, J = 7.5 Hz), 7.48 (m, 4H), 7.83 (dd, 1H, J = 9.1 Hz, 2.9 Hz), 8.17 (d, 1H, J = 2.7 Hz); MS (ESI+): m/z = 494.8, 496.8 [M+H]⁺.

### Example 39: Preparation of 2-{3-[2-(4-chlorophenyl)ethyl]-5-oxo-1-phenyl-2-sulfanylideneimidazolidin-4-yl}-N-[4-(trifluoromethyl)phenyl]acetamide.

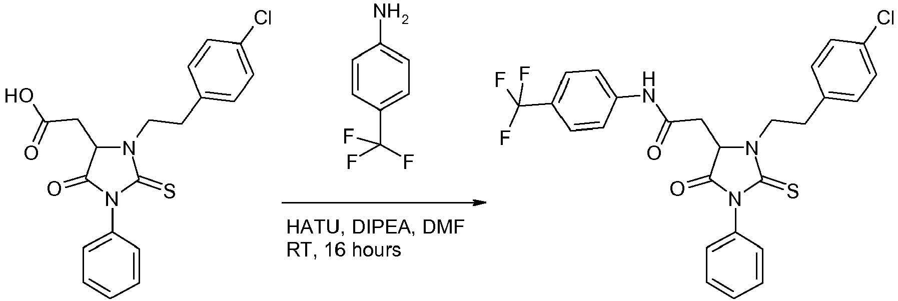

4-(trifluoromethyl)aniline (181 µL; 1.44 mmol; 2.8 eq) followed by HATU (272 mg; 0.71 mmol; 1.4 eq), and DIPEA (266 µL; 1.53 mmol; 3 eq) were added to a solution of 2-{3-[2-(4-chlorophenyl)ethyl]-5-oxo-1-phenyl-2-sulfanylideneimidazolidin-4-yl}acetic acid (1-3) (200 mg; 0.5 mmol; 1 eq) in dimethylformamide (6 mL). The reaction mixture was stirred at room temperature for 16 hours. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. After trituration in dichloromethane/pentane/diethyl ether and lyophilisation, the title compound 2-{3-[2-(4-chlorophenyl)ethyl]-5-oxo-1-phenyl-2-sulfanylideneimidazolidin-4-yl}-*N*-[4- trifluoromethyl)phenyl]acetamide was obtained in 11% yield (28.6 mg) as a white solid. ¹H-NMR (DMSO-d₆): δ (ppm) 2.87 (m, 1H), 3.02 (m, 1H), 3.22 (m, 1H), 3.69 (m, 1H), 3.85 (m, 1H), 4.17 (m, 1H), 4.8 (t, 1H, J = 4.3 Hz), 7.35 (m, 6H), 7.5 (m, 3H), 7.72 (dt, 4H, J = 17.6 Hz, 1.6 Hz), 10.57 (s, 1H); MS (ESI+): m/z = 529.8, 531.7 [M+H]⁺.

### Example 60: Preparation of 2-{3-[2-(4-chlorophenyl)ethyl]-5-oxo-1-phenyl-2-sulfanylideneimidazolidin-4-yl}-N-(6-methylpyridazin-3-yl)acetamide.

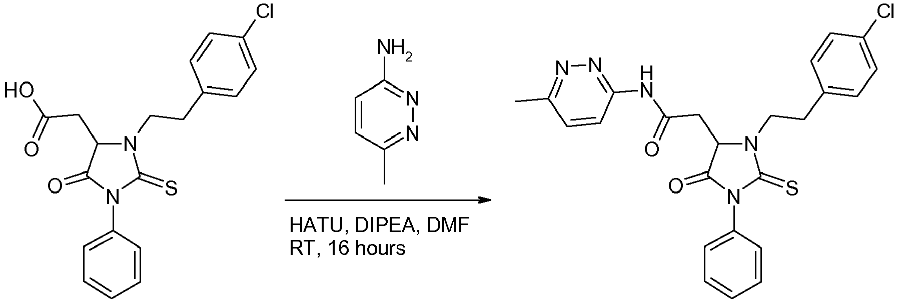

6-methylpyridazin-3-amine (78.1 mg; 0.72 mmol; 2.8 eq) followed by HATU (136 mg; 0.36 mmol; 1.4 eq), and DIPEA (134 µL; 0.77 mmol; 3 eq) were added to a solution of 2-{3-[2-(4-chlorophenyl)ethyl]-5-oxo-1-phenyl-2-sulfanylideneimidazolidin-4-yl}acetic acid (1-3) (100 mg; 0.26 mmol; 1 eq) in dimethylformamide (3 mL). The reaction mixture was stirred at room temperature for 16 hours. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on preparative TLC on silica gel using dichloromethane/methanol/ammonium hydroxide (95/4/1). The title compound, 2-{3-[2-(4-chlorophenyl)ethyl]-5-oxo-1-phenyl-2-sulfanylideneimidazolidin-4-yl}-*N*-(6-methylpyridazin-3-yl)acetamide was obtained in 35% yield (31 mg) as an off-white solid. ¹H-NMR (DMSO-d₆): δ (ppm) 2.55 (s, 3H), 2.9 (m, 1H), 3.06 (m, 1H), 3.23 (m, 1H), 3.48 (m, 1H), 3.75 (m, 1H), 4.18 (m, 1H), 4.81 (t, 1H, J = 4.2 Hz), 7.33 (d, 4H, J = 8.3 Hz), 7.37 (d, 2H, J = 8.2 Hz), 7.45 (td, 1H, J = 6.8 Hz, 1.4 Hz), 7.51 (t, 2H, J = 7.6 Hz), 7.57 (d, 1H, J = 9.1 Hz), 8.14 (d, 1H, J = 9.2 Hz), 11.29 (d, 1H, J = 1.5 Hz); MS (ESI+): m/z = 479.8, 481.8 [M+H]⁺.

### Example 61: Preparation of 2-{3-[2-(4-chlorophenyl)ethyl]-5-oxo-1-phenyl-2-sulfanylideneimidazolidin-4-yl}-N-(5-methoxypyridin-2-yl)acetamide.

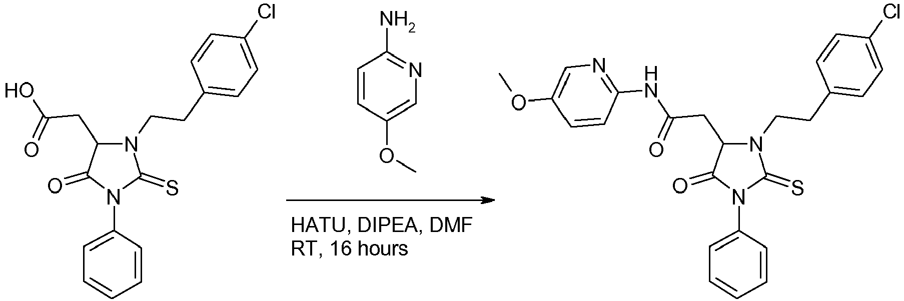

5-Methoxypyridin-2-amine (89 mg; 0.72 mmol; 2.8 eq), followed by HATU (136 mg; 0.36mmol; 1.4 eq), and DIPEA (134 µL; 0.77 mmol; 3 eq) were added to a solution of 2- {3-[2-(4-chlorophenyl)ethyl]-5-oxo-1-phenyl-2-sulfanylideneimidazolidin-4-yl}acetic acid (1-3) (100 mg; 0.26 mmol; 1 eq) in dimethylformamide (3 mL). The reaction mixture was stirred at room temperature for 16 hours. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified by flash chromatography using dichloromethane/ethyl acetate (98/2 to 90/10). After lyophilisation, the title compound 2- {3-[2-(4-chlorophenyl)ethyl]-5-oxo-1-phenyl-2-sulfanylideneimidazolidin-4-yl}-*N*-(5-methoxypyridin-2-yl)acetamide was obtained in 13% yield (16.8 mg) as a yellow powder. ¹H-NMR (Acetone-d₆): δ (ppm) 3.03 (m, 1H), 3.2 (m, 1H), 3.31 (dd, 1H, J = 17.0 Hz, 4.6 Hz), 3.55 (dd, 1H, J = 17.0 Hz, 4.5 Hz), 3.85 (m, 4H), 4.35 (m, 1H), 4.73 (t, 1H, J = 4.4 Hz), 7.41 (m, 10H), 7.96 (d, 1H, J = 3.0 Hz), 8.12 (m, 1H), 9.59 (m, 1H); MS (ESI+): m/z = 495.2, 497.2 [M+H]⁺.

### Example 62: Preparation of 2-{3-[2-(4-chlorophenyl)ethyl]-5-oxo-1-phenyl-2-sulfanylideneimidazolidin-4-yl]-N-(6-methoxypyridazin-3-yl)acetamide.

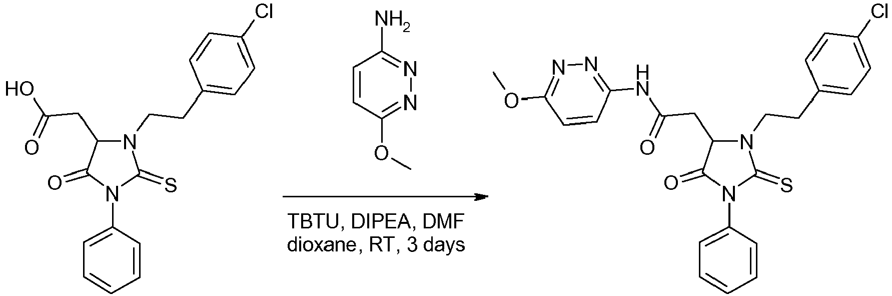

TBTU (253.3 mg; 0.77 mmol; 1.5 eq) and DIPEA (179 µL; 1.03 mmol; 2 eq) were added to a solution of 2-{3-[2-(4-chlorophenyl)ethyl]-5-oxo-1-phenyl-2-sulfanylideneimidazolidin-4-yl}acetic acid (1-3) (200 mg; 0.51 mmol; 1 eq) in dioxane (5 mL). After 20 minutes, 6-methoxypyridazin-3-amine (128.9 mg; 1.03 mmol; 2 eq) in dimethylformamide (0.1 mL) was added. The reaction mixture was stirred at room temperature over the week-end. After concentration to dryness, saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was precipitated in methanol. The title compound, the title compound 2-{3-[2-(4-chlorophenyl)ethyl]-5-oxo-1-phenyl-2-sulfanylideneimidazolidin-4-yl}-*N*-(6-methoxypyridazin-3-yl)acetamide was obtained in 22 % yield (55.8 mg) as a grey-white powder. ¹H-NMR (CDCl₃): δ (ppm) 2.9 (m, 1H), 3.03 (m, 1H), 3.22 (m, 1H), 3.45 (m, 1H), 3.74 (m, 1H), 3.98 (s, 3H), 4.17 (m, 1H), 4.8 (t, 1H, J = 4.2 Hz), 7.38 (m, 10H), 8.17 (m, 1H), 11.19 (s, 1H); MS (ESI+): m/z = 495.9, 497.8 [M+H]⁺.

### Example 63: Preparation of 2-{3-[2-(4-chlorophenyl)ethyl]-4-hydroxy-5-oxo-1-phenyl-2-sulfanylideneimidazolidin-4-yl}-N-[4-(dimethylamino)phenyl]acetamide.

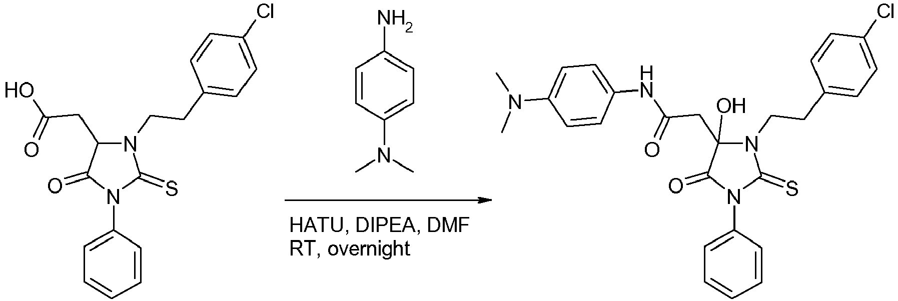

1-N,1-N-dimethylbenzene-1,4-diamine (196 mg; 1.44 mmol; 2.8 eq), followed by HATU (272 mg; 0.71 mmol; 1.4 eq) and DIPEA (266 µL; 1.53 mmol; 3 eq) were added to a solution of 2-{3-[2-(4-chlorophenyl)ethyl]-5-oxo-1-phenyl-2-sulfanylidene imidazolidin-4-yl}acetic acid (1-3) (200 mg; 0.51 mmol; 1 eq) in dimethylformamide (6 mL). The reaction mixture was stirred at room temperature overnight. Saturated ammonium chloride (10 mL) was added and the aqueous layer was extracted with ethyl acetate (3 x 10 mL). The combined organic layers were washed with saturated sodium chloride (3 x 10 mL), dried over sodium sulfate, filtered and concentrated under reduced pressure. The crude was purified on silica gel using petroleum ether/ethyl acetate (50/50 to 20/80) as an eluent. After trituration in dichloromethane/pentane and lyophilisation, 2- {3-[2-(4-chlorophenyl)ethyl]-4-hydroxy-5-oxo-1-phenyl-2-sulfanylideneimidazolidin-4-yl}-N-[4-(dimethylamino)phenyl]acetamide was obtained in 41 % yield (81.14 mg) as a grey powder. ¹H-NMR (DMSO-d₆): δ (ppm) 2.82 (s, 6H), 3.02 (dd, 2H, J = 10.3 Hz, 7.3 Hz), 3.22 (m, 2H), 3.83 (m, 2H), 6.67 (d, 2H, J = 8.8 Hz), 7.4 (m, 11H), 7.64 (s, 1H), 9.88 (s, 1H); MS (ESI+): m/z = 505.15 [(M-H₂O)+H]⁺.

### Example 64: Preparation of 2-{3-[2-(4-chlorophenyl)ethyl]-5-oxo-1-phenyl-2-sulfanylideneimidazolidin-4-yl}-N-(1,2-oxazol-3-yl)acetamide.

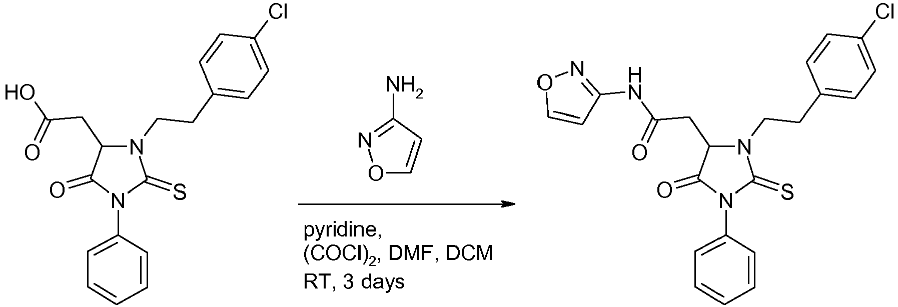

Oxalyl chloride (44.8 µL; 0.51 mmol; 2 eq) and dimethylformamide (0.3 mL) were **added to a solution of** 2-{3-[2-(4-chlorophenyl)ethyl]-5-oxo-1-phenyl-2-sulfanylideneimidazolidin-4-yl}acetic acid (1-3) (100 mg; 0.26 mmol; 1 eq) in dichloromethane (6 mL). The reaction mixture was stirred at room temperature for 3 hours. Then, pyridine (62 µL; 0.77 mmol; 3 eq) and 1,2-oxazol-3-amine (38 µL; 0.51 mmol; 2 eq) were added. The mixture was stirred at room temperature over the week-end. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified on silica gel using dichloromethane/ethyl acetate (95/5 to 50/50) as an eluent. After trituration in diethyl ether and lyophilisation, the title compound 2- {3-[2-(4-chlorophenyl)ethyl]-5-oxo-1-phenyl-2-sulfanylideneimidazolidin-4-yl}-*N*-(1,2-oxazol-3-yl)acetamide was obtained in 25% yield (29.25 mg) as a white powder. ¹H-NMR (DMSO-d₆): δ (ppm) 2.89 (m, 1H), 3.03 (m, 1H), 3.18 (m, 1H), 3.39 (m, 1H), 3.71 (m, 1H), 4.17 (m, 1H), 4.8 (t, 1H), 6.9 (d, 1H), 7.32 (m, 4H), 7.38 (m, 2H), 7.48 (m, 3H), 8.81 (d, 1H), 11.34 (s, 1H); MS (ESI+): m/z = 454.8, 456.8 [M+H]⁺.

### Example 40: Preparation of 4-(2-{3-[2-(4-chlorophenyl)ethyl)-5-oxo-1-phenyl-2-sulfanylideneimidazolidin-4-yl}acetamido)benzamide.

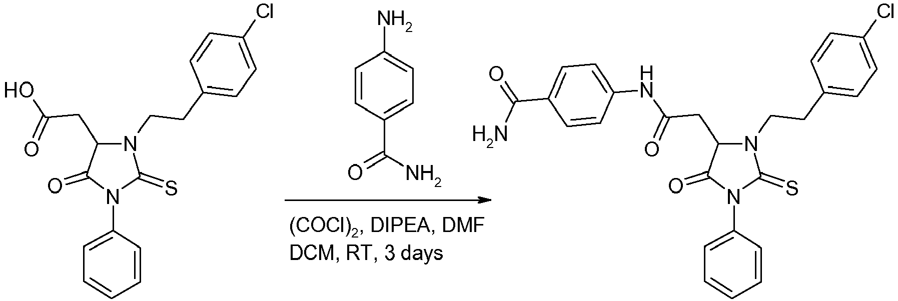

Oxalyl chloride (224 µL; 2.56 mmol; 2 eq) and dimethylformamide (1.5 mL) were added to a solution of 2-{3-[2-(4-chlorophenyl)ethyl]-5-oxo-1-phenyl-2-sulfanylideneimidazolidin-4-yl}acetic acid (I-3) (500 mg; 1.28 mmol; 1 eq) in dichloromethane (20 mL). The reaction mixture was stirred at room temperature for 2 hours. Then, 4-aminobenzamide (348 mg; 2.56 mmol; 2 eq) and DIPEA (560 µL; 3.20 mmol; 2.5 eq) were added. The reaction mixture was stirred at room temperature over the week-end. Saturated ammonium chloride (70 mL) was added and the aqueous layer was extracted with ethyl acetate (3 x 70 mL). The combined organic layers were washed with saturated sodium chloride (3 x 70 mL), dried over sodium sulfate, filtered and concentrated under reduced pressure. The crude was purified on silica gel using ethyl acetate/dichloromethane (70/30 to 100/0) as an eluent. After trituration in dichloromethane/pentane/diethyl ether and lyophilisation, the title compound 4-(2-{3-[2-(4-chlorophenyl)ethyl]-5-oxo-1-phenyl-2-sulfanylideneimidazolidin-4-yl}acetamido) benzamide was obtained in 13% yield (16 mg) as an off-white powder. ¹H-NMR (DMSO-d₆): δ (ppm) 2.86 (m, 1H), 3.04 (m, 1H), 3.18 (m, 1H), 3.31 (m, 1H), 3.71 (m, 1H), 4.16 (m, 1H), 4.79 (m, 1H), 7.35 (m, 12H), 7.84 (m, 3H), 10.42 (s, 1H); MS (ESI+): m/z = 504.7, 506.8 [M+H]⁺.

### Example 65: Preparation of 2-{3-[2-(4-chlorophenyl)ethyl]-1-(4-fluorophenyl)-4-hydroxy-5-oxo-2-sulfanylideneimidazolidin-4-yl}-N-[4-(dimethylamino) phenyl]acetamide.

### a) Ethyl 2-{3-[2-(4-chlorophenyl)ethyl]-1-(4-fluorophenyl)-5-oxo-2-sulfanylideneimidazolidin-4-yl}acetate (I-4)

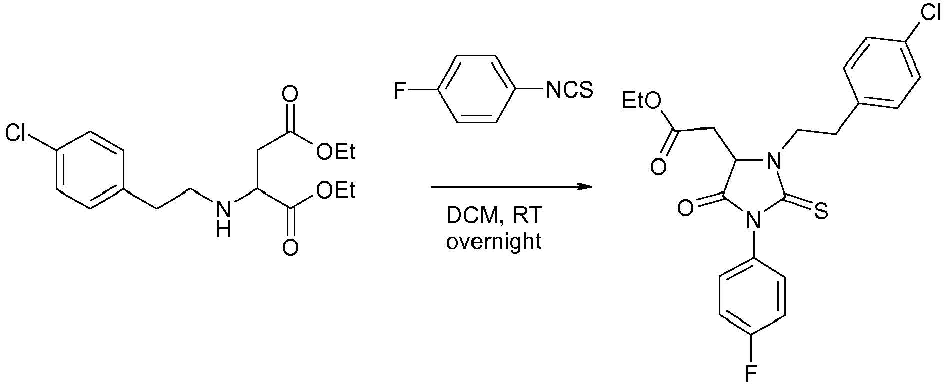

4-Fluorophenyl isothiocyanate (1.3 g; 8.8 mmol; 1.15 eq) was added to a solution of 1,4-diethyl 2-{[2-(4-chlorophenyl)ethyl]amino}butanedioate (I-1) (2.5 g; 7.6 mmol; 1 eq) in dichloromethane (25 mL). The reaction mixture was stirred at room temperature overnight and then concentrated to dryness. After trituration in diethyl ether/cyclohexane, ethyl 2- {3-[2-(4-chlorophenyl)ethyl]-1-(4-fluorophenyl)-5-oxo-2-sulfanylideneimidazolidin-4-yl}acetate was obtained in 88% yield (2.93 g) as a white solid. ¹H-NMR (DMSO-d₆): δ (ppm) 1.16 (t, 3H, J = 7.1 Hz), 2.85 (m, 1H), 3.02 (m, 1H), 3.13 (m, 1H), 3.31 (m, 1H), 3.74 (m, 1H), 4.1 (m, 3H), 4.72 (t, 1H, J = 4.0 Hz), 7.36 (m, 8H).

### b) 2-{3-[2-(4-chlorophenyl)ethyl]-1-(4-fluorophenyl)-5-oxo-2-sulfanylideneimidazolidin-4-yl}acetic acid (1-5)

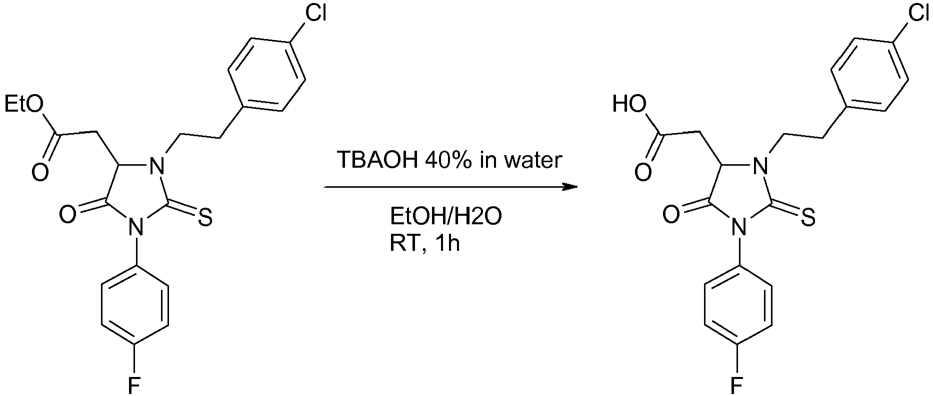

Tetrabutyl ammonium hydroxide 40% in water (4.52 mL; 6.90 mmol; 3 eq) was added to a suspension of ethyl 2-{3-[2-(4-chlorophenyl)ethyl]-1-(4-fluorophenyl)-5-oxo-2-sulfanylideneimidazolidin-4-yl} acetate (1-4) (1 g; 2.30 mmol; 1 eq) in ethanol/water (30 mL/10 mL). The reaction mixture was stirred at room temperature for 1 hour. The reaction mixture was acidified with hydrochloric acid 1N, and then, concentrated to remove ethanol. The aqueous layer was extracted with dichloromethane (3 x 50 mL). The combined organic layers were washed with saturated sodium chloride (3 x 50 mL), dried over sodium sulfate, filtered and concentrated under reduced pressure. The crude was purified on silica gel using dichloromethane/methanol (100% to 95/5) as an eluent. **The title** compound, 2-{3-[2-(4-chlorophenyl)ethyl]-1-(4-fluorophenyl)-5-oxo-2-sulfanylideneimidazolidin-4-yl}acetic acid was obtained in 60% yield (559 mg) as a yellow solid. ¹H-NMR (DMSO-d₆): δ (ppm) 2.86 (m, 1H), 3.04 (m, 1H), 3.21 (m, 2H), 3.72 (m, 1H), 4.15 (m, 1H), 4.67 (t, 1H, J = 4.2 Hz), 7.35 (m, 8H), 12.89 (m, 1H).

### c) 2-{3-[2-(4-chlorophenyl)ethyl]-1-(4-fluorophenyl)-4-hydroxy-5-oxo-2-sulfanylideneimidazolidin-4-yl}-N-[4-(dimethylamino)phenyl]acetamide (example 65)

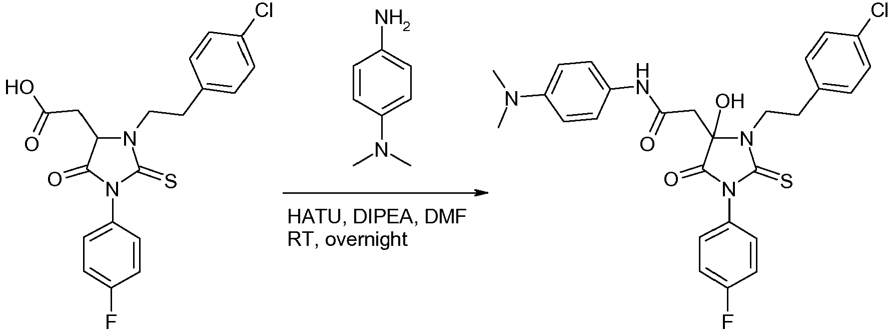

HATU (157 mg; 0.41 mmol; 1.4 eq) and DIPEA (154 µL; 0.89 mmol; 3 eq) were added to a solution of 2-{3-[2-(4-chlorophenyl)ethyl]-1-(4-fluorophenyl)-5-oxo-2-sulfanylideneimidazolidin-4-yl}acetic acid (I-5) (120 mg; 0.30 mmol; 1 eq) in dimethylformamide (3 mL). The reaction mixture was stirred at room temperature for 1 hour. Then, 1-*N*,1-*N*-dimethylbenzene-1,4-diamine (112.5 mg; 0.83 mmol; 2.8 eq) in dimethylformamide (0.2 mL) was added and the reaction mixture was stirred at room temperature overnight. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified by flash chromatography on silica gel using cyclohexane/ethyl acetate (70/30 to 40/60) as an eluent. After trituration in dichloromethane/pentane/diethyl ether and lyophilisation, the title compound 2-{3-[2-(4-chlorophenyl)ethyl]-1-(4-fluorophenyl)-4-hydroxy-5-oxo-2-sulfanylideneimidazolidin-4-yl}-*N*-[4-(dimethylamino)phenyl]acetamide was obtained in 32 % yield (28.06 mg) as a grey solid. ¹H-NMR (DMSO-d₆): δ (ppm) 2.82 (s, 6H), 3.01 (m, 2H), 3.24 (m, 2H), 3.82 (m, 2H), 6.66 (d, 2H, J = 8.8 Hz), 7.25 (d, 2H, J = 8.8 Hz), 7.37 (m, 7H), 7.65 (s, 1H), 9.89 (s, 1H); MS (ESI+): m/z = 522.8, 524.8 [(M-H₂O)+H]⁺.

### Example 66: Preparation of 2-{3-[2-(4-chlorophenyl)ethyl]-1-(4-fluorophenyl)-5-oxo-2-sulfanylideneimidazolidin-4-yl}-N-(5-methoxypyridin-2-yl)acetamide.

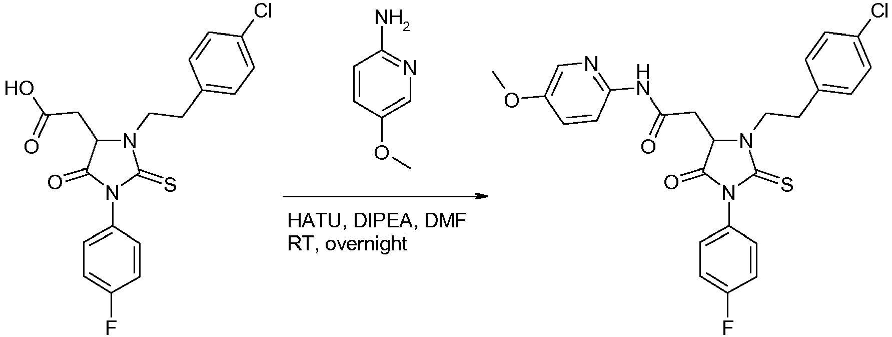

HATU (262 mg; 0.69 mmol; 1.4 eq) and DIPEA (257 µL; 1.47 mmol; 3 eq) were added to a solution of 2- {3-[2-(4-chlorophenyl)ethyl]-1-(4-fluorophenyl)-5-oxo-2-sulfanylidene imidazolidin-4-yl}acetic acid (1-5) (200 mg; 0.49 mmol; 1 eq) in dimethylformamide (6 mL). The reaction mixture was stirred at room temperature for 1 hour. Then, 5-methoxypyridin-2-amine (171 mg; 1.38 mmol; 2.8 eq) was added and the reaction mixture was stirred at room temperature overnight. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified by flash chromatography on silica gel using dichloromethane/ethyl acetate (95/5 to 90/10) as an eluent. After trituration in methanol/diethyl ether, the title compound 2-{3-[2-(4-chlorophenyl)ethyl]-1-(4-fluorophenyl)-5-oxo-2-sulfanylidene imidazolidin-4-yl}-*N*-(5-methoxypyridin-2-yl)acetamide was obtained in 7% yield (19.70 mg) as a white solid. ¹H-NMR (DMSO-d₆): δ (ppm) 2.89 (m, 1H), 3.04 (m, 1H), 3.10 (m, 1H), 3.19 (m, 1H), 3.38 (m, 1H), 3.73 (m, 1H), 3.8 (s, 3H), 4.16 (m, 1H), 4.77 (dd, 1H, J = 4.4 Hz, 3.7 Hz), 7.27 (m, 8H), 7.94 (d, 1H, J = 9.0 Hz), 8.03 (d, 1H, J = 3.1 Hz), 10.64 (d, 1H, J = 3.1Hz); MS (ESI+): m/z = 512.7, 514.8 [M+H]⁺.

### Example 67: Preparation of 2-{3-[2-(4-chlorophenyl)ethyl]-1-(4-fluorophenyl)-5-oxo-2-sulfanylideneimidazolidin-4-yl}-N-(5-methoxypyridin-2-yl)acetamide.

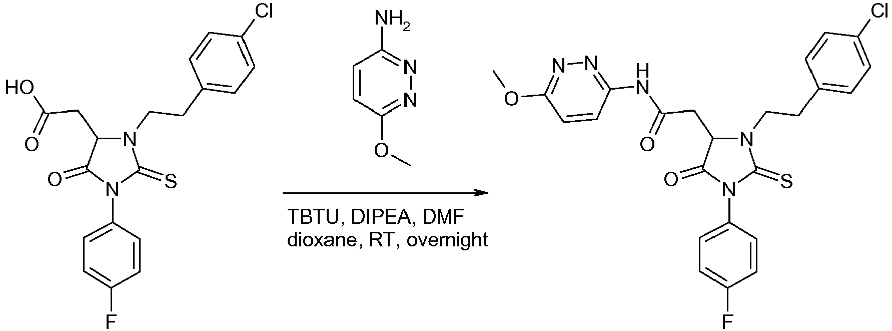

TBTU (18.2 mg; 0.55 mmol; 1.5 eq) and DIPEA (129 µL; 0.74 mmol; 2 eq) were added to a solution of 2-{3-[2-(4-chlorophenyl)ethyl]-1-(4-fluorophenyl)-5-oxo-2-sulfanylideneimidazolidin-4-yl}acetic acid (1-5) (150 mg; 0.37 mmol; 1 eq) in dioxane (2 mL). The reaction mixture was stirred at room temperature for 30 minutes, before adding 6-methoxypyridazin-3-amine (93 mg; 0.74 mmol; 2 eq) in dimethylformamide (0.1 mL). The reaction mixture was stirred at room temperature overnight. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was triturated in methanol/diethyl ether. The title compound, 2-{3-[2-(4-chlorophenyl)ethyl]-1-(4-fluorophenyl)-5-oxo-2-sulfanylideneimidazolidin-4-yl}-N-(5-methoxypyridin-2-yl)acetamide was obtained in 17 % yield (33.14 mg) as a white powder. ¹H-NMR (DMSO-d₆): δ (ppm) 2.9 (m, 1H), 3.04 (m, 1H), 3.22 (dd, 1H, J = 16.9 Hz, 3.8 Hz), 3.46 (m, 1H), 3.75 (m, 1H), 3.98 (s, 3H), 4.16 (m, 1H), 4.8 (t, 1H, J = 4.2 Hz), 7.35 (m, 9H), 8.16 (d, 1H, J = 9.6 Hz), 11.22 (s, 1H); MS (ESI+): m/z = 513.7, 515.7 [M+H]⁺.

### Example 41: Preparation of 2-{3-[2-(4-chlorophenyl)ethyl]-1-(4-fluorophenyl)-5-oxo-2-sulfanylideneimidazolidin-4-yl}-N-[4-(morpholin-4-yl)phenyl]acetamide.

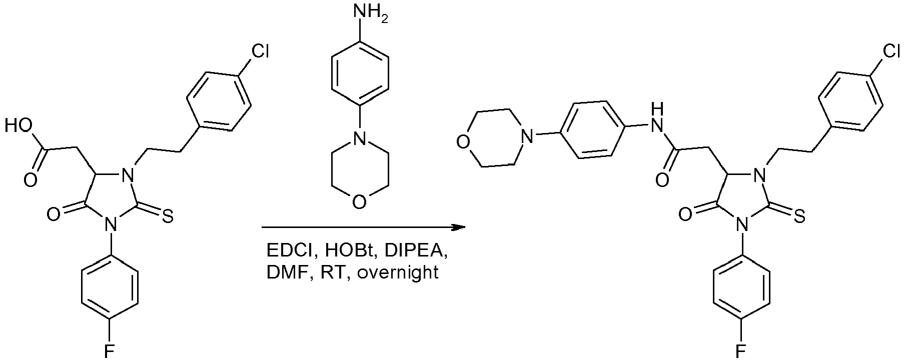

EDCI (113.1 mg; 0.59 mmol; 1.2 eq), followed by HOBt (90.3 mg; 0.59 mmol; 1.2 eq) and DIPEA (214 µL; 1.23 mmol; 2.5 eq) were added to a solution of 2-{3-[2-(4-chlorophenyl)ethyl]-1-(4-fluorophenyl)-5-oxo-2-sulfanylideneimidazolidin-4-yl}acetic acid (1-5) (200 mg; 0.49 mmol; 1 eq) in dimethylformamide (4 mL). After 20 minutes, 4-(morpholin-4-yl)aniline (105 mg; 0.59 mmol; 1.2 eq) was added and the reaction mixture was stirred at room temperature overnight. Saturated ammonium chloride (30 mL) was added and the aqueous layer was extracted with ethyl acetate (3 x 30 mL). The combined organic layers were washed with saturated sodium chloride (3 x 30 mL), dried over sodium sulfate, filtered and concentrated under reduced pressure. The crude was purified by flash chromatography on silica gel using dichloromethane/methanol (100/0 to 95/5) as an eluent. After trituration in dichloromethane/pentane, the title compound 2- {3-[2-(4-chlorophenyl)ethyl]-1-(4-fluorophenyl)-5-oxo-2-sulfanylidene imidazolidin-4-yl}-*N*-[4-(morpholin-4-yl)phenyl]acetamide was obtained in 4 % yield (11.8 mg) as an off-white solid. ¹H-NMR (DMSO-d₆): δ (ppm) 2.88 (m, 1H), 3.06 (m, 6H), 3.25 (m, 1H), 3.71 (m, 5H), 4.16 (m, 1H), 4.75 (m, 1H), 6.89 (m, 2H), 7.35 (m, 10H), 10.02 (s, 1H); MS (ESI+): m/z = 566.9, 568.9 [M+H]⁺.

### Example 68: Preparation of 2-{3-[2-(4-methoxyphenyl)ethyl]-5-oxo-1-phenyl-2-sulfanylideneimidazolidin-4-yl}-N-(6-methylpyridazin-3-yl)acetamide.

### a) 1,4-diethyl 2-{[2-(4-methoxyphenyl)ethyl]amino}butanedioate (I-6)

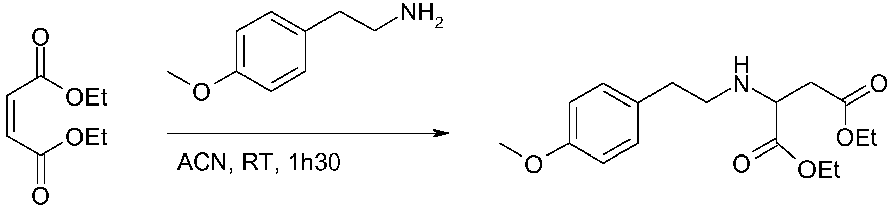

2-(4-methoxyphenyl)ethan-1-amine (10 g; 64.25 mmol; 4 eq) was added to a solution of 1,4-diethyl (2Z)-but-2-enedioate (2.77 g; 16.06 mmol; 1 eq) in acetonitrile (56 mL). The reaction mixture was stirred at room temperature for 1 hour and 30 minutes, and then, concentrated to dryness. The crude was purified on silica gel chromatography using cyclohexane/ethyl acetate (90/10 to 60/40) as an eluent. The title compound, 1,4-diethyl 2- {[2-(4-methoxyphenyl)ethyl]amino}butanedioate was obtained in 93% yield (4.81 g) as yellow oil. ¹H-NMR (CDCl₃): δ (ppm) 1.24 (m, 6H), 1.72 (s, 1H), 2.61 (dd, 1H, J = 15.7 Hz, 6.9 Hz), 2.72 (m, 4H), 2.9 (m, 1H), 3.65 (t, 1H, J = 6.5 Hz), 3.78 (s, 3H), 4.15 (m, 4H), 6.82 (d, 2H, J = 8.5 Hz), 7.11 (d, 2H, J = 8.5 Hz).

### b) Ethyl 2- {3-[2-(4-methoxyphenyl)ethyl]-5-oxo-1-phenyl-2-sulfanylidene imidazolidin-4-yl}acetate (I-7)

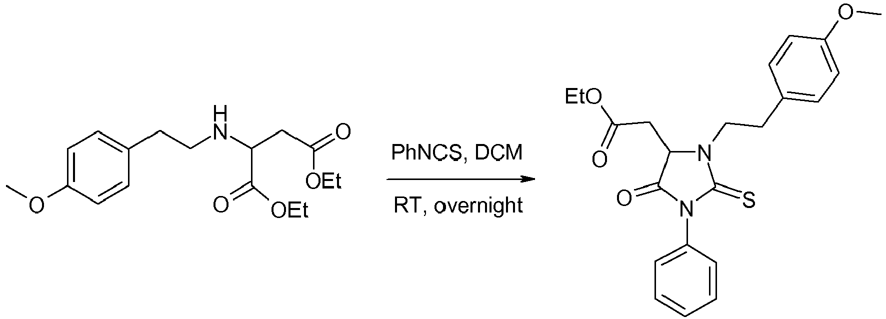

Phenyl isothiocyanate (1.5 mL; 12.93 mmol; 1.1 eq) was added to a solution of 1,4-diethyl 2-{[2-(4-methoxyphenyl)ethyl]amino}butanedioate (3.8 g; 11.75 mmol; 1 eq) in dichloromethane (40 mL). The reaction mixture was stirred at room temperature overnight, and then, concentrated to dryness. The crude was purified on silica gel using cyclohexane/ethyl acetate (70/30 to 50/50) as an eluent. The title compound, ethyl 2-{3-[2-(4-methoxyphenyl)ethyl]-5-oxo-1-phenyl-2-sulfanylideneimidazolidin-4-yl} acetate was obtained in 99 % yield (4.8 g) as colorless oil. ¹H-NMR (CDCl₃): δ (ppm) 1.26 (t, 3H, J = 7.4 Hz), 2.89 (m, 1H), 2.97 (d, 2H, J = 4.6 Hz), 3.12 (m, 1H), 3.58 (m, 1H), 3.8 (s, 3H), 4.17 (m, 3H), 4.39 (m, 1H), 6.87 (d, 2H, J = 8.5 Hz), 7.2 (d, 2H, J = 8.4 Hz), 7.36 (dd, 2H, J = 8.0 Hz, 1.6 Hz), 7.48 (m, 3H).

### c) 2-{3-[2-(4-methoxyphenyl)ethyl]-5-oxo-1-phenyl-2-sulfanylideneimidazolidin-4-yl}acetic acid (1-8)

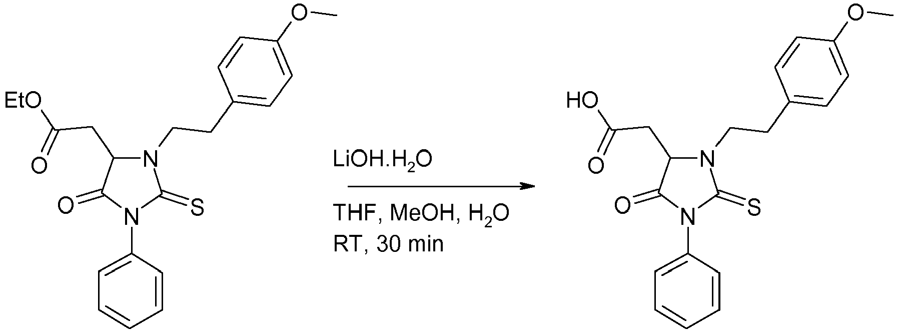

An aqueous solution of lithium hydroxide monohydrate (10.6 mL; 2.2 M aqueous solution; 23.27 mmol; 2 eq) was added to a solution of ethyl 2-{3-[2-(4-methoxyphenyl)ethyl]-5-oxo-1-phenyl-2-sulfanylideneimidazolidin-4-yl} acetate (1-7) (4.8 g; 11.64 mmol; 1 eq) in tetrahydrofuran (30.6 mL) and methanol (21.1 mL). The reaction mixture was stirred at room temperature for 30 minutes, and then, concentrated to dryness. Ethyl acetate (30 mL) was added. The layers were separated and the aqueous layer was acidified with hydrochloric acid 1N until pH 2, and was extracted with ethyl acetate (2 x 30 mL). The combined organic layers was dried over sodium sulfate, filtered and concentrated under reduced pressure. The crude was purified on silica gel using cyclohexane/ethyl acetate/acetic acid (79/20/1 to 50/50/1) as an eluent. The title compound, 2-{3-[2-(4-methoxyphenyl)ethyl]-5-oxo-1-phenyl-2-sulfanylidene imidazolidin-4-yl}acetic acid was obtained in 34 % yield (1.52 g) as a yellow solid. ¹H-NMR (CDCl₃): δ (ppm) 2.93 (m, 1H), 3.01 (dt, 2H, J = 4.6 Hz, 1.6 Hz), 3.12 (m, 1H), 3.59 (dt, 1H, J = 14.1 Hz, 7.7 Hz), 3.79 (s, 3H), 4.09 (t, 1H, J = 4.3 Hz), 4.42 (m, 1H), 6.86 (d, 2H, J = 8.5 Hz), 7.18 (d, 3H, J = 7.1 Hz), 7.29 (m, 2H), 7.47 (m, 2H).

### d) 2-2+{3-[2-(4-methoxyphenyl)ethyl]-5-oxo-1-phenyl-2-sulfanylideneimidazolidin-4-yl}-N-(6-methylpyridazin-3-yl)acetamide (example68)

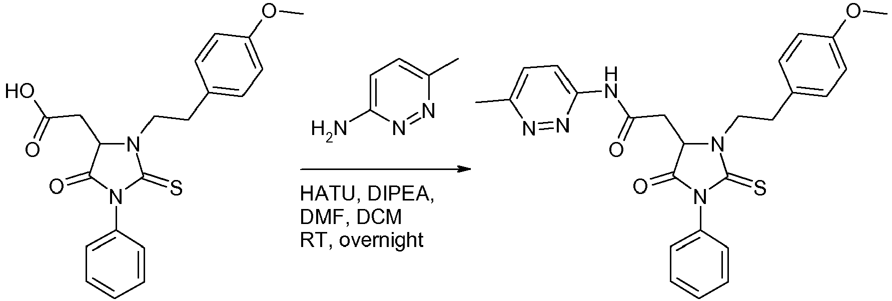

HATU (475 mg; 1.25 mmol; 1.6 eq) and DIPEA (410 µL; 2.34 mmol; 3 eq) were added to a solution of 2-{3-[2-(4-methoxyphenyl)ethyl]-5-oxo-1-phenyl-2-sulfanylideneimidazolidin-4-yl}acetic acid (I-8) (300 mg; 0.78 mmol; 1 eq) in dimethylformamide (3 mL). The reaction mixture was stirred at room temperature for 1 hour. Then, 6-methylpyridazin-3-amine (128 mg; 1.17 mmol; 1.5 eq) in dimethylformamide (0.3 mL) and dichloromethane (0.3 mL) was added to the solution. The reaction mixture was stirred at room temperature overnight. Saturated ammonium chloride (80 mL) was added and the aqueous layer was extracted with ethyl acetate (3 x 80 mL). The combined organic layers were washed with saturated sodium chloride (3 x 80 mL), dried over sodium sulfate, filtered and concentrated under reduced pressure. The crude was purified on silica gel using cyclohexane/ethyl acetate (40/60 to 0/100) and dichloromethane/methanol (99/1 to 80/20). After trituration in methanol, the title compound 2-{3-[2-(4-methoxyphenyl)ethyl]-5-oxo-1-phenyl-2-sulfanylidene imidazolidin-4-yl}-*N*-(6-methylpyridazin-3-yl)acetamide was obtained in 9 % yield (35 mg) as a white powder. ¹H-NMR (CDCl₃): δ (ppm) 2.66 (s, 3H), 2.95 (m, 1H), 3.16 (m, 1H), 3.3 (m, 1H), 3.68 (m, 2H), 3.76 (s, 3H), 4.36 (m, 2H), 6.79 (d, 2H, J = 8.5 Hz), 7.12 (d, 2H, J = 7.9 Hz), 7.46 (m, 6H), 8.48 (d, 1H, J = 9.4 Hz), 10.8 (s, 1H); MS (ESI+): m/z = 475.8 [M+H]⁺.

### Example 69: Preparation of 2-{3-[2-(4-methoxyphenyl)ethyl]-5-oxo-1-phenyl-2-sulfanylideneimidazolidin-4yl}-N-(6-methoxypyridazin-3-yl)acetamide.

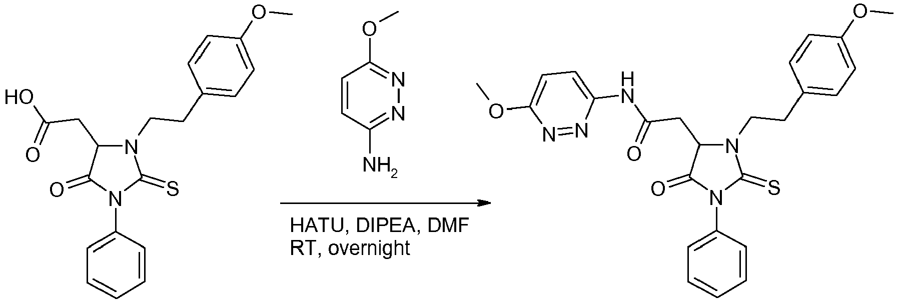

HATU (475 mg; 1.25 mmol; 1.6 eq) and DIPEA (410 µL; 2.34 mmol; 3 eq) were added to a solution of 2-{3-[2-(4-methoxyphenyl)ethyl]-5-oxo-1-phenyl-2-sulfanylideneimidazolidin-4-yl}acetic acid (I-8) (300 mg; 0.78 mmol; 1 eq) in dimethylformamide (3 mL). The reaction mixture was stirred at room temperature for 1 hour. Then, 6-methoxypyridazin-3-amine (146 mg; 1.17 mmol; 1.5 eq) in dimethylformamide (0.5 mL) was added and the reaction mixture was stirred at room temperature overnight. Saturated ammonium chloride (80 mL) was added and the aqueous layer was extracted with ethyl acetate (3 x 80 mL). The combined organic layers were washed with saturated sodium chloride (3 x 80 mL), dried over sodium sulfate, filtered and concentrated under reduced pressure. The crude was purified on silica gel using dichloromethane/methanol (99/1 to 95/5). After trituration in diethyl ether/methanol, the title compound 2-{3-[2-(4-methoxyphenyl)ethyl]-5-oxo-1-phenyl-2-sulfanylideneimidazolidin-4-yl}-*N*-(6-methoxypyridazin-3-yl)acetamide was obtained in 3 % yield (12 mg) as a white powder. ¹H-NMR (CDCl₃): δ (ppm) 2.96 (m, 1H), 3.12 (m, 1H), 3.25 (m, 1H), 3.46 (m, 1H), 3.65 (m, 1H), 3.75 (s, 3H), 4.02 (s, 3H), 4.38 (m, 2H), 6.79 (d, 2H, J = 8.5 Hz), 7.11 (m, 3H), 7.38 (dd, 2H, J = 7.5 Hz, 1.6 Hz), 7.49 (m, 3H), 8.49 (d, 1H, J = 9.7 Hz), 10.25 (m, 1H); MS (ESI+): m/z = 491.8 [M+H]⁺.

### Example 71: Preparation of 2-[1-(4-fluorophenyl)-3-[2-(4-methoxyphenyl)ethyl]-2,5-dioxoimidazolidin-4-yl]-N-(6-methoxypyridazin-3-yl)acetamide.

### a) Ethyl 2-[1-(4-fluorophenyl)-3-[2-(4-methoxyphenyl)ethyl]-2,5-dioxoimidazolidin-4-yl]acetate (1-9)

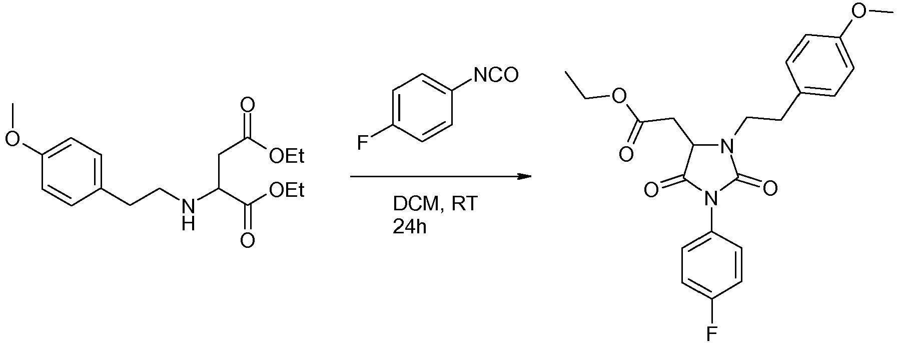

4-Fluorophenyl isocyanate (2 mL; 17.7 mmol; 1.15 eq) was added to a solution of 1,4-diethyl 2-{[2-(4-methoxyphenyl)ethyl]amino}butanedioate (1-6) (5 g; 15.4 mmol; 1 eq) in dichloromethane (50 mL) and the reaction mixture was stirred at room temperature for 24 hours. Then, the reaction mixture was concentrated to dryness. After trituration in cyclohexane/diethyl ether, the title compound was obtained in 99 % yield (6.3 g) as a white solid. ¹H-NMR (CDCl₃): δ (ppm) 1.28 (t, 3H, J = 7.1 Hz), 2.78 (m, 1H), 2.89 (t, 2H, J = 6.4 Hz), 3.29 (dd, 1H, J = 17.4 Hz, 5.5 Hz), 3.41 (m, 1H), 3.68 (m, 1H),3.80 (s, 3H), 4.2 (m, 2H), 4.53 (dd, 1H, J = 7.7 Hz, 5.5 Hz), 6.92 (m, 6H), 7.23 (d, 2H, J = 8.6 Hz).

### b) 2-[1-(4-Fluorophenyl)-3-[2-(4-methoxyphenyl)ethyl]-2,5-dioxoimidazolidin-4-yl]acetic acid (1-10)

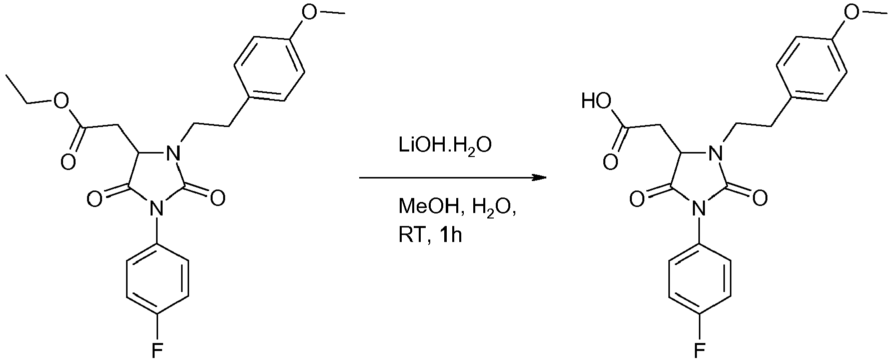

An aqueous solution of lithium hydroxide monohydrate (13.7 mL; 2.2 M aqueous solution; 30.4 mmol; 2 eq) was added to a solution of ethyl 2-[1-(4-fluorophenyl)-3-[2-(4-methoxyphenyl)ethyl]-2,5-dioxoimidazolidin-4-yl]acetate (6.3 g; 15 mmol; 1 eq) in tetrahydrofuran (39.4 mL) and methanol (27.3 mL). The reaction mixture was stirred at room temperature for 1 hour, and then concentrated to dryness. Aqueous solution of hydrochloric acid 1N was added until pH 3, to obtain a white solid as precipitate which was filtered to yield the title compound 2-[1-(4-fluorophenyl)-3-[2-(4-methoxyphenyl)ethyl]-2,5-dioxoimidazolidin-4-yl]acetic acid in a quantitative yield. The crude was used without any further purification. ¹H-NMR (DMSO-d₆): δ (ppm) 2.87 (m, 4H), 3.32 (m, 2H), 3.72 (s, 3H), 4.42 (t, 1H, J = 4.2 Hz), 6.87 (d, 2H, J = 8.7 Hz), 7.19 (d, 2H, J = 8.5 Hz), 7.33 (m, 4H), 12.81 (s, 1H).

### c) 2-[1-(4-fluorophenyl)-3-[2-(4-methoxyphenyl)ethyl]-2,5-dioxoimidazolidin-4-yl]-N-(6-methoxypyridazin-3-yl)acetamide (example 71)

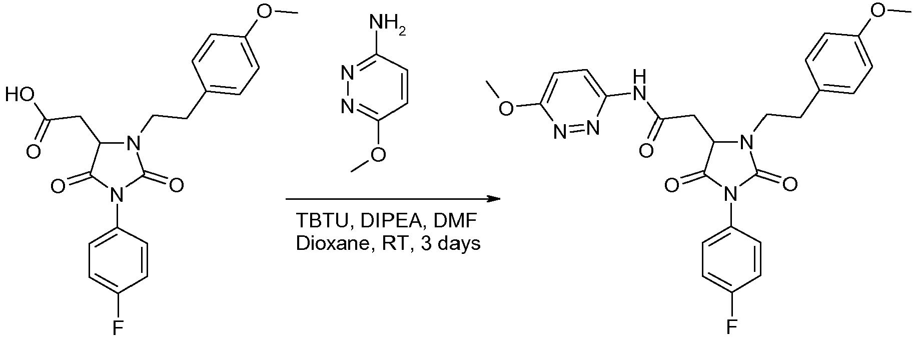

TBTU (380.8 mg; 1.16 mmol; 1.5 eq) and DIPEA (270 µL; 1.55 mmol; 2 eq) were added to a suspension of 2-[1-(4-fluorophenyl)-3-[2-(4-methoxyphenyl)ethyl]-2,5-dioxoimidazolidin-4-yl]acetic acid (I-10) (300 mg; 0.78 mmol; 1 eq) in dioxane (4 mL). The reaction mixture was stirred at room temperature for 20 minutes. Then, 6-methoxypyridazin-3-amine (145.9 mg; 1.16 mmol; 1.2 eq) in dimethylformamide (0.2 mL) was added and the reaction mixture was stirred at room temperature for 3 days. The reaction mixture was concentrated to dryness. Saturated ammonium chloride (80 mL) was added and the aqueous layer was extracted with ethyl acetate (3 x 80 mL). The combined organic layers were washed with saturated sodium chloride (3 x 80 mL), dried over sodium sulfate, filtered and concentrated under reduced pressure. The title compound, 2-[1-(4-fluorophenyl)-3-[2-(4-methoxyphenyl)ethyl]-2,5-dioxoimidazolidin-4-yl]-N-(6-methoxypyridazin-3-yl)acetamide, was obtained in 45% yield (173.8 mg) as a white solid. ¹H-NMR (DMSO-d₆): δ (ppm) 2.8 (m, 2H), 3.09 (m, 1H), 3.28 (m, 1H), 3.7 (s, 4H), 3.98 (s, 3H), 4.54 (t, 1H, J = 4.1 Hz), 6.84 (d, 2H, J = 8.7 Hz), 7.26 (m, 8H), 8.17 (dt, 1H, J = 9.5 Hz, 1.4Hz), 11.18 (s, 1H); MS (ESI+): m/z = 493.9 [M+H]⁺.

### Example 72: Preparation of 2-[1-(4-fluorophenyl)-3-[2-(4-methoxyphenyl)ethyl]-2,5-dioxoimidazolidin-4-yl]-N-(5-methoxypyridin-2-yl)acetamide.

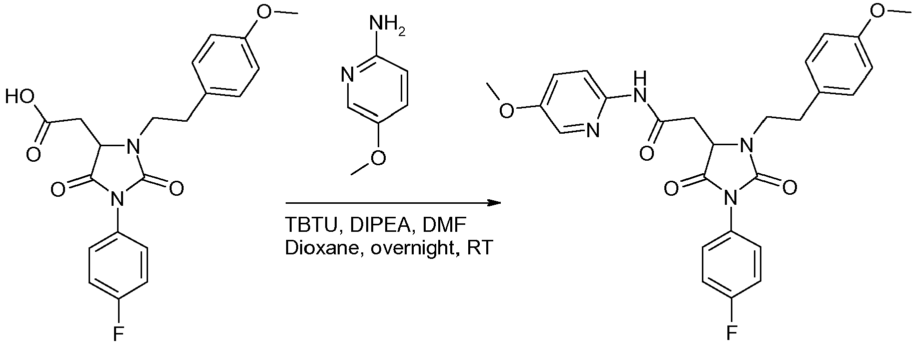

TBTU (351 mg; 1.07 mmol; 1.5 eq) and DIPEA (248 µL; 1.42 mmol; 2 eq) were added **to a suspension of** 2-[1-(4-fluorophenyl)-3-[2-(4-methoxyphenyl)ethyl]-2,5-dioxoimidazolidin-4-yl]acetic acid (1-10) (275 mg; 0.71 mmol; 1 eq) in dioxane (10 mL). The reaction mixture was stirred at room temperature for 20 minutes. Then, 5-methoxypyridin-2-amine (132 mg; 1.07 mmol; 1.5 eq) in dimethylformamide (0.2 mL) was added and the reaction mixture was stirred at room temperature overnight. The reaction mixture was concentrated to dryness. Saturated ammonium chloride (80 mL) was added and the aqueous layer was extracted with ethyl acetate (3 x 80 mL). The combined organic layers were washed with saturated sodium chloride (3 x 80 mL), dried over sodium sulfate, filtered and concentrated under reduced pressure. After trituration in diethyl ether/dichloromethane, the title compound 2-[1-(4-fluorophenyl)-3-[2-(4-methoxyphenyl)ethyl]-2,5-dioxoimidazolidin-4-yl]-*N*-(5-methoxypyridin-2-yl) acetamide was obtained in 17% yield (65.5 mg) as an orange solid. ¹H-NMR (DMSO-d₆): δ (ppm) 2.79 (m, 2H), 3.02 (dd, 1H, J = 16.5 Hz, 4.0 Hz), 3.24 (dd, 1H, J = 16.6 Hz, 4.5 Hz), 3.33 (m, 1H), 3.67 (m, 1H), 3.7 (s, 3H), 3.79 (s, 3H), 4.52 (t, 1H, J = 4.2 Hz), 6.84 (d, 2H, J = 8.4 Hz), 7.16 (d, 2H, J = 8.7 Hz), 7.38 (m, 5H), 7.99 (m, 2H), 10.63 (s, 1H); MS (ESI+): m/z = 493 [M+H]⁺.

### Example 42: Preparation of N-[4-(dimethylamino)phenyl]-2-[1-(4-fluorophenyl)-3-[2-(4-methoxyphenyl)ethyl]-2,5-dioxoimidazolidin-4-yl]acetamide.

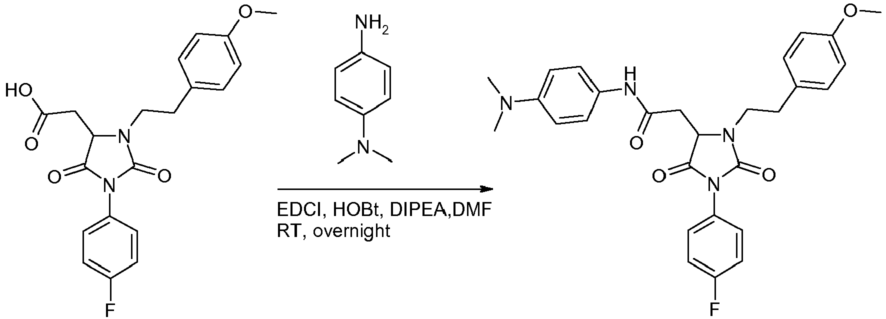

EDCI (337 mg; 1.77 mmol; 1.3 eq), followed by HOBt (270 mg; 1.77 mmol; 1.3 eq), DIPEA (599 mg; 3.11 mmol; 3 eq) and 1*-N,*1-*N*-dimethylbenzene-1,4-diamine (240 mg; 1.77 mmol; 1.3 eq) were added to a solution of 2-[1-(4-fluorophenyl)-3-[2-(4-methoxyphenyl)ethyl]-2,5-dioxoimidazolidin-4-yl]acetic acid (1-10) (400 mg; 1.04 mmol; 1 eq) in dimethylformamide (5 mL). The reaction mixture was stirred at room **temperature overnight. The crude was purified on silica gel using ethyl** acetate/cyclohexane (20/80 to 50/50) as an eluent. The title compound, N-[4-(dimethylamino)phenyl]-2-[1-(4-fluorophenyl)-3-[2-(4-methoxyphenyl)ethyl]-2,5-dioxoimidazolidin-4-yl]acetamide was obtained in 29% (150 mg) as a white solid. ¹H-NMR (DMSO-d₆): δ (ppm) 2.83 (m, 8H), 3.01 (m, 2H), 3.31 (m, 1H), 3.71 (m, 4H), 4.5 (t, 1H, J = 4.1 Hz), 6.68 (d, 2H, J = 9.2 Hz), 6.84 (d, 2H, J = 8.8 Hz), 7.17 (d, 2H, J = 8.1 Hz), 7.36 (m, 6H), 9.89 (s, 1H). MS (ESI+): m/z = 505 [M+H]⁺.

### Example 73: Preparation of N-[5-(dimethylamino)pyridin-2-yl]-2-[1-(4-fluorophenyl)-3-[2-(4-methoxyphenyl)ethyl]-2,5-dioxoimidazolidin-4-yl]acetamide.

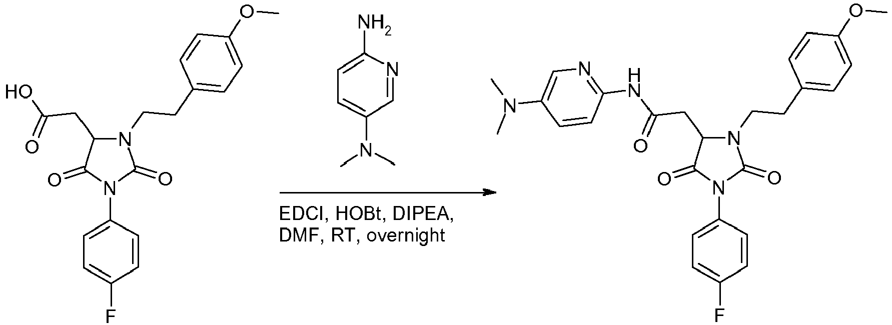

EDCI (237 mg; 1.24 mmol; 1.2 eq), followed by HOBt (189.7 mg; 1.24 mmol; 1.2 eq), DIPEA (599.3 mg; 3.11 mmol; 3 eq) and 5-*N*,5-*N*-dimethylpyridine-2,5-diamine (142 mg; 1.14 mmol; 1.1 eq) were added to a solution of 2-[1-(4-fluorophenyl)-3-[2-(4-methoxyphenyl)ethyl]-2,5-dioxoimidazolidin-4-yl]acetic acid (1-10) (400 mg; 1.04 mmol; 1 eq) in dimethylformamide (5 mL). The reaction mixture was stirred at room temperature overnight. Saturated ammonium chloride (50 mL) was added and the aqueous layer was extracted with ethyl acetate (3 x 50 mL). The combined organic layers were washed with saturated sodium chloride (3 x 20 mL), dried over magnesium sulfate, filtered and concentrated under reduced pressure. The crude was purified on silica gel using cyclohexane/ethyl acetate (80/20 to 50/50) as an eluent. After trituration in diethyl ether, the title compound N-[5-(dimethylamino)pyridin-2-yl]-2-[1-(4-fluorophenyl)-3-[2-(4-methoxyphenyl)ethyl]-2,5-dioxoimidazolidin-4-yl]acetamide was obtained in 33% yield (173 mg). ¹H-NMR (DMSO-d₆): δ (ppm) 2.65 (m, 1H), 2.87 (m, 7H), 2.94 (m, 1H), 3.33 (m, 2H), 3.7 (m, 4H), 4.51 (t, 1H, J = 4.4 Hz), 6.84 (d, 2H, J = 8.4 Hz), 7.18 (dd, 3H, J = 9.5 Hz, 8.6 Hz), 7.36 (m, 4H), 7.83 (m, 2H), 10.45 (s, 1H). MS (ESI+): m/z = 506.0 [M+H]⁺.

### Example 74: Preparation of 2-[1-(4-fluorophenyl)-3-[2-(4-methoxyphenyl)ethyl]-2,5-dioxoimidazolidin-4-yl]-N-(6-methylpyridazin-3-yl)acetamide.

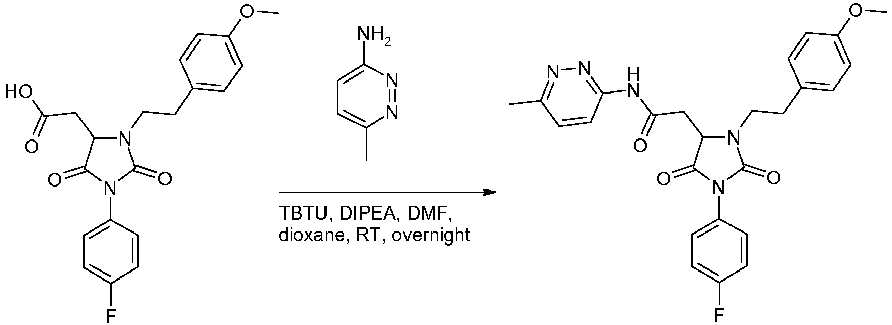

TBTU (351 mg; 1.07 mmol; 1.5 eq) and DIPEA (248 µL; 1.42 mmol; 2 eq) were added to a suspension of 2-[1-(4-fluorophenyl)-3-[2-(4-methoxyphenyl)ethyl]-2,5-dioxoimidazolidin-4-yl]acetic acid (1-10) (275 mg; 0.71 mmol; 1 eq) in dioxane (10 mL). The reaction mixture was stirred at room temperature for 20 minutes. Then, 6-methylpyridazin-3-amine (117 mg; 1.07 mmol; 1.5 eq) in dimethylformamide (0.2 mL) was added and the reaction mixture was stirred at room temperature overnight. Saturated ammonium chloride (80 mL) was added and the aqueous layer was extracted with ethyl acetate (3 x 80 mL). The combined organic layers were washed with saturated sodium chloride (3 x 80 mL), dried over sodium sulfate, filtered and concentrated under reduced pressure. After trituration in diethyl ether and lyophilisation, the title compound 2-[1-(4-fluorophenyl)-3-[2-(4-methoxyphenyl)ethyl]-2,5-dioxoimidazolidin-4-yl]-N-(6-methylpyridazin-3-yl)acetamide was obtained in 31 % yield (106.3 mg) as an off-white solid. ¹H-NMR (DMSO-d₆): δ (ppm) 2.60 (s, 3H), 2.81 (m, 2H), 3.11 (m, 1H), 3.32 (m, 2H), 3.7 (m, 4H), 4.54 (t, 1H, J = 3.8 Hz), 6.83 (dt, 2H, J = 8.1 Hz, 1.8 Hz), 7.28 (m, 7H), 8.14 (dt, 1H, J = 9.2 Hz, 1.5 Hz), 11.26 (s, 1H); MS (ESI+): m/z = 478 [M+H]⁺.

### Example 43: Preparation of 2-[1-(4-fluorophenyl)-3-[2-(4-methoxyphenyl)ethyl]-2,5-dioxoimidazolidin-4-yl]-N-{4-[(2-methoxyethyl)(methyl)amino]phenyl}acetamide.

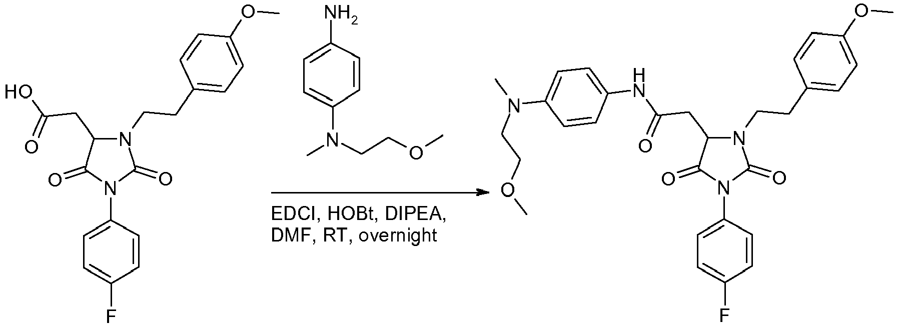

EDCI (221 mg; 1.15 mmol; 1.5 eq) followed by HOBt (156 mg; 1.15 mmol; 1.5 eq), DIPEA (298 mg; 2.31 mmol; 3 eq) and 1-*N*-(2-methoxyethyl)-1-N-methylbenzene-1,4-diamine (167 mg; 0.93 mmol; 1.2 eq) were added to a solution of 2-[1-(4-fluorophenyl)-3-[2-(4-methoxyphenyl)ethyl]-2,5-dioxoimidazolidin-4-yl]acetic acid (1-10) (300 mg; 0.77 mmol; 1 eq) in dimethylformamide (5 mL). The reaction mixture was stirred at room temperature overnight. Saturated ammonium chloride (80 mL) was added and the aqueous layer was extracted with ethyl acetate (3 x 80 mL). The combined organic layers were washed with saturated sodium chloride (3 x 80 mL), dried over magnesium sulfate, filtered and concentrated under reduced pressure. The crude was purified on silica gel using dichloromethane/ethyl acetate (90/10) as an eluent. After trituration in diethyl ether, the title compound 2-[1-(4-fluorophenyl)-3-[2-(4-methoxyphenyl)ethyl]-2,5-dioxoimidazolidin-4-yl]-*N*-{4-[(2-methoxyethyl)(methyl)amino]phenyl}acetamide was obtained in 8% yield (33 mg) as a white solid. ¹H-NMR (DMSO-d₆): δ (ppm) 2.76 (m, 1H), 2.84 (m, 4H), 3.08 (m, 2H), 3.22 (s, 3H), 3.3 (d, 1H, J = 4.7 Hz), 3.44 (m, 4H), 3.7 (m, 4H), 4.5 (m, 1H), 6.65 (m, 2H), 6.84 (m, 2H), 7.17 (m, 2H), 7.29 (m, 6H), 9.87 (s, 1H); MS (ESI+): m/z = 549.0 [M+H]⁺.

### Example 44: Preparation of N-[4-(dimethylamino)-3-methylphenyl]-2-[1-(4-fluorophenyl)-3-[2-(4-methoxyphenyl)ethyl]-2,5-dioxoimidazolidin-4-yl]acetamide.

### a) N,N,2-trimethyl-4-nitroaniline (I-13)

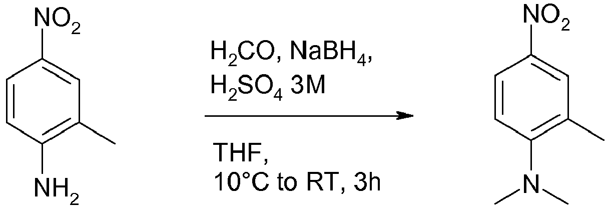

A solution of 2-methyl-4-nitroaniline (4g; 0.026 mmol; 1 eq) and sodium borohydride (4.95 g; 0.131 mmol; 5 eq) in tetrahydrofuran (230 mL) was added dropwise to an efficiently stirred solution of 3M sulfuric acid (131 mL, 393 mmol) and 35 % aq formaldehyde (7.8 mL; 0.105 mmol; 4 eq) in tetrahydrofuran (58 mL) at 10-15°C. After the first half of the addition was performed, the mixture was acidified with 3M sulfuric acid (131 mL; 393 mmol). The reaction mixture was stirred for 15 minutes, before adding dropwise the remainder of the solution. The reaction mixture was stirred for 2 hours at room temperature. Sodium hydroxide 3M was added to raise the pH to about 9-10. The organic phase was separated and the aqueous layer was extracted with ethyl acetate (3 x 200 mL). The combined organic layers were washed with saturated sodium chloride (150 mL), dried over sodium sulfate, filtered and concentrated under reduced pressure. The crude was purified on silica gel using ethyl acetate/cyclohexane (10/90) as an eluent. The title compound N,N,2-trimethyl-4-nitroaniline was obtained in 63% yield (2.98 g) as yellow oil. ¹H-NMR (CDCl₃): δ (ppm) 1.62 (s, 3H), 2.1 (s, 6H), 6.16 (m, 1H), 6.5 (m, 1H), 7.25 (dd, 1H, J = 5.1Hz, 2.4Hz).

### b) 1-N,1-N,2-trimethylbenzene-1,4-diamine (I-14)

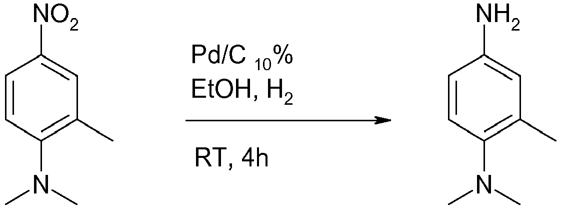

Palladium on carbon 10% (596 mg; 5.60 mmol; 0.3 eq) under argon atmosphere was added to a solution of N,N,2-trimethyl-4-nitroaniline (2.98 g; 16.53 mmol; 1 eq) in ethanol (165 mL). The reaction mixture was submitted to 5 cycles vacuum/hydrogen and stirred at room temperature under hydrogen atmosphere for 4 hours. The reaction mixture was filtered on Clarcel^{®}, washed with ethanol and concentrated to dryness. The title compound, 1-*N*,1-*N*,2-trimethylbenzene-1,4-diamine was obtained in 95% yield (2.35 g) as a pink oil. ¹H-NMR (CDCl₃): δ (ppm) 2.97 (s, 3H), 3.32 (s, 6H), 4.14 (m, 2H), 7.22 (dd, 1H, J = 8.9 Hz, 2.4Hz), 7.61 (dt, 1H, J = 8.2 Hz, 1.6 Hz), 7.97 (s, 1H).

### c) N-[4-(dimethylamino)-3-methylphenyl]-2-[1-(4-fluorophenyl)-3-[2-(4-methoxyphenyl)ethyl]-2,5-dioxoimidazolidin-4-yl]acetamide (example 44)

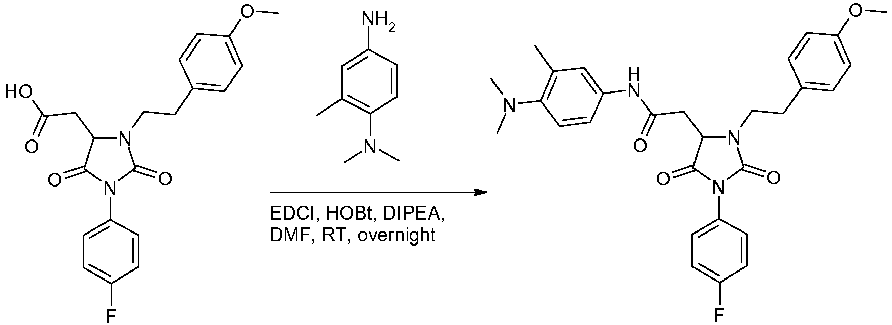

EDCI (74.3 mg; 0.39 mmol; 1.5 eq) followed by HOBt (59.4 mg; 0.39 mmol; 1.5 eq), DIPEA (215.3 µL; 1.29 mmol; 5 eq) and 1-*N*,1-*N*,2-trimethylbenzene-1,4-diamine (50.5 mg; 0.37 mmol; 1.3 eq) were added to a solution of 2-[1-(4-fluorophenyl)-3-[2-(4-methoxyphenyl)ethyl]-2,5-dioxoimidazolidin-4-yl] acetic acid (1-10) (100 mg; 0.26 mmol; 1 eq) in dimethylformamide (1.5 mL). The reaction mixture was stirred at room temperature overnight. Saturated ammonium chloride (10 mL) was added and the aqueous layer was extracted with ethyl acetate (3 x 10 mL). The combined organic layers were washed with saturated sodium chloride (3 x 30 mL), dried over sodium sulfate, filtered and concentrated under reduced pressure. The crude was purified by flash chromatography on silica gel using cyclohexane/ethyl acetate (100/0 to 50/50) as an eluent. After trituration in diethyl ether, the title compound, *N*-[4-(dimethylamino)-3-methylphenyl]-2-[1-(4-fluorophenyl)-3-[2-(4-methoxyphenyl)ethyl]-2,5-dioxo imidazolidin-4-yl]acetamide was obtained in 59% yield (79 mg) as a white solid. ¹H-NMR (CDCl₃): δ (ppm) 2.31 (s, 3H), 2.65 (s, 6H), 2.67 (m, 1H), 2.94 (m, 3H), 3.42 (m, 1H), 3.77 (s, 3H), 3.97 (m, 1H), 4.39 (m, 1H), 6.82 (m, 2H), 6.99 (m, 1H), 7.14 (m, 4H), 7.22 (m, 2H), 7.38 (m, 2H); MS (ESI+): m/z = 519.3 [M+H]⁺.

### Example 45: Preparation of N-[4-(dimethylamino)phenyl]-2-[1-(4-fluorophenyl)-2,5-dioxo-3-[2-(thiophen-2-yl)ethyl]imidazolidin-4-yl]acetamide.

### a) 1,4-diethyl 2-{[2-(thiophen-2-yl)ethyl]amino}butanedioate (I-15)

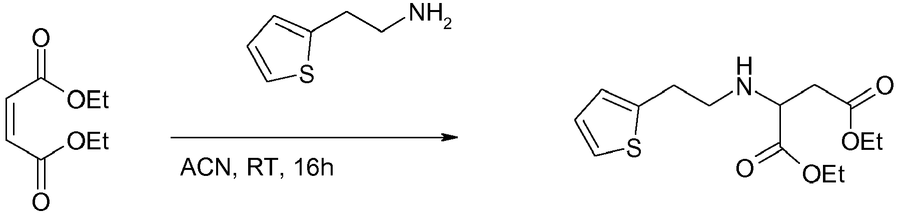

2-(Thiophen-2-yl)ethan-1-amine (4.08 g; 32.1 mmol; 2.5 eq) was added to a solution of 1,4-diethyl (2Z)-but-2-enedioate (2.08 mL; 12.8 mmol; 1 eq) in acetonitrile (45 mL). The reaction mixture was stirred at room temperature overnight and then, concentrated to dryness. The crude was purified by flash chromatography on silica gel using cyclohexane/ethyl acetate (100/0 to 80/20) as an eluent. The title compound, 2-[1-(4-fluorophenyl)-2,5-dioxo-3-[2-(thiophen-2-yl)ethyl]imidazolidin-4-yl]-N-(4-methoxyphenyl)acetamide, was obtained in 99% yield (3.80 g) as a yellow oil. ¹H-NMR (CDCl₃): δ (ppm) 1.25 (m, 6H), 1.81 (m, 1H), 2.72 (m, 3H), 2.99 (s, 3H), 3.66 (m, 1H), 4.16 (m, 4H), 6.88 (m, 2H), 7.13 (dd, 1H, J = 5.0 Hz, 2.3 Hz).

### b) ethyl 2-[1-(4-fluorophenyl)-2,5-dioxo-3-[2-(thiophen-2-yl)ethyl]imidazolidin-4-yl]acetate (I-16)

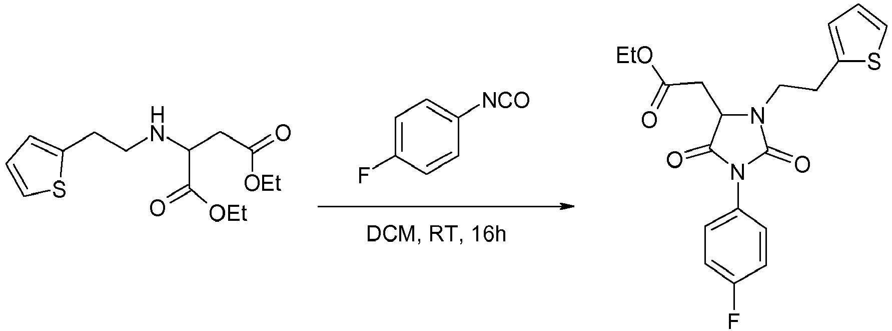

4-Fluorophenyl isocyanate (0.82 mL; 7.30 mmol; 1.15 eq) was added to a solution of 1,4-diethyl 2-{[2-(thiophen-2-yl)ethyl]amino}butanedioate (1-15) (1.9 g; 6.35 mmol; 1 eq) in dichloromethane (20 mL). The reaction mixture was stirred at room temperature overnight and concentrated to dryness. The crude was purified by flash chromatography on silica gel using cyclohexane/ethyl acetate (100/0 to 80/20) as an eluent. The title compound, ethyl 2-[1-(4-fluorophenyl)-2,5-dioxo-3-[2-(thiophen-2-yl)ethyl] imidazolidin-4-yl]acetate, was obtained in quantitative amount as a yellow gum. ¹H-NMR (CDCl₃): δ (ppm) 1.21 (m, 3H), 2.82 (m, 1H), 3.15 (m, 2H), 3.31 (m, 1H), 3.68 (m, 1H), 3.93 (m, 1H), 4.19 (m, 3H), 6.88 (m, 4H), 7.14 (m, 2H), 7.41 (m, 1H).

### c) 2-[1-(4-fluorophenyl)-2,5-dioxo-3-[2-(thiophen-2-yl)ethyl]imidazolidin-4-yl] acetic acid (I-17)

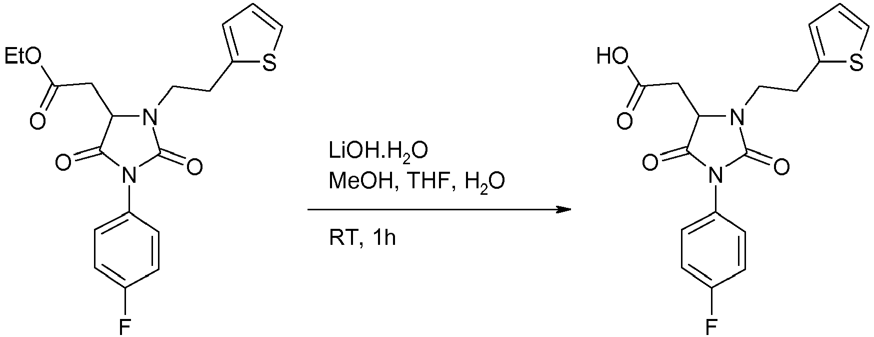

Lithium hydroxide monohydrate (498 mg; 11.88 mmol; 2 eq) was added to a solution of ethyl 2-[1-(4-fluorophenyl)-2,5-dioxo-3-[2-(thiophen-2yl)ethyl]imidazolidin-4-yl] acetate (1-16) (2.32 g; 5.94 mmol; 1 eq) in tetrahydrofuran (15 mL), methanol (10 mL) and water (5 mL). The reaction mixture was stirred at room temperature for 1 hour. The reaction mixture was concentrated to dryness. Water (20 mL) was added and the solution was acidified with hydrochloric acid 3M until pH 2. The aqueous layer was extracted with dichloromethane (2 x 20 mL), the combined organic layers were dried over sodium sulfate and concentrated to dryness. The title compound, 2-[1-(4-fluorophenyl)-2,5-dioxo-3-[2-(thiophen-2-yl)ethyl]imidazolidin-4-yl]acetic acid, was obtained in 86 % yield (1.85 g) as white foam. ¹H-NMR (CDCl₃): δ (ppm) 2.97 (m, 1H), 3.15 (m, 1H), 3.24 (m, 1H), 3.41 (m, 1H), 4.06 (m, 1H), 4.10 (m, 2H), 6.88(m, 1H), 6.92 (m, 1H), 7.16 (m, 3H), 7.36 (m, 2H).

### d) N-[4-(dimethylamino)phenyl]-2-[1-(4-fluorophenyl)-2,5-dioxo-3-[2-(thiophen-2-yl)ethyl]imidazolidin-4-yl]acetamide (example 45)

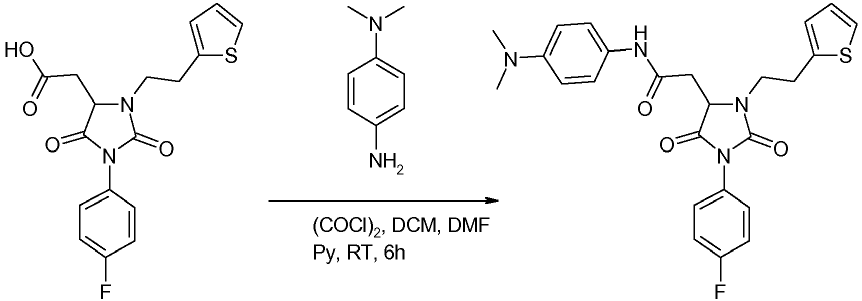

Dimethylformamide (catalytic amount) and oxalyl chloride (108.7 µL; 1.24 mmol; 3 eq) were added to a solution of 2-[1-(4-fluorophenyl)-2,5-dioxo-3-[2-(thiophen-2-yl)ethyl]imidazolidin-4-yl]acetic acid (1-17) (150 mg; 0.41 mmol; 1 eq) in dichloromethane (5 mL). The reaction mixture was stirred at room temperature for 2 hours and 30 minutes. Then, 1-N,1-N-dimethylbenzene-1,4-diamine (111 mg; 0.82 mmol; 2 eq) and pyridine (77 µL; 1.24 mmol; 3 eq) were added. The reaction mixture was stirred at room temperature for 3 hours. Water (20 mL), dichloromethane (20 mL) and saturated sodium bicarbonate were added. The aqueous layer was extracted with dichloromethane (3 x 20 mL). The combined organic layers were dried over sodium sulfate, filtered and concentrated under reduced pressure. The crude was purified on silica gel using dichloromethane/methanol (100/0 to 98/2) as an eluent. After trituration in dichloromethane/pentane, the title compound, *N*-[4-(dimethylamino)phenyl]-2-[1-(4-fluorophenyl)-2,5-dioxo-3-[2-(thiophen-2-yl)ethyl]imidazolidin-4-yl]acetamide, was obtained in 54 % yield (108 mg) as a pale yellow solid. ¹H-NMR (DMSO-d₆): δ (ppm) 2.83 (s, 6H), 2.96 (m, 1H), 3.12 (m, 3H), 3.41 (m, 1H), 3.77 (m, 1H), 4.51 (m, 1H), 6.67 (m, 2H), 6.95 (m, 2H), 7.35 (m, 7H), 9.86 (s, 1H); MS (ESI+): m/z = 480.9 [M+H]⁺.

### Example 80: Preparation of 2-[1-(4-fluorophenyl)-2,5-dioxo-3-[2-(thiophen-2-yl)ethyl]imidazolidin-4-yl]-N-(2-methoxypyrimidin-5-yl)acetamide.

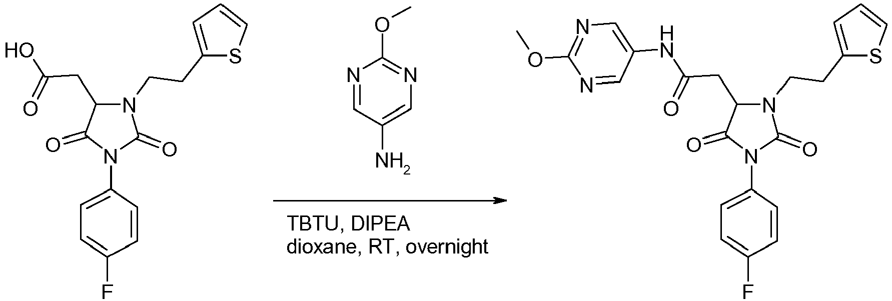

TBTU (265.8 mg; 0.83 mmol; 1.5 eq), followed by DIPEA (288 µL; 1.66 mmol; 3 eq) and after 10 minutes, 2-methoxypyrimidin-5-amine (103.6 mg; 0.83 mmol; 1.5 eq) were added to a solution of 2-[1-(4-fluorophenyl)-2,5-dioxo-3-[2-(thiophen-2-yl)ethyl] imidazolidin-4-yl] acetic acid (1-17) (200 mg; 0.55 mmol; 1 eq) in dioxane/dimethylformamide (3 mL/300 µL). The reaction mixture was stirred at room temperature overnight. Saturated sodium hydrogenocarbonate (15 mL) was added and the aqueous layer was extracted with ethyl acetate (3 x 15 mL). The combined organic layers were washed with saturated sodium chloride (3 x 5 mL), dried over sodium sulfate, filtered and concentrated under reduced pressure. The crude was purified by flash chromatography on silica gel using dichloromethane/methanol (100/0 to 97/3) as an eluent. After washing in hydrochloric acid 1M and trituration in diethyl ether, the title compound, 2-[1-(4-fluorophenyl)-2,5-dioxo-3-[2-(thiophen-2-yl)ethyl] imidazolidin-4-yl]-*N*-(2-methoxypyrimidin-5-yl)acetamide, was obtained in 55% yield (143 mg) as beige solid. ¹H-NMR (CDCl₃): δ (ppm) 2.83 (m, 1H), 2.96 (m, 1H), 3.25 (m, 2H), 3.5 (m, 1H), 4.01 (m, 4H), 4.31 (m, 1H), 6.92 (m, 2H), 7.16 (m, 3H), 7.39 (m, 2H), 7.61 (s, 1H), 8.66 (m, 2H); MS (ESI+): m/z = 469.9 [M+H]⁺.

### Example 46: Preparation of 2-[1-(4-fluorophenyl)-5-oxo-2-sulfanylidene-3-[2-(thiophen-2-yl)ethyl]imidazolidin-4-yl]-N-(4-methoxyphenyl)acetamide.

### a) Ethyl 2-[1-(4-fluorophenyl)-5-oxo-2-sulfanylidene-3-[2-(thiophen-2-yl)ethyl] imidazolidin-4-yl]acetate (I-18)

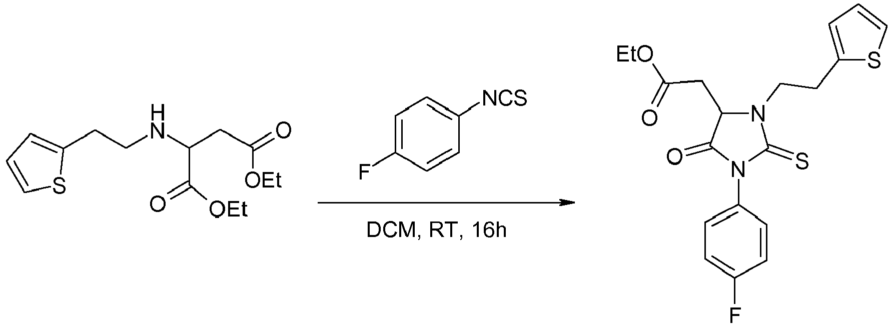

4-Fluorophenyl isothiocyanate (1.12 g; 7.30 mmol; 1.15 eq) was added to a solution of 1,4-diethyl 2-{[2-(thiophen-2-yl)ethyl]amino}butanedioate (1-15) (1.9 g; 6.35 mmol; 1 eq) in dichloromethane (20 mL). The reaction mixture was stirred at room temperature overnight and concentrated to dryness. The crude was purified by flash chromatography on silica gel using cyclohexane/ethyl acetate (100/0 to 80/20) as an eluent. The title compound, 2-[1-(4-fluorophenyl)-5-oxo-2-sulfanylidene-3-[2-(thiophen-2-yl)ethyl] imidazolidin-4-yl]-N-(4-methoxyphenyl)acetamide, was obtained in 99% yield (2.56 g) as a yellow gum. ¹H-NMR (CDCl₃): δ (ppm) 1.27 (t, 3H, J = 7.0Hz), 2.99 (m, 2H), 3.16 (m, 1H), 3.48 (m, 1H), 3.64 (m, 1H), 4.05 (m, 1H), 4.19 (m, 2H), 4.46 (m, 1H), 6.91 (m, 1H), 6.95 (m, 1H), 7.19 (m, 3H), 7.36 (m, 2H).

### b) 2-[1-(4-fluorophenyl)-5-oxo-2-sulfanylidene-3-[2-(thiophen-2-yl)ethyl] imidazolidin-4-yl]acetic acid (I-19)

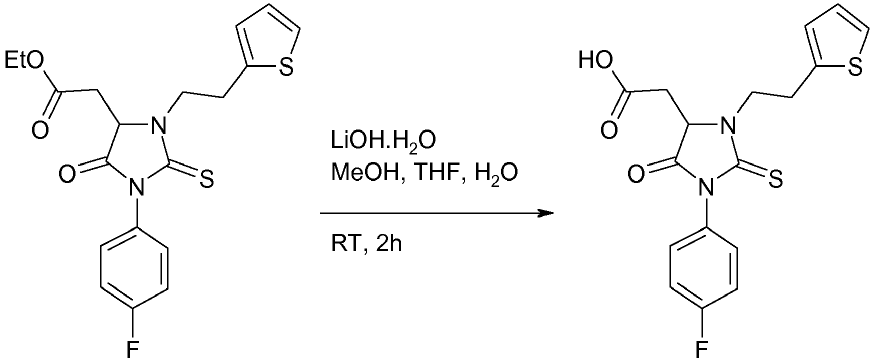

Lithium hydroxide (6 mL; 2.1 M aqueous solution; 12.60 mmol; 2 eq) was added to a **solution of ethyl** 2-[1-(4-fluorophenyl)-2,5-dioxo-3-[2-(thiophen-2yl)ethyl] imidazolidin-4-yl]acetate (I-18) (2.56 g; 6.30 mmol; 1 eq) in tetrahydrofuran (16 mL) and methanol (11 mL). The reaction mixture was stirred at room temperature for 2 hours, then concentrated to dryness. Water (40 mL) and hydrochloric acid 3M were added until pH 2 to afford a yellow gum. The layers were separated. The yellow gum was solubilized in diethyl ether and concentrated to dryness. The title compound, 2-[1-(4-fluorophenyl)-5-oxo-2-sulfanylidene-3-[2-(thiophen-2-yl)ethyl]imidazolidin-4-yl]acetic acid, was obtained in 94% yield (2.24 g) as yellow solid. ¹H-NMR (CDCl₃): δ (ppm) 3.04 (m, 2H,), 3.18 (m, 1H), 3.48 (m, 2H), 3.64 (m, 1H), 4.01 (m, 1H), 4.5 (m, 1H), 7.06 (m, 6H), 7.3 (m, 1H).

### c) 2-[1-(4-fluorophenyl)-5-oxo-2-sulfanylidene-3-[2-(thiophen-2-yl)ethyl]imidazolidin-4-yl]-N-(4-methoxyphenyl)acetamide (example 46)

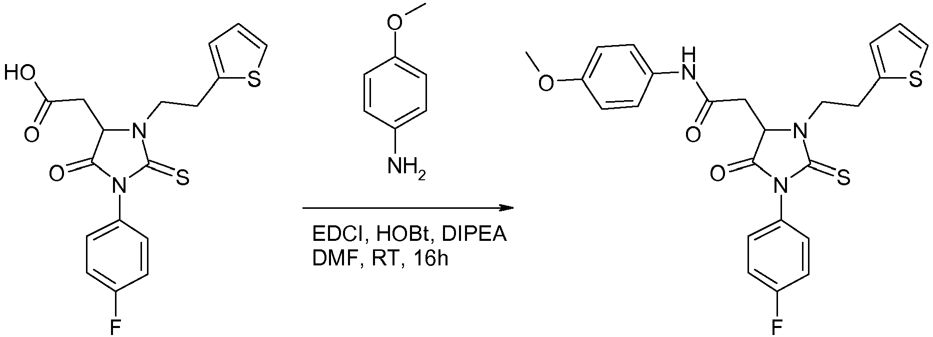

4-Methoxyaniline (62 mg; 0.50 mmol; 1.3), followed by EDCI (114 mg; 0.59 mmol; 1.5 eq), HOBt (90 mg; 0.59 mmol; 1.5 eq) and DIPEA (339 µL; 1.95 mmol; 5 eq) were added to a solution of 2-[1-(4-fluorophenyl)-5-oxo-2-sulfanylidene-3-[2-(thiophen-2-yl)ethyl]imidazolidin-4-yl]acetic acid (I-19) (150 mg; 0.39 mmol; 1 e q) in dimethylformamide (5 mL). The reaction mixture was stirred at room temperature overnight. Water (20 mL) was added and the aqueous layer was extracted with ethyl acetate (2 x 30 mL). The combined organic layers were washed with saturated sodium chloride (3 x 10 mL), dried over sodium sulfate, filtered and concentrated under reduced pressure. The crude was purified on silica gel using dichloromethane/methanol (100/0 to 98/2) as an eluent. The title compound, 2-[1-(4-fluorophenyl)-5-oxo-2-sulfanylidene-3-[2-(thiophen-2-yl)ethyl]imidazolidin-4-yl]-*N*-(4-methoxyphenyl)acetamide, was obtained in 32 % yield (60 mg) as a pale yellow solid. ¹H-NMR (DMSO-d₆): δ (ppm) 3.11 (m, 2H), 3.26 (m, 2H), 3.71 (s, 3H), 3.72 (m, 1H), 4.24 (m, 1H), 4.71 (m, 1H), 6.88 (m, 2H), 6.96 (m, 2H), 7.4 (m, 7H), 10.06 (s, 1H); MS (ESI+): m/z = 483.9 [M+H]⁺.

### Example 48: Preparation of N-[4-(dimethylamino)phenyl]-2-[1-(4-fluorophenyl)-5-oxo-2-sulfanylidene-3-[2-(thiophen-2-yl)ethyl]imidazolidin-4-yl]acetamide.

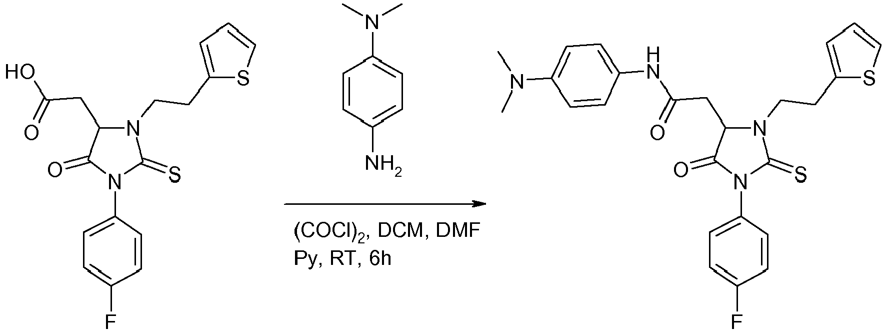

Dimethylformamide (catalytic amount) and oxalyl chloride (104 µL; 1.19 mmol; 3 eq) were added to a solution of 2-[1-(4-fluorophenyl)-5-oxo-2-sulfanylidene-3-[2-(thiophen-2-yl)ethyl]imidazolidin-4-yl]acetic acid (I-19) (150 mg; 0.39 mmol; 1 eq) in dichloromethane (5 mL). The reaction mixture was stirred at room temperature for 2 hours and 30 minutes. Then, 1-*N*,1-*N*-dimethylbenzene-1,4-diamine (106.23 mg; 0.78 mmol; 2 eq) and pyridine (73 µL; 1.17 mmol; 3 eq) were added. The reaction mixture was stirred at room temperature for 3 hours. Water (20 mL), dichloromethane (20 mL) and saturated sodium hydrogenocarbonate until a basic pH were added and the aqueous layer was extracted with dichloromethane (2 x 30 mL). The combined organic layers were washed with saturated ammonium chloride (3 x 10 mL), dried over sodium sulfate, filtered and concentrated under reduced pressure. The crude was purified on silica gel using dichloromethane/ethyl acetate (100/0 to 80/20) as an eluent. After trituration in pentane/dichloromethane, the title compound, *N*-[4-(dimethylamino)phenyl]-2-[1-(4-fluorophenyl)-5-oxo-2-sulfanylidene-3-[2-(thiophen-2-yl)ethyl]imidazolidin-4-yl] acetamide, was obtained in 5% yield (10 mg) as an orange solid. ¹H-NMR (DMSO-d₆): δ (ppm) 2.86 (s, 6H), 3.17 (m, 4H), 3.74 (m, 1H), 4.23 (m, 1H), 4.71 (m, 1H), 6.68 (m, 2H), 6.98 (m, 2H), 7.32 (m, 7H), 9.94 (m, 1H); MS (ESI+): m/z = 496.9 [M+H]⁺.

### Example 81: Preparation of 2-[1-(4-fluorophenyl)-5-oxo-2-sulfanylidene-3-[2-(thiophen-2-yl)ethyl]imidazolidin-4-yl]-N-(2-methoxypyrimidin-5-yl)acetamide.

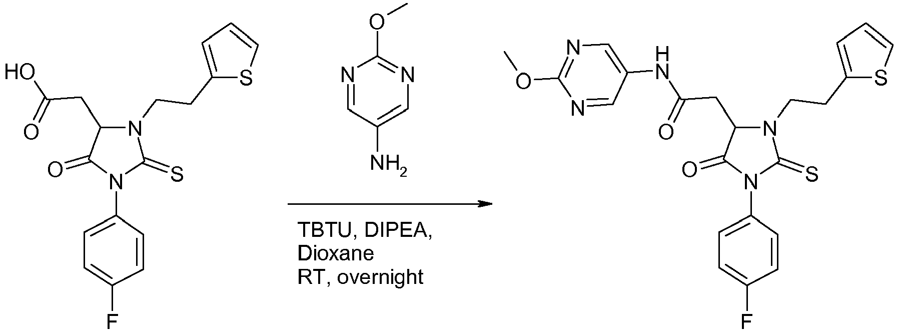

TBTU (254.5 mg; 0.79 mmol; 1.5 eq), followed by DIPEA (276 µL; 1.59 mmol; 3 eq) and after 10 minutes, 2-methoxypyrimidin-5-amine (99.12 mg; 0.79 mmol; 1.5 eq) were added to a solution of 2-[1-(4-fluorophenyl)-5-oxo-2-sulfanylidene-3-[2-(thiophen-2-yl) ethyl]imidazolidin-4-yl]acetic acid (I-19) (200 mg; 0.53 mmol; 1 eq) in dioxane/dimethylformamide (3 mL/300 µL). The reaction mixture was stirred at room temperature overnight. Saturated sodium hydrogenocarbonate (15 mL) was added and the aqueous layer was extracted with ethyl acetate (3 x 15 mL). The combined organic layers were washed with saturated sodium chloride (3 x 5 mL), dried over sodium sulfate, filtered and concentrated under reduced pressure. The crude was purified by flash chromatography on silica gel using dichloromethane/methanol (100/0 to 98/2) as an eluent. After washing with 1M hydrochloric acid and trituration in diethyl ether, the title compound, 2-[1-(4-fluorophenyl)-5-oxo-2-sulfanylidene-3-[2-(thiophen-2-yl) ethyl]imidazolidin-4-yl]-*N*-(2-methoxypyrimidin-5-yl)acetamide, was obtained in 28 % yield (71 mg) as beige solid. ¹H-NMR (DMSO-d₆): δ (ppm) 3.2 (m, 4H), 3.75 (m, 1H), 3.88 (s, 3H), 4.26 (m, 1H), 4.75 (m, 1H), 6.97 (m, 2H), 7.37 (m, 5H), 8.71 (m, 2H), 10.47 (s, 1H); MS (ESI+): m/z = 485.8 [M+H]⁺.

### Example 53: Preparation of 2-[1-(4-fluorophenyl)-3-[2-(4-methoxyphenyl)ethyl]-5-oxo-2-sulfanylideneimidazolidin-4-yl]-N-[4-(2-methoxyethoxy)phenyl]acetamide.

### a) Ethyl 2-[1-(4-fluorophenyl)-3-[2-(4-methoxyphenyl)ethyl]-5-oxo-2-sulfanylideneimidazolidin-4-yl]acetate (I-20).

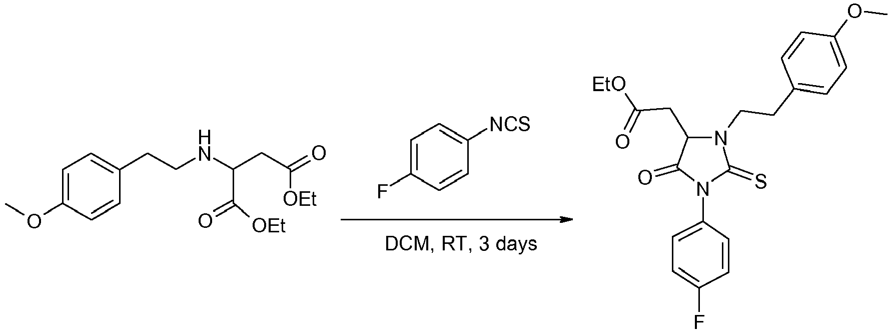

1-Fluoro-4-isothiocyanatobenzene (2.8 g; 18.25 mmol; 1.15 eq) was added to a solution of 1,4-diethyl 2-{[2-(4-methoxyphenyl)ethyl]amino}butanedioate (1-6) (5.13 g; 15.87 mmol; 1 eq) in dichloromethane (50 mL), and the reaction mixture was stirred at room temperature 3 days. Then, the reaction mixture was concentrated to dryness. The crude was purified on silica gel using ethyl acetate/cyclohexane (30/70) as an eluent. The title compound, 2-[1-(4-fluorophenyl)-3-[2-(4-methoxyphenyl)ethyl]-5-oxo-2-sulfanylidene imidazolidin-4-yl]-N-[4-(2-methoxyethoxy)phenyl]acetamide, was obtained in 94% yield (6.53 g) as a white solid. ¹H-NMR (DMSO-d₆): δ (ppm) 1.17 (t, 3H, J = 6 Hz), 2.78 (m, 1H), 2.96 (m, 1H), 3.12 (m, 1H), 3.28 (m, 1H), 3.71 (m, 4H), 4.09 (m, 3H), 4.67 (m, 1H), 6.89 (m, 2H), 7.2 (m, 2H), 7.31 (m, 4H).

### b) 2-[1-(4-fluorophenyl)-3-[2-(4-methoxyphenyl)ethyl]-5-oxo-2-sulfanylidene imidazolidin-4-yl]acetic acid (1-21).

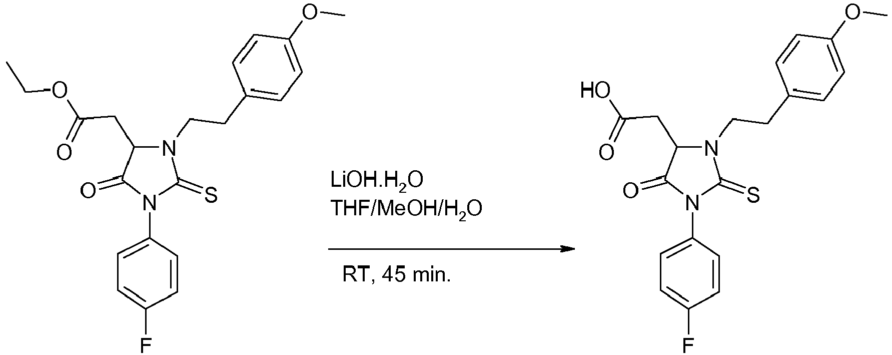

Lithium hydroxide (11 mL; 2.7 M aqueous solution; 30.34 mmol; 2 eq) was added to a solution of ethyl 2-[1-(4-fluorophenyl)-3-[2-(4-methoxyphenyl)ethyl]-5-oxo-2-sulfanylideneimidazo lidin-4-yl] acetate (1-20) (6.53 g; 15.17 mmol; 1 eq) in tetrahydrofuran (38 mL) and methanol (23 mL). The reaction mixture was stirred at room temperature for 45 minutes. Hydrochloric acid 3M (10.5 mL) was added until pH 2. The reaction mixture was evaporated to remove tetrahydrofuran and methanol, then water (50 mL) was added. The aqueous layer was extracted with ethyl acetate (2 x 150 mL). The combined organic layers were washed with saturated sodium chloride (3 x 20 mL), dried over sodium sulfate, filtered and concentrated to dryness. The title compound, 2-[1-(4-fluorophenyl)-3-[2-(4-methoxyphenyl)ethyl]-5-oxo-2-sulfanylideneimidazolidin-4-yl]acetic acid, was obtained in quantitative yield as a yellow foam. ¹H-NMR (DMSO-d₆): δ (ppm) 2.78 (m, 1H), 3.0 (m, 2H), 3.21 (m, 1H), 3.70 (m, 1H), 3.73 (s, 3H), 4.09 (m, 1H), 4.64 (m, 1H), 6.89 (m, 2H), 7.28 (m, 6H), 12.88 (s, 1H).

### c) 2-[1-(4-fluorophenyl)-3-[2-(4-methoxyphenyl)ethyl]-5-oxo-2-sulfanylidene imidazolidin-4-yl]-N-[4-(2-methoxyethoxy)phenyl]acetamide (example 53)

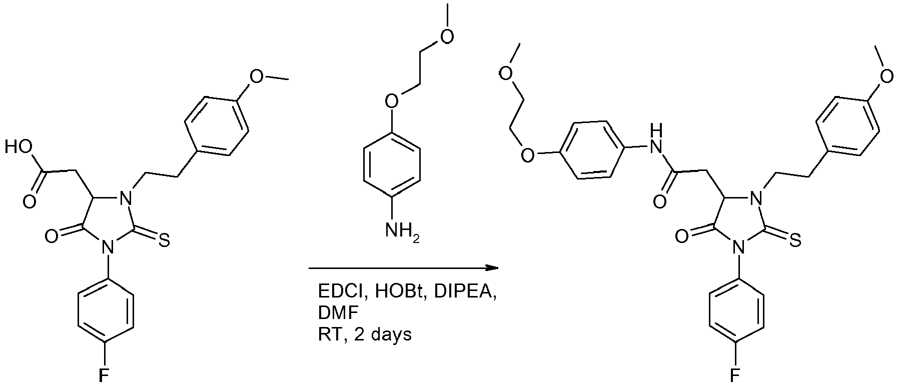

EDCI (107.4 mg; 0.55 mmol; 1.5 eq), followed by HOBt (84 mg; 0.55 mmol; 1.5 eq), DIPEA (323 µL; 1.85 mmol; 5 eq) and 4-(2-methoxyethoxy)aniline (81 mg; 0.48 mmol; 1.3 eq) were added to a solution of 2-[1-(4-fluorophenyl)-3-[2-(4-methoxyphenyl)ethyl]-5-oxo-2-sulfanylideneimidazolidin-4-yl]acetic acid (1-21) (150 mg; 0.37 mmol; 1 eq) in dimethylformamide (5 mL). The reaction mixture was stirred at room temperature for 2 days. Ethyl acetate (10 mL) and water (5 mL) were added and the layers were separated. The organic layer was washed with saturated ammonium chloride (10 mL), water (10 mL) and sodium chloride (2 x 15 mL), dried over sodium sulfate, filtered and concentrated to dryness. The crude was triturated in ethyl acetate, washed with petroleum ether, and concentrated to dryness. The residue was taken up in ethyl acetate/cyclohexane (200 µL) until precipitation, filtration. The title compound, 2-[1-(4-fluorophenyl)-3-[2-(4-methoxyphenyl)ethyl]-5-oxo-2-sulfanylideneimidazolidin-4-yl]-*N*-[4-(2-methoxyethoxy)phenyl]acetamide, was obtained in 25 % yield (50 mg) as a yellow solid. ¹H-NMR (Acetone-d₆): δ (ppm) 2.85 (m, 1H), 3.17 (m, 2H), 3.36 (s, 3H), 3.40 (m, 1H), 3.65 (m, 2H), 3.68 (s, 3H), 3.70 (m, 1H), 4.09 (m, 2H), 4.31 (m, 1H), 4.63 (m, 1H), 6.87 (m, 4H), 7.25 (m, 4H), 7.49 (m, 4H), 9.34 (s, 1H); MS (ESI+): m/z = 551.9 [M+H]⁺.

### Example 77: Preparation of N-[4-(dimethylamino)phenyl]-2-[1-(4-fluorophenyl)-4-hydroxy-3-[2-(4-methoxyphenyl)ethyl]-5-oxo-2-sulfanylideneimidazolidin-4 yl]acetamide.

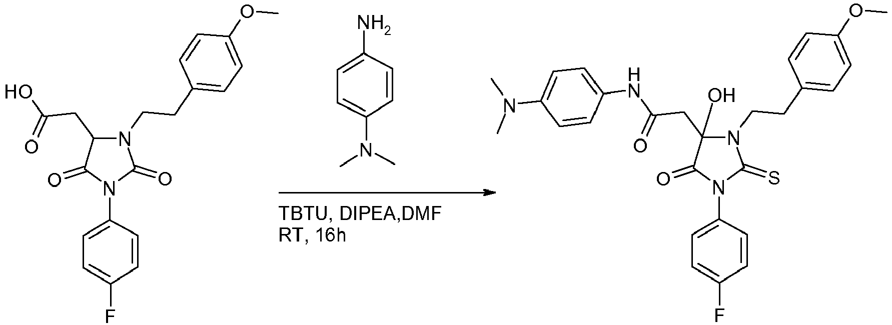

TBTU (262 mg; 0.819 mmol; 1.1 eq) and DIPEA (232 mg; 1.80 mmol; 2.2 eq), followed by 1-*N*,1-*N-*dimethylbenzene-1,4-diamine (1.21 mg; 0.819 mmol; 1.1 eq) were added to a solution of 2-[1-(4-fluorophenyl)-3-[2-(4-methoxyphenyl)ethyl]-2,5-dioxoimidazolidin-4-yl]acetic acid (1-21) (300 mg; 0.745 mmol; 1 e q) in dimethylformamide (3 mL). The reaction mixture was stirred at room temperature overnight. Saturated ammonium chloride (100 mL) was added and the aqueous layer was extracted with ethyl acetate (3 x 50 mL). The combined organic layers were washed with saturated sodium chloride (100 mL), dried over sodium sulfate, filtered and concentrated under reduced pressure. The crude was purified by flash chromatography on silica gel using cyclohexane/ethyl acetate (80/20 to 50/50) as an eluent to afford the title compound N-[4-(dimethylamino)phenyl]-2-[1-(4-fluorophenyl)-4-hydroxy-3-[2-(4-methoxyphenyl)ethyl]-5-oxo-2-sulfanylideneimidazolidin-4-yl]acetamide in 72 % yield (50 mg) as an off-white solid. ¹H-NMR (DMSO-d₆): δ (ppm) 2.83 (s, 6H), 2.95 (m, 2H), 3.25 (m, 2H), 3.73 (s, 3H), 3.78 (m, 2H), 6.67 (d, 2H), 6.91 (d, 2H), 7.25 (t, 4H), 7.38 (m, 4H), 7.67 (s, 1H), 9.94 (s, 1H); MS (ESI+): m/z = 519.3 [(M ― H₂O]+H]⁺.

### Example 86: Preparation of 4-methoxyphenyl 2-[1-(4-fluorophenyl)-3-[2-(4-methoxyphenyl)ethyl]-5-oxo-2-sulfanylideneimidazolidin-4-yl]acetate.

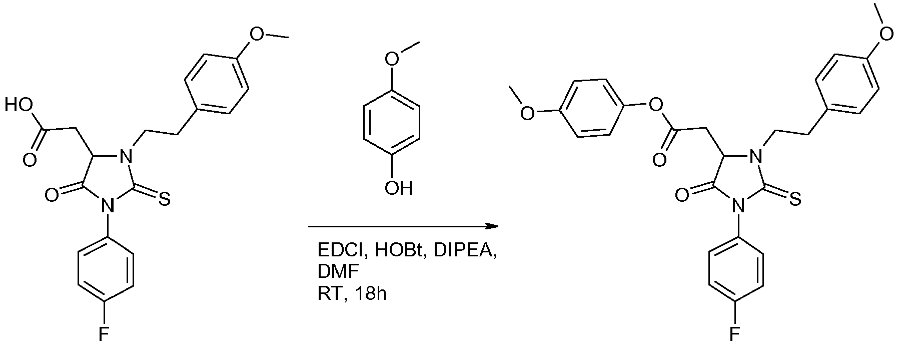

EDCI (112 mg; 0.59 mmol; 1.5 eq), followed by HOBt (89 mg; 0.59 mmol; 1.5 eq), DIPEA (200 µL; 1.17 mmol; 3 eq) and 4-methoxyphenol (97 mg; 0.78 mmol; 2 eq) were added to a solution of 2-[1-(4-fluorophenyl)-3-[2-(4-methoxyphenyl)ethyl]-5-oxo-2-sulfanylideneimidazolidin-4-yl]acetic acid (1-21) (150 mg; 0.39 mmol; 1 eq) in dimethylformamide (6 mL). The reaction mixture was stirred at room temperature for 18 hours. Saturated ammonium chloride (25 mL) was added and the aqueous layer was extracted with ethyl acetate (3 x 40 mL). The combined organic layers were washed with saturated sodium chloride (1 x 25 mL), dried over sodium sulfate, filtered and concentrated to dryness. The crude was purified on silica gel using ethyl acetate/cyclohexane (20/80 to 30/70) as an eluent. After trituration in diethyl ether/ethanol, the title compound, 4-methoxyphenyl 2-[1-(4-fluorophenyl)-3-[2-(4-methoxyphenyl)ethyl]-5-oxo-2-sulfanylideneimidazolidin-4-yl]acetate, was obtained with 35 % yield (92 mg) as a white powder. ¹H-NMR (DMSO-d₆): δ (ppm) 2.84 (m, 1H), 3.01 (m, 1H), 3.52 (m, 2H), 3.73 (s, 6H), 3.74 (m, 1H), 4.16 (m, 1H), 4.82 (m, 1H), 6.96 (m, 6H), 7.29 (m, 6H); MS (ESI+): m/z = 508.9 [M+H]⁺.

### Example 82: Preparation of 2-[1-(4-fluorophenyl)-3-[2-(4-methoxyphenyl)ethyl]-5-oxo-2-sulfanylideneimidazolidin-4-yl]-N-(2-methoxypyrimidin-5-yl)acetamide.

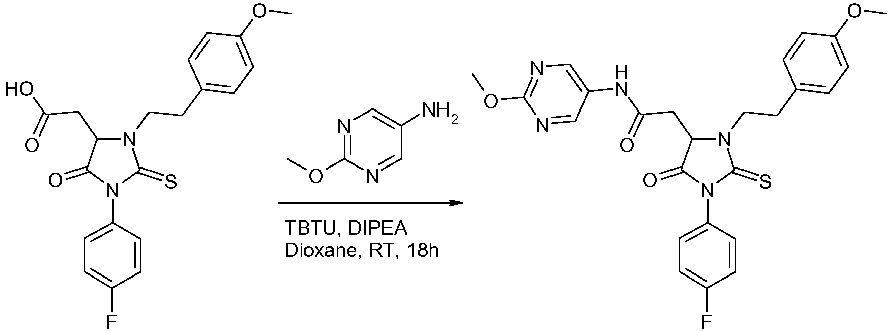

TBTU (244 mg; 0.74 mmol; 1.5 eq) and DIPEA (170 µL; 0.99 mmol; 2 eq) were added to a solution of 2-[1-(4-fluorophenyl)-3-[2-(4-methoxyphenyl)ethyl]-5-oxo-2-sulfanylideneimidazolidin-4-yl]acetic acid (1-21) (200 mg; 0.50 mmol; 1 eq) in anhydrous dioxane (8 mL). The reaction mixture was stirred at room temperature for 30 minutes. 2-Methoxypyrimidin-5-amine (124 mg; 0.99 mmol; 2 eq) was added and the reaction mixture was stirred at room temperature for 18 hours. Saturated ammonium chloride (20 mL) was added and the aqueous layer was extracted with ethyl acetate (2 x 30 mL). The combined organic layers were washed with saturated sodium chloride (1 x 20 mL), filtered and evaporated to dryness. The crude was purified on silica gel using dichloromethane/methanol (98/2) as an eluent. After trituration in diethyl ether, the title compound 2-[1-(4-fluorophenyl)-3-[2-(4-methoxyphenyl)ethyl]-5-oxo-2-sulfanylidene imidazolidin-4-yl]-*N*-(2-methoxypyrimidin-5-yl)acetamide was obtained in 25 % yield (79 mg) as a white solid. ¹H-NMR (Acetone-d₆): δ (ppm) δ 2.96 (m, 1H), 3.09 (m, 1H), 3.24 (m, 1H), 3.43 (m, 1H), 3.75 (s, 3H), 3.78 (m, 1H), 3.92 (s, 3H), 4.30 (m, 1H), 4.65 (s, 1H), 6.86 (m, 2H), 7.25 (m, 4H), 7.45 (m, 2H), 8.75 (m, 2H) 9.62 (m, 1H); MS (ESI+): m/z = 509.9 [M+H]⁺.

### Example 83: Preparation of 2-[1-(4-fluorophenyl)-3-[2-(4-methoxyphenyl)ethyl]-5-oxo-2-sulfanylideneimidazolidin-4-yl]-N-(5-methoxypyridin-2-yl)acetamide.

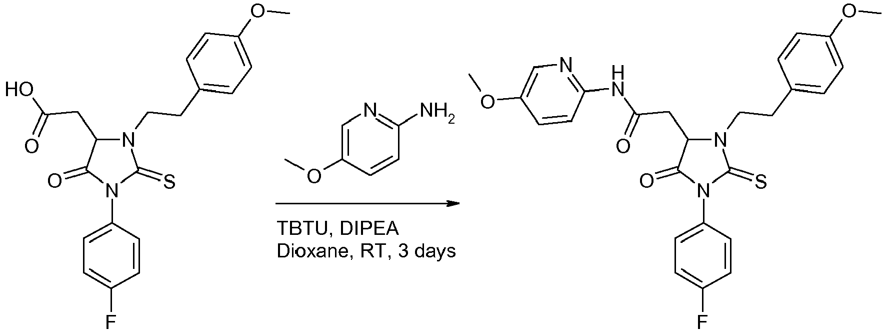

TBTU (244 mg; 0.74 mmol; 1.5 eq) and DIPEA (170 µL; 0.99 mmol; 2 eq) were added to a solution of 2-[1-(4-fluorophenyl)-3-[2-(4-methoxyphenyl)ethyl]-5-oxo-2-sulfanylideneimidazolidin-4-yl]acetic acid (1-21) (200 mg; 0.50 mmol; 1 eq) in anhydrous dioxane (8 mL). The reaction mixture was stirred at room temperature for 30 minutes. 5-methoxypyridin-2-amine (124 mg; 0.99 mmol; 2 eq) in solution in dioxane (2 mL) was added and the reaction mixture was stirred at room temperature for 3 days. Saturated ammonium chloride (30 mL) was added and the aqueous layer was extracted with ethyl acetate (3 x 40 mL). The combined organic layers were washed with saturated sodium chloride (30 mL), filtered and evaporated to dryness. The crude was purified on silica gel using dichloromethane/ethyl acetate (90/10) as an eluent. The residue was triturated with diethyl ether to afford the title compound 2-[1-(4-fluorophenyl)-3-[2-(4-methoxyphenyl)ethyl]-5-oxo-2-sulfanylideneimidazolidin-4-yl]-N-(5-methoxypyridin-2-yl)acetamide in 27% yield (85 mg) as a yellow solid. ¹H-NMR (Acetone-d₆): δ (ppm) 2.98 (m, 1H), 3.11 (m, 1H), 3.28 (m, 1H), 3.5 (m, 1H), 3.74 (s, 3H), 3.78 (m, 1H), 3.84 (s, 3H), 4.32 (m, 1H), 4.65 (m, 1H), 6.86 (m, 2H), 7.25 (m, 4H), 7.38 (m, 1H), 7.45 (m, 2H), 7.96 (s, 1H), 8.11 (m, 1H), 9.6 (m, 1H); MS (ESI+): m/z = 508.9 [M+H]⁺.

### Example 85: Preparation of N-(2-fluoro-4-methoxyphenyl)-2-[1-(4-fluorophenyl)-3-[2-(4-methoxyphenyl)ethyl]-5-oxo-2-sulfanylideneimidazolidin-4-yl]acetamide.

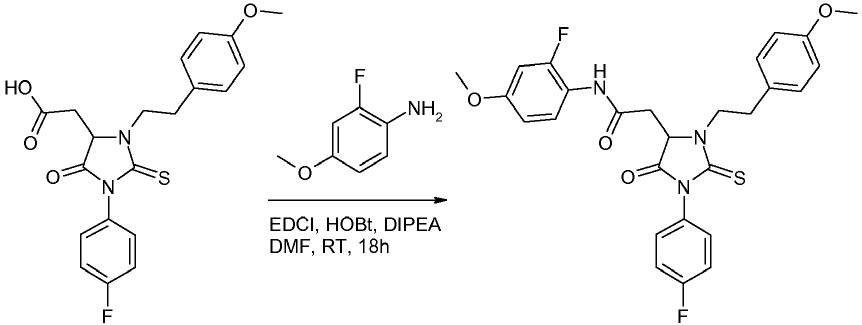

EDCI (143 mg; 0.74 mmol; 2 eq), followed by HOBt (113 mg; 0.74 mmol; 2 eq), DIPEA (322 µL; 1.85 mmol; 5 eq) and 2-fluoro-4-methoxyaniline (84.4 mg; 0.60 mmol; 1.6 eq) were added to a solution of 2-[1-(4-fluorophenyl)-3-[2-(4-methoxyphenyl)ethyl]-5-oxo-2-sulfanylideneimidazolidin-4-yl]acetic acid (1-21) (150 mg; 0.37 mmol; 1 eq) in dimethylformamide (5 mL). The reaction mixture was stirred at room temperature for 18 hours. Saturated ammonium chloride (5 mL) was added and the aqueous layer was extracted with ethyl acetate (3 x 20 mL). The combined organic layers were washed with saturated sodium chloride (3 x 20 mL), dried over sodium sulfate, filtered and concentrated to dryness. The crude was purified on silica gel using dichloromethane/ethyl acetate (95/5) as an eluent. The title compound *N-*(2-fluoro-4-methoxyphenyl)-2-[1-(4-fluorophenyl)-3-[2-(4-methoxyphenyl)ethyl]-5-oxo-2-sulfanylideneimidazolidin-4-yl]acetamide was obtained with 13 % yield (25 mg) as an off-white solid. ¹H-NMR (Acetone-d₆): δ (ppm) 2.83 (m, 1H), 2.98 (m, 1H), 3.12 (m, 1H), 3.38 (m, 1H), 3.62 (s, 3H), 3.66 (s, 3H), 3.70 (m, 1H), 4.17 (m, 1H), 4.5 (m, 1H), 6.61 (m, 2H), 6.73 (m, 2H), 7.18 (m, 4H), 7.27 (m, 2H), 7.73 (m, 1H), 8.97 (s, 1H); MS (ESI+): m/z = 525.9 [M+H]⁺.

### Example 54: Preparation of 2-[1-(4-fluorophenyl)-3-[2-(4-methoxyphenyl)ethyl]-5-oxo-2-sulfanylideneimidazolidin-4-yl]-N-[4-(propan-2-yloxy)phenyl]acetamide.

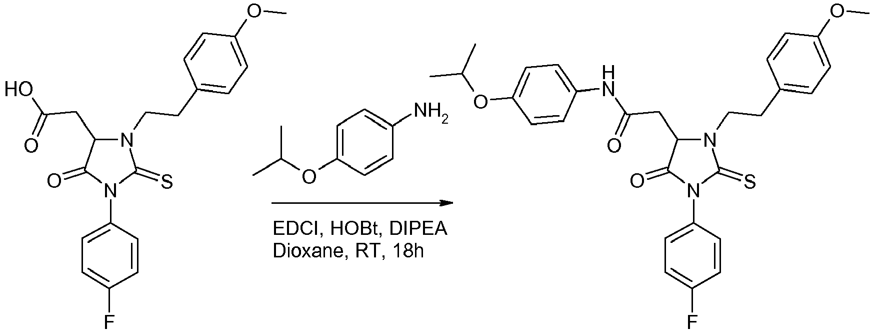

EDCI (143 mg; 0.74 mmol; 1.5 eq), followed by HOBt (113 mg; 0.74 mmol; 1.5 eq), DIPEA (256 µL; 1.49 mmol; 3 eq) and 4-(propan-2-yloxy)aniline (150 mg; 0.99 mmol; 2 eq) were added to a solution of 2-[1-(4-fluorophenyl)-3-[2-(4-methoxyphenyl)ethyl]-5-oxo-2-sulfanylideneimidazolidin-4-yl]acetic acid (I-21) (200 mg; 0.50 mmol; 1 eq) in anhydrous dioxane (8 mL). The reaction mixture was stirred at room temperature for 18 hours. Saturated ammonium chloride (20 mL) was added and the aqueous layer was extracted with ethyl acetate (3 x 25 mL). The combined organic layers were washed with saturated sodium chloride (3 x 20 mL), dried over sodium sulfate, filtered and concentrated to dryness. The crude was purified on silica gel using dichloromethane/ethyl acetate (95/5) as an eluent. After trituration in diethyl ether/ethanol, the title compound 2-[1-(4-fluorophenyl)-3-[2-(4-methoxyphenyl)ethyl]-5-oxo-2-sulfanylideneimidazolidin-4-yl]-*N-*[4-(propan-2-yloxy)phenyl]acetamide was obtained with 43% yield (114 mg) as a white solid. ¹H-NMR (Acetone-d₆): δ (ppm), 1.26 (d, 6H), 2.94 (m, 1H), 3.15 (m, 2H), 3.36 (m, 1H), 3.74 (s, 3H), 3.78 (m, 1H), 4.31 (m, 1H), 4.59 (m, 2H), 6.86 (d, 4H), 7.25 (m, 4H), 7.48 (m, 4H), 9.32 (s, 1H); MS (ESI+): m/z = 535.9 [M+H]⁺.

### Example 84: Preparation of 2-[1-(4-fluorophenyl)-3-[2-(4-methoxyphenyl)ethyl]-5-oxo-2-sulfanylideneimidazolidin-4-yl]-N-(5-methoxypyrimidin-2-yl)acetamide.

### a) 5-Methoxypyrimidin-2-amine (I-22).

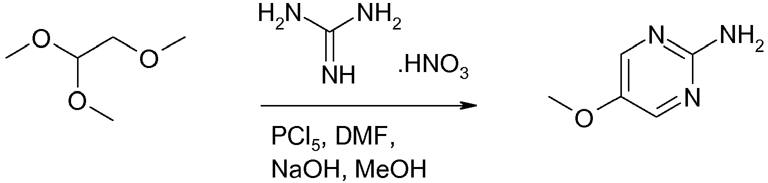

Phosphorus pentachloride (8.07 g; 38.78 mmol; 1 eq) was added portion-wise to methoxyacetaldehyde dimethyl acetal (5 mL; 38.78 mmol; 1 eq) kept at 20°C. The reaction mixture was heated at 60°C for 1 hour and 15 minutes, then cooled down to 0°C, before adding anhydrous dimethylformamide (9 mL; 116.3 mmol; 3 eq) dropwise. The reaction mixture was heated at 70°C for 45 minutes, then cooled at 0°C before adding methanol (40 mL) followed by sodium hydroxide (20.1 g; 504 mmol; 13 eq) and guanidine nitrate (9.46 g; 77.56 mmol; 2 eq). The reaction mixture was stirred at 0°C for 15 minutes. The reaction mixture was allowed to reach at room temperature and methanol was evaporated. The resulting solution was heated at 100°C for 1 hour and 30 minutes. Water (200 mL) and ice was added and the aqueous layer was extracted with dichloromethane (3 x 250 mL). The combined organic layers were washed with saturated sodium chloride (150 mL), dried over sodium sulfate, filtered and concentrated to dryness. The title compound 5-methoxypyrimidin-2-amine was obtained in 53 % yield (2.6 g) as a brown solid. ¹H-NMR (DMSO-d₆): δ (ppm) 3.73 (s, 3H), 6.16 (s, 2H), 8.04 (s, 2H).

### b) 2-[1-(4-Fluorophenyl)-3-[2-(4-methoxyphenyl)ethyl]-5-oxo-2-sulfanylideneimidazolidin-4-yl]-N-(5-methoxypyrimidin-2-yl)acetamide (example 84).

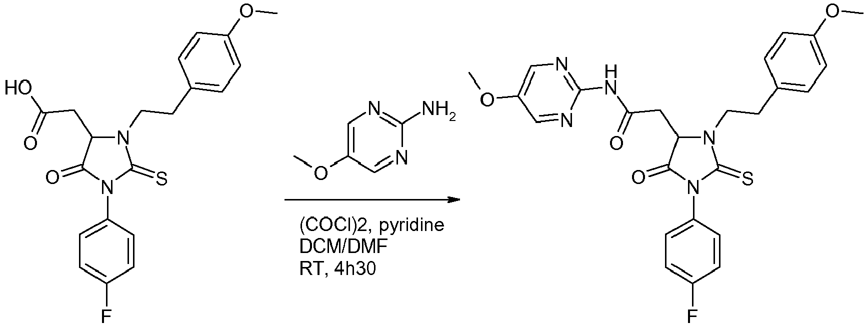

Oxalyl chloride (130 µL; 1.49 mmol; 3 eq) and anhydrous dimethylformamide (catalytic amount) were added dropwise to a solution of 2-[1-(4-fluorophenyl)-3-[2-(4-methoxyphenyl)ethyl]-5-oxo-2-sulfanylideneimidazolidin-4-yl] acetic acid (1-21) (200 mg; 0.50 mmol; 1 eq) in anhydrous dichloromethane (8 mL). The reaction mixture was stirred at room temperature for 2 hours. 5-Methoxypyrimidin-2-amine (1-22) (124 mg; 0.99 mmol; 2 eq) and pyridine (93µL; 1.49 mmol; 3 eq) were added and the reaction mixture was stirred at room temperature for 2 hours and 30 minutes. Water (20 mL) and saturated ammonium chloride (15 mL) were added and the aqueous layer was extracted with ethyl acetate (3 x 40 mL). The combined organic layers were washed with saturated sodium chloride (30 mL), dried over sodium sulfate, filtered and concentrated to dryness. The crude was purified on silica gel using dichloromethane/ethyl acetate (90/10) as an eluent. After trituration in ethanol/diethyl ether, the title compound 2-[1-(4-fluorophenyl)-3-[2-(4-methoxyphenyl)ethyl]-5-oxo-2-sulfanylideneimidazolidin-4-yl]-*N-*(5-methoxypyrimidin-2-yl)acetamide was obtained in 7 % yield (23 mg) as an orange solid. ¹H-NMR (Acetone-d₆): δ (ppm) 2.93 (m, 1H), 3.1 (m, 1H), 3.43 (m, 1H), 3.72 (s, 3H), 3.81 (m, 2H), 3.95 (s, 3H), 4.27 (m, 1H), 4.65 (s, 1H), 6.85 (d, 2H), 7.25 (m, 4H), 7.45 (m, 2H), 8.36 (s, 2H), 9.58 (s, 1H); MS (ESI+): m/z = 509.9 [M+H]⁺.

### Example 50: Preparation of N-[4-(dimethylamino)phenyl]-2-[1-(4-fluorophenyl)-3-[2-(1H-imidazol-4-yl)ethyl]-2,5-dioxoimidazolidin-4-yl]acetamide.

### a) 1,4-diethyl 2-{[2-(1H-imidazol-4-yl)ethyl]amino}butanedioate (I-23).

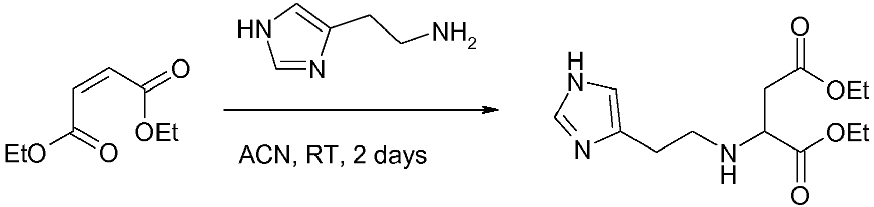

2-(1H-imidazol-4-yl)ethan-1-amine (2.60 g; 23.4 mmol; 2.5 eq) was added to a solution of 1,4-diethyl (2Z)-but-2-enedioate (1.51 mL; 9.35 mmol; 1 eq) in acetonitrile (35 mL). The reaction mixture was stirred at room temperature overnight. The reaction mixture was stirred at 60°C for 2 hours. The layers were separated and the organic layer was concentrated to dryness. The crude was purified by flash chromatography on silica gel using dichloromethane/methanol/30 % ammonium hydroxide (99/0/1 to 95/4/1) as an eluent. The title compound 1,4-diethyl 2-{[2-(1H-imidazol-4-yl)ethyl] amino}butanedioate was obtained in 75 % yield (1.98 g) as a yellow gum. ¹H-NMR (CDCl₃): δ (ppm) 1.27 (m, 6H), 2.61 (m, 1H), 2.75 (m, 4H), 3.02 (m, 1H), 3.69 (m, 1H), 4.18 (m, 4H), 6.79 (s, 1H), 7.52 (s, 1H).

### b) Ethyl 2-[1-(4-fluorophenyl)-3-[2-(1H-imidazol-4-yl)ethyl]-2,5-dioxoimidazolidin-4-yl]acetate (I-24).

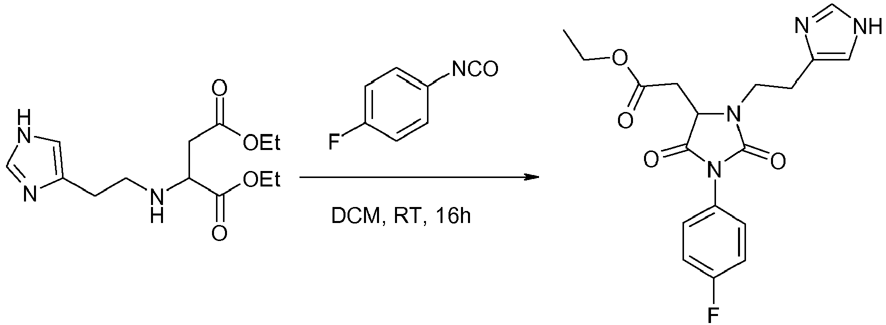

4-Fluorophenyl isocyanate (0.44 mL; 3.91 mmol; 1.15 eq) was added to a solution of 1,4-diethyl 2-{[2-(1H-imidazol-4-yl)ethyl]amino}butanedioate (1-23) (964 mg; 3.40 mmol; 1 eq) in dichloromethane (12 mL). The reaction mixture was stirred at room temperature overnight and concentrated to dryness. The crude was purified on silica gel using dichloromethane/methanol (100% to 90/10) as an eluent. The title compound ethyl 2-[1-(4-fluorophenyl)-3-[2-(1*H-*imidazol-4-yl)ethyl]-2,5-dioxoimidazolidin-4-yl] acetate was obtained in 82% yield (1.05 g) as a white foam. ¹H-NMR (CDCl₃): δ (ppm) 1.23 (m, 3H), 2.90 (m, 2H), 2.98 (m, 2H), 3.71 (m, 1H), 3.95 (m, 1H), 4.13 (m, 2H), 4.20 (m, 1H), 6.78 (m, 1H), 7.13 (m, 2H), 7.36 (m, 2H) 7.51 (m, 1H).

### c) 2-[1-(4-Fluorophenyl)-3-[2-(1H-imidazol-4-yl)ethyl]-2,5-dioxoimidazolidin-4-yl]acetic acid (1-25).

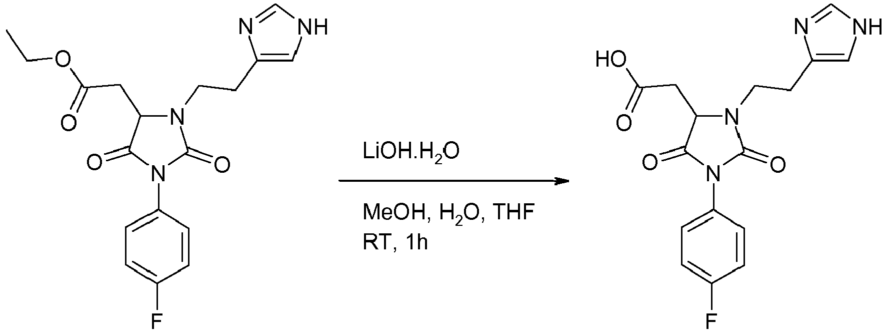

Lithium hydroxide (235 mg; 5.6 mmol; 2 eq) was added to a solution of ethyl 2-[1-(4-fluorophenyl)-3-[2-(1H-imidazol-4-yl)ethyl]-2,5-dioxoimidazolidin-4-yl]acetate (I-24) (1.05 g; 2.80 mmol; 1 eq) in tetrahydrofuran/methanol/water (7 mL/5 mL/2 mL),. The reaction mixture was stirred at room temperature for 1 hour and then, was concentrated to dryness. Water (10 mL) was added followed by 3M HCl until pH 1. The aqueous layers were concentrated to dryness and triturated with a mixture of dichloromethane/methanol (85/15, 150 mL). The salts were filtered and the filtrate was concentrated to dryness. The title compound 2-[1-(4-fluorophenyl)-3-[2-(1*H-*imidazol-4-yl)ethyl]-2,5-dioxoimidazolidin-4-yl]acetic acid was obtained in quantitative yield as a yellow oil. ¹H-NMR (DMSO-d₆): δ (ppm) 2.97 (m, 1H), 3.12 (m, 1H), 3.47 (m, 2H), 3.81 (m, 2H), 4.56 (m, 1H), 7.32 (m, 4H), 7.5 (s, 1H), 9.01 (s, 1H).

### d) N-[4-(Dimethylamino)phenyl]-2-[1-(4-fluorophenyl)-3-[2-(1H-imidazol-4-yl)ethyl]-2,5-dioxoimidazolidin-4-yl]acetamide (example 50).

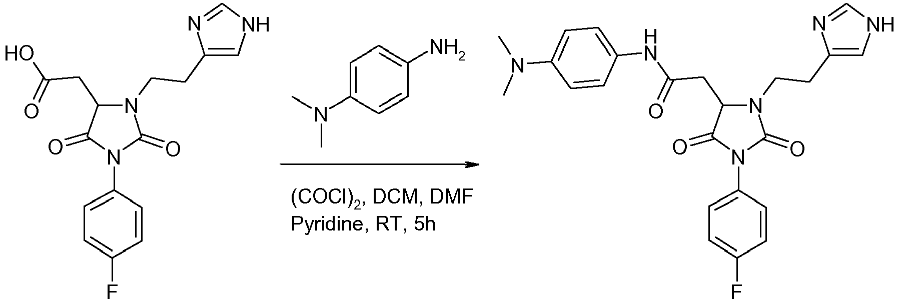

Oxalyl chloride (113 µL; 1.29 mmol; 3 eq) and dimethylformamide (catalytic amount) were added to a suspension of 2-[1-(4-fluorophenyl)-3-[2-(1H-imidazol-4-yl)ethyl]-2,5-dioxoimidazolidin-4-yl] acetic acid (1-25) (150 m g ; 0.43 mmol; 1 eq) in dichloromethane (5 mL). The reaction mixture was stirred at room temperature for 2 hours. A solution of 1-*N*,1-*N-*dimethylbenzene-1,4-diamine (118 mg; 0.86 mmol; 2 eq) in dichloromethane (500 µL) and pyridine (80 µL; 1.29 mmol; 3 eq) were added. The reaction mixture was stirred at room temperature for 3 hours. Water (20 mL) was added and the aqueous layer was extracted with dichloromethane (3 x 20 mL). The combined organic layers were washed with saturated ammonium chloride (3 x 15 mL), dried over sodium sulfate and concentrated to dryness. The crude was purified by flash chromatography on silica gel using ethyl acetate/methanol (100/0 to 85/15) as an eluent. The title compound *N*-[4-(dimethylamino)phenyl]-2-[1-(4-fluorophenyl)-3-[2-(1*H-*imidazol-4-yl)ethyl]-2,5-dioxoimidazolidin-4-yl]acetamide was obtained in 25% yield (51 mg) as an off-white solid. ¹H-NMR (Acetone-d₆): δ (ppm) 2.82 (s, 6H), 2.84 (m, 2H) 2.99 (m, 1H), 3.18 (m, 1H), 3.50 (m, 1H), 3.85 (m, 1H), 4.34 (m, 1H), 6.57 (d, 2H), 6.8 (s, 1H), 7.07 (m, 2H), 7.35 (m, 4H), 7.43 (s, 1H), 9.12 (s, 1H). MS (ESI+): m/z = 465 [M+H]⁺.

### Example 51: Preparation of N-[4-(dimethylamino)phenyl]-2-{3-[2-(4-methoxyphenyl)ethyl]-5-oxo-1-phenyl-2-sulfanylideneimidazolidin-4-yl}acetamide.

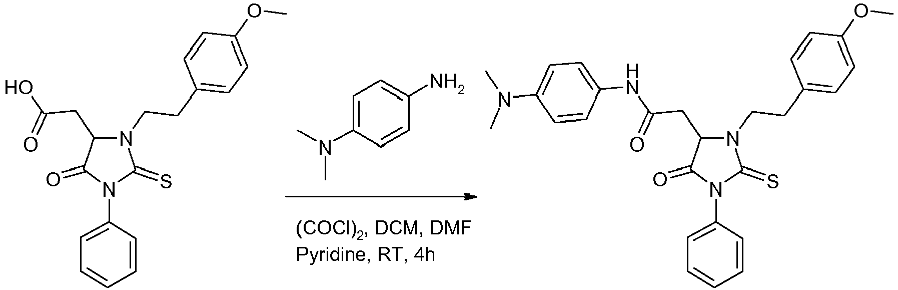

Anhydrous dimethylformamide (catalytic amount) and oxalyl chloride (275 µL; 3.12 mmol; 3 eq) were added to a solution of 2-{3-[2-(4-methoxyphenyl)ethyl]-5-oxo-1-phenyl-2-sulfanylideneimidazolidin-4-yl} acetic acid (1-8) (600 mg; 1.04 mmol; 1 eq) in anhydrous dichloromethane (6 mL). The reaction mixture was stirred at room temperature for 2 hours. Then, 1-*N,*1-N-dimethylbenzene-1,4-diamine (283 mg; 2.08 mmol; 2 eq) and pyridine (194 µL; 3.12 mmol; 3 eq) were added and the reaction mixture was stirred at room temperature for 2 hours. Water (35 mL) and saturated sodium hydrogenocarbonate (25 mL) were added and the aqueous layer was extracted with ethyl acetate (3 x 50 mL). The combined organic layers were washed with saturated sodium chloride (3 x 15 mL), dried over sodium sulfate, filtered and concentrated to dryness. After trituration in diethyl ether/pentane, the title compound *N-*[4-(dimethylamino)phenyl]-2- {3-[2-(4-methoxyphenyl)ethyl]-5-oxo-1-phenyl-2-sulfanylideneimidazolidin-4-yl}acetamide was obtained in 43% yield (27 mg) as a brown solid. ¹H-NMR (DMSO-d₆): δ (ppm) 2.78 (m, 1H), 2.82 (s, 6H), 2.95 (m, 2H), 3.23 (m, 1H), 3.68 (m, 1H), 3.71 (s, 3H), 4.11 (m, 1H), 4.71 (s, 1H), 6.68 (m, 2H), 6.87 (m, 2H) 7.19 (m, 2H), 7.33 (m, 4H), 7.44 (m, 1H), 7.49 (m, 2H), 9.25 (s, 1H); MS (ESI+): m/z = 502.9 [M+H]⁺.

### Example 70: Preparation of N-[4-(dimethylamino)phenyl]-2-{4-hydroxy-3-[2-(4-methoxyphenyl)ethyl]-5-oxo-1-phenyl-2-sulfanylideneimidazolidin-4-yl}acetamide.

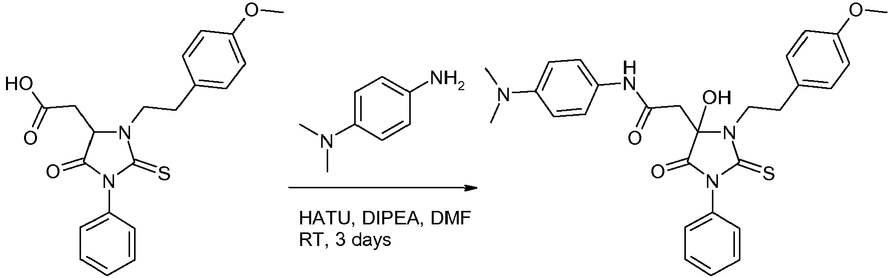

HATU (1.18 g; 3.12 mmol; 1.5 eq), followed by 1-*N*,1-*N-*dimethylbenzene-1,4-diamine (566 mg; 4.16 mmol; 2eq) and DIPEA (668 µL; 4.16 mmol; 2 eq) were added to a solution of 2-{3-[2-(4-methoxyphenyl)ethyl]-5-oxo-1-phenyl-2-sulfanylidene imidazolidin-4-yl}acetic acid (1-8) (800 mg; 2.08 mmol; 1 eq) in anhydrous dimethylformamide (16 mL). The reaction mixture was stirred at room temperature for 3 days. Saturated ammonium chloride (30 mL) was added and the aqueous layer was extracted with ethyl acetate (3 x 50 mL). The combined organic layers were washed with saturated sodium chloride (3 x 40 mL), dried over sodium sulfate and concentrated to dryness. The crude was purified on C₁₈ reversed-phase chromatography using water/acetonitrile (50/50) as eluent. The title compound *N*-[4-(dimethylamino)phenyl]-2- {4-hydroxy-3-[2-(4-methoxyphenyl)ethyl]-5-oxo-1-phenyl-2-sulfanylidene imidazolidin-4-yl}acetamide was obtained in 31 % in yield (373 mg) as a grey solid. ¹H-NMR (DMSO-d₆): δ (ppm) 2.82 (s, 6H), 2.95 (m, 2H), 3.23 (m, 2H), 3.71 (s, 3H), 3.77 (m, 2H), 6.66 (d, 2H), 6.89 (dd, 2H), 7.23 (m, 4H), 7.33 (m, 2H), 7.46 (m, 1H), 7.53 (m, 2H), 7.64 (m, 1H), 9.91 (s, 1H); MS (ESI+): m/z = 500.9 [(M-H₂O)+H]⁺.

### Example 52: Preparation of 2-[1-(4-fluorophenyl)-3-{2-[4-(morpholin-4-yl)phenyl]ethyl}-5-oxo-2-sulfanylideneimidazolidin-4-yl]-N-(4-methoxyphenyl) acetamide.

### a) 2-[4-(Morpholin-4-yl)phenyl]acetonitrile (I-26).

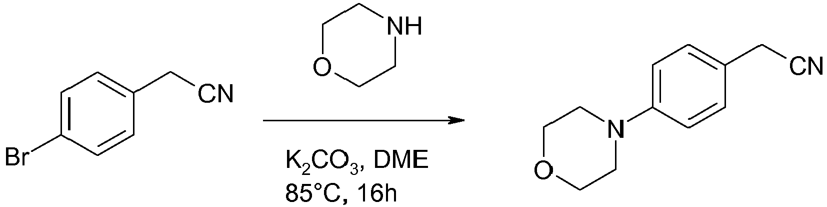

Morpholine (8.7 mL; 99.5 mmol; 1.3 eq) and potassium carbonate (14.8g; 107.1 mmol; 1.4eq) were added to a solution of 2-(4-bromophenyl)acetonitrile (15 g; 76.5 mmol; 1 eq) in dimethoxyethane (200 mL). The reaction mixture was stirred at 85°C overnight. Water (20 mL) was added and the aqueous layer was extracted with dichloromethane (3 x 50 mL). The combined organic layers were dried over sodium sulfate, filtered and concentrated to dryness. The crude was purified on silica gel using cyclohexane/ethyl acetate (80/20 to 60/40) as an eluent. The title compound 2-[4-(morpholin-4-yl)phenyl]acetonitrile was obtained in 20% yield (3.52 g) as a yellow solid. ¹H-NMR (CDCl₃): δ (ppm) 3.14 (m, 4H), 3.65 (s, 2H), 3.83 (m, 4H), 6.87 (d, 2H), 7.20 (d, 2H); MS (ESI+): m/z = 203.1 [M+H]⁺.

### b) 2-[4-(Morpholin-4-yl)phenyl]ethan-1-amine (I-27).

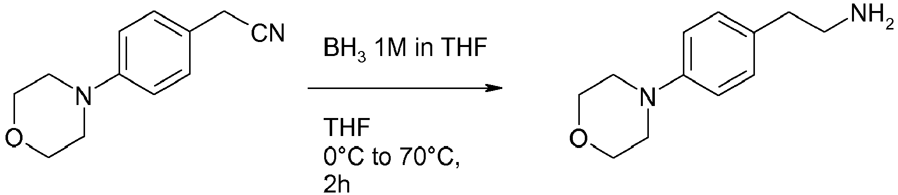

Borane (59 mL, 1M in THF, 59.33 mmol, 6 eq) was added at 0°C to a solution of 2-[4-(morpholin-4-yl)phenyl]acetonitrile (1-26) (2.0 g; 9.89 mmol; 1 eq) in tetrahydrofuran (15 mL). The reaction mixture was stirred at room temperature for 30 minutes, and then heated at 70°C for 2 hours. The reaction mixture was acidified with hydrochloric acid 3M until pH 2 and extracted with ethyl acetate (3 x 20 mL). The aqueous layer was basified with sodium hydroxide (50 mL) until pH 12 and extracted with ethyl acetate (3 x 30 mL). The combined organic layers were washed with water (30 mL), dried over sodium sulfate, filtered and concentrated to dryness. The title compound 2-[4-(morpholin-4-yl)phenyl]ethan-1-amine was obtained in 65% yield (1.32 g) as an orange oil. ¹H-NMR (DMSO-d₆): δ (ppm) 2.53 (m, 2H), 2.68 (m, 2H), 3.05 (m, 4H), 3.72 (m, 4H), 6.85 (m, 2H), 7.05 (m, 2H).

### c) 1,4-Diethyl 2-({2-[4-(morpholin-4-yl)phenyl]ethyl}amino)butanedioate (I-28).

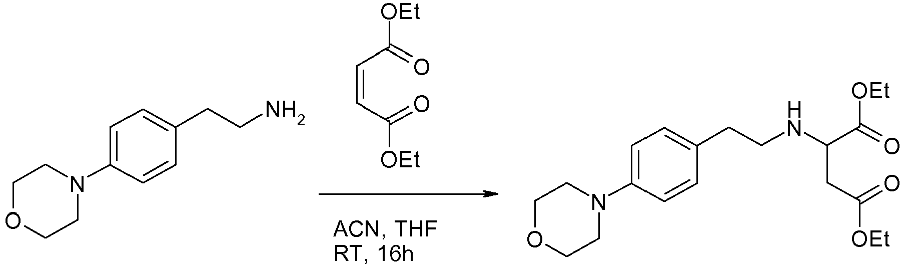

1,4-Diethyl (2Z)-but-2-enedioate (931 µL; 5.75 mmol ; 1 eq) was added to a solution of 2-[4-(morpholin-4-yl)phenyl]ethan-1-amine (I-28) (1.78 g; 8.63 mmol; 1.5 eq) in acetonitrile/tetrahydrofuran (50 mL/2 mL). The reaction mixture was stirred at room temperature overnight. The mixture was concentrated to dryness and was purified by flash chromatography using cyclohexane/ethyl acetate (80/20 to 60/40) as an eluent. The title compound 1,4-diethyl 2-({2-[4-(morpholin-4-yl)phenyl] ethyl}amino)butanedioate was obtained in 57% yield (1.24 g). ¹H-NMR (DMSO-d₆): δ (ppm) 1.16 (m, 6H), 2.09 (m, 1H), 2.59 (m, 4H), 2.73 (m, 1H), 3.03 (m, 4H), 3.34 (m, 1H), 3.54 (m, 1H), 3.72 (m, 4H), 4.05 (m, 4H), 6.84 (d, 2H), 7.04 (d, 2H).

### d) Ethyl 2-[1-(4-fluorophenyl)-3-{2-[4-(morpholin-4-yl)phenyl]ethyl}-5-oxo-2-sulfanylideneimidazolidin-4-yl]acetate (1-29).

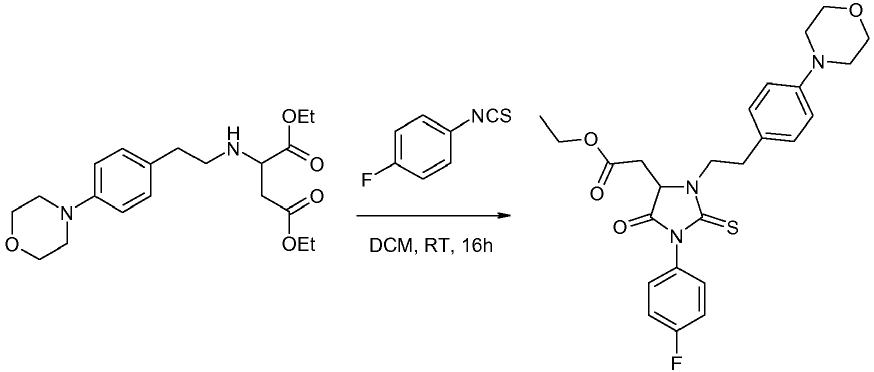

4-Fluoro-isothiocyanate (577 mg; 3.78 mmol; 1.15 eq) was added to a solution of 1,4-diethyl 2-({2-[4-(morpholin-4-yl)phenyl]ethyl}amino)butanedioate (1-29) (1.24 g; 3.28 mmol; 1 eq) in dichloromethane (1.5 mL), and the reaction mixture was stirred at room temperature for 16 hours. Methanol (40 mL) was added to the reaction mixture to obtain a precipitate which was filtered and dried to afford the title compound Ethyl 2-[1-(4-fluorophenyl)-3- {2-[4-(morpholin-4-yl)phenyl]ethyl}-5-oxo-2-sulfanylidene imidazolidin-4-yl]acetate in 81% yield (1.29 g) as a white solid. ¹H-NMR (DMSO-d₆): δ (ppm) 1.16 (m, 3H), 2.74 (m, 1H), 2.94 (m, 1H), 3.06 (m, 4H), 3.12 (m, 1H), 3.28 (m, 1H), 3.68 (m, 1H), 3.72 (m, 4H), 4.12 (m, 2H), 4.67 (m, 1H), 6.90 (m, 2H), 7.16 (m, 2H), 7.31 (m, 4H).

### e) 2-[1-(4-Fluorophenyl)-3-{2-[4-(morpholin-4-yl)phenyl]ethyl}-5-oxo-2-sulfanylideneimidazolidin-4-yl]acetic acid (1-30).

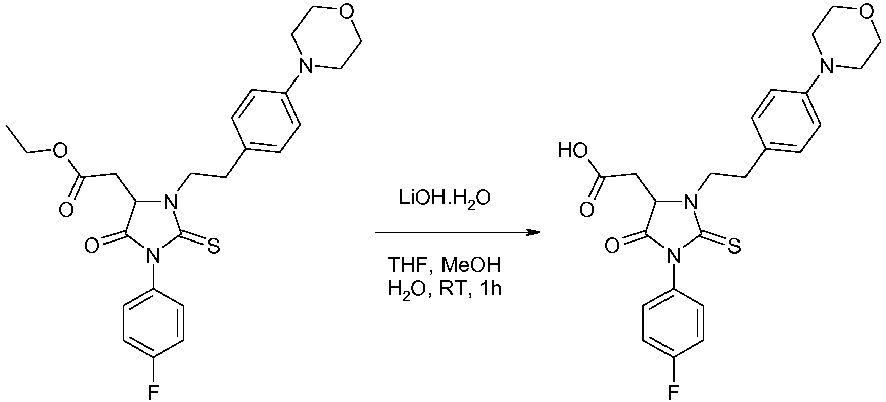

Lithium hydroxide (2.1 mL; 2.5M aqueous solution; 5.27 mmol; 2 eq) was added to a solution of ethyl 2-[1-(4-fluorophenyl)-3-{2-[4-(morpholin-4-yl)phenyl]ethyl}-5-oxo-2-sulfanylideneimidazolidin-4-yl]acetate (1-29) (1.28g; 2.6 mmol; 1eq) in tetrahydrofuran/methanol (6.5 mL/4.3 mL). The reaction mixture was stirred at room temperature for 1 hour. The mixture was concentrated to dryness. Hydrochloric acid 1M was added until pH 2 to obtain a precipitate which was filtered to afford the title compound 2-[1-(4-fluorophenyl)-3-{2-[4-(morpholin-4-yl)phenyl]ethyl}-5-oxo-2-sulfanylideneimidazolidin-4-yl]acetic acid in 83 % yield (1.0 g) as a white solid. 1H-NMR (DMSO-d6): δ (ppm) 2.74 (m, 1H), 2.97 (m, 2H), 3.06 (m, 4H), 3.15 (m, 1H), 3.67 (m, 1H), 3.72 (m, 4H), 4.08 (m, 1H), 4.59 (m, 1H), 6.9 (d, 2H), 7.16 (d, 2H), 7.33 (m, 4H).

### f) 2-[1-(4-Fluorophenyl)-3-{2-[4-(morpholin-4-yl)phenyl]ethyl}-5-oxo-2-sulfanylideneimidazolidin-4-yl]-N-(4-methoxyphenyl)acetamide (example 52).

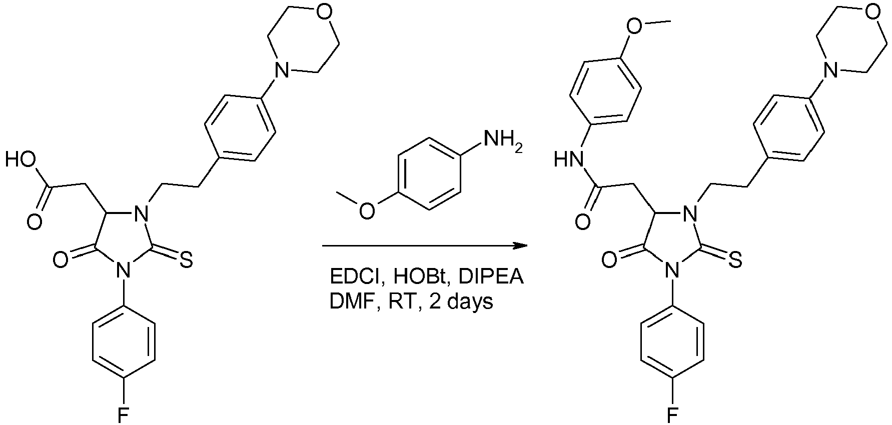

4-Methoxyaniline (61 mg; 0.49 mmol; 1.5 eq), followed by EDCI (94 mg; 0.49 mmol; 1.5 eq), HOBt (75 mg; 0.49 mmol; 1.5 eq) and DIPEA (285 µL; 1.63 mmol; 5 eq) were added to a solution of 2-[1-(4-fluorophenyl)-3-{2-[4-(morpholin-4-yl)phenyl]ethyl}-5-oxo-2-sulfanylideneimidazolidin-4-yl]acetic acid of (1-30) (150 mg; 0.33 mmol; 1 eq) in dimethylformamide (5 mL). The reaction mixture was stirred at room temperature for 2 days. Ethyl acetate (10 mL) and water (5 mL) were added and the aqueous layer was extracted with ethyl acetate (2 x 20 mL). The combined organic layers were washed with saturated sodium chloride (3 x 10 mL), dried over sodium sulfate and concentrated to dryness. The crude was taken up in dichloromethane/diethyl ether and pentane was added. After trituration in ethyl acetate, the title compound 2-[1-(4-fluorophenyl)-3-{2-[4-(morpholin-4-yl)phenyl]ethyl}-5-oxo-2-sulfanylideneimidazolidin-4-yl]-*N*-(4-methoxyphenyl)acetamide was obtained in 34% yield (62.1 mg) as an off-white solid. ¹H-NMR (DMSO-d₆): δ (ppm) 2.76 (m, 1H), 2.95 (m, 1H), 3.05 (m, 4H), 3.12 (m, 1H), 3.24 (m, 1H), 3.66 (m, 1H), 3.72 (m, 7H), 4.09 (m, 1H), 4.71 (m, 1H), 6.88 (m, 4H), 7.14 (m, 2H), 7.4 (m, 5H), 10.09 (s, 1H); MS (ESI+): m/z = 562.9 [M+H]⁺.

### Example 75: Preparation of 2-{3-[2-(4-methoxyphenyl)ethyl]-2-oxo-1-(pyridin-3-yl)-5-sulfanylideneimidazolidin-4-yl}-N-(6-methylpyridazin-3-yl)acetamide.

### a) Ethyl 2-{3-[2-(4-methoxyphenyl)ethyl]-5-oxo-1-(pyridin-3-yl)-2-sulfanylidene imidazolidin-4-yl}acetate (I-31).

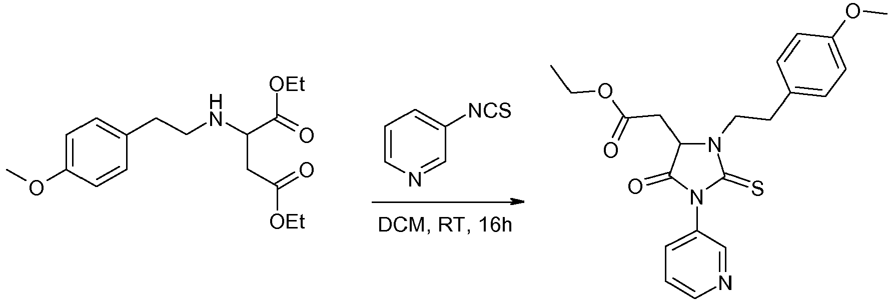

3-Pyridyl isothiocyanate (1.19 mL; 10.67 mmol; 1.15 eq) was added to a solution of 1,4-diethyl 2-{[2-(4-methoxyphenyl)ethyl]amino}butanedioate (3.8 g; 11.75 mmol; 1 eq) in dichloromethane (1-6) (3.0 g; 9.28 mmol; 1 eq) in dichloromethane (10 mL). The reaction mixture was stirred at room temperature overnight. The mixture was concentrated to dryness. The crude was purified on silica gel using cyclohexane/ethyl acetate (90/10 to 40/60) as an eluent. The title compound ethyl 2-{3-[2-(4-methoxyphenyl)ethyl]-5-oxo-1-(pyridin-3-yl)-2-sulfanylideneimidazolidin-4-yl}acetate was obtained in 98.2% (3.81 g) as a white solid. ¹H-NMR (DMSO-d₆): δ (ppm) 1.16 (t, 3H), 2.78 (m, 1H), 2.97 (m, 1H), 3.15 (m, 1H), 3.32 (m, 1H), 3.68 (m 1H), 3.71 (s, 3H), 4.09 (m, 3H), 4.74 (m, 1H), 6.89 (d, 2H), 7.22 (d, 2H), 7.59 (m, 1H), 7.76 (m, 1H), 8.5 (m, 1H), 8.63 (m, 1H).

### b) 2-{3-[2-(4-Methoxyphenyl)ethyl]-5-oxo-1-(pyridin-3-yl)-2-sulfanylidene imidazolidin-4-yl}acetic acid (I-32).

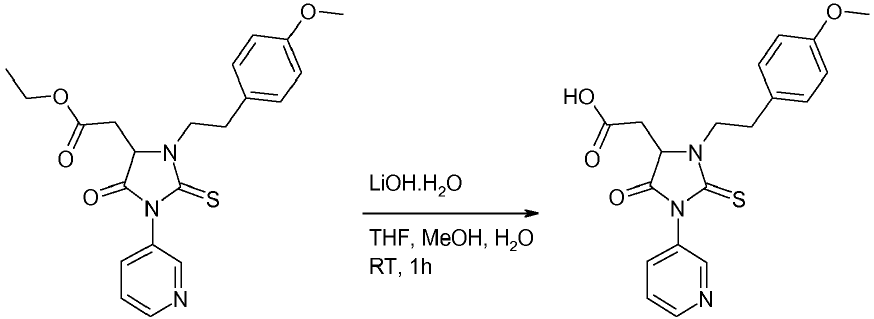

Lithium hydroxide (7 mL; 2.6M aqueous solution; 18.43 mmol; 2eq) was added to a solution of ethyl 2-{3-[2-(4-methoxyphenyl)ethyl]-5-oxo-1-(pyridin-3-yl)-2-sulfanylideneimidazolidin-4-yl}acetate (I-31) (3.81 g; 9.21 mmol; 1 eq) in tetrahydrofuran/methanol (25 mL/16 mL). The reaction mixture was stirred at room temperature for 20 minutes. The mixture was concentrated to dryness. Hydrochloric acid 1M and water was added until pH 1 to obtain a precipitate which was filtered to afford the title compound 2-{3-[2-(4-methoxyphenyl)ethyl]-5-oxo-1-(pyridin-3-yl)-2-sulfanylideneimidazolidin-4-yl}acetic acid in 93% yield (3.31 g) as a white solid. ¹H-NMR (DMSO-d₆): δ (ppm) 2.79 (m, 1H), 3.02 (m, 2H), 3.24 (m, 1H), 3.69 (m, 1H), 3.73 (s, 3H), 4.12 (m, 1H), 4.7 (m, 1H), 6.9 (d, 2H), 7.23 (d, 2H), 7.58 (m, 1H), 7.74 (m, 1H), 8.49 (m, 1H), 8.63 (m, 1H), 12.9 (s, 1H).

### c) 2-{3-[2-(4-Methoxyphenyl)ethyl]-2-oxo-1-(pyridin-3-yl)-5-sulfanylidene imidazolidin-4-yl}-N-(6-methylpyridazin-3-yl)acetamide (example 75).

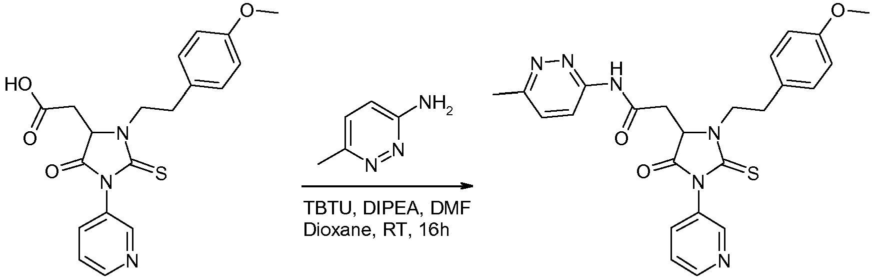

TBTU (383 mg; 1.17 mmol; 1.5 eq) and DIPEA (271 µL; 1.56 mmol; 2 eq) were added to a suspension of 2-{3-[2-(4-methoxyphenyl)ethyl]-5-oxo-1-(pyridin-3-yl)-2-sulfanylideneimidazolidin-4-yl}acetic acid (1-32) (300 mg; 0.78 mmol; 1eq) in dioxane (10 mL). The reaction mixture was stirred at room temperature for 20 minutes. Then, 6-methylpyridazin-3-amine (128 mg; 1.17 mmol; 1.5 eq) and dimethylformamide (0.3 mL) were added and the reaction mixture was stirred at room temperature overnight. Saturated ammonium chloride (20 mL) and ethyl acetate (20 mL) were added. The organic layer was washed with water (20 mL), then with a saturated solution of sodium chloride (20 mL) and dried over sodium sulfate. After concentration to dryness, the crude was triturated in ethyl acetate to give the title compound 2-{3-[2-(4-Methoxyphenyl)ethyl]-2-oxo-1-(pyridin-3-yl)-5-sulfanylideneimidazolidin-4-yl}-*N*-(6-methylpyridazin-3-yl)acetamide in 59% yield (217 mg) as a white solid. ¹H-NMR (DMSO-d₆): δ (ppm) 2.54 (s, 3H), 2.82 (m, 1H), 2.98 (m, 1H), 3.27 (m, 1H), 3.49 (m, 1H), 3.71 (s, 3H), 3.75 (m, 1H), 4.13 (m, 1H), 4.83 (m, 1H), 6.87 (d, 2H), 7.2 (d, 2H), 7.57 (m, 2H), 7.8 (m, 1H), 8.13 (m, 1H), 8.55 (m, 1H), 8.63 (m, 1H), 11.34 (s, 1H); MS (ESI+): m/z = 476.9 [M+H]⁺.

### Example 76: Preparation of 2-{3-[2-(4-methoxyphenyl)ethyl]-2-oxo-1-(pyridin-3-yl)-5-sulfanylideneimidazolidin-4-yl}-N-(6-methoxypyridazin-3-yl)acetamide.

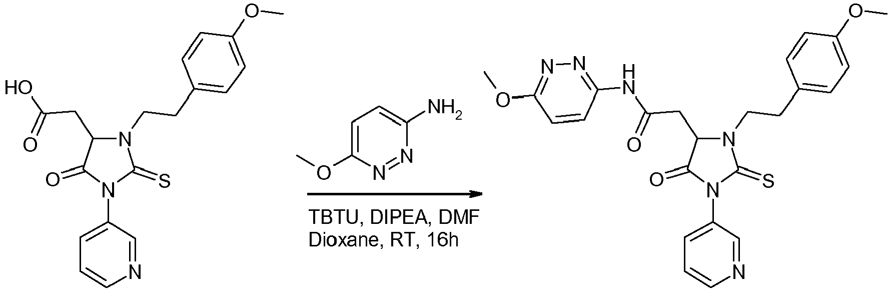

TBTU (383 mg; 1.17 mmol; 1.5 eq) and DIPEA (271 µL; 1.56 mmol; 2 eq) were added to a suspension of 2-{3-[2-(4-methoxyphenyl)ethyl]-5-oxo-1-(pyridin-3-yl)-2-sulfanylideneimidazolidin-4-yl}acetic acid (1-32) (300 mg; 0.78 mmol; 1eq) in dioxane (10 mL). The reaction mixture was stirred at room temperature for 20 minutes. Then, 6-methoxypyridazin-3-amine (145 mg; 1.17 mmol; 1.5 eq) and dimethylformamide (0.3 mL) were added and the reaction mixture was stirred at room temperature overnight. Saturated ammonium chloride (20 mL) and ethyl acetate (20 mL) were added. The organic layer was separated, washed with water (20 mL), then with a saturated solution of sodium chloride (20 mL) and dried over sodium sulfate After concentration to dryness, the crude was triturated in ethyl acetate to give the title compound the title compound 2-{3-[2-(4-methoxyphenyl)ethyl]-2-oxo-1-(pyridin-3-yl)-5-sulfanylideneimidazolidin-4-yl}-*N-*(6-methoxypyridazin-3-yl)acetamide in 42% yield (168 mg) as a white solid. ¹H-NMR (Acetone-d₆): δ (ppm) 2.99 (m, 1H), 3.14 (m, 1H), 3.39 (m, 1H), 3.63 (m, 1H), 3.75 (s, 3H), 3.86 (m, 1H), 4.01 (s, 3H), 4.34 (m, 1H), 4.73 (m, 1H), 6.87 (d, 2H), 7.16 (d, 1H), 7.24 (d, 2H), 7.53 (m, 1H), 7.83 (m, 1H), 8.33 (m, 1H), 8.63 (m, 2H), 10.19 (s, 1H); MS (ESI+): m/z = 492.9 [M+H]⁺.

### Example 55: Preparation of N-(4-methoxyphenyl)-2-{3-[2-(4-methoxyphenyl)ethyl]-5-oxo-1-(pyridin-4-yl)-2-sulfanylideneimidazolidin-4-yl}acetamide.

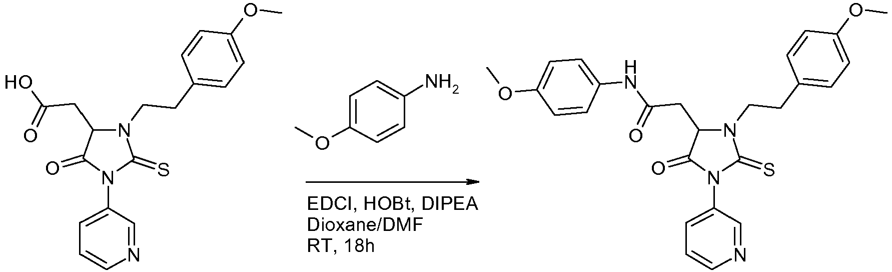

4-Methoxyaniline (128 mg; 1.04 mmol; 2 eq), followed by EDCI (149 mg; 0.78 mmol; 1.5 eq), HOBt (119 mg; 0.78 mmol; 1.5 eq) and DIPEA (266 µL; 1.56 mmol ; 3 eq) were added to a solution of 2-{3-[2-(4-methoxyphenyl)ethyl]-5-oxo-1-(pyridin-3-yl)-2-sulfanylideneimidazolidin-4-yl}acetic acid (I-32) (200 mg; 0.52 mmol; 1eq) in anhydrous dioxane/dimethylformamide (8 mL/2 mL). The reaction mixture was stirred at room temperature for 18 hours. Saturated ammonium chloride (45 mL) was added and the aqueous layer was extracted with ethyl acetate (3 x 30 mL). The combined organic layers were washed with saturated sodium chloride (2 x 20 mL), dried over sodium sulfate and concentrated to dryness. The crude was purified on silica gel using dichloromethane/methanol (98/2) as an eluent. After trituration in diethyl ether/ethanol, the title compound *N*-(4-methoxyphenyl)-2-{3-[2-(4-methoxyphenyl)ethyl]-5-oxo-1-(pyridin-4-yl)-2-sulfanylideneimidazolidin-4-yl}acetamide was obtained in 62 % yield (157 mg) as a white powder. ¹H-NMR (Acetone-d₆): δ (ppm) 2.95 (m, 1H), 3.13 (m, 1H), 3.22 (m, 1H), 3.38 (m, 1H), 3.75 (s, 6H), 3.81 (m, 1H), 4.32 (m, 1H), 4.7 (s, 1H), 6.87 (m, 4H), 7.24 (m, 2H), 7.53 (m, 3H), 7.84 (d, 1H), 8.64 (m, 2H), 9.35 (s, 1H); MS (ESI+): m/z = 490.9 [M+H]⁺.

### Example 47: Preparation of 2-(3-{2-[4-(dimethylamino)phenyl]ethyl}-1-(4-fluorophenyl)-5-oxo-2-sulfanylideneimidazolidin-4-yl)-N-(4-methoxyphenyl)acetamide.

### a) N,N-Dimethyl-4-[(E)-2-nitroethenyl]aniline (1-33).

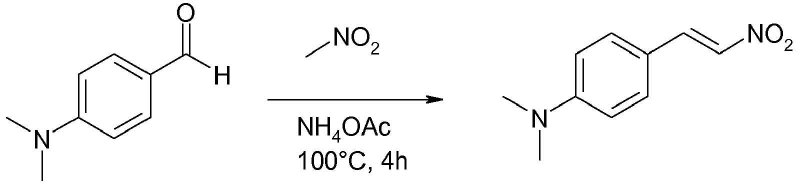

Ammonium acetate (7.23 g; 93.8 mmol; 1.4 eq) in nitromethane (520 mL) was added to a suspension of 4-(dimethylamino)benzaldehyde (10 g; 67.0 mmol; 1 eq) and. The reaction mixture was stirred at 100°C for 4 hours and then, concentrated to dryness. The solid was taken up in dichloromethane and washed with water (50 mL). The organic layer was dried over sodium sulfate, filtered and concentrated to dryness. The title compound *N,N*-dimethyl-4-[(E)-2-nitroethenyl]aniline was obtained in 92% yield (11.85 g) as a red solid. ¹H-NMR (DMSO-d₆): δ (ppm) 3.03 (s, 6H), 6.75 (d, 2H), 7.68 (d, 2H), 8.01 (m, 2H).

### b) 4-(2-Aminoethyl)-N,N-dimethylaniline (1-34).

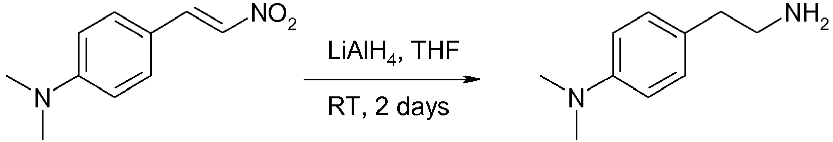

A solution of *N,N*-dimethyl-4-[(E)-2-nitroethenyl]aniline (1-33) (3 g; 15.6 mmol; 1 eq) in anhydrous tetrahydrofuran (120 mL) was added to a suspension of lithium aluminum hydride (1.45 g; 39 mmol; 2.5 eq) in anhydrous tetrahydrofuran (30 mL). The reaction mixture was stirred at room temperature for 2 days. Ethyl acetate (80 mL) and ethanol (40 mL) were added to the reaction mixture followed by 1M sodium hydroxide (80 mL). The organic layer was washed with saturated sodium chloride, dried over sodium sulfate, filtered and concentrated to dryness. The title compound 4-(2-Aminoethyl)-*N,N-*dimethylaniline was obtained in a quantitative yield as a yellow oil. ¹H-NMR (DMSO-d₆): δ (ppm) 2.5 (m, 1H), 2.68 (m, 1H), 2.78 (m, 2H), 2.83 (s, 6H), 6.65 (m, 2H), 7.0 (m, 2H).

### c) 1,4-Diethyl 2-({2-[4-(dimethylamino)phenyl]ethyl}amino)butanedioate (I-35).

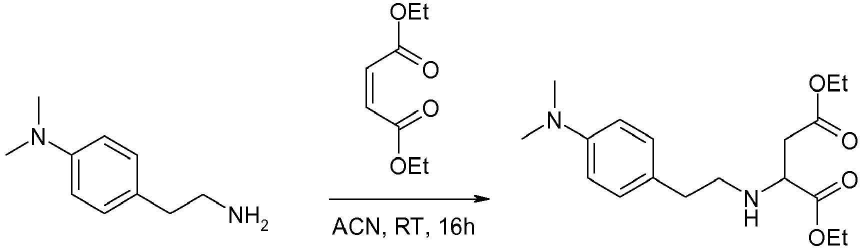

1,4-diethyl (2Z)-but-2-enedioate (6.57 mL; 40.6 mmol; 1 eq) was added to a solution of 4-(2-aminoethyl)-*N,N-*dimethylaniline (1-34) (10 g; 60.9 mmol; 1.5 eq) in acetonitrile (200 mL). The reaction mixture was stirred at room temperature for 16 hours. The reaction mixture was concentrated to dryness and the residue was purified by flash chromatography on silica gel using (80/20 to 60/40) as an eluent. The column was washed with ethyl acetate/methanol/ammoniac (89/10/1). The title compound 1,4-diethyl 2-({2-[4-(dimethylamino)phenyl]ethyl}amino)butanedioate was obtained in 32% yield (6.55 g) as an orange oil. ¹H-NMR (DMSO-d₆): δ (ppm) 1.16 (m, 6H), 2.05 (m, 1H), 2.59 (m, 4H), 2.72 (m, 1H), 2.83 (s, 6H), 2.85 (m, 1H), 3.54 (m, 1H), 4.06 (m, 4H), 6.64 (m, 2H), 6.99 (m, 2H).

### d) Ethyl 2-(3- {2-[4-(dimethylamino)phenyl]ethyl}-1-(4-fluorophenyl)-5-oxo-2-sulfanylideneimidazolidin-4-xl)acetate (I-36).

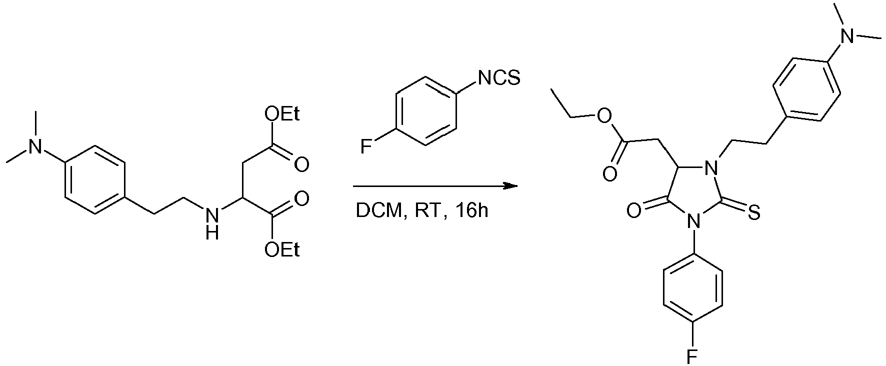

4-Fluoro-phenylisothiocyanate (1.73 g; 11.3 mmol; 1.15 eq) was added to a solution of 1,4-diethyl 2-({2-[4-(dimethylamino)phenyl]ethyl}amino)butanedioate (1-35) (3.3 g; 9.81 mmol; 1 eq) in dichloromethane (20 mL), and the reaction mixture was stirred at room temperature for 16 hours. The reaction mixture was concentrated to dryness and the residue was purified by flash chromatography on silica gel using cyclohexane/ethyl acetate (80/20 to 60/40) as an eluent. The title compound ethyl 2-(3-{2-[4-(dimethylamino)phenyl]ethyl}-1-(4-fluorophenyl)-5-oxo-2-sulfanylideneimidazolidin-4-yl)acetate was obtained in 84% yield (3.89 g) as a dark brown oil. ¹H-NMR (DMSO-d₆): δ (ppm) 1.16 (t, 3H), 2.69 (m, 1H), 2.85 (s, 6H), 2.91 (m, 1H), 3.12 (m, 1H), 3.29 (m, 1H), 3.67 (m, 1H), 4.08 (m, 3H), 4.66 (m, 1H), 6.69 (d, 2H), 7.11 (d, 2H), 7.35 (m, 4H).

### e) 2-(3-{2-[4-(Dimethylamino)phenyl]ethyl}-1-(4-fluorophenyl)-5-oxo-2-sulfanylideneimidazolidin-4-yl)acetic acid (1-37).

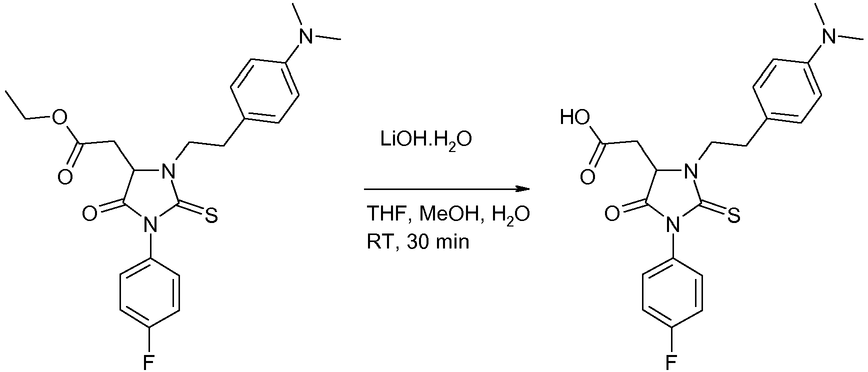

Lithium hydroxide (7 mL; 2.4M aqueous solution; 16.7 mmol; 2eq) was added to a solution of ethyl 2-(3- {2-[4-(dimethylamino)phenyl]ethyl}-1-(4-fluorophenyl)-5-oxo-2-sulfanylideneimidazolidin-4-yl)acetate (I-36) (3.7 g; 8.34 mmol; 1 eq) in tetrahydrofuran/methanol (25 mL/16 mL), and the reaction mixture was stirred at room temperature for 30 minutes. 1M Hydrochloric acid was added to the reaction mixture until pH 7. The aqueous layer was extracted with ethyl acetate (3 x 20 mL). The combined organic layers were washed with saturated sodium chloride (3 x 20 mL), dried over sodium sulfate, filtered and concentrated to dryness. The title compound 2-(3- {2-[4-(dimethylamino)phenyl]ethyl}-1-(4-fluorophenyl)-5-oxo-2-sulfanylidene imidazolidin-4-yl)acetic acid was obtained in 53% yield (1.88 g) as a beige solid. ¹H-NMR (DMSO-d₆): δ (ppm) 2.69 (m, 1H), 2.85 (s, 6H), 2.87 (m, 2H), 3.19 (m, 1H), 3.65 (m, 1H), 4.09 (m, 1H), 4.6 (m, 1H), 6.69 (d, 2H), 7.11 (d, 2H), 7.33 (m, 4H).

### f) 2-(3-{2-[4-(Dimethylamino)phenyl]ethyl}-1-(4-fluorophenyl)-5-oxo-2-sulfanylideneimidazolidin-4-yl)-N-(4-methoxyphenyl)acetamide (example 47).

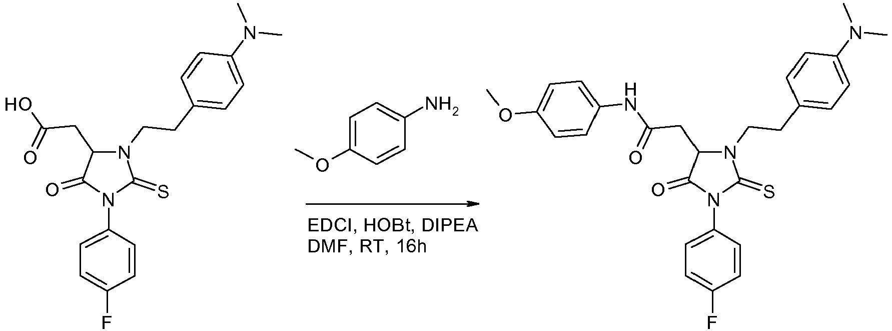

4-Methoxyaniline (58 mg; 0.47 mmol; 1.3 eq), followed by EDCI (103.5 mg; 0.54 mmol; 1.5 eq), HOBt (83 mg; 0.54 mmol; 1.5 eq) and DIPEA (313 µL; 1.80 mmol; 5 eq) were added to a solution of 2-(3-{2-[4-(dimethylamino)phenyl]ethyl}-1-(4-fluorophenyl)-5-oxo-2-sulfanylideneimidazolidin-4-yl)acetic acid (1-37) (150 mg; 0.36 mmol; 1 eq) in dimethylformamide (5 mL). The reaction mixture was stirred at room temperature overnight. Water (10 mL) and ethyl acetate (10 mL) was added and the aqueous layer was extracted with ethyl acetate (3 x 20 mL). The combined organic layer were dried over sodium sulfate, filtered and concentrated to dryness. After trituration in diethyl ether, the title compound 2-(3-{2-[4-(dimethylamino)phenyl]ethyl}-1-(4-fluorophenyl)-5-oxo-2-sulfanylideneimidazolidin-4-yl)-N-(4-methoxyphenyl)acetamide was obtained in 30% yield (55.1 mg) as an off-white solid. ¹H-NMR (DMSO-d₆): δ (ppm) 2.75 (m, 1H), 2.88 (s, 6H), 2.92 (m, 1H), 3.1 (d, 1H), 3.24 (d, 1H), 3.63 (m, 1H), 3.70 (s, 3H), 4.08 (m, 1H), 4.69 (m, 1H), 6.69 (d, 2H), 6.91 (d, 2H), 7.09 (d, 2H), 7.46 (m, 6H), 10.1 (s, 1H); MS (ESI+): m/z = 520.9 [M+H]⁺.

### Example 79: Preparation of 2-(3-{2-[4-(dimethylamino)phenyl]ethyl}-1-(4-fluorophenyl)-5-oxo-2-sulfanylideneimidazolidin-4-yl)-N-(2-methoxypyrimidin-5-yl)acetamide.

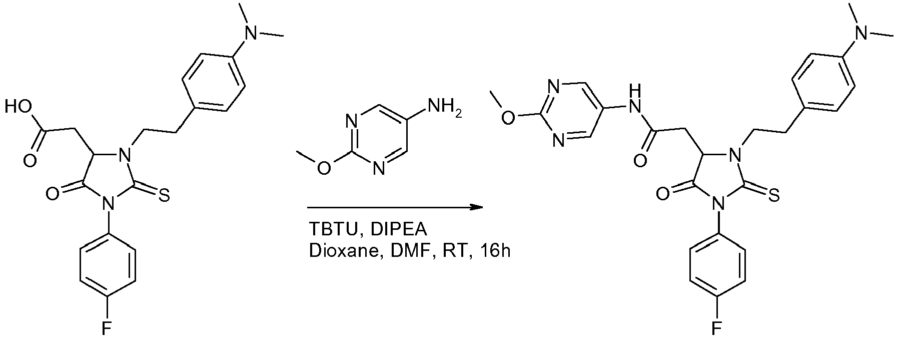

TBTU (177.2 mg; 0.54 mmol; 1.5 eq) and DIPEA (126 µL; 0.72 mmol; 2 eq) were added to a solution of 2-(3- {2-[4-(dimethylamino)phenyl]ethyl}-1-(4-fluorophenyl)-5-oxo-2-sulfanylideneimidazolidin-4-yl)acetic acid (1-37) (150 mg; 0.36 mmol; 1 eq) in dioxane (6 mL). The reaction mixture was stirred at room temperature for 30 minutes. A solution of 2-methoxypyrimidin-5-amine (90.3 mg; 0.72 mmol; 2 eq) in dimethylformamide (2 mL) was added and the reaction mixture was stirred at room temperature overnight. The mixture was concentrated to dryness. Saturated ammonium chloride was added (15 mL) and the aqueous layer was extracted with ethyl acetate (3 x 20 mL). The combined organic layers were washed with saturated sodium chloride (3 x 20 mL), dried over sodium sulfate and concentrated to dryness. The crude was purified by flash chromatography on silica gel using cyclohexane/ethyl acetate (25/75 to 0/100) as an eluent. After trituration in diethyl ether/dichloromethane, the title compound 2-(3-{2-[4-(dimethylamino)phenyl]ethyl}-1-(4-fluorophenyl)-5-oxo-2-sulfanylidene imidazolidin-4-yl)-*N*-(2-methoxypyrimidin-5-yl)acetamide was obtained in 28 % yield (52 mg) as an off-white solid. ¹H-NMR (DMSO-d₆): δ (ppm) 2.75 (m, 1H), 2.84 (s, 6H), 2.94 (m, 1H), 3.15 (d, 1H), 3.28 (m, 1H), 3.64 (m, 1H), 3.89 (s, 3H), 4.13 (m, 1H), 4.73 (m, 1H), 6.65 (d, 2H), 7.07 (d, 2H), 7.37 (m, 4H), 8.72 (d, 2H), 10.5 (s, 1H); MS (ESI+): m/z = 522.9 [M+H]⁺.

### Example 78: Preparation of 2-(3-{2-[4-(dimethylamino)phenyl]ethyl}-1-(4-fluorophenyl)-2,5-dioxoimidazolidin-4-yl)-N-(2-methoxypyrimidin-5-yl)acetamide.

### a) Ethyl 2-(3-{2-[4-(dimethylamino)phenyl]ethyl}-1-(4-fluorophenyl)-2,5-dioxoimidazolidin-4-yl)acetate (I-38).

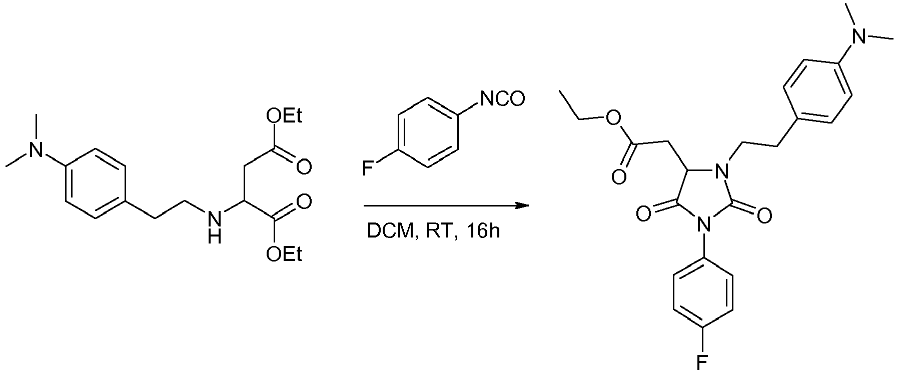

4-Fluoro-phenylisocyanate (1.55 g; 11.3 mmol; 1.15 eq) was added to a solution of 1,4-diethyl 2-({2-[4-(dimethylamino)phenyl]ethyl}amino)butanedioate (I-35) (3.3 g; 9.81 mmol; 1 eq) in dichloromethane (20 mL), and the reaction mixture was stirred at room temperature for 16 hours. Then, the mixture was concentrated to dryness. The crude was purified by flash chromatography on silica gel using cyclohexane/ethyl acetate (80/20 to 60/40) as an eluent. The title compound ethyl 2-(3-{2-[4-(dimethylamino)phenyl]ethyl}-1-(4-fluorophenyl)-2,5-dioxoimidazolidin-4-yl)acetate was obtained in 41 % yield (1.72 g) as a black oil. ¹H-NMR (DMSO-d₆): δ (ppm) 1.15 (t, 3H), 2.66 (m, 1H), 2.78 (m, 1H), 2.84 (s, 6H), 2.99 (d, 1H), 3.08 (d, 1H), 3.26 (m, 1H), 3.64 (m, 1H), 4.06 (m, 2H), 4.43 (m, 1H), 6.67 (d, 2H), 7.07 (d, 2H), 7.35 (m, 4H).

### b) 2-(3-{2-[4-(Dimethylamino)phenyl]ethyl}-1-(4-fluorophenyl)-2,5-dioxoimidazolidin-4-yl)acetic acid (1-39).

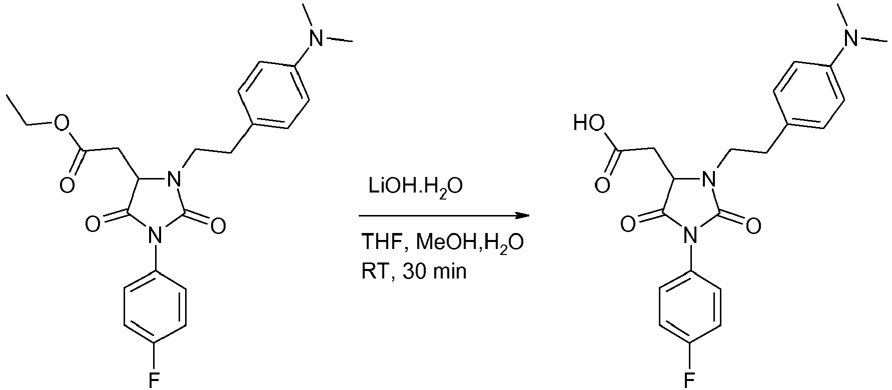

Lithium hydroxide (3 mL; 2.6M aqueous solution; 7.95 mmol, 2eq) was added to a solution of ethyl 2-(3- {2-[4-(dimethylamino)phenyl]ethyl}-1-(4-fluorophenyl)-5-oxo-2-sulfanylideneimidazolidin-4-yl)acetate (I-38) (1.7 g; 3.98 mmol; 1 eq) in tetrahydrofuran/methanol (10 mL/6 mL), and the reaction mixture was stirred at room temperature for 30 minutes. The mixture was concentrated to dryness. Water (15 mL) and hydrochloric acid 1M were added until pH 7. The aqueous layer was extracted with ethyl acetate (3 x 20 mL). The combined organic layers were dried over sodium sulfate, filtered and concentrated to dryness to afford the title compound 2-(3-{2-[4-(dimethylamino)phenyl]ethyl}-1-(4-fluorophenyl)-2,5-dioxoimidazolidin-4-yl)acetic acid in 85% yield (1.40 g) as a brown-green solid. ¹H-NMR (DMSO-d₆): δ (ppm) 2.63 (m, 1H), 2.78 (m, 1H), 2.84 (s, 6H), 2.93 (m, 2H), 3.27 (m, 1H), 3.65 (m, 1H), 4.4 (m, 1H), 6.67 (d, 2H), 7.08 (d, 2H), 7.33 (m, 4H), 12.74 (s, 1H).

### c) 2-(3-{2-[4-(Dimethylamino)phenyl]ethyl}-1-(4-fluorophenyl)-2,5-dioxoimidazolidin-4-yl)-N-(2-methoxypyrimidin-5-yl)acetamide (example 78).

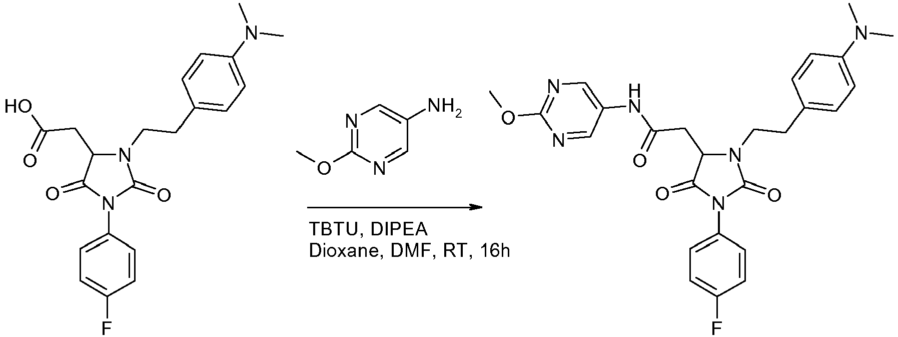

TBTU (187.1 mg; 0.57 mmol; 1.5 eq) and DIPEA (131 µL; 0.75 mmol; 2 eq) were added to a solution of 2-(3-{2-[4-(dimethylamino)phenyl]ethyl}-1-(4-fluorophenyl)-5-oxo-2-sulfanylideneimidazolidin-4-yl)acetic acid (1-39) (150 mg; 0.38 mmol; 1 eq) in dioxane (6 mL). The reaction mixture was stirred at room temperature for 30 minutes. Then, 2-methoxypyrimidin-5-amine (93.8 mg; 0.75 mmol; 2 eq) in dimethylformamide (2 mL) was added and the reaction mixture was stirred at room temperature overnight. The mixture was concentrated to dryness. Saturated ammonium chloride was added (15 mL) and the aqueous layer was extracted with ethyl acetate (3 x 20 mL). The combined organic layers were washed with saturated sodium chloride (3 x 20 mL), dried over sodium sulfate and concentrated to dryness. The crude was purified by flash chromatography on silica gel using cyclohexane/ethyl acetate (25/75 to 0/100) as an eluent. After trituration in diethyl ether/dichloromethane/pentane, the title compound 2-(3-{2-[4-(dimethylamino)phenyl]ethyl}-1-(4-fluorophenyl)-2,5-dioxoimidazolidin-4-yl)-N-(2-methoxypyrimidin-5-yl)acetamide was obtained in 33% yield (63.8 mg) as an off-white solid. ¹H-NMR (DMSO-d₆): δ (ppm) 2.69 (m, 1H), 2.80 (m, 1H), 2.83 (s, 6H), 3.02 (d, 1H), 3.12 (d, 1H), 3.28 (m, 1H), 3.68 (m, 1H), 3.88 (s, 3H), 4.51 (m, 1H), 6.64 (d, 2H), 7.05 (d, 2H), 7.36 (m, 4H), 8.71 (s, 2H), 10.39 (s, 1H); MS (ESI+): m/z = 506.9 [M+H]⁺.

### Example 49: Preparation of N-[4-(dimethylamino)phenyl]-2-(3-{2-[4-(dimethylamino)phenyl]ethyl}-1-(4-fluorophenyl)-2,5-dioxoimidazolidin-4-yl)acetamide.

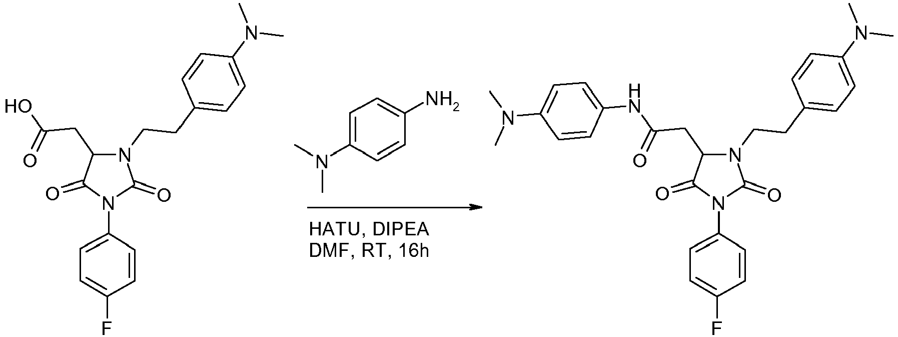

HATU (266 mg; 0.70 mmol; 1.4 eq) and DIPEA (174 µL; 1.0 mmol; 2 eq) were added to a solution of 2-(3-{2-[4-(dimethylamino)phenyl]ethyl}-1-(4-fluorophenyl)-5-oxo-2-sulfanylideneimidazolidin-4-yl)acetic acid (1-39) (200 mg; 0.50 mmol; 1 eq) in dimethylformamide (4 mL). The reaction mixture was stirred at room temperature for 20 minutes. Then, 1-*N*,1-*N-*dimethylbenzene-1,4-diamine (102 mg; 0.75 mmol; 1.5 eq) was added and the reaction mixture was stirred at room temperature overnight. Saturated ammonium chloride was added (15 mL) and the aqueous layer was extracted with ethyl acetate (3 x 20 mL). The combined organic layers were dried over sodium sulfate and concentrated to dryness. After trituration in diethyl ether/methanol at 0°C, the title compound *N-*[4-(dimethylamino)phenyl]-2-(3-{2-[4-(dimethylamino) phenyl]ethyl}-1-(4-fluorophenyl)-2,5-dioxoimidazolidin-4-yl)acetamide was obtained in 74 % yield (192 mg) as a grey solid. ¹H-NMR (DMSO-d₆): δ (ppm) 2.67 (m, 1H), 2.80 (m, 1H), 2.83 (s, 12H), 2.95 (d, 1H), 3.07 (d, 1H), 3.25 (m, 1H), 3.67 (m, 1H), 4.47 (m, 1H), 6.66 (m, 4H), 7.05 (d, 2H), 7.35 (m, 6H), 9.87 (s, 1H); MS (ESI+): m/z = 518 [M+H]⁺.

### Example 89: Preparation of 2-[1-(4-fluorophenyl)-3-[2-(4-methoxyphenyl)ethenyl]-5-oxo-2-sulfanylideneimidazolidin-4-yl]-N-(5-methoxypyridin-2-yl)acetamide.

### a) 1,4-Diethyl 2-{[(2,4-dimethoxyphenyl)methyl]amino}butanedioate (I-40).

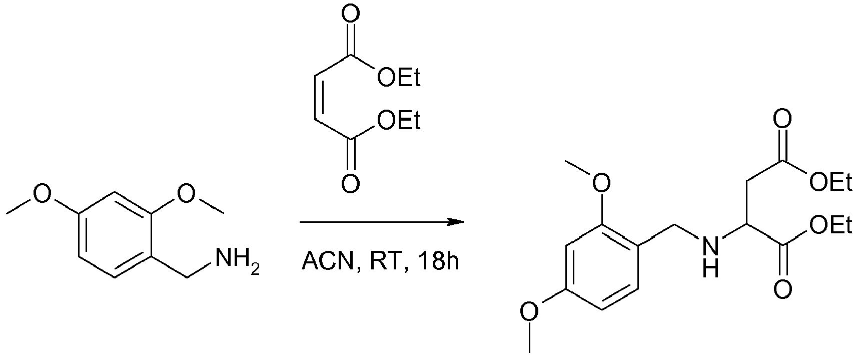

(2,4-Dimethoxyphenyl)methanamine (3.6 mL; 23.9 mmol; 1.5 eq) was added to a solution of 1,4-diethyl (2Z)-but-2-enedioate (2.6 mL; 15.9 mmol; 1 eq) in acetonitrile (120 mL). The reaction mixture was stirred at room temperature for 18 hours. The mixture was concentrated to dryness. The crude was purified on silica gel using ethyl acetate/cyclohexane (20/80 to 30/70) as an eluent. The title compound 1,4-diethyl 2-{[(2,4-dimethoxyphenyl)methyl]amino}butanedioate was obtained in 79% yield (4.28 g) as a yellow pale oil. ¹H-NMR (CDCl₃): δ (ppm) 1.25 (m, 6H), 2.69 (m, 2H), 3.63 (m, 1H), 3.72 (s, 2H), 3.77 (s, 6H), 4.13 (m, 4H), 6.42 (m, 2H), 7.14 (d, 1H); MS (ESI+): m/z = 340.0 [M+H]⁺.

### b) Ethyl 2-13-[(2,4-dimethoxyphenyl)methyl]-1-(4-fluorophenyl)-5-oxo-2-sulfanylideneimidazolidin-4-yl}acetate (I-41).

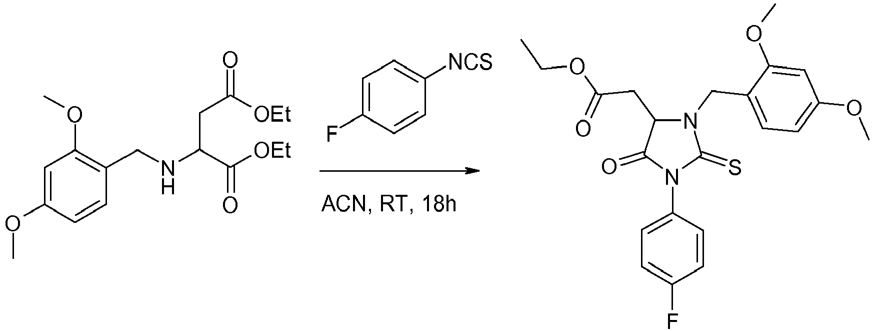

4-Fluorophenyl isothiocyanate (2.7 g; 17.6 mmol; 1.15 eq) was added to a solution of 1,4-diethyl 2-{[(2,4-dimethoxyphenyl)methyl]amino}butanedioate (I-40) (5.20 g; 15.3 mmol; 1 eq) in acetonitrile (40 mL). The reaction mixture was stirred at room temperature for 18 hours. The mixture was concentrated to dryness. The crude was purified on silica gel using ethyl acetate/cyclohexane (30/70) as an eluent. The title compound ethyl 2- {3-[(2,4-dimethoxyphenyl)methyl]-1-(4-fluorophenyl)-5-oxo-2-sulfanylideneimidazolidin-4-yl}acetate was obtained in 96% yield (6.89 g) as a yellow gum. ¹H-NMR (CDCl₃): δ (ppm) 1.24 (t, 3H), 3.13 (d, 2H), 3.81 (m, 3H), 3.86 (m, 3H), 4.12 (m, 2H), 4.25 (m, 1H), 4.63 (d, 1H), 5.45 (d, 1H), 6.48 (m, 2H), 7.17 (m, 2H), 7.37 (m, 2H), 7.53 (d, 1H); MS (ESI+): m/z = 446.9 [M+H]⁺.

### c) Ethyl 2-[1-(4-fluorophenyl)-5-oxo-2-sulfanylideneimidazolidin-4-yl]acetate (I-42).

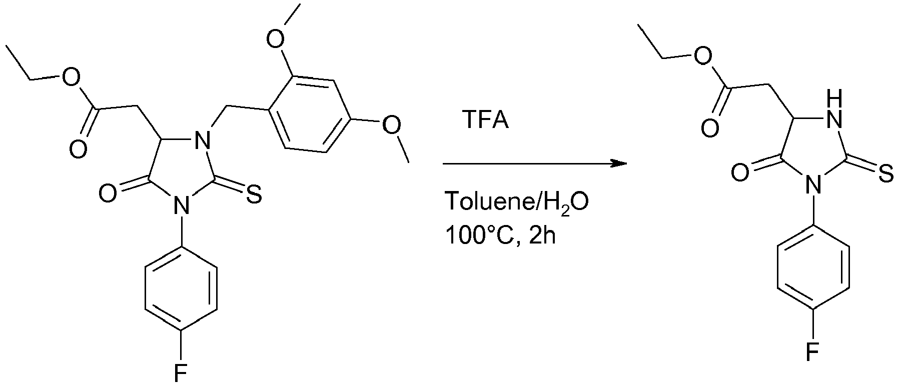

Water (22 mL) and trifluoroacetic acid (163 mL) were added to a solution of ethyl 2-{3-[(2,4-dimethoxyphenyl)methyl]-1-(4-fluorophenyl)-5-oxo-2-sulfanylideneimidazolidin-4-yl}acetate (I-41) (6.57 g; 14.7 mmol; 1 eq) in toluene (163 mL). The reaction mixture was stirred at 100°C on a sealed tube for 2 hours. The reaction mixture was cooled at room temperature and concentrated to dryness and the residue was taken up in dichloromethane (500 mL). The organic layer was washed with saturated sodium hydrogenocarbonate (200 mL), saturated sodium chloride (200 mL), dried over sodium sulfate, filtered and concentrated to dryness. The crude was purified on silica gel using ethyl acetate/cyclohexane (40/60) as an eluent. The title compound ethyl 2-[1-(4-fluorophenyl)-5-oxo-2-sulfanylideneimidazolidin-4-yl]acetate was obtained in 77 % yield (3.34 g) as a yellow solid. ¹H-NMR (CDCl₃): δ (ppm) 1.31 (t, 3H), 2.84 (dd, 1H), 3.13 (d, 1H), 4.24 (q, 2H), 4.57 (m, 1H), 7.19 (m, 2H), 7.32 (m, 2H), 7.51 (s, 1H); MS (ESI+): m/z = 297.0 [M+H]⁺.

### d) Ethyl 2-[1₋(4-fluorophenyl)-3-[2₋(4-methoxyphenyl)ethenyl]-5-oxo-2-sulfanylideneimidazolidin-4-yl]acetate, E & Z isomers (I-43a & I-43b).

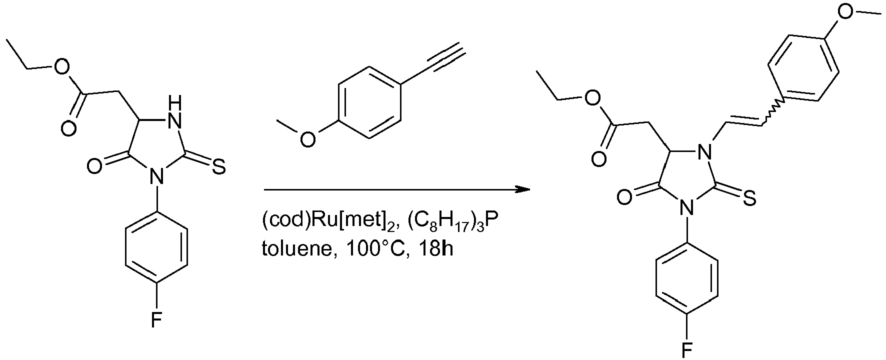

Trioctylphosphine (72 µL; 0.16 mmol; 0.06 eq) and 1-ethyl-4-ethynylbenzene (700 µL; 5.39 mmol; 2 eq) were added to a solution of ethyl 2-[1-(4-fluorophenyl)-5-oxo-2-sulfanylideneimidazolidin-4-yl]acetate (1-42) (799 mg; 2.69 mmol; 1 eq) and bis(2-methylallyl)cycloocta-1,5-dieneruthenium(II) ((cod)Ru[met]₂; 17 mg; 0.053 mmol; 0.02 eq) in anhydrous toluene (17 mL) over argon atmosphere. The reaction mixture was stirred at 100°C for 18 hours. The mixture was cooled at room temperature, filtered on Clarcel® and washed with ethyl acetate. The filtrate was diluted with saturated sodium hydrogenocarbonate (45 mL) and extracted with ethyl acetate (3 x 45 mL). The combined organic layers were washed with saturated sodium chloride (3 x 15 mL), dried over sodium sulfate, filtered and concentrated to dryness. The crude was purified on silica gel using ethyl acetate/cyclohexane (10/90 to 30/70) as an eluent. The title compound ethyl 2-[1-(4-fluorophenyl)-3-[2-(4-methoxyphenyl)ethenyl]-5-oxo-2-sulfanylideneimidazolidin-4-yl]acetate was obtained in 82 % yield (335 mg of isomer E (I-43a) and 615 mg of isomer Z (I43b) as a brown oil. ¹H-NMR (CDCl₃) Z isomer (I-43b): δ (ppm) 1.23 (t, 3H), 2.62 (d, 1H), 2.74 (d, 1H), 3.84 (s, 3H), 4.13 (m, 2H), 4.41 (m, 1H), 6.35 (d, 1H), 6.83 (d, 1H), 6.92 (d, 2H), 7.21 (m, 4H), 7.41 (m, 2H); MS (ESI+): m/z = 429.0 [M+H]⁺. 1H-NMR (CDCl3) *E* isomer (I-43a): δ (ppm) 1.21 (m, 3H), 3.20 (m, 1H), 3.35 (m, 1H), 3.79 (s, 3H), 4.13 (m, 2H), 4.74 (m, 1H), 6.07 (d, 1H), 6.86 (d, 2H), 7.18 (m, 2H), 7.35 (m, 4H), 7.93 (d, 1H).

### e) 2-[1-(4-Fluorophenyl)-3-[(Z)-2-(4-methoxyphenyl)ethenyl]-5-oxo-2-sulfanylideneimidazolidin-4-yl]acetic acid (1-44).

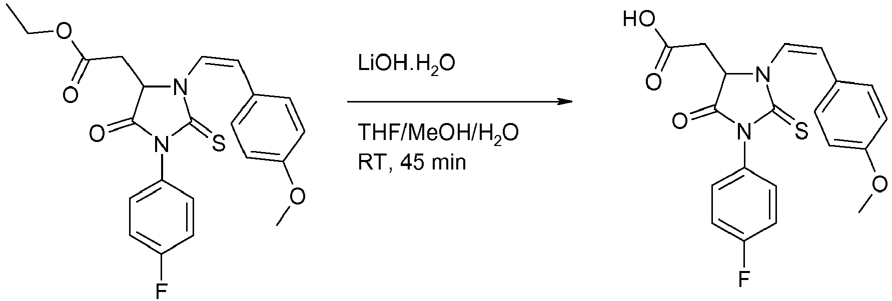

Lithium hydroxide (650 µL; 2.5 M aqueous solution; 2 eq) was added to a solution of ethyl 2-[1-(4-fluorophenyl)-3-[(Z)-2-(4-methoxyphenyl)ethenyl]-5-oxo-2-sulfanylidene imidazolidin-4-yl] acetate (I-43b) (349 mg; 0.81 mmol; 1 eq) in tetrahydrofuran/methanol (2.2 mL/1.3 mL). The reaction mixture was stirred at room temperature for 45 minutes. Saturated hydrochloric acid 1M (1.7 mL) was added until pH 2 and the aqueous layer was extracted with ethyl acetate (3 x 25 mL). The combined organic layers were washed with saturated sodium chloride (3 x 10 mL), dried over sodium sulfate, filtered and concentrated to dryness. The title compound 2-[1-(4-fluorophenyl)-3-[(Z)-2-(4-methoxyphenyl)ethenyl]-5-oxo-2-sulfanylideneimidazolidin-4-yl]acetic acid was obtained in a quantitative yield as a brown foam. ¹H-NMR (DMSO-d₆): δ (ppm) 2.39 (m, 1H), 2.75 (m, 1H), 3.79 (s, 3H), 4.6 (m, 1H), 6.35 (m, 1H), 6.65 (m, 1H), 6.95 (m, 3H), 7.37 (m, 5H), 12.84 (s, 1H); MS (ESI+): m/z = 401.0 [M+H]⁺.

### f) 2-[1-(4-Fluorophenyl)-3-[(Z)-2-(4-methoxyphenyl)ethenyl]-5-oxo-2-sulfanylideneimidazolidin-4-yl]-N-(5-methoxypyridin-2-yl)acetamide (example 89).

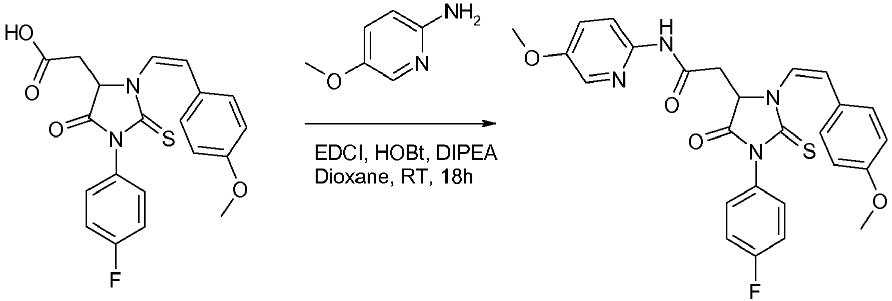

5-Methoxypyridin-2-amine (124 mg; 1.0 mmol; 2 eq), followed by EDCI (143 mg; 0.75 mmol; 1.5 eq), HOBt (115 mg; 0.75 mmol; 1.5 eq) and DIPEA (255 µL; 1.50 mmol; 3 eq) were added to a solution of 2-[1-(4-fluorophenyl)-3-[(Z)-2-(4-methoxyphenyl)ethenyl]-5-oxo-2-sulfanylideneimidazolidin-4-yl]acetic acid (1-47) (200 mg; 0.50 mmol; 1 eq) in dioxane (8 mL). The reaction mixture was stirred at room temperature for 18 hours. Saturated ammonium chloride (25 mL) was added and the aqueous layer was extracted with ethyl acetate (3 x 30 mL). The combined organic layers were washed with saturated sodium chloride (3 x 20 mL), dried over sodium sulfate, filtered and concentrated to dryness. The crude was purified on C18 reversed phase chromatography using methanol/water (50/50 to 80/20) as an eluent. The title compound 2-[1-(4-fluorophenyl)-3-[(Z)-2-(4-methoxyphenyl)ethenyl]-5-oxo-2-sulfanylideneimidazolidin-4-yl]-*N-*(5-methoxypyridin-2-yl)acetamide was obtained in 17% yield (35 mg) as a yellow solid. ¹H-NMR (Acetone-d₆): δ (ppm) 2.95 (d, 1H), 3.03 (d, 1H), 3.80 (s, 3H), 3.84 (s, 3H), 4.65 (m, 1H), 6.37 (d, 1H), 6.77 (d, 1H), 6.95 (d, 2H), 7.29 (t, 2H), 7.38 (m, 3H), 7.48 (m, 2H), 7.95 (s, 1H), 8.04 (d, 1H), 9.54 (s, 1H); MS (ESI+): m/z = 506.9 [M+H]⁺.

### Example 90: Preparation of N-[4-(dimethylamino)phenyl]-2-[(4E)-3-[2-(4-methoxyphenyl)ethyl]-5-oxo-1-phenyl-2-sulfanylideneimidazolidin-4-ylidene]acetamide.

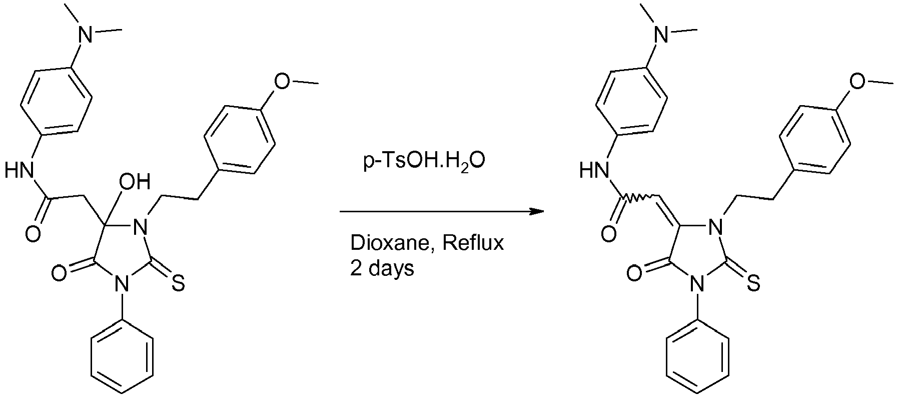

*p*-Toluenesulfonic acid monohydrate (136.5 mg; 0.723 mmol; 7.5 eq) was added to a suspension of N-[4-(dimethylamino)phenyl]-2-{4-hydroxy-3-[2-(4-methoxyphenyl)ethyl]-5-oxo-1-phenyl-2-sulfanylideneimidazolidin-4-yl}acetamide of example 70 (50 mg; 0.096 mmol; 1 eq) in dioxane (3 mL). The reaction mixture was stirred at room temperature for 2 days. Saturated sodium hydrogenocarbonate (20 mL) was added and the aqueous layer was extracted with ethyl acetate (3 x 30 mL). The combined organic layers were washed with saturated sodium chloride (3 x 15 mL), dried over sodium sulfate, filtered and concentrated to dryness. The crude was purified on unmetallated silica gel using dichloromethane/methanol (99/1) as an eluent. The title compound *N*-[4-(dimethylamino)phenyl]-2-[(4*E*)-3-[2-(4-methoxyphenyl)ethyl]-5-oxo-1-phenyl-2-sulfanylideneimidazolidin-4-ylidene]acetamide was obtained in 62% yield (30 mg) as a purple solid as mixture of E and Z (50:50). ¹H-NMR (DMSO-d₆): δ (ppm) 2.84 (s, 6H), 2.88 (s, 6H), 2.92 (m, 4H), 3.70 (s, 3H), 3.73 (s, 3H), 4.24 (m, 2H), 4.81 (m, 2H), 6.26 (s, 1H), 6.59 (s, 1H), 6.69 (m, 2H), 6.75 (m, 2H), 6.82 (m, 2H), 6.90 (m, 2H), 7.15 (m, 2H), 7.42 (m, 16H), 10.26 (s, 1H), 10.53 (s, 1H); MS (ESI+): m/z = 500.9 [M+H]⁺.

### Example 87: Preparation of 2-[1-(4-fluorophenyl)-3-[2-(4-methoxyphenyl)ethyl]-5-oxo-2-sulfanylideneimidazolidin-4-yl]-N-[4-(2-oxopyrrolidin-1-yl)phenyl]acetamide.

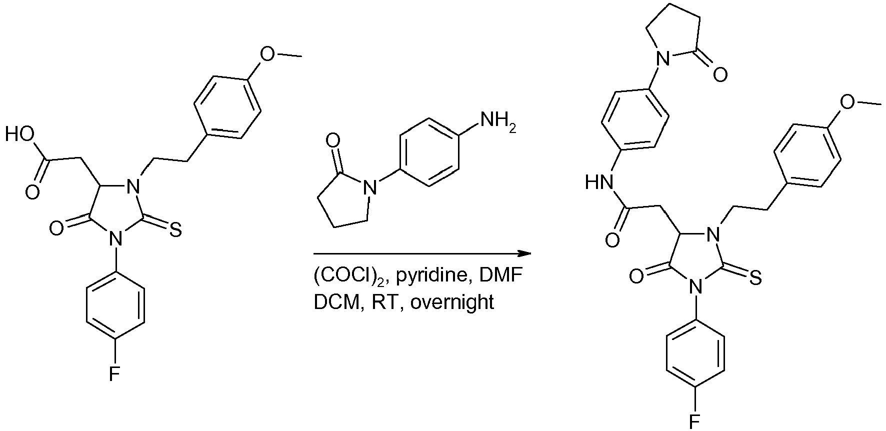

Oxalyl chloride (81 µL; 0.93 mmol; 2.5 eq), followed by dimethylformamide (catalytic amount) were added to a solution of 2-[1-(4-fluorophenyl)-3-[2-(4-methoxyphenyl)ethyl]-5-oxo-2-sulfanylideneimidazolidin-4-yl]acetic acid (1-10) (150 mg; 0.37 mmol; 1 eq) in dichloromethane (5 mL). The reaction mixture was stirred at room temperature for 2 hours. Pyridine (93 µL; 1.49 mmol; 4 eq) and 1-(4-aminophenyl)pyrrolidin-2-one (98.5 mg; 0.56 mmol; 1.5 eq) were added. The reaction mixture was stirred at room temperature overnight. Dichloromethane (10 mL) was added and the organic layer was washed with saturated sodium hydrogenocarbonate (15 mL), dried over sodium sulfate, filtered and concentrated to dryness. The crude was purified by flash chromatography on silica gel using dichloromethane/methanol (100/0 to 99/1) as an eluent. After trituration in diethyl ether/dichloromethane/acetone, the title compound 2-[1-(4-fluorophenyl)-3-[2-(4-methoxyphenyl)ethyl]-5-oxo-2-sulfanylidene imidazolidin-4-yl]-*N*-[4-(2-oxopyrrolidin-1-yl)phenyl]acetamide was obtained in 5% yield (10 mg) as a white solid. ¹H-NMR (DMSO-d₆): δ (ppm) 2.07 (m, 2H), 2.48 (m, 2H), 2.87 (m, 1H), 2.98 (m, 1H), 3.11 (d, 1H), 3.24 (d, 1H), 3.74 (m, 1H), 3.78 (s, 3H), 3.81 (m, 2H), 4.18 (m, 1H), 4.68 (m, 1H), 6.87 (m, 2H), 7.12 (m, 2H), 7.18(m, 4H), 7.38 (m, 4H), 9.92 (s, 1H); MS (ESI+): m/z = 560.9 [M+H]⁺.

### Example 88: Preparation of 2-[1-(4-fluorophenyl)-3-[2-(4-methoxyphenyl)ethyl]-5-oxo-2-sumanylideneimidazolidin-4-yl]-N-(5-methoxy-1,3-thiazol-2-yl)acetamide.

### a) 5-Methoxy-1,3-thiazol-2-amine (I-45).

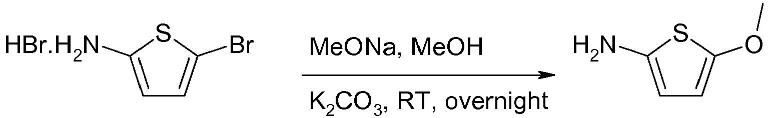

Potassium carbonate (4.7 g; 34.4 mmol; 3 eq) and sodium methanoate (1.8 g; 34.4 mmol; 3 eq) were added to a solution of 5-bromo-1,3-thiazol-2-amine hydrobromide (2.9 g; 11.5 mmol; 1 eq) in methanol (37 mL). The reaction mixture was stirred at room temperature overnight. The mixture was concentrated to dryness. The crude was purified on silica gel using ethyl acetate/dichloromethane (80/20 to 100/0) as an eluent. The title compound 5-methoxy-1,3-thiazol-2-amine was obtained in 38% yield (564 mg) as a purple solid. ¹H-NMR (CDCl₃): δ (ppm) 3.82 (s, 3H), 4.48 (m, 2H), 6.41 (s, 1H).

### b) 2-[1-(4-Fluorophenyl)-3-[2-(4-methoxyphenyl)ethyl]-5-oxo-2-sulfanylideneimidazolidin-4-yl]-N-(5-methoxy-1,3-thiazol-2-yl)acetamide (example 88).

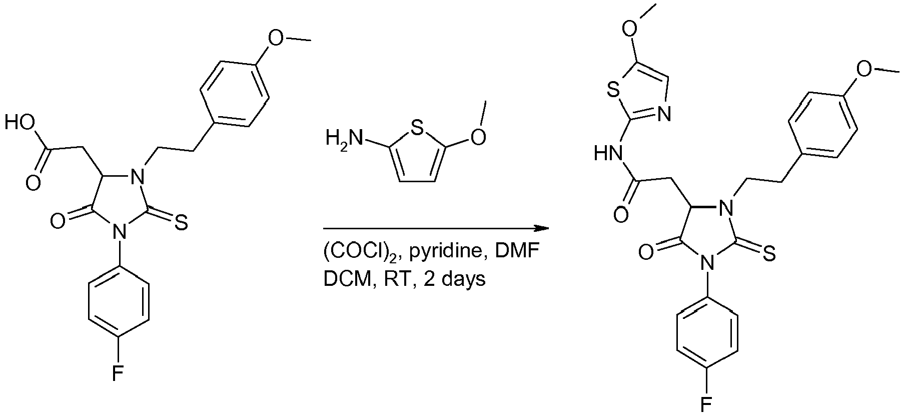

Oxalyl chloride (66 µL; 0.75 mmol; 2.5 eq), followed by dimethylformamide (catalytic amount) was added to a solution of 2-[1-(4-fluorophenyl)-3-[2-(4-methoxyphenyl)ethyl]-5-oxo-2-sulfanylideneimidazolidin-4-yl]acetic acid (1-10) (120 mg; 0.30 mmol; 1 eq) in dichloromethane (6 mL). The reaction mixture was stirred at room temperature for 1 hour and 30 minutes. Pyridine (75 µL; 1.2 mmol; 4 eq) and 5-methoxy-1,3-thiazol-2-amine (1-48) (58.2 mg; 0.44 mmol; 1.5 eq) were added. The reaction mixture was stirred at room temperature for 2 days. Ammonium chloride and ethyl acetate (20 mL) were added and the aqueous layer was extracted with ethyl acetate. The combined organic layers were washed with saturated sodium chloride (3 x 15 mL), dried over sodium sulfate, filtered and concentrated to dryness. The crude was purified by preparative HPLC and the title compound 2-[1-(4-fluorophenyl)-3-[2-(4-methoxyphenyl)ethyl]-5-oxo-2-sulfanylideneimidazolidin-4-yl]-*N*-(5-methoxy-1,3-thiazol-2-yl)acetamide was obtained in 8% yield (12 mg) as a white powder. ¹H-NMR (DMSO-d₆): δ (ppm) 2.78 (m, 1H), 2.85 (m, 1H), 3.16 (d, 1H), 3.33 (m, 1H), 3.63 (m, 1H), 3.72 (s, 3H), 3.85 (s, 3H), 4.10 (m, 1H), 4.81 (m, 1H), 6.83 (m, 3H), 7.15 (d, 2H), 7.39 (m, 4H), 12.14 (s, 1H); MS (ESI+): m/z = 514.8 [M+H]⁺.

### Example 91: Preparation of 2-[1-(4-fluorophenyl)-3-[2-(4-methoxyphenyl)ethyl]-5-oxo-2-sulfanylideneimidazolidin-4-yl]-N-(4-hydroxyphenyl)acetamide.

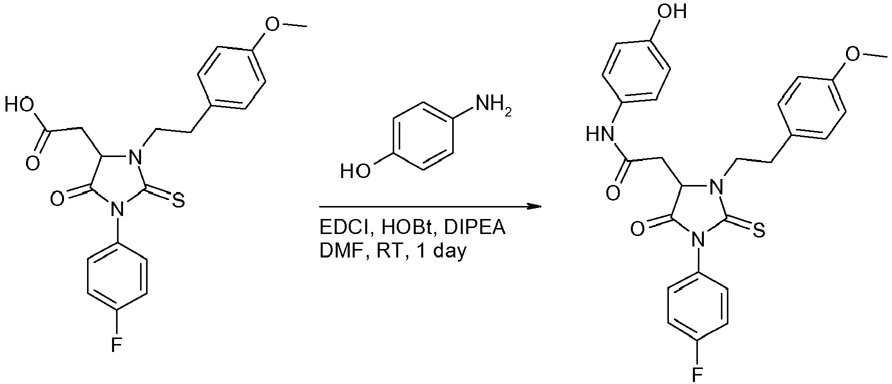

4-Aminophenol (60 mg; 0.54 mmol; 1.1 eq), followed by EDCI (140 mg; 0.73 mmol; 1.5 eq), HOBt (112 mg; 0.73 mmol; 1.5 eq) and DIPEA (426 µL; 2.45 mmol; 5 eq) were added to a solution of 2-[1-(4-fluorophenyl)-3-[2-(4-methoxyphenyl)ethyl]-5-oxo-2-sulfanylideneimidazolidin-4-yl]acetic acid (1-10) (200 mg; 0.49 mmol; 1 eq) in dimethylformamide (20 mL). The reaction mixture was stirred at room temperature for 1 day. Water an ethyl acetate were added and the organic layer was washed with saturated sodium chloride (3 x 20 mL), dried over sodium sulfate, filtered and concentrated to dryness. The crude was purified on silica gel using ethyl acetate/dichloromethane (30/70) as an eluent. After trituration in diethyl ether, the title compound 2-[1-(4-fluorophenyl)-3-[2-(4-methoxyphenyl)ethyl]-5-oxo-2-sulfanylidene imidazolidin-4-yl]-*N*-(4-hydroxyphenyl)acetamide was obtained in 32% yield (75.5 mg) as a yellow solid. ¹H-NMR (DMSO-d₆): δ (ppm) 2.86 (m, 1H), 3.02 (m, 1H), 3.08 (d, 1H), 3.23 (m, 1H), 3.63 (m, 1H), 3.68 (s, 3H), 4.11 (m, 1H), 4.71 (m, 1H), 6.69 (d, 2H), 6.88 (d, 2H), 7.28 (m, 8H), 9.24 (s, 1H), 9.97 (s, 1H); MS (ESI+): m/z = 493.9 [M+H]⁺.

### Example 93: Preparation of 2-[1-(4-fluorophenyl)-3-[4-(4-methoxyphenyl)butyl]-5-oxo-2-sumanylideneimidazolidin-4-yl]-N-(5-methoxypyridin-2-yl)acetamide.

### a) 4-Tert-butyl 1-methyl 2-{[(9H-fluoren-9-ylmethoxy)carbonyl]amino}butanedioate (1-46).

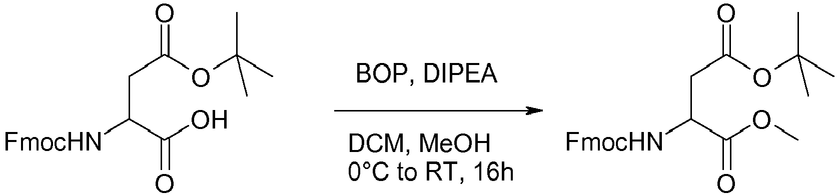

BOP (7.63 g; 17.3 mmol; 1.1 eq) and DIPEA (5.22 mL; 31.4 mmol; 2 eq) were added to a solution of 4-*(tert*-butoxy)-2-{[(9H-fluoren-9-ylmethoxy)carbonyl]amino}-4-oxobutanoic acid (6.46 g; 15.7 mmol; 1 eq) in dichloromethane/methanol (103 mL/5.2 mL), at 0°C. The reaction mixture was stirred at room temperature for 16 hours. The organic layer was washed with saturated sodium hydrogenocarbonate (10 mL), saturated ammonium chloride (10 mL), water (10 mL), dried over sodium sulfate, filtered and concentrated to dryness. The crude was purified on silica gel using cyclohexane/ethyl acetate (80/20) as an eluent. The title compound 4-*tert*-butyl 1-methyl 2-{[(9*H*-fluoren-9-ylmethoxy)carbonyl]amino}butanedioate was obtained in 97% yield (6.19 g) as a white solid. ¹H-NMR (CDCl₃): δ (ppm) 1.45 (s, 9H), 2.76 (d, 1H), 2.95 (d, 1H), 3.75 (s, 3H), 4.25 (m, 1H), 4.39 (m, 2H), 4.61 (m, 1H), 5.75 (d, 1H), 7.32 (m, 2H), 7.40 (t, 2H), 7.60 (t, 2H), 7.77 (d, 2H).

### b)3-{[(9H-Fluoren-9-ylmethoxy)carbonyl]amino}-4-methoxy-4-oxobutanoic acid (I-47).

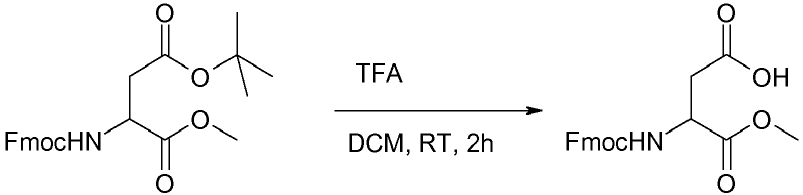

Trifluoroacetic acid (10 mL) was added to a solution of 4-tert-butyl 1-methyl 2-{[(9*H-*fluoren-9-ylmethoxy)carbonyl]amino}butanedioate (1-46) (1.9 g; 4.47 mmol; 1 eq) in dichloromethane (40 mL). The reaction mixture was stirred at room temperature for 2 hours. The mixture was concentrated to dryness. The title compound 3-{[(9*H*-fluoren-9-ylmethoxy)carbonyl]amino}-4-methoxy-4-oxobutanoic acid was obtained in a quantitative yield as a beige solid. ¹H-NMR (DMSO-d₆): δ (ppm) 2.6 (dd, 1H), 2.72 (dd, 1H), 3.62 (s, 3H), 4.23 (m, 1H), 4.31 (m, 2H), 4.41 (m, 1H), 7.33 (t, 2H), 7.42 (t, 2H), 7.70 (d, 2H), 7.83 (d, 1H), 7.89 (d, 2H).

### c) Methyl 2-{[(9H-fluoren-9-ylmethoxy)carbonyl]amino}-3-[(5-methoxypyridin-2-yl)carbamoyl]propanoate (1-48).

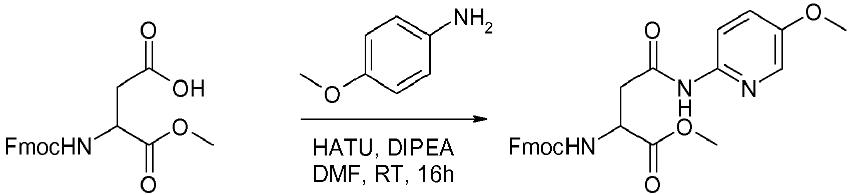

HATU (1.27 g; 3.34 mmol; 1.2 eq) and DIPEA (1.41 mL; 8.1 mmol; 3 eq) were added to a solution of 3-([(9*H*-fluoren-9-ylmethoxy)carbonyl]amino)-4-methoxy-4-oxobutanoic acid (1-47) (1.03 g; 2.7 mmol; 1 eq) in dimethylformamide (51 mL). After 20 minutes, 5-methoxypyridin-2-amine (415.5 mg; 3.34 mmol; 1.2 eq) was added and the reaction mixture was stirred at room temperature overnight. Water (50 mL) and ethyl acetate (50 mL) were added and the aqueous layer was extracted with ethyl acetate (3 x 30 mL). The combined organic layers were washed with saturated sodium chloride (3 x 50 mL), dried over sodium sulfate, filtered and concentrated to dryness. The crude was purified on silica gel using dichloromethane/ethyl acetate (90/10 to 50/50) as an eluent. After washing in ethyl acetate and saturated sodium chloride, the title compound methyl 2-{[(9*H*-fluoren-9-ylmethoxy)carbonyl]amino}-3-[(5-methoxypyridin-2-yl) carbamoyl]propanoate was obtained in 75% yield (1.00 g). ¹H-NMR (CDCl₃): δ (ppm) 2.97 (m, 1H), 3.19 (d, 1H), 3.78 (s, 3H), 3.82 (s, 3H), 4.23 (m, 1H), 4.37 (m, 2H), 4.69 (m, 1H), 6.04 (m, 1H), 7.30 (m, 2H), 7.38 (m, 3H), 7.59 (m, 2H), 7.76 (m, 2H), 7.95 (m, 2H), 8.07 (d, 1H).

### d) Methyl 2-amino-3-[(5-methoxypyridin-2-yl)carbamoyl]propanoate (1-49).

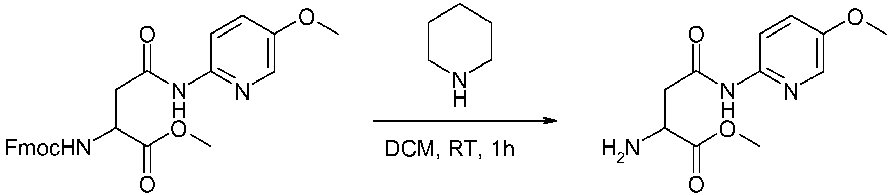

Piperidine (2.5 mL) was added to a solution of methyl 2-{[(9*H*-fluoren-9-ylmethoxy)carbonyl]amino}-3-[(5-methoxypyridin-2-yl)carbamoyl]propanoate (I-48) (792 mg; 1.67 mmol; 1 eq) in dichloromethane (25 mL). The reaction mixture was stirred at room temperature for 1 hour and the solvent removed in vacuum. The crude was purified by flash chromatography on silica gel using dichloromethane/methanol (100/0 to 95/5) as an eluent. The title compound methyl 2-amino-3-[(5-methoxypyridin-2-yl)carbamoyl]propanoate was obtained in 72% yield (382 mg) as a yellow gum. ¹H-NMR (CDCl₃): δ (ppm) 2.65 (dd, 1H), 2.82 (d, 1H), 3.76 (s, 3H), 3.83 (s, 3H), 3.89 (d, 1H), 7.24 (d, 1H), 7.97 (s, 1H), 8.12 (d, 1H), 9.82 (s, 1H).

### e) 4₋(4-Methoxyphenyl)butanal (I-50).

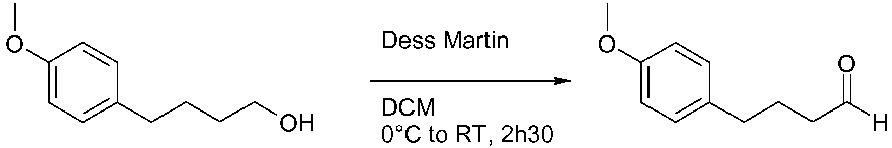

Dess-Martin periodinane (1.23 g; 2.91 mmol; 1.05 eq) was added to a solution of 4-(4-methoxyphenyl)butan-1-ol (500 mg; 2.77 mmo; 1 eq) in dichloromethane (15 mL) at 0°C. The reaction mixture was stirred at 0°C for 30 minutes and at room temperature for 2 hours. Dichloromethane was added (30 mL) and the organic layer was washed with sodium thiosulfate 10% in water (45 mL) and sodium hydroxide 1M (50 mL), dried over sodium sulfate, filtered and concentrated to dryness. The title compound 4-(4-methoxyphenyl)butanal was obtained in quantitative yield as a yellow oil. ¹H-NMR (CDCl₃): δ (ppm) 1.9 (m, 2H), 2.41 (t, 2H), 2.57 (t, 2H), 3.76 (s, 3H), 6.81 (d, 2H), 7.06 (d, 2H), 9.72 (s, 1H).

### f) Methyl 2-{[4-(4-methoxyphenyl)butyl]amino}-3-[(5-methoxypyridin-2-yl)carbamoyl]propanoate (I-51).

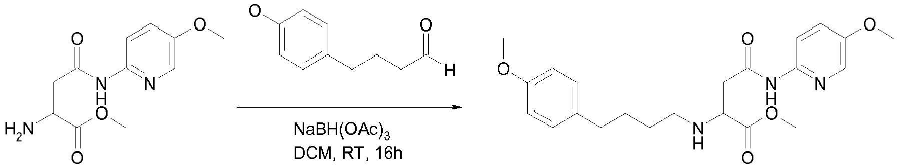

Sodium triacetoxyborohydride (174 mg; 0.82 mmol; 1.1 eq) was added to a solution of methyl 2-amino-3-[(5-methoxypyridin-2-yl)carbamoyl]propanoate (1-49) (189 mg; 0.75 mmol; 1 eq) and 4-(4-methoxyphenyl)butanal (I-50) (146 mg; 0.82 mmol; 1.1 eq) in dichloromethane (15 mL). The reaction mixture was stirred at room temperature for 16 hours. Dichloromethane (30 mL) was added and the organic layer was washed with water (45 mL), dried over sodium sulfate, filtered and concentrated to dryness. The crude was purified by flash chromatography on silica gel using dichloromethane/methanol (100/0 to 98/2) as an eluent. The title compound methyl 2-{[4-(4-methoxyphenyl)butyl]amino}-3-[(5-methoxypyridin-2-yl)carbamoyl]propanoate was obtained in 71% yield (220 mg) as a yellow gum. ¹H-NMR (CDCl₃): δ (ppm) 1.61 (m, 2H), 1.71 (m, 2H), 2.59 (m, 4H), 2.67 (m, 1H), 2.74 (m, 2H), 3.64 (m, 1H), 3.76 (s, 3H), 3.78 (s, 3H), 3.83 (s, 3H), 6.8 (d, 2H), 7.08 (d, 2H), 7.22 (m, 1H), 7.89 (s, 1H), 8.09 (d, 1H), 10.43 (s, 1H).

### g) 2-[1-(4-Fluorophenyl)-3-[4-(4-methoxyphenyl)butyl]-5-oxo-2-sulfanylidene imidazolidin-4-yl]-N-(5-methoxypyridin-2-yl)acetamide (example 93).

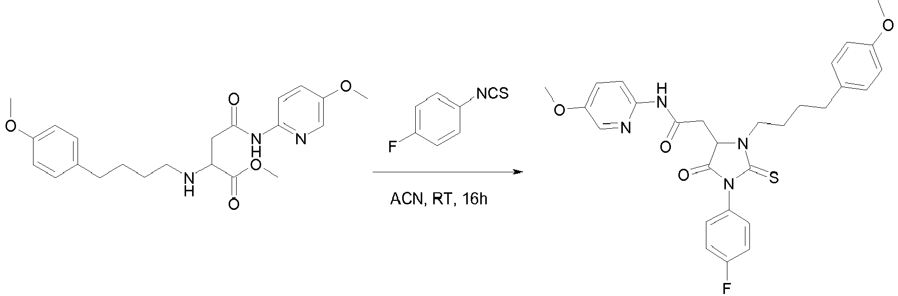

4-Fluorophenyl isothiocyanate (96.4 mg; 0.63 mmol; 1.2 eq) was added to a solution of methyl 2-{[4-(4-methoxyphenyl)butyl]amino}-3-[(5-methoxypyridin-2-yl)carbamoyl] propanoate (I-54) (218 mg; 0.52 mmol; 1 eq) in acetonitrile (15 mL). The reaction mixture was stirred at room temperature for 16 hours, and then, concentrated to dryness. The crude was purified by flash chromatography on silica gel using dichloromethane/ethyl acetate (100/0 to 90/10) as an eluent. The title compound 2-[1-(4-fluorophenyl)-3-[4-(4-methoxyphenyl)butyl]-5-oxo-2-sulfanylideneimidazolidin-4-yl]-*N*-(5-methoxypyridin-2-yl)acetamide was obtained in 85% yield (241 mg) as a beige solid. ¹H-NMR (DMSO-d₆): δ (ppm) 1.61 (m, 4H), 2.58 (m, 2H), 3.12 (d, 1H), 3.35 (d, 1H), 3.57 (m, 1H), 3.69 (s, 3H), 3.8 (s, 3H), 4.02 (m, 1H), 4.73 (s, 1H), 6.77 (d, 2H), 7.03 (d, 2H), 7.38 (m, 4H), 7.42 (d, 1H), 7.94 (d, 1H), 8.05 (s, 1H), 10.64 (s, 1H); MS (ESI+): m/z = 536.9 [M+H]⁺.

### Example 92: Preparation of 2-[1-(4-fluorophenyl)-3-[3-(4-methoxyphenyl)propyl]-5-oxo-2-sulfanylideneimidazolidin-4-yl]-N-(5-methoxypyridin-2-yl)acetamide

### a) Methyl 2-{[3-(4-methoxyphenyl)propyl]amino}-3-[(5-methoxypyridin-2-yl) carbamoyl]propanoate (I-52)

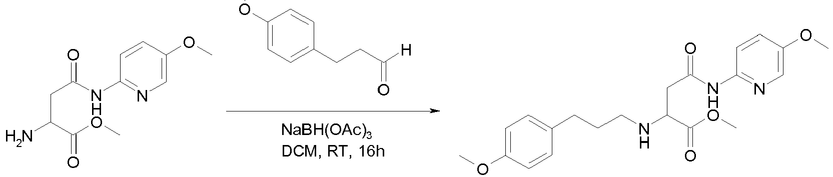

Sodium triacetoxy borohydride (174 mg; 0.82 mmol; 1.1 eq) was added to a solution of methyl 2-amino-3-[(5-methoxypyridin-2-yl)carbamoyl]propanoate (1-49) (189 mg; 0.75 mmol; 1 eq) and 3-(4-methoxyphenyl)propanal (134.8 mg; 0.82 mmol; 1.1 eq) in dichloromethane (14 mL). The reaction mixture was stirred at room temperature overnight. The reaction mixture was diluted with water (15 mL) and extracted with ethylacetate (3 x 15 mL). The organic layers were collected and washed with brine, dried over sodium sulphate, filtrated and concentrated in vacuum. The residue was purified by flash chromatography on silica gel using dichoromethane/methanol (100:0 to 98:2) as eluent to afford the title compound methyl 2-{[3-(4-methoxyphenyl)propyl]amino}-3-[(5-methoxypyridin-2-yl)carbamoyl]propanoate as a yellow gum in 71% yield (212 mg). ¹H-NMR (CDC13): δ (ppm) 2.64 (m, 8H), 3.61 (m, 1H), 3.71 (s, 3H), 3.74 (s, 3H), 3.78 (s, 3H), 6.77 (d, 2H), 7.07 (d, 2H), 7.2 (m, 2H), 7.91 (s, 1H), 8.07 (d, 1H), 10.4 (s, 1H).

### b) 2-[1-(4-Fluorophenyl)-3-[2-(4-methoxyphenyl)ethyl]-5-oxo-2-sulfanylidene imidazolidin-4-yl]-N-(5-methoxypyridin-2-yl)acetamide (example 92)

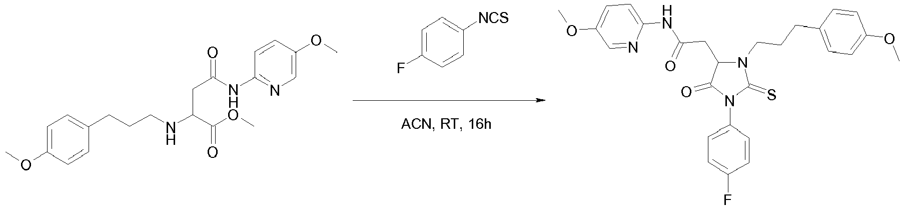

1-Fluoro-4-isothiocyanatobenzoate (96 mg; 0.63 mmol; 1.2 eq) was added to a solution of **methyl** 1 2- {[3-(4-methoxyphenyl)propyl]amino}-3-[(5-methoxypyridin-2-yl) carbamoyl]propanoate (1-49) (212mg; 0.52 mmol; 1.0 eq) in acetonitrile (15 mL) at room temperature. The reaction mixture was stirred at room temperature overnight. Upon concentration to dryness, the residue was purified by flash chromatography using dichloromethane/ethylacetate (100:0 to 90:10) as eluent to afford the title compound 2-[1-(4-fluorophenyl)-3-[2-(4-methoxyphenyl)ethyl]-5-oxo-2-sulfanylideneimidazolidin-4-yl]-N-(5-methoxypyridin-2-yl)acetamide as a beige solid in 73% yield (163 mg). ¹H-NMR (CDC13): δ (ppm) 1.93 (m, 1H), 2.07 (m, 1H), 2.61 (m, 2H), 2.92 (d, 2H), 3.04 (d, 2H), 3.45 (m, 1H), 3.74 (s, 3H), 3.81 (s, 3H), 4.23 (m, 1H), 4.41 (m, 1H), 6.77 (d, 2H), 7.05 (d, 2H), 7.12 (m, 2H), 7.28 (m, 1H), 7.90 (s, 1H), 8.03 (m, 2H); MS (ESI+): m/z = 522.9 [M+H]⁺.

### Example 99: Preparation of N-{[1-(4-fluorophenyl)-3-[2-(4-methoxyphenyl)ethyl]-5-oxo-2-sulfanylideneimidazolidin-4-yl]methyl}-5-methoxypyridine-2-carboxamide

### a) Methyl2,3-diaminopropanoate (I-53)

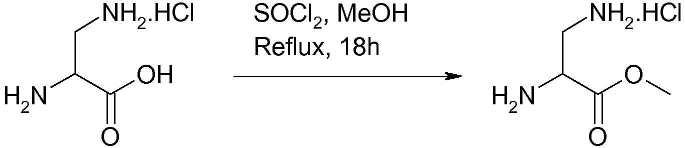

Thionyl chloride (4.15 mL; 56.91 mmol; 4 eq) was added dropwise, at 0°C, to a suspension of 2,3-diaminopropanoic acid hydrochloride (2 g; 14.23 mmol; 1 eq) in methanol (89 mL). The reaction mixture was slowly allowed to warm at room temperature then, refluxed for 18h. After concentration to dryness, the title compound, methyl 2,3-diaminopropanoate was obtained in a quantitative yield (2.79 g) as a white solid. ¹H NMR (D₂O): δ 3.48 (m, 1H), 3.58 (m, 1H), 3.86 (s, 3H), 4.47 (m, 1H).

### b) methyl2-amino-3-{[(tert-butoxy)carbonyl]amino}propanoate (I-54)

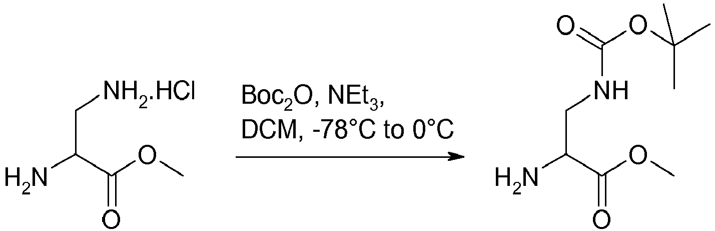

Triethylamine (10 mL; 72.16 mmol; 4 eq.) was added dropwise, at -78°C, to a suspension of methyl 2,3-diaminopropanoate (I-56) (2.79 g; 18.04 mmol; 1 eq) in dichloromethane (600 mL). Then, a solution of di-tert-butyl dicarbonate (3.54 g; 16.24 mmol; 0.9 eq.) in dichloromethane (30 mL) was added slowly. After 10 minutes at - 78°C, the reaction mixture was stirred for 3 hours at 0°C. After concentration to dryness, the crude was purified on silica gel using dichloromethane/methanol (98/2) as an eluent. The title compound, methyl 2-amino-3-{[(*tert*-butoxy) carbonyl]amino}propanoate was obtained in 58% yield (2.07 g) as a yellow oil. ¹H NMR (CDCl₃): δ 1.5 (s, 9H), 1.76 (s, 2H), 3.33 (m, 1H), 3.55 (m, 1H), 3.66 (m, 1H), 3.81 (s, 3H), 5.06 (m, 1H).

### c) 2-(4-methoxyphenyl)acetaldehyde (I-55)

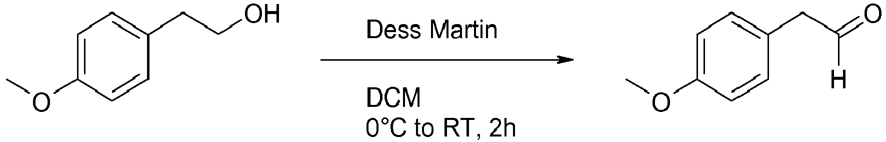

Dess-Martin periodinane (5.8 g; 13.79 mmol; 1.05 eq) was added to a solution of 2-(4-methoxyphenyl)ethan-1-ol (2 g; 13.14 mmol; 1 eq) in dichloromethane (90 mL) at 0°C. The reaction mixture was stirred at 0°C for 30 minutes and at room temperature for 1.5 hours. Dichloromethane (100 mL) was added and the organic layer was washed with sodium thiosulfate 10% in water (100 mL) and sodium hydroxide 1M (100 mL), dried over sodium sulfate, filtrated and concentrated to dryness. The title compound, 2-(4-methoxyphenyl)acetaldehyde was obtained in quantitative yield (2.1 g) as a colorless oil. ¹H NMR (CDCl₃): δ 3.68 (s, 2H), 3.87 (s, 3H), 6.97 (d, 2H), 7.19 (d, 2H), 9.78 (s, 1H).

### d) Methyl 3-{[(tert-butoxy)carbonyl]amino}-2-{[2-(4-methoxyphenyl) ethyl]amino)propanoate (I-56)

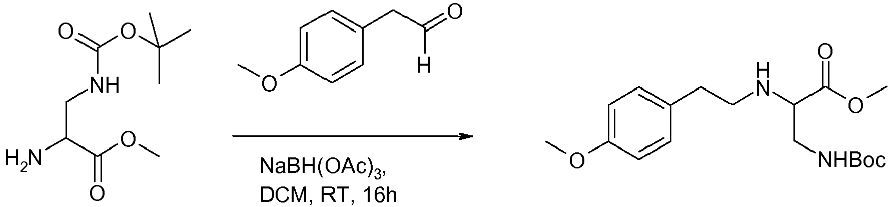

Sodium triacetoxyborohydride (2.21 g; 10.43 mmol; 1.1 eq) was added to a solution of 2-(4-methoxyphenyl)acetaldehyde (I-55) (1.28 g; 8.53 mmol; 0.9 eq) and methyl 2-amino-3-{[(*tert*-butoxy)carbonyl]amino}propanoate (I-57) (2.07 g; 9.48 mmol; 1 eq) in dichloromethane (135 mL). The reaction mixture was stirred at room temperature for 16 hours. Saturated sodium hydrogenocarbonate (100 mL) was added. The aqueous layer was extracted with dichloromethane (2 x 130 mL). The combined organic layers were dried over sodium sulfate, filtered and concentrated under reduced pressure. The crude was first purified on silica gel using ethyl acetate/dichloromethane (1/9) as an eluent to afford the title compound, methyl 3-{[(*tert*-butoxy)carbonyl]amino}-2-{[2-(4-methoxyphenyl)ethyl]amino}propanoate in 80% yield (2.4 g) as an yellow oil. ¹H NMR (CDCl₃): δ 1.49 (s, 9H), 2.8 (m, 3H), 2.94 (m, 1H), 3.3 (m, 1H), 3.44 (m, 1H), 3.52 (m, 1H), 3.78 (s, 3H), 3.85 (s, 3H), 5.04 (m, 1H), 6.89 (d, 2H), 7.18 (d, 2H); MS (ESI+): m/z = 353.2 [(M+H]⁺

### e) tert-butylN-{[1-(4-fluorophenyl)-3-[2-(4-methoxyphenyl)ethyl]-5-oxo-2-sulfanylideneimidazolidin-4-yl]methyl}carbamate (I-57)

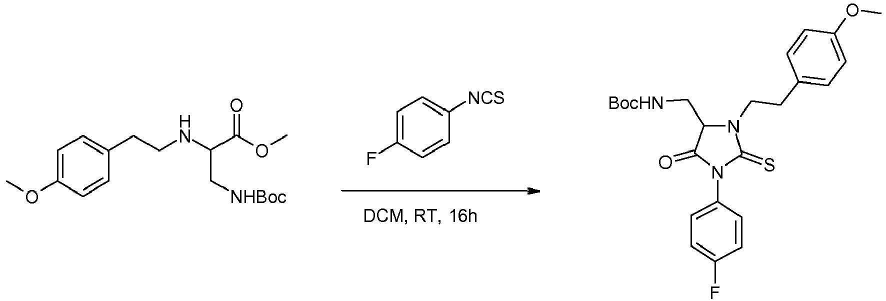

4-Fluorophenyl isothiocyanate (1.2 g; 7.83 mmol; 1.15 eq) was added to a solution of methyl 3-{[(*tert*-butoxy)carbonyl]amino}-2-{[2-(4-methoxyphenyl) ethyl]amino}propanoate (I-56) (2.4 g; 6.8 mmol; 1 eq) in dichloromethane (48 mL). The reaction mixture was stirred at room temperature for 16 hours, then, concentrated to dryness. The crude was triturated in dichloromethane/diethyl ether to afford the title compound, *tert*-butyl N-{[1-(4-fluorophenyl)-3-[2-(4-methoxyphenyl)ethyl]-5-oxo-2-sulfanylideneimidazolidin-4-yl]methyl}carbamate in 87 % yield (2.84 g) as a white solid. ¹H NMR (DMSO-d₆): δ 1.36 (s, 9H), 2.86 (m, 1H), 2.96 (m, 1H), 3.48 (m, 1H), 3.65 (m, 2H), 3.75 (s, 3H), 4.3 (m, 1H), 4.5 (m, 1H), 6.91 (d, 2H), 7.22 (d, 2H), 7.36 (m, 5H); MS (ESI+): m/z = 418.1 [(M-tBu)+H]⁺.

### f) 5-(aminomethyl)-3-(4-fluorophenyl)-1-[2-(4-methoxyphenyl)ethyl]-2-sulfanylideneimidazolidin-4-one (I-58)

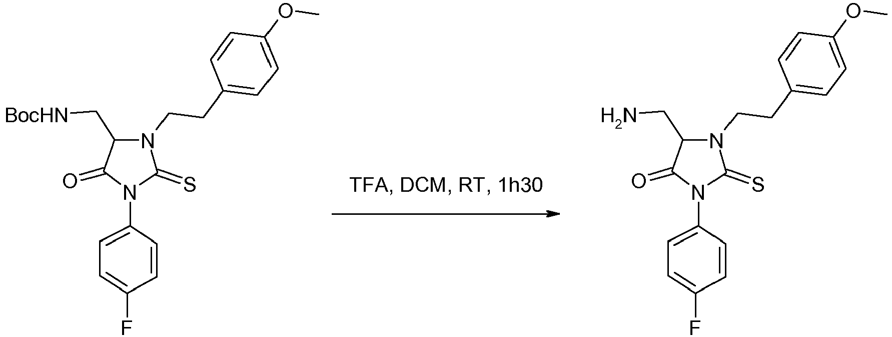

Trifluoroacetic acid (2.5 mL) was added to a solution of *tert*-butyl N-{[1-(4-fluorophenyl)-3-[2-(4-methoxyphenyl)ethyl]-5-oxo-2-sulfanylideneimidazolidin-4-yl] methyl}carbamate (I-57) (300 mg; 0.633 mmol; 1 eq) in dichloromethane (2.5 mL). The reaction mixture was stirred at room temperature for 1.5 hours, then, concentrated to dryness. Saturated sodium hydrogenocarbonate (20 mL) was added. The aqueous layer was extracted with dichloromethane (2 x 45 mL). The combined organic layers were washed with saturated sodium chloride (30 mL), dried over sodium sulfate, filtered and concentrated under reduced pressure. The title compound 5-(aminomethyl)-3-(4-fluorophenyl)-1-[2-(4-methoxyphenyl)ethyl]-2-sulfanylideneimidazolidin-4-one was obtained in a quantitative yield (288 mg) as an yellow oil. MS (ESI+): m/z = 357.1 [(M - NH₃)+H]⁺.

### g) N-{[1-(4-fluorophenyl)-3-[2-(4-methoxyphenyl)ethyl]-5-oxo-2-sulfanylidene imidazolidin-4-yl]methyl}-5-methoxypyridine-2-carboxamide (example 99)

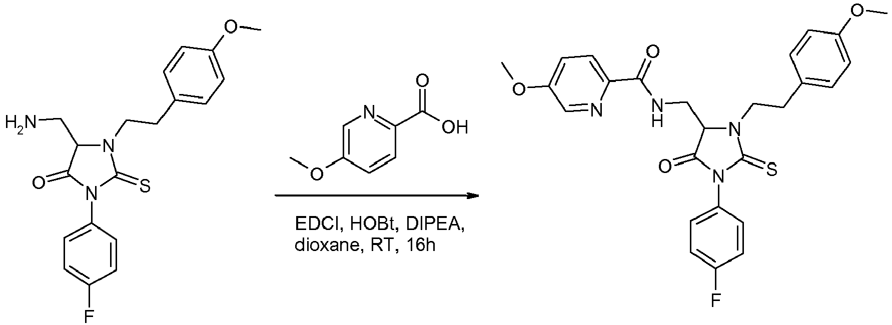

5-Methoxypyridine-2-carboxylic acid (86 mg; 0.56 mmol; 1.1 eq), followed by EDCI (146 mg; 0.76 mmol; 1.5 eq), HOBt (117 mg; 0.76 mmol; 1.5 eq) and DIPEA (261 µL; 1.52 mmol; 3 eq) were added to a solution of 5-(aminomethyl)-3-(4-fluorophenyl)-1-[2-(4-methoxyphenyl)ethyl]-2-sulfanylideneimidazolidin-4-one (I-58) (190 mg; 0.57 mmol; 1 eq) in dioxane (8 mL). The reaction mixture was stirred at room temperature overnight. Saturated sodium hydrogenocarbonate (25 mL) was added and the aqueous layer was extracted with ethyl acetate (3 x 35 mL). The combined organic layers were washed with saturated sodium chloride (30 mL), dried over sodium sulfate, filtered and concentrated under reduced pressure. The crude was purified on silica gel using dichloromethane/ethyl acetate (10/0 to 90/10) as an eluent. After trituration in diethyl ether/pentane, the title compound, *N*-{[1-(4-fluorophenyl)-3-[2-(4-methoxyphenyl)ethyl]-5-oxo-2-sulfanylideneimidazolidin-4-yl]methyl}-5-methoxypyridine-2-carboxamide was obtained as a white solid. ¹H NMR (Acetone-d₆): δ 3.01 (m, 1H), 3.12 (m, 1H), 3.79 (s, 3H), 3.94 (m, 2H), 3.99 (s, 3H), 4.25 (m, 1H), 4.47 (m, 1H), 4.6 (m, 1H), 6.89 (d, 2H), 7.25 (m, 4H), 7.36 (m, 2H), 7.54 (d, 1H), 8.09 (d, 1H), 8.31 (s, 1H), 8.42 (s, 1H); MS (ESI+): m/z = 509.1 [M+H]⁺.

### II Biological testing of the compounds according to the present invention

The compounds according to the present invention were tested for their ability to inhibit HCV replication as follows:

### Materials and methods

### Cell culture:

Huh-7 cell lines containing HCV replicon Luc Neo ET (Reblikon) were maintained in DMEM supplemented with 10% SVF, 2 mM glutamine, and 1 X NEAA, 100 U / ml penicillin, and 100 µg / ml streptomycine. Cells were maintained in medium supplemented with 0.5 mg/ml G418, at 37°C and 5% CO₂ in a humidified atmosphere. Cells were dissociated at sub confluence with trypsin EDTA 1X.

### Replicon assay:

Replicon Luc Neo ET is bicistronic expression constructs (Lohmann et al, 1999, Science 285, 110-113). In brief, the structural genes of the HCV genome were replaced by heterologous sequences; the gene encoding the neomycin phosphotransferase (NPT) and the internal ribosome entry site (IRES) of the encephalomyocarditis virus (EMCV). The bicistronic construct is therefore composed of the following elements: HCV-IRES nucleotides 1-389 for Luc Neo ET, the NPT gene, the EMCV-IRES directing translation of downstream HCV sequences from NS2 or NS3 up to the authentic 3' end of the genome. HCV Polyprotein of Luc Neo ET replicon harbours the cell culture adaptive mutations E1202G, T1280I, K1846T. G418-resistance is only possible with cells containing high amounts of replicon.

### Experiment

Cells were dissociated the day before addition of compounds and seeded in 96 well-plates at a final concentration of 27 777.77 cells.ml⁻¹.cm⁻² in 90µl final volume of culture medium per well and were maintained at 37°C and 5% CO₂ in a humidified atmosphere for 24 hours. 10 µl of compounds to be tested were added 24 hours after seeding. Final concentration of DMSO was 1 %. Cells were incubated in the presence of compounds for 72 hours after which expression of firefly luciferase reporter gene was quantified. Briefly, culture medium was removed and cells were lysed by addition of 100 µl of lysis buffer containing 125 mM Tris Phosphate ph 7.8, 10 mM EDTA, 5 mM DTT, 50 % glycerol and 5 % Triton. Plates were vortexed 10 min at 1300 rpm. 100µl of luciferin solution 1X were added to each well. Luciferin solution contained 40 mM Tris Phosphate ph 7.8, 0.2 mM EDTA, 67 mM DTT, 2.14 mM MgCl₂, 5.4 mM MgSO₄, 4.7 x 10⁻⁴ M luciferin, 5.3 x 10⁻⁴ M ATP and 2.7 x 10⁻⁴ M Acetyl co enzyme A. Luminescence was immediately measured with Berthold Microlumat Plus LB 96V luminometer with an integration of 1 sec. Inhibition was calculated using the formula: % inhibition = 1-[(RLUsample-RLUbackground)/(RLUsignal-RLUbackground)].

### Results

The Table 1 below illustrates data on the biological activity of compounds of the present invention. Results are expressed as EC₅₀ which is the concentration that produces 50 % of replication inhibition.

**Table 1. HCV replication inhibition assay**

| **Example** | **EC50 Replicon LucneoET (µM)** | | **Example** | **EC50 Replicon LucneoET (µM)** |
|---|---|---|---|---|
| 1 | < 0.5 | | 49 | < 0.5 |
| 2 | < 0.5 | | 51 | < 0.5 |
| 3 | < 0.5 | | 52 | < 0.5 |
| 4 | <5 | | 53 | < 0.5 |
| 5 | < 0.5 | | 54 | < 0.5 |
| 6 | < 0.5 | | 55 | <5 |
| 7 | < 0.5 | | 56 | <5 |
| 8 | < 0.5 | | 57 | < 0.5 |
| 9 | < 0.5 | | 59 | <5 |
| 10 | <5 | | 61 | < 0.5 |
| 11 | <5 | | 63 | < 0.5 |
| 12 | < 0.5 | | 64 | <5 |
| 13 | < 0.5 | | 65 | < 0.5 |
| 14 | < 0.5 | | 66 | < 0.5 |
| 15 | <5 | | 67 | <5 |
| 16 | <5 | | 68 | < 30 |
| 17 | <5 | | 69 | <5 |
| 18 | <5 | | 70 | < 0.5 |
| 19 | <5 | | 71 | < 30 |
| 20 | < 30 | | 72 | <5 |
| 22 | <5 | | 73 | < 0.5 |
| 23 | <5 | | 74 | < 30 |
| 24 | <5 | | 77 | < 0.5 |
| 25 | <5 | | 78 | < 30 |
| 26 | <5 | | 79 | <5 |
| 27 | < 30 | | 82 | <5 |
| 29 | < 30 | | 83 | < 0.5 |
| 31 | < 30 | | 84 | < 0.5 |
| 32 | < 30 | | 85 | < 0.5 |
| 37 | <5 | | 86 | < 30 |
| 38 | <5 | | 87 | <5 |
| 40 | <5 | | 88 | < 0.5 |
| 41 | < 0.5 | | 89 | Non Active |
| 42 | <5 | | 90 | < 0.5 |
| 43 | <5 | | 91 | < 0.5 |
| 44 | < 30 | | 92 | < 0.5 |
| 45 | <5 | | 93 | < 0.5 |
| 46 | < 0.5 | | 95 | < 0.5 |
| 47 | < 0.5 | | 97 | < 0.5 |
| 48 | < 0.5 | | 98 | < 0.5 |

## Claims

1. A compound of the following formula (I), or a salt, solvate, tautomer, enantiomer, diastereoisomer or racemic mixture thereof: 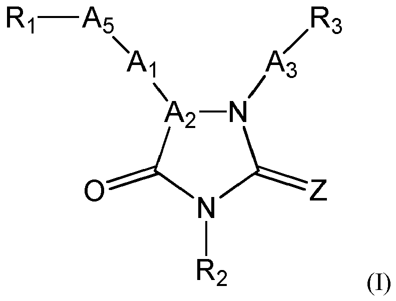 wherein:
- Z represents O or S,
- A₁ represents -OC(O)-, -C(O)O-, -NHC(O)- or -C(O)NH-, notably -NHC(O)-,
- A₂ represents 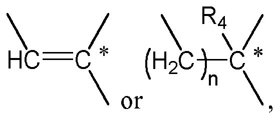 the carbon atom marked with a star being comprised in the hydantoin or thiohydantoin ring,
- A₃ represents -(CH₂)ₒ-, -CH=CH-, -C≡C-, or -(CH₂)ₘ-A₄-(CH₂)ₚ-,
- A₄ represents O, NH or 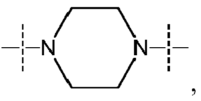
- A₅ represents 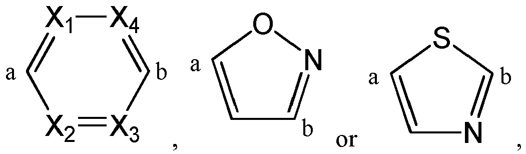 the carbon atoms marked with a being bound to R₁ and the carbon atoms marked with b being bound to (CH₂)rA₁,
- X₁ represents N or CR₅,
- X₂ represents N or CR₆,
- X₃ represents N or CR₇,
- X₄ represents N or CR₈,
- R₁ represents a hydrogen atom, a halogen atom, R₉, OR₁₀, NR₁₁R₁₂, C(O)R₁₀, C(O)OR₁₀, OC(O)R₉, C(O)NR₁₁R₁₂ or NR₁₃C(O)R₁₄,
- R₂ represents a (C₁-C₆)alkyl or -(CH₂)_{q}-R₂₂ group,
- R₃ represents a (C₁-C₆)alkyl group, a (C₂-C₆)alkenyl group or a cycloalkyl, cycloalkenyl, heterocyclic, aryl or heteroaryl ring, the said ring being optionally substituted with one or several groups, preferably with one, chosen among a halogen atom, a oxo group, (C₁-C₆)alkyl, (C₁-C₆)haloalkyl, OR₂₃, NR₁₇R₁₈ and SO₂NR₁₉R₂₀,
- R₄ represents a hydrogen atom or an OH, (C₁-C₆)alkyl or (C₁-C₆)alkoxy group,
- R₅ and R₆ represent, independently of each other, a hydrogen atom, a halogen atom, a (C₁-C₆)alkyl or a (C₁-C₆)alkoxy group,
- R₇ and R₈ represent, independently of each other, H or F,
- R₉ represents a (C₁-C₆)alkyl, (C₁-C₆)haloalkyl, or (C₁-C₆)alkyl-O-(C₁-C₆)alkanediyl group,
- R₁₀ represents a hydrogen atom or a (C₁-C₆)alkyl, (C₁-C₆)haloalkyl, or (C₁-C₆)alkyl-O-(C₁-C₆)alkanediyl group,
- R₁₁ and R₁₂ represent, independently of each other, a hydrogen atom or a (C₁-C₆)alkyl, (C₁-C₆)haloalkyl or (C₁-C₆)alkyl-O-(C₁-C₆)alkanediyl group, or R₁₁ and R₁₂ form, together with the nitrogen atom bearing them, a heterocycle optionally comprising another heteroatom chosen among O, S and N and optionally substituted with a (C₁-C₆)alkyl or oxo group,
- R₁₃ represents a hydrogen atom or a (C₁-C₆)alkyl, (C₁-C₆)haloalkyl or (C₁-C₆)alkyl-O-(C₁-C₆)alkanediyl group,
- R₁₄ represents a (C₁-C₆)alkyl, (C₁-C₆)haloalkyl, or (C₁-C₆)alkyl-O-(C₁-C₆)alkanediyl group, or R₁₃ and R₁₄ form together a -(CH₂)₂- or -(CH₂)₃- chain,
- R₁₅ and R₁₆ represent, independently of each other, a hydrogen atom or a (C₁-C₆)alkyl or (C₁-C₆)haloalkyl group, or R₁₅ and R₁₆ form, together with the nitrogen atom bearing them, a heterocycle optionally comprising another heteroatom chosen among O, S and N and optionally substituted with a (C₁-C₆)alkyl or oxo group,
- R₁₇ and R₁₈ represent, independently of each other, a hydrogen atom or a (C₁-C₆)alkyl or (C₁-C₆)haloalkyl group, or R₁₇ and R₁₈ form, together with the nitrogen atom bearing them, a heterocycle optionally comprising another heteroatom chosen among O, S and N and optionally substituted with a (C₁-C₆)alkyl or oxo group,
- R₁₉ and R₂₀ represent, independently of each other, a hydrogen atom or a (C₁-C₆)alkyl or (C₁-C₆)haloalkyl group, or R₁₉ and R₂₀ form, together with the nitrogen atom bearing them, a heterocycle optionally comprising another heteroatom chosen among O, S and N and optionally substituted with a (C₁-C₆)alkyl or oxo group,
- R₂₂ represents a cycloalkyl, aryl or heteroaryl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆,
- R₂₃ represents a hydrogen atom or a (C₁-C₆)alkyl, (C₁-C₆)haloalkyl or (C₁-C₆)alkyl-OC(O)NH-(C₁-C₆)alkanediyl group,
- n represents 0 or 1, notably 1,
- m represents 0, 1, 2 or 3,
- o represents 1, 2, 3, 4, 5 or 6,
- p represents 0, 1, 2 or 3, and
- q represents 0, 1, 2 or 3, preferably 0,
for use as a drug.

2. The compound according to claim 1, wherein it corresponds to a compound of the following formula (Ia):

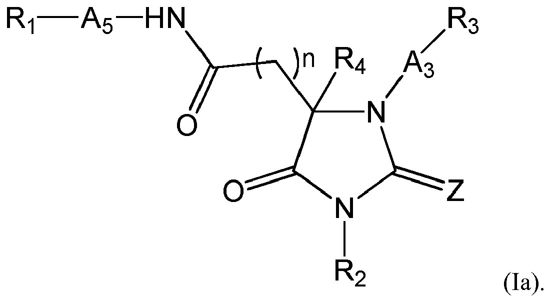

3. The compound according to any one of claims 1 and 2, wherein A₅ represents 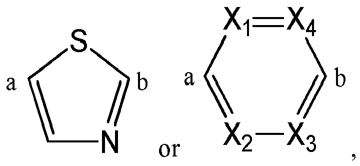 the latter being one of the following rings:

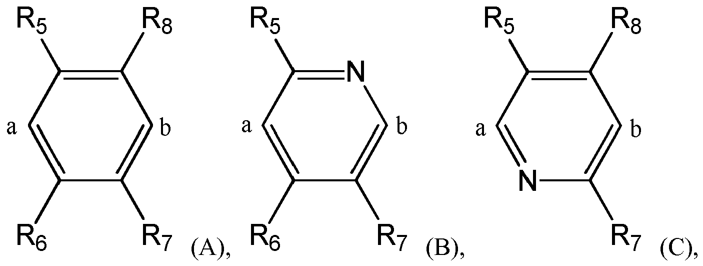

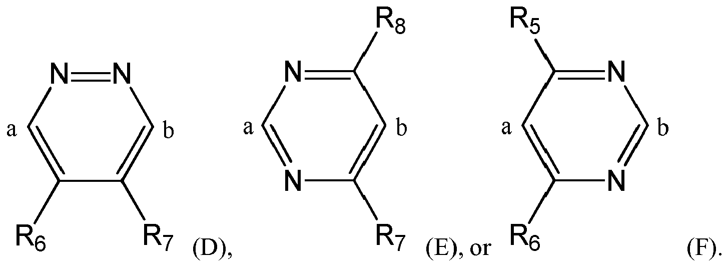

4. The compound according to any one of claims 1 to 3, wherein R₁ represents a hydrogen atom, a halogen atom, R₉, OR₁₀, NR₁₁R₁₂, C(O)OR₁₀ or C(O)NR₁₁R₁₂; advantageously a hydrogen atom, a halogen atom, R₉, OR₁₀, or NR₁₁R₁₂.

5. The compound according to any one of claims 1 to 4, wherein R₂ represents an aryl or heteroaryl ring, notably a phenyl or pyridinyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy and NR₁₅R₁₆.

6. The compound according to any one of claims 1 to 5, wherein R₃ represents a cycloalkyl, cycloalkenyl, heterocyclic, aryl or heteroaryl ring, notably a phenyl, pyridinyl, thienyl, imidazolyl, or indolyl ring, the said ring being optionally substituted with one or several groups, preferably with one, chosen among a halogen atom, OH, (C₁-C₆)alkyl, (C₁-C₆)haloalkyl, (C₁-C₆)alkoxy, (C₁-C₆)haloalkoxy, NR₁₇R₁₈ and SO₂NR₁₉R₂₀.

7. The compound according to claim 1, wherein it is chosen among the following compounds:
Example 1: 2-[5-oxo-1-phenyl-3-(2-phenylethyl)-2-sulfanylideneimidazolidin-4-yl]-N-(4-propoxyphenyl)acetamide,
Example 2: 2-{3-[2-(4-chlorophenyl)ethyl]-5-oxo-1-phenyl-2-sulfanylideneimidazolidin-4-yl}-N-(4-propoxyphenyl)acetamide,
Example 3: N-(4-methoxyphenyl)-2- {3-[2-(4-methoxyphenyl)ethyl]-5-oxo-1-phenyl-2-sulfanylideneimidazolidin-4-yl} acetamide,
Example 4: 2-{3-[2-(3,4-dimethoxyphenyl)ethyl]-5-oxo-1-phenyl-2-sulfanylideneimidazolidin-4-yl}-N-(4-methoxyphenyl)acetamide,
Example 5: 2-{3-[2-(4-chlorophenyl)ethyl]-5-oxo-1-phenyl-2-sulfanylideneimidazolidin-4-yl}-N-(4-methoxyphenyl)acetamide,
Example 6: 2-[1-(4-fluorophenyl)-3-[2-(4-methoxyphenyl)ethyl]-5-oxo-2-sulfanylideneimidazolidin-4-yl]-N-(4-methoxyphenyl)acetamide,
Example 7: N-(4-ethoxyphenyl)-2-{3-[2-(2-fluorophenyl)ethyl]-5-oxo-1-phenyl-2-sulfanylideneimidazolidin-4-yl} acetamide,
Example 8: 2- {3-[2-(4-chlorophenyl)ethyl]-1-(4-fluorophenyl)-5-oxo-2-sulfanylideneimidazolidin-4-yl} -N-phenylacetamide,
Example 9: 2-{3-[2-(4-chlorophenyl)ethyl]-1-(4-fluorophenyl)-5-oxo-2-sulfanylideneimidazolidin-4-yl}-N-(4-methoxyphenyl)acetamide,
Example 10:2-{3-[3-(4-benzylpiperazin-1-yl)propyl]-5-oxo-1-phenyl-2-sulfanylideneimidazolidin-4-yl}-N-(4-propoxyphenyl)acetamide,
Example 11: 2-{3-[2-(4-chlorophenyl)ethyl]-5-oxo-1-phenyl-2-sulfanylideneimidazolidin-4-yl} -N-phenylacetamide,
Example 12:2-{3-[2-(cyclohex-1-en-1-yl)ethyl]-2,5-dioxo-1-phenylimidazolidin-4-yl}-N-(4-methoxyphenyl)acetamide,
Example 13:2-{3-[3-(4-benzylpiperazin-1-yl)propyl]-1-(4-fluorophenyl)-5-oxo-2-sulfanylideneimidazolidin-4-yl}-N-(4-methoxyphenyl)acetamide,
Example 14: 2-[1-(4-fluorophenyl)-5-oxo-3-[3-(4-phenylpiperazin-1-yl)propyl]-2-sulfanylideneimidazolidin-4-yl]-N-(4-methoxyphenyl)acetamide,
Example 15: N-(4-ethoxyphenyl)-2-[3-(3-methylbutyl)-2,5-dioxo-1-phenylimidazolidin-4-yl]acetamide,
Example 16: 2-[1-(4-fluorophenyl)-2,5-dioxo-3-[2-(thiophen-2-yl)ethyl]imidazolidin-4-yl]-N-(4-methoxyphenyl)acetamide,
Example 17: 2-{3-[2-(5-methoxy-1H-indol-3-yl)ethyl]-5-oxo-1-phenyl-2-sulfanylideneimidazolidin-4-yl}-N-phenylacetamide,
Example 18: N-(3-ethoxyphenyl)-2-{3-[2-(4-fluorophenyl)ethyl]-5-oxo-1-phenyl-2-sulfanylideneimidazolidin-4-yl} acetamide,
Example 19: N-(4-ethoxyphenyl)-2-{3-[2-(2-fluorophenyl)ethyl]-5-oxo-2-sulfanylidene-1-[3-(trifluoromethyl)phenyl]imidazolidin-4-yl} acetamide,
Example 20: 2-[1-(4-chlorophenyl)-3-[2-(4-chlorophenyl)ethyl]-5-oxo-2-sulfanylideneimidazolidin-4-yl]-N-(4-fluorophenyl)acetamide,
Example 21: N-(4-methoxyphenyl)-2-{5-oxo-1-phenyl-3-[2-(pyridin-2-yl)ethyl]-2-sulfanylideneimidazolidin-4-yl} acetamide,
Example 22: N-(4-chlorophenyl)-2-[5-oxo-1-phenyl-3-(2-phenylethyl)-2-sulfanylideneimidazolidin-4-yl]acetamide,
Example 23: N-(4-fluorophenyl)-2- {3-[2-(3-methylthiophen-2-yl)ethyl]-5-oxo-1-phenyl-2-sulfanylideneimidazolidin-4-yl} acetamide,
Example 24: N-(4-chlorophenyl)-2-{3-[2-(4-chlorophenyl)ethyl]-5-oxo-1-phenyl-2-sulfanylideneimidazolidin-4-yl} acetamide,
Example 25: methyl 4-(2- {3-[2-(4-chlorophenyl)ethyl]-5-oxo-1-phenyl-2-sulfanylideneimidazolidin-4-yl} acetamido)benzoate,
Example 26: N-(4-methoxyphenyl)-2-[1-(4-methylphenyl)-5-oxo-3-(2-phenylethyl)-2-sulfanylideneimidazolidin-4-yl]acetamide,
Example 27: 2-[1-(4-fluorophenyl)-3-(3-methoxypropyl)-5-oxo-2-sulfanylideneimidazolidin-4-yl]-N-(4-methoxyphenyl)acetamide,
Example 28: N-(4-methoxyphenyl)-2-{3-[3-(morpholin-4-yl)propyl]-5-oxo-1-phenyl-2-sulfanylideneimidazolidin-4-yl} acetamide,
Example 29: 2- {1-cyclohexyl-3-[(4-methoxyphenyl)methyl]-5-oxo-2-sulfanylideneimidazolidin-4-yl}-N-(4-propoxyphenyl)acetamide,
Example 30: 2-{5-oxo-1-phenyl-3-[2-(4-sulfamoylphenyl)ethyl]-2-sulfanylideneimidazolidin-4-yl}-N-(4-propoxyphenyl)acetamide,
Example 31: 2-{3-[3-(4-ethylpiperazin-1-yl)propyl]-1-(4-fluorophenyl)-5-oxo-2-sulfanylideneimidazolidin-4-yl}-N-(4-propoxyphenyl)acetamide,
Example 32: 2-{1-benzyl-3-[2-(4-chlorophenyl)ethyl]-5-oxo-2-sulfanylideneimidazolidin-4-yl}-N-(4-methoxyphenyl)acetamide,
Example 33: N-(4-ethoxyphenyl)-2- {3-[2-(morpholin-4-yl)ethyl]-5-oxo-1-phenyl-2-sulfanylideneimidazolidin-4-yl} acetamide,
Example 34: 1-{3-[3-(4-fluorophenyl)-4-oxo-5-([(4-propoxyphenyl) carbamoyl]methyl}-2-sulfanylideneimidazolidin-1-yl]propyl}piperidine-4-carboxamide,
Example 35: 2-[1-(4-fluorophenyl)-3-[3-(4-methylpiperazin-1-yl)propyl]-5-oxo-2-sulfanylideneimidazolidin-4-yl]-N-(4-methoxyphenyl)acetamide,
Example 36: 2-[1-(4-fluorophenyl)-3-[3-(4-methylpiperidin-1-yl)propyl]-5-oxo-2-sulfanylideneimidazolidin-4-yl]-N-(4-methoxyphenyl)acetamide,
Example 37: 2-{3-[2-(4-chlorophenyl)ethyl]-5-oxo-1-phenyl-2-sulfanylideneimidazolidin-4-yl}-N-(4-methylphenyl)acetamide,
Example 38: 2-{3-[2-(4-chlorophenyl)ethyl]-5-oxo-1-phenyl-2-sulfanylideneimidazolidin-4-yl}-N-(3,4-dichlorophenyl)acetamide,
Example 39: 2-{3-[2-(4-chlorophenyl)ethyl]-5-oxo-1-phenyl-2-sulfanylideneimidazolidin-4-yl}-N-[4-(trifluoromethyl)phenyl]acetamide,
Example 40: 4-(2-{3-[2-(4-chlorophenyl)ethyl]-5-oxo-1-phenyl-2-sulfanylideneimidazolidin-4-yl} acetamido)benzamide,
Example 41: 2-{3-[2-(4-chlorophenyl)ethyl]-1-(4-fluorophenyl)-5-oxo-2-sulfanylideneimidazolidin-4-yl}-N-[4-(morpholin-4-yl)phenyl]acetamide,
Example 42: N-[4-(dimethylamino)phenyl]-2-[1-(4-fluorophenyl)-3-[2-(4-methoxyphenyl)ethyl]-2,5-dioxoimidazolidin-4-yl]acetamide,
Example 43: 2-[1-(4-fluorophenyl)-3-[2-(4-methoxyphenyl)ethyl]-2,5-dioxoimidazolidin-4-yl]-N-{4-[(2-methoxyethyl)(methyl)amino]phenyl}acetamide,
Example 44: N-[4-(dimethylamino)-3-methylphenyl]-2-[1-(4-fluorophenyl)-3-[2-(4-methoxyphenyl)ethyl]-2,5-dioxoimidazolidin-4-yl]acetamide,
Example 45: N-[4-(dimethylamino)phenyl]-2-[1-(4-fluorophenyl)-2,5-dioxo-3-[2-(thiophen-2-yl)ethyl]imidazolidin-4-yl] acetamide,
Example 46: 2-[1-(4-fluorophenyl)-5-oxo-2-sulfanylidene-3-[2-(thiophen-2-yl)ethyl]imidazolidin-4-yl]-N-(4-methoxyphenyl)acetamide,
Example 47: 2-(3- {2-[4-(dimethylamino)phenyl]ethyl}-1-(4-fluorophenyl)-5-oxo-2-sulfanylideneimidazolidin-4-yl)-N-(4-methoxyphenyl)acetamide,
Example 48: N-[4-(dimethylamino)phenyl]-2-[1-(4-fluorophenyl)-5-oxo-2-sulfanylidene-3-[2-(thiophen-2-yl)ethyl]imidazolidin-4-yl]acetamide,
Example 49: N-[4-(dimethylamino)phenyl]-2-(3-{2-[4-(dimethylamino)phenyl]ethyl}-1-(4-fluorophenyl)-2,5-dioxoimidazolidin-4-yl)acetamide,
Example 50: N-[4-(dimethylamino)phenyl]-2-[1-(4-fluorophenyl)-3-[2-(1H-imidazol-4-yl)ethyl]-2,5-dioxoimidazolidin-4-yl]acetamide,
Example 51: N-[4-(dimethylamino)phenyl]-2- {3-[2-(4-methoxyphenyl)ethyl]-5-oxo-1-phenyl-2-sulfanylideneimidazolidin-4-yl} acetamide,
Example 52: 2-[1-(4-fluorophenyl)-3-(2-[4-(morpholin-4-yl)phenyl]ethyl)-5-oxo-2-sulfanylideneimidazolidin-4-yl]-N-(4-methoxyphenyl)acetamide,
Example 53: 2-[1-(4-fluorophenyl)-3-[2-(4-methoxyphenyl)ethyl]-5 oxo-2-sulfanylideneimidazolidin-4-yl]-N-[4-(2-methoxyethoxy)phenyl]acetamide,
Example 54: 2-[1-(4-fluorophenyl)-3-[2-(4-methoxyphenyl)ethyl]-5-oxo-2-sulfanylideneimidazolidin-4-yl]-N-[4-(propan-2-yloxy)phenyl]acetamide,
Example 55: N-(4-methoxyphenyl)-2- {3-[2-(4-methoxyphenyl)ethyl]-5-oxo-1-(pyridin-4-yl)-2-sulfanylideneimidazolidin-4-yl} acetamide,
Example 56: 2-[1-(4-fluorophenyl)-3-[2-(4-methoxyphenyl)ethyl]-5 oxo-2-sulfanylideneimidazolidin-4-yl] -N-(5 -methoxy-1, 3 -thiazol-2-yl)acetamide,
Example 57: 2-{3-[2-(4-chlorophenyl)ethyl]-5-oxo-1-phenyl-2-sulfanylideneimidazolidin-4-yl}-N-(pyridin-2-yl)acetamide,
Example 58: 2-{3-[2-(4-chlorophenyl)ethyl]-5-oxo-1-phenyl-2-sulfanylidene imidazolidin-4-yl}-N-[4-(trifluoromethoxy)phenyl]acetamide,
Example 59: 2-{3-[2-(4-chlorophenyl)ethyl]-5-oxo-1-phenyl-2-sulfanylidene imidazolidin-4-yl}-N-(6-methoxypyridin-3-yl)acetamide,
Example 60: 2-{3-[2-(4-chlorophenyl)ethyl]-4-hydroxy-5-oxo-1-phenyl-2-sulfanylideneimidazolidin-4-yl}-N-(6-methylpyridazin-3-yl)acetamide,
Example 61: 2-{3-[2-(4-chlorophenyl)ethyl]-5-oxo-1-phenyl-2-sulfanylidene imidazolidin-4-yl}-N-(5-methoxypyridin-2-yl)acetamide,
Example 62: 2-{3-[2-(4-chlorophenyl)ethyl]-5-oxo-1-phenyl-2-sulfanylidene imidazolidin-4-yl}-N-(6-methoxypyridazin-3-yl)acetamide,
Example 63: 2-{3-[2-(4-chlorophenyl)ethyl]-4-hydroxy-5-oxo-1-phenyl-2-sulfanylideneimidazolidin-4-yl}-N-[4-(dimethylamino)phenyl]acetamide,
Example 64: 2-{3-[2-(4-chlorophenyl)ethyl]-5-oxo-1-phenyl-2-sulfanylidene imidazolidin-4-yl}-N-(1,2-oxazol-3-yl)acetamide,
Example 65: 2- {3-[2-(4-chlorophenyl)ethyl]-1-(4-fluorophenyl)-4-hydroxy-5-oxo-2-sulfanylideneimidazolidin-4-yl}-N-[4-(dimethylamino)phenyl]acetamide,
Example 66: 2-{3-[2-(4-chlorophenyl)ethyl]-1-(4-fluorophenyl)-5-oxo-2-sulfanylideneimidazolidin-4-yl}-N-(5-methoxypyridin-2-yl)acetamide,
Example 67: 2-{3-[2-(4-chlorophenyl)ethyl]-1-(4-fluorophenyl)-5-oxo-2-sulfanylideneimidazolidin-4-yl}-N-(5-methoxypyridin-2-yl)acetamide,
Example 68: 2- (3-[2-(4-methoxyphenyl)ethyl]-5-oxo-1-phenyl-2-sulfanylidene imidazolidin-4-yl}-N-(6-methylpyridazin-3-yl)acetamide,
Example 69: 2-{3-[2-(4-methoxyphenyl)ethyl]-5-oxo-1-phenyl-2-sulfanylidene imidazolidin-4-yl}-N-(6-methoxypyridazin-3-yl)acetamide,
Example 70: N-[4-(dimethylamino)phenyl]-2-{4-hydroxy-3-[2-(4-methoxyphenyl) ethyl]-5-oxo-1-phenyl-2-sulfanylideneimidazolidin-4-yl} acetamide,
Example 71: 2-[1-(4-fluorophenyl)-3-[2-(4-methoxyphenyl)ethyl]-2,5-dioxoimidazolidin-4-yl]-N-(6-methoxypyridazin-3-yl)acetamide,
Example 72: 2-[1-(4-fluorophenyl)-3-[2-(4-methoxyphenyl)ethyl]-2,5-dioxoimidazolidin-4-yl]-N-(5-methoxypyridin-2-yl)acetamide,
Example 73: N-[5-(dimethylamino)pyridin-2-yl]-2-[1-(4-fluorophenyl)-3-[2-(4-methoxyphenyl)ethyl]-2,5-dioxoimidazolidin-4-yl]acetamide,
Example 74: 2-[1-(4-fluorophenyl)-3-[2-(4-methoxyphenyl)ethyl]-2,5-dioxoimidazolidin-4-yl]-N-(6-methylpyridazin-3-yl)acetamide,
Example 75: 2- (3-[2-(4-methoxyphenyl)ethyl]-2-oxo-1-(pyridin-3-yl)-5-sulfanylideneimidazolidin-4-yl}-N-(6-methylpyridazin-3-yl)acetamide,
Example 76: 2-{3-[2-(4-methoxyphenyl)ethyl]-2-oxo-1-(pyridin-3-yl)-5-sulfanylidene imidazolidin-4-yl}-N-(6-methoxypyridazin-3-yl)acetamide,
Example 77: N-[4-(dimethylamino)phenyl]-2-[1-(4-fluorophenyl)-4-hydroxy-3-[2-(4-methoxyphenyl)ethyl]-5-oxo-2-sulfanylideneimidazolidin-4-yl]acetamide,
Example 78: 2-(3- {2-[4-(dimethylamino)phenyl]ethyl}-1-(4-fluorophenyl)-2,5-dioxoimidazolidin-4-yl)-N-(2-methoxypyrimidin-5-yl)acetamide,
Example 79: 2-(3- {2-[4-(dimethylamino)phenyl]ethyl}-1-(4-fluorophenyl)-5-oxo-2-sulfanylideneimidazolidin-4-yl)-N-(2-methoxypyrimidin-5-yl)acetamide,
Example 80: 2-[1-(4-fluorophenyl)-2,5-dioxo-3-[2-(thiophen-2-yl)ethyl]imidazolidin-4-yl]-N-(2-methoxypyrimidin-5-yl)acetamide,
Example 81: 2-[1-(4-fluorophenyl)-5-oxo-2-sulfanylidene-3-[2-(thiophen-2-yl)ethyl]imidazolidin-4-yl]-N-(2-methoxypyrimidin-5-yl)acetamide,
Example 82: 2-[1-(4-fluorophenyl)-3-[2-(4-methoxyphenyl)ethyl]-5-oxo-2-sulfanylideneimidazolidin-4-yl]-N-(2-methoxypyrimidin-5-yl)acetamide,
Example 83: 2-[1-(4-fluorophenyl)-3-[2-(4-methoxyphenyl)ethyl]-5-oxo-2-sulfanylideneimidazolidin-4-yl]-N-(5-methoxypyridin-2-yl)acetamide,
Example 84: 2-[1-(4-fluorophenyl)-3-[2-(4-methoxyphenyl)ethyl]-5-oxo-2-sulfanylideneimidazolidin-4-yl]-N-(5-methoxypyrimidin-2-yl)acetamide,
Example 85: N-(2-fluoro-4-methoxyphenyl)-2-[1-(4-fluorophenyl)-3-[2-(4-methoxyphenyl)ethyl]-5-oxo-2-sulfanylideneimidazolidin-4-yl]acetamide,
Example 86: 4-methoxyphenyl 2-[1-(4-fluorophenyl)-3-[2-(4-methoxyphenyl)ethyl]-5-oxo-2-sulfanylideneimidazolidin-4-yl] acetate,
Example 87: 2-[1-(4-fluorophenyl)-3-[2-(4-methoxyphenyl)ethyl]-5-oxo-2-sulfanylideneimidazolidin-4-yl]-N-[4-(2-oxopyrrolidin-1-yl)phenyl]acetamide,
Example 88: 2-[1-(4-fluorophenyl)-3-[2-(4-methoxyphenyl)ethyl]-5-oxo-2-sulfanylideneimidazolidin-4-yl] -N-(5 -methoxy-1, 3 -thiazol-2-yl)acetamide,
Example 89: 2-[1-(4-fluorophenyl)-3-[(Z)-2-(4-methoxyphenyl)ethenyl]-5-oxo-2-sulfanylideneimidazolidin-4-yl]-N-(5-methoxypyridin-2-yl)acetamide,
Example 90: N-[4-(dimethylamino)phenyl]-2-[(4E)-3-[2-(4-methoxyphenyl)ethyl]-5-oxo-1-phenyl-2-sulfanylideneimidazolidin-4-ylidene]acetamide,
Example 91: 2-[1-(4-fluorophenyl)-3-[2-(4-methoxyphenyl)ethyl]-5-oxo-2-sulfanylideneimidazolidin-4-yl]-N-(4-hydroxyphenyl)acetamide,
Example 92: 2-[1-(4-fluorophenyl)-3-[3-(4-methoxyphenyl)propyl]-5-oxo-2-sulfanylideneimidazolidin-4-yl]-N-(5-methoxypyridin-2-yl)acetamide,
Example 93: 2-[1-(4-fluorophenyl)-3-[4-(4-methoxyphenyl)butyl]-5-oxo-2-sulfanylideneimidazolidin-4-yl]-N-(5-methoxypyridin-2-yl)acetamide,
Example 94: N-[4-(dimethylamino)phenyl]-2-{3-[2-(4-methoxyphenyl)ethyl]-5-oxo-1-phenyl-2- sulfanylideneimidazolidin-4-yl} acetamide,
Example 95: 2-[1-(4-fluorophenyl)-3-[(E)-2-(4-methoxyphenyl)ethenyl]-5-oxo-2-sulfanylideneimidazolidin-4-yl]-N-(5-methoxypyridin-2-yl)acetamide,
Example 96: 2-[1-(4-fluorophenyl)-3-[2-(4-hydroxyphenyl)ethyl]-5-oxo-2-sulfanylideneimidazolidin-4-yl]-N-(5-methoxypyridin-2-yl)acetamide,
Example 97: N-(2,6-difluoro-4-methoxyphenyl)-2-[1-(4-fluorophenyl)-3-[2-(4-methoxyphenyl)ethyl]-5-oxo-2-sulfanylideneimidazolidin-4-yl]acetamide,
Example 98: tert-butyl N-[2-(4- {2-[3-(4-fluorophenyl)-5-{[(5-methoxypyridin-2-yl)carbamoyl]methyl} -4-oxo-2-sulfanylideneimidazolidin-1-yl]ethyl} phenoxy)ethyl] carbamate,
Example 99: N-{[1-(4-fluorophenyl)-3-[2-(4-methoxyphenyl)ethyl]-5-oxo-2-sulfanylideneimidazolidm-4-yl]methyl}-5-methoxypyridme-2-carboxamide, and
Example 100: 1-(4-fluorophenyl)-3-[2-(4-methoxyphenyl)ethyl]-N-(5-methoxypyridin-2-yl)-5-oxo-2-sulfanylideneimidazolidine-4-carboxamide.

8. A compound as defined in any one of claims 1 to 7, as an antiviral drug.

9. A compound as defined in any one of claims 1 to 7, for use in the treatment of hepatitis C.

10. A pharmaceutical composition comprising a compound as defined in any one of claims 1 to 7, in combination with pharmaceutically acceptable excipients.

11. The pharmaceutical composition according to claim 10, wherein it contains a further antiviral agent, in particular selected in the group consisting of ribavirin, interferon, inhibitors of HCV helicase, inhibitors of HCV protease, inhibitors of HCV NS4A, inhibitors of HCV NS5B, inhibitors of HCV NS5A, anti-HIV agent and mixture thereof.

12. A compound of the following formula (I), or a salt, solvate, tautomer, enantiomer, diastereoisomer or racemic mixture thereof: 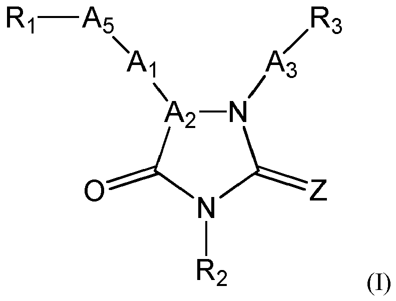 wherein Z, A₁, A₂, A₃, A₅, R₁, R₂, and R₃ are as defined in any one of claims 1 to 6, with the exception of:
■ the compounds for which Z = O or S; A₁ = -NHC(O)-, C(O)- being bound to A₂; 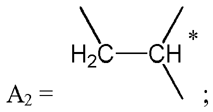 A₃ = -(CH₂)ₒ-; 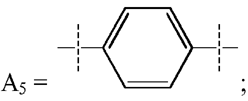 R₁, R₂ and R₃ are as defined above, and
■ the compound of the following formula:

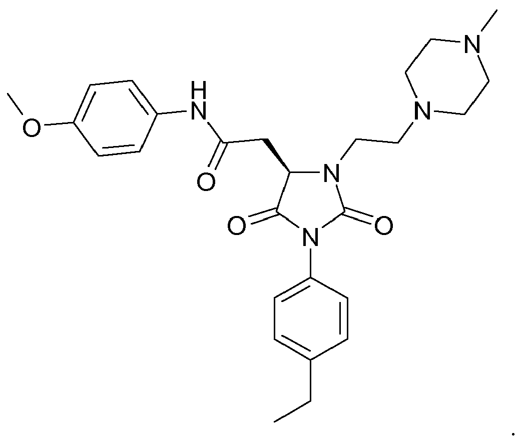

13. The compound according to claim 12, wherein it is chosen among the compounds 37 to 100 as defined in claim 7.

14. A method to prepare a compound of formula (I) according to claim 12 for which A₁ = -OC(O)- or -NHC(O)- and 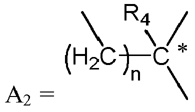 with R4 = H, OH or (C₁-C₆)alkoxy, comprising the coupling of a compound of the following formula (II): 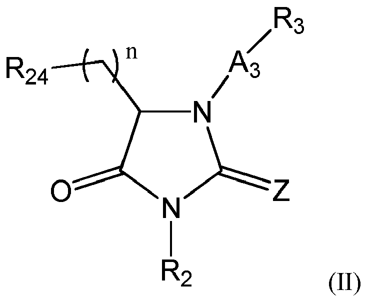 wherein Z, A₃, R₂, R₃ and n are as defined in claim 12 and R₂₄ represents a carboxylic acid function (COOH) optionally in an activated form,
with a compound of the following formula (III):
R₁—A₅—R₂₅ (III)
wherein R₁ and A₅ are as defined in claim 12 and R₂₅ represents OH or NH₂,
to give a compound of formula (I) according to claim 12 with A₁ = -OC(O)- or -
NHC(O)- and 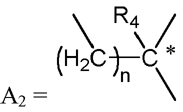 with R4 = H or OH, followed optionally, when R₄ = OH, by a substitution of the OH function to give a compound of formula (I) according to claim 12 with A₁ = -OC(O)- or -NHC(O)- and 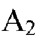 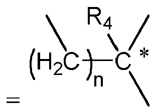 with R4 = (C₁-C₆)alkoxy.

15. A method to prepare a compound of formula (I) according to claim 12 for which 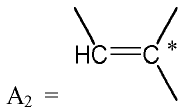 by dehydration of the corresponding compound of formula (I) for which 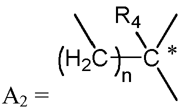 with R4 = OH.
